(19) Europäisches Patentamt
European Patent Office
Office européen des brevets

(11) EP 4 268 855 A1

(12) **EUROPEAN PATENT APPLICATION**
published in accordance with Art. 153(4) EPC

(43) Date of publication:
**01.11.2023 Bulletin 2023/44**

(21) Application number: **20966837.5**

(22) Date of filing: **22.12.2020**

(51) International Patent Classification (IPC):
***A61L 27/38*** *(2006.01)* ***A61K 35/39*** *(2015.01)*
***A61L 27/20*** *(2006.01)* ***A61P 3/10*** *(2006.01)*

(52) Cooperative Patent Classification (CPC):
**A61K 35/39; A61L 27/20; A61L 27/38; A61P 3/10**

(86) International application number:
**PCT/JP2020/047944**

(87) International publication number:
**WO 2022/137345 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Mochida Pharmaceutical Co., Ltd.**
  **Tokyo 160-8515 (JP)**
- **National Center for Global Health and Medicine**
  **Tokyo 162-8655 (JP)**

(72) Inventors:
- **SHIMODA Masayuki**
  **Tokyo 162-8655 (JP)**
- **AJIMA Kumiko**
  **Tokyo 162-8655 (JP)**
- **FURUSAKO Shoji**
  **Tokyo 160-8515 (JP)**
- **TSUDA Naoto**
  **Tokyo 160-8515 (JP)**

(74) Representative: **Grünecker Patent- und Rechtsanwälte PartG mbB**
**Leopoldstraße 4**
**80802 München (DE)**

(54) **TRANSPLANTATION DEVICE USING CHEMICALLY CROSSLINKED ALGINIC ACID**

(57) Provided is a transplantation device comprising a hydrogel in which insulin-secreting cells or pancreatic islets are enclosed, wherein the hydrogel is prepared by gelatinizing an alginic acid derivative by a chemical crosslinking. Accordingly, a novel transplantation device is provided.

Fig. 5-1

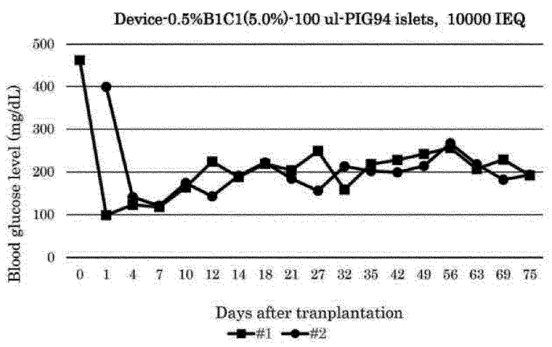


EP 4 268 855 A1

## Description

[Technical Field]

The present invention relates to a device for transplanting cells and the like into a living body. More specifically, the present invention relates to a transplantation device using a chemically crosslinked alginic acid, and to a method of manufacturing the same.

[Background Art]

In addition to conventional insulin injection and pancreas transplantation, pancreas islet transplantation is being performed both in Japan and abroad as a method for treating type I diabetes, but in Japan in particular the number of cases thereof has been small due to a shortage of donors and the like. Although xenotransplantation using pig islets could be an effective technology for solving the donor shortage, immune rejection reactions are unavoidable in both allogenic transplantation and xenotransplantation, necessitating long-term administration of immune suppressants, and complications from immune suppressants as well as possible adverse effects from residual transplanted islets have been reported (Non Patent Literature 1: Organ Biology Vol. 24, No. 1, pp. 7-12, 2017).

To resolve these problems, there has already been research into bioartificial pancreas (BAP) technology, also called bioartificial islet technology, in which pancreatic islets for transplantation are encapsulated in a polymer gel, semipermeable membrane or the like capable of isolating the islets from the recipient's immune cells and the like while allowing permeation of nutrients, insulin and the like (Patent Literature 1: Japanese Patent Application Publication No. S55-157502, Patent Literature 2: Japanese Patent Application Publication No. S60-258121, Patent Literature 3: WO 95/28480, Patent Literature 4: WO 92/19195, Patent Literature 5: Japanese Patent Application Publication No. 2017-196150).

Bioartificial pancreatic islets are classified generally into (1) a "microcapsule'" type comprising individual islets encapsulated in a polymer gel or the like, (2) a "macrocapsule" type comprising multiple islets encapsulated in a polymer gel, semipermeable membrane or the like, and (3) a "hemoperfusion" type comprising islets encapsulated in a hollow fiber module or immunoisolation device prepared from a semipermeable membrane or the like, which is then perfused with blood (Non Patent Literature 1).

The microcapsule type is a technology whereby individual islets are encapsulated using a polymer gel capable isolating the islets from the immune cells and the like while allowing permeation of nutrients, insulin and the like, and then transplanted into the body (mainly intraperitoneally) as in normal pancreatic islet transplantation. In addition to isolating the islets from the recipient's immune cells, this also has the advantage of rapid nutrient permeation and cell response because the permeation time from diffusion is short due to the relative thinness of the isolation membrane, but on the other hand the microcapsules are difficult to collect when the islet function declines.

Applying a combination of technologies including artificial dialysis and bioartificial liver technology, the hemoperfusion type involves perfusing blood in a channel containing islets isolated by a semipermeable membrane, but there are problems with the large size of the device and the severe risk of thrombus formation, and this technique has not yet been put into practical use due the likelihood of thrombus formation and clogging during long-term use.

The macrocapsule type is an improved technology that aims to solve a drawback of microcapsule technology by allowing extraction when islet function declines. However, no outstanding results have yet been reported from studies of islet transplantation using macrocapsule-type xenogenic islets, and no bioartificial pancreatic islets using xenogenic islets have yet been discovered that overcome the problems of pancreatic islet transplantation, including the donor shortage, the use of immune suppressants, and the long-term survival and functional maintenance of the pancreatic islets.

There has been a report on the synthesis of alginic acid hydrogel capsules by a click reaction and on the stability, water swelling and diffusion properties of these, compared with those of ionically crosslinked alginate capsules (Non Patent Literature 2: Journal of Biomedical Materials Research, Part B/Vol. 103B, Issue 5, pp. 1120-1132 (2015)). According to this literature, alginate capsules formed by click reactions are more stable than ionically ($C^+$) crosslinked capsules.

[Citation List]

[Patent Literature]

[Patent Literature 1] Japanese Patent Application Publication No. S55-157502
[Patent Literature 2] Japanese Patent Application Publication No. S60-258121

[Patent Literature 3] WO 95/28480
[Patent Literature 4] WO 92/19195
[Patent Literature 5] Japanese Patent Application Publication No. 2017-196150

[Non Patent Literature]

[Non Patent Literature 1] Organ Biology Vol. 24, No. 1, pp. 7-12, 2017
[Non Patent Literature 2] Journal of Biomedical Materials Research, Part B/Vol. 103B, Issue 5, pp. 1120-1132 (2015)

[Summary of Invention]

[Technical Problem]

Under these circumstances, there is demand for new practical transplantation devices.

[Solution to Problem]

The inventors discovered (1) to (10) below as a result of exhaustive research aimed at solving these problems, and perfected the present invention based on these findings.

(1) The novel alginic acid derivatives disclosed here (for example, the alginic acid derivatives of formula (HA-I) and formula (HA-II)) are hydrogelled by chemical crosslink formation for example, and when an alginic acid gel prepared in flat form using such chemically crosslinking alginic acid derivatives was transplanted in vivo (intraperitoneally in a healthy mouse), there was no great change in the size of the flat gel even after 5 weeks, and the gel maintained its shape without dissolving and had excellent in vivo stability.
(2) Said mouse exhibited no intraperitoneal adhesion or inflammation.
(3) When Min6 cells were enveloped in the flat gel and cultured for 3 to 4 weeks, survival of Min6 clusters could be confirmed, with good cell proliferation and no cytotoxicity.
(4) When a transplantation device obtained by enveloping a chemically crosslinked alginic acid gel containing pig pancreatic islets in a semipermeable membrane was transplanted into a diabetes model mouse, a blood glucose suppression effect was observed for 75 days.
(5) When the transplanted device of (4) above was extracted 10 weeks after transplantation, no harmful effects such as adhesion, angiogenesis or inflammation were observed. When the survival of the pancreatic islets in the transplantation device was confirmed by dithizone staining, moreover, adequate survival was confirmed. When the transplantation device extracted 10 weeks after transplantation was opened, moreover, the alginate gel in the device was confirmed to have maintained its shape.
(6) Substance permeability into and out of the device is excellent.
(7) A thin device with a hydrogel thickness of less than 500 $\mu$m has a higher cure rate in animals implanted with the device than a device with a thickness of 500 $\mu$m or more.
(8) In the case of a thin device with a hydrogel thickness of less than 500 $\mu$m, the ratio of the oxygen concentration of the center part to the oxygen concentration of the device surface is higher than when the thickness is over 500 $\mu$m.
(9) The hydrogel does not collapse or hardly collapses when the hydrogel is shaken.
(10) There is little shedding of islet cells contained in the hydrogel when the hydrogel is shaken.

The novel alginic acid derivatives used here (for example, the alginic acid derivatives of formula (HA-I) and formula (HA-II)) can be used for example in chemical crosslink formation, or in other words have an introduced reactive group that can be used in chemical crosslink formation or an introduced reactive group complementary to that reactive group.
This chemical crosslink formation may also occur for example between an alginic acid derivative having an introduced reactive group that can be used in chemical crosslink formation and an alginic acid derivative having an introduced reactive group complementary to that reactive group. The reactive group and the reactive group complementary to that reactive group may both be introduced into one molecule of an alginic acid, or may be introduced separately. Chemical crosslink formation may be performed within molecules of the alginic acid derivatives, or between molecules, or with other molecules into which the reactive group or the reactive group complementary to that reactive group has been introduced.
This chemical crosslink formation is accomplished for example by crosslinking using a Huisgen reaction (1,3-dipolar cycloaddition reaction) and can be performed for example between the alginic acid derivatives of formula (HA-I) and formula (HA-II), or between the alginic acid derivative of formula (HA-I) and another molecule having an azide group, or between the alginic acid derivative of formula (HA-II) and another molecule having an alkyne group.

This chemical crosslink formation may also be accomplished for example by a crosslinking reaction using a Huisgen reaction (1,3-dipolar cycloaddition reaction), and may be performed for example between the alginic acid derivatives of formula (HB-I) and formula (HB-II), or between the alginic acid derivative of formula (HB-I) and another molecule having an azide group, or between the alginic acid derivative of formula (HB-II) and another molecule having an alkyne group for example.

Provided here is a device for transplanting cells or the like in vivo, prepared using an alginic acid derivative that is gelled by chemical crosslinking, and more specifically a transplantation device comprising a chemically crosslinked alginic acid gel enclosing insulin-secreting cells, pancreatic islets or the like together with a semipermeable membrane encapsulating the gel as necessary and a method for manufacturing the device are provided. The alginic acid derivative that is gelled by chemical crosslinking is for example an alginic acid derivative of formula (HA-I) or formula (HA-II) comprising a cyclic alkyne group or azide group introduced via an amide bond and a divalent linker at any one or more carboxyl groups of alginic acid, and a novel crosslinked alginic acid can be obtained by performing a Huisgen reaction (1,3-dipolar cycloaddition reaction) using the alginic acid derivatives of formula (HA-I) and formula (HA-II).

Alternatively, the alginic acid derivative that is gelled by chemical crosslinking is for example an alginic acid derivative of formula (HB-I) or formula (HB-II) comprising a cyclic alkyne group or azide group introduced via an amide bond and a divalent linker at any one or more carboxyl groups of alginic acid, and a novel crosslinked alginic acid can be obtained by performing a Huisgen reaction (1,3-dipolar cycloaddition reaction) using the alginic acid derivatives of formula (HB-I) and formula (HB-II).

Exemplary embodiments may be as shown in [1-1] to [3-4] below.

In this Description, the alginic acid derivatives of formula (HA-I) and formula (HB-I) both have the common structure of formula (I). Consequently, the general formula for the alginic acid derivatives of formula (HA-I) and formula (HB-I) is represented by formula (I) in both cases.

In this Description, moreover, the alginic acid derivatives of formula (HA-II) and formula (HB-II) both have the common structure of formula (II). Consequently, the general formula for the alginic acid derivatives of formula (HA-II) and formula (HB-II) is represented by formula (II) in both cases.

Furthermore, in this description the crosslinked alginic acids of formula (HA-III-L) and formula (HB-III-L) both have the common structure of formula (III-L). Consequently, the general formula for the crosslinked alginic acids of formula (HA-III-L) and formula (HB-III-L) is represented by formula (III-L) in both cases.

The first embodiment of the present invention is as shown in [1-1] to [1-34] below.

[1-1] A transplantation device comprising insulin-secreting cells or pancreatic islets enclosed in a hydrogel, wherein the hydrogel is an alginic acid derivative that has been gelled by chemical crosslinking.

[1-2] The transplantation device according to [1-1] above, wherein the hydrogel comprises, as crosslinking, chemical crosslinking formed between alginic acid derivatives.

[1-3] The transplantation device according to [1-1] or [1-2] above, wherein the hydrogel comprises, as crosslinking, chemical crosslinking by triazole rings formed by a Huisgen reaction.

[1-4] The transplantation device according to any one of [1-1] to [1-3] above, wherein the chemical crosslinking is chemical crosslinking by cyclic alkyne groups introduced at any one or more carboxyl groups of alginic acid and azide groups introduced at any one or more carboxyl groups of alginic acid.

[1-5] The transplantation device according to [1-4] above, wherein the chemical crosslinking is chemical crosslinking by cyclic alkyne groups introduced at any one or more carboxyl groups of a first alginic acid and azide groups introduced at any one or more carboxyl groups of a second alginic acid.

[1-6] The transplantation device according to any one of [1-1] or [1-5] above, wherein the chemical crosslinking is chemical crosslinking by a combination of alginic acid derivatives described under (A) and (B) below:

(A) an alginic acid derivative represented by formula (HA-I) below, including a cyclic alkyne group (Akn) introduced via an amide bond and a divalent linker ($-L^1-$) at any one or more carboxyl groups of alginic acid:

[C1]

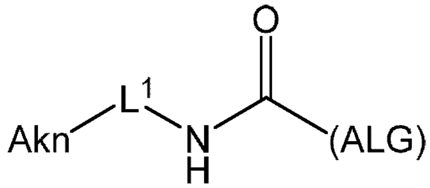

(I)

[in formula (HA-I), (ALG) represents alginic acid; -NHCO- represents an amide bond via any carboxyl group of alginic acid; $-L^1-$ represents a divalent linker selected from the group consisting of the following partial structural formulae [excluding the parts outside the dashed lines at both ends of each formula]:

[C2]

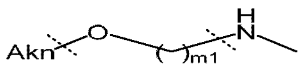

(LN-1) m1=2-6

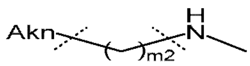

(LN-2) m2=1-6

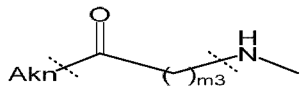

(LN-3) m3=1-6

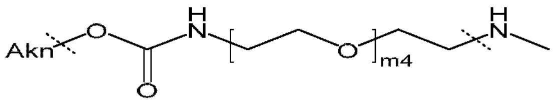

(LN-4) m4=1-6

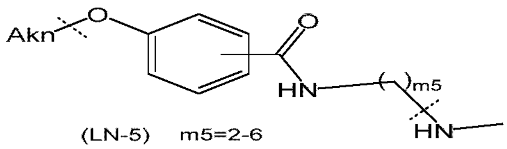

(LN-5) m5=2-6

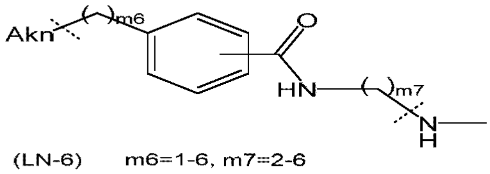

(LN-6) m6=1-6, m7=2-6

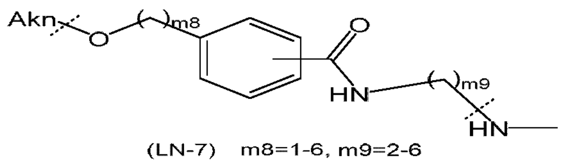

(LN-7) m8=1-6, m9=2-6

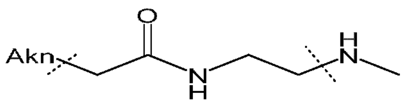

(LN-8)

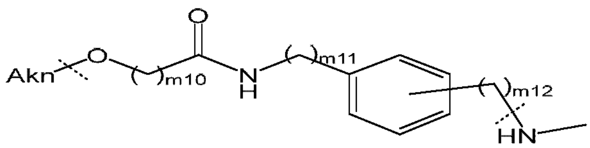

(LN-9) m10=1-4, m11=1-6,m12=1-6

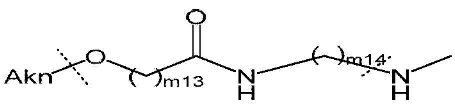

m13=1-4, m14=2~6

(LN-10)

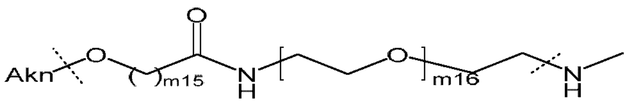

m15=1-4, m16=1-6

(LN-11)

and Akn represents a cyclic alkyne group selected from the group consisting of following partial structural formulae [excluding the part to the right of the dashed line in each formula]:

[C3]

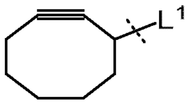
(AK-1)

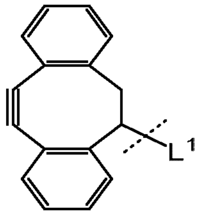
(AK-2)

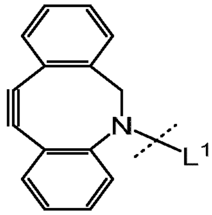
(AK-3)

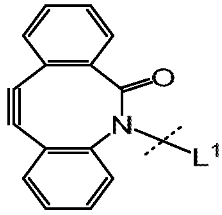
(AK-4)

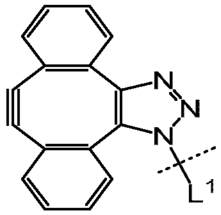
(AK-5)

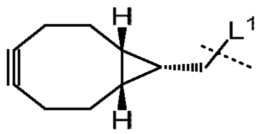
(AK-6)

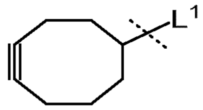
(AK-7)

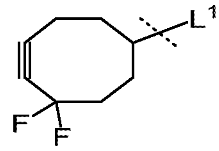
(AK-8)

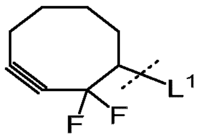
(AK-9)

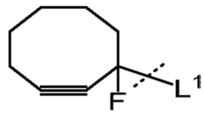
(AK-10)

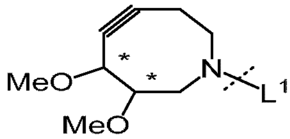
(AK-11)

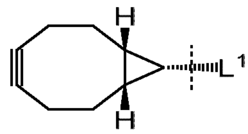
(AK-12)

which the asterisks represent chiral centers];

(B) an alginic acid derivative represented by formula (HA-II) below, comprising an azide group introduced via an amide bond and a divalent linker (-$L^2$-) at any one or more carboxyl groups of alginic acid:

[C4]

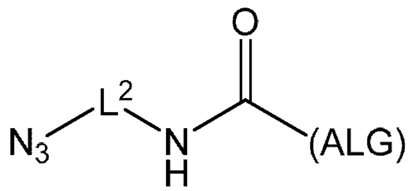
(II)

[in formula (HA-II), (ALG) represents alginic acid; -NHCO- represents an amide bond via any carboxyl group of alginic acid; and -$L^2$- represents a divalent linker selected from the group consisting of following partial structural formulae [excluding the parts outside the dashed lines at both ends of each formula]]:

[C5]

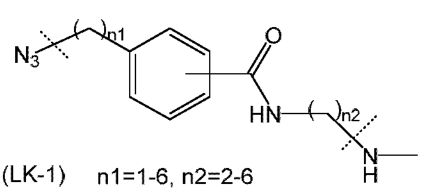

(LK-1) n1=1-6, n2=2-6

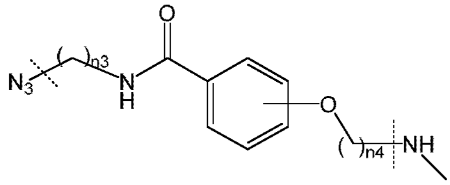

(LK-2) n3=2-6, n4=2-6

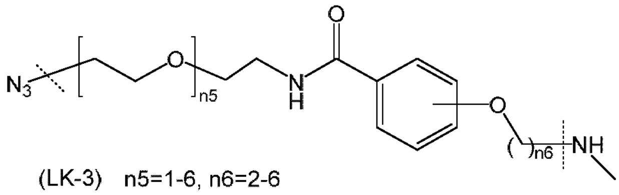

(LK-3) n5=1-6, n6=2-6

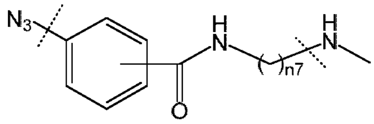

(LK-4) n7=2-6

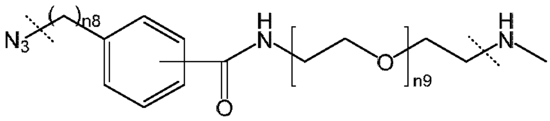

(LK-5) n8=1-4, n9=1-6

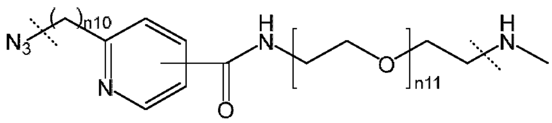

(LK-6) n10=1-4, n11=1-6

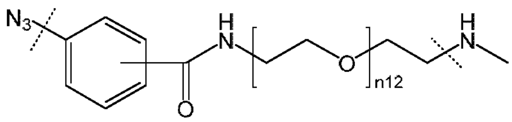

(LK-7) n12=1-6

[1-7] The transplantation device according to [1-6] above, wherein the chemically crosslinked alginic acid derivative is a crosslinked alginic acid in which any carboxyl group of a first alginic acid and any carboxyl group of a second alginic acid are bound together via following formula (HA-III-L):

[C6]

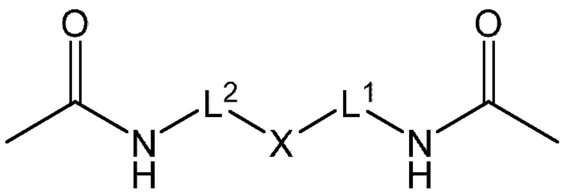

(III-L)

[in formula (HA-III-L), the -CONH- and -NHCO- at either end represent amide bonds via any carboxyl group of alginic acid;

- $-L^1-$ is defined as in the Embodiment [1-6];
- $-L^2-$ is defined as in the Embodiment [1-6]; and
- X is a cyclic group selected from the group consisting of following partial structural formulae (excluding the parts outside the dashed lines at both ends of each formula):

[C7]

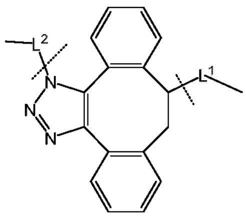

(TZ-1)

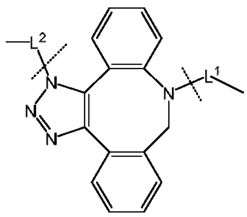

(TZ-2)

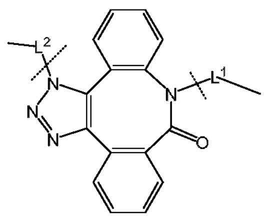

(TZ-3)

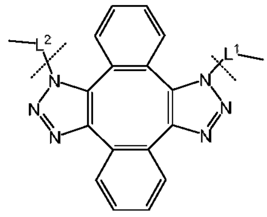

(TZ-4)

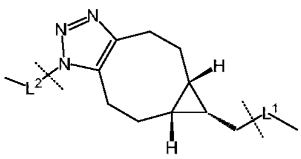

(TZ-5)

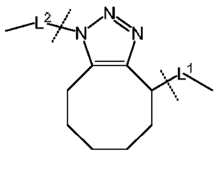

(TZ-6)

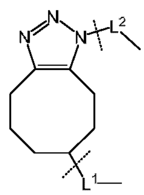

(TZ-7)

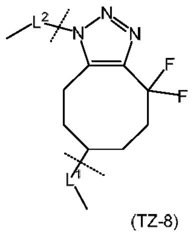

(TZ-8)

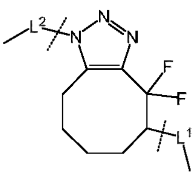

(TZ-9)

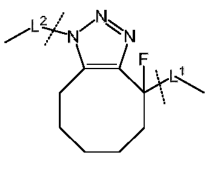

(TZ-10)

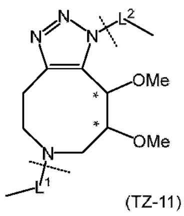

(TZ-11)

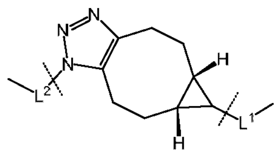

(TZ-12)

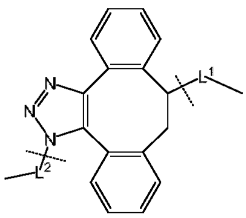

(TZ-1-r)

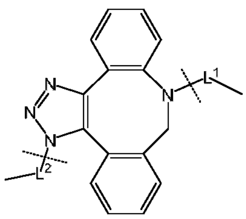

(TZ-2-r)

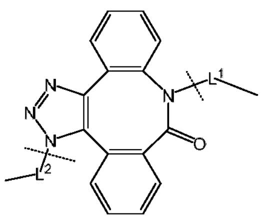

(TZ-3-r)

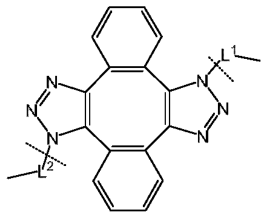

(TZ-4-r)

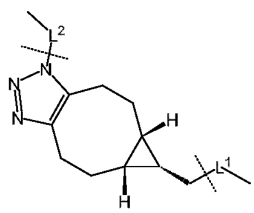

(TZ-5-r)

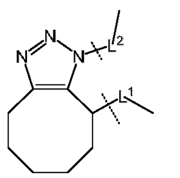

(TZ-6-r)

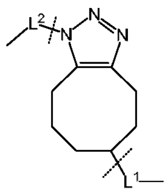

(TZ-7-r)

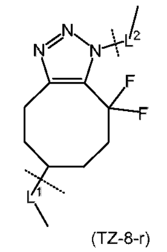

(TZ-8-r)

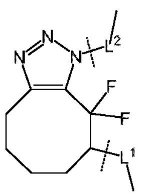

(TZ-9-r)

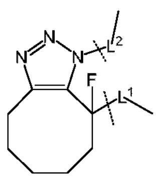

(TZ-10-r)

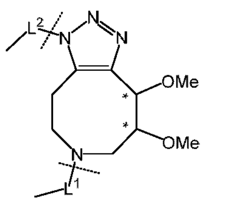

(TZ-11-r)

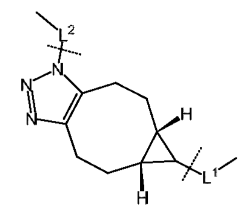

(TZ-12-r)

in which the asterisks represent chiral centers].

[1-8] The transplantation device according to any one of [1-1] to [1-5] above, wherein the chemical crosslinking is chemical crosslinking by a combination of the alginic acid derivatives according to (A) and (B) below.
(A): an alginic acid derivative represented by formula (HB-I) below, comprising a cyclic alkyne group (Akn) introduced via an amide bond and a divalent linker (-$L^1$-) at any one or more carboxyl group groups of alginic acid:

[C8]

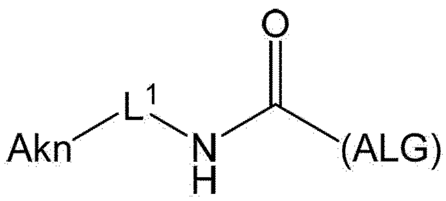

(I)

[in the formula (HB-I), (ALG) represents alginic acid; -NHCO- represents an amide bond via any carboxyl group of alginic acid; -$L^1$- represents a linker selected from the group consisting of the partial structural formulae shown in the following tables (excluding the parts outside the dashed lines at both ends of each formula);

[Table 1-1]

| No. | -$L^1$- | |
|---|---|---|
| (L1-1) | 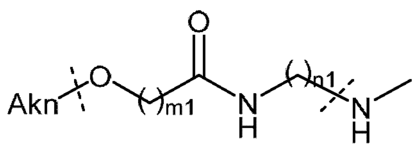 | m1=1-6<br>n1=2-6 |
| (L1-2a) | 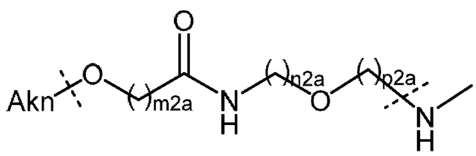 | m2a=1-6<br>n2a=2-6<br>p2a=2-6 |
| (L1-2b) | 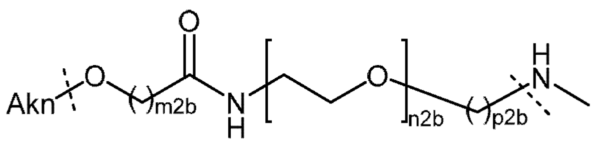 | m2b=1-6<br>n2b=1-6<br>p2b=2-6 |
| (L1-3) | 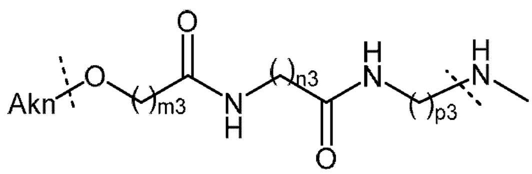 | m3=1-6<br>n3=1-6<br>p3=2-6 |
| (L1-4a) | 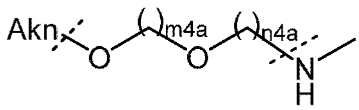 | m4a=2-6<br>n4a=2-6 |
| (L1-4b) | 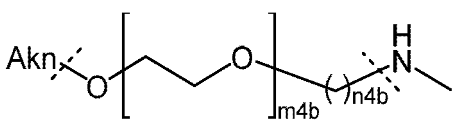 | m4b=0-6<br>n4b=2-6 |

(continued)

| No. | -L[1]- | |
|---|---|---|
| (L1-5a) | 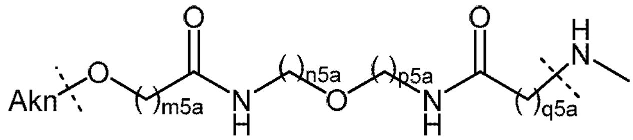 | m5a=1-6<br>n5a=2-6<br>p5a=2-6<br>q5a=1-6 |
| (L1-5b) | 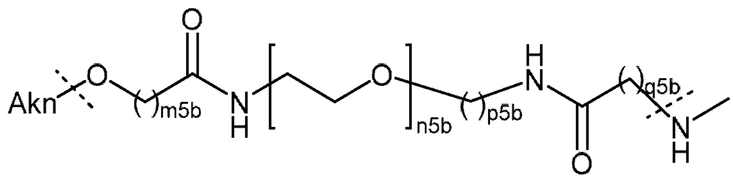 | m5b=1-6<br>n5b=1-6<br>p5b=2-6<br>q5b=1-6 |
| (L1-6a) | 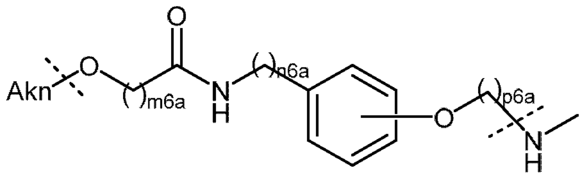 | m6a=1-6<br>n6a=0-6<br>p6a=2-6 |
| (L1-6b) | 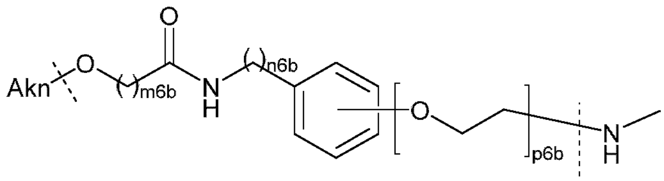 | m6b=1-6<br>n6b=1-6<br>p6b=2-6 |

[Table 1-2]

| No. | -L[1]- | |
|---|---|---|
| (L1-7) | 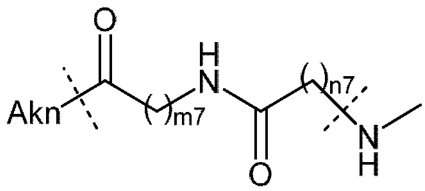 | m7=1-6<br>n7=1-6 |
| (L1-8a) | 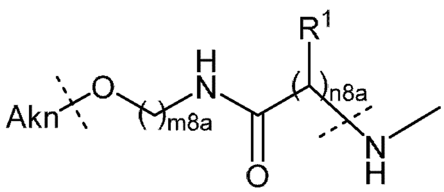 | m8a=2-6<br>n8a=1-6<br>$R^1$=H, Me, Et, Bn |
| (L1-8b) | 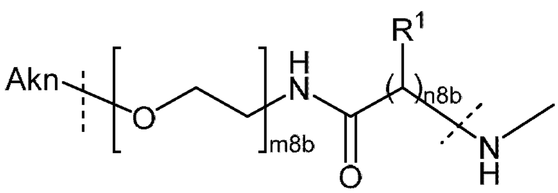 | m8b=1-6<br>n8b=1-6<br>$R^1$=H, Me, Et, Bn |
| (L1-9a) | 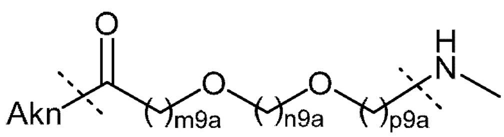 | m9a=1-6<br>n9a=2-6<br>p9a=2-6 |

(continued)

| No. | $-L^1-$ | |
|---|---|---|
| (L1-9b) | 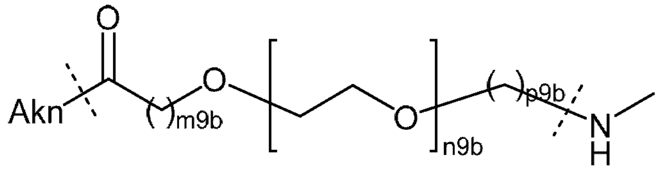 | m9b=1-6<br>n9b=1-6<br>p9b=2-6 |
| (L1-10) | 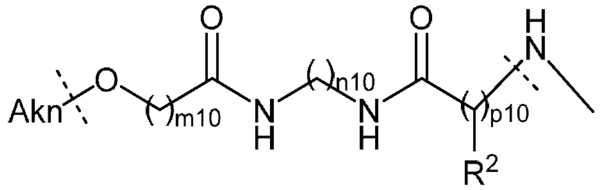 | m10=1-6<br>n10=2-6<br>p10=1-6<br>$R^2$=H, Me, Et, Bn |
| (L1-11) | 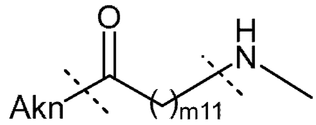 | m11=2-6 |
| (L1-12) | 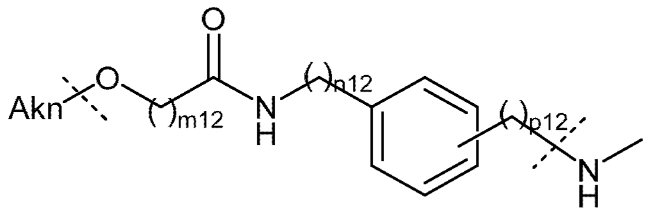 | m12=1-6<br>n 12=0-6<br>p12=1-6 |

and Akn is a cyclic alkyne selected from the group consisting of the partial structural formula shown in the following table (excluding the part to the right of the dashed line in each formula)]:

[Table 2]

| No. | Akn | No. | Akn | No. | Akn |
|---|---|---|---|---|---|
| (AK-1) | 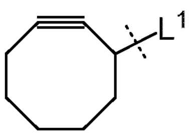 | (AK-5) | 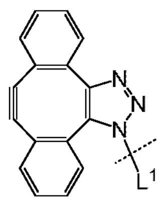 | (AK-9) | 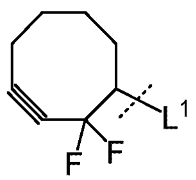 |
| (AK-2) | 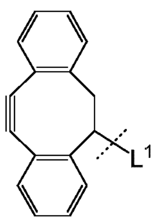 | (AK-6) | 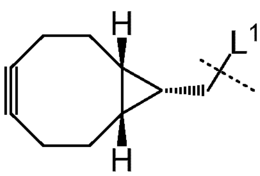 | (AK-10) | 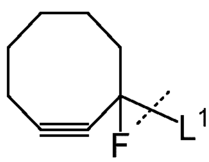 |
| (AK-3) | 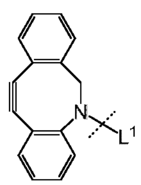 | (AK-7) | 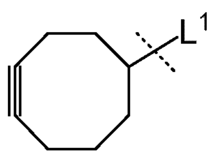 | (AK-11) | 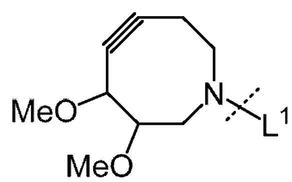 |

(continued)

| No. | Akn | No. | Akn | No. | Akn |
|---|---|---|---|---|---|
| (AK-4) | 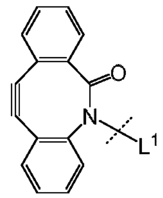 | (AK-8) | 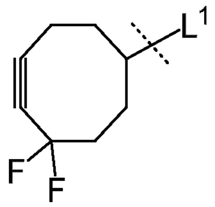 | (AK-12) | 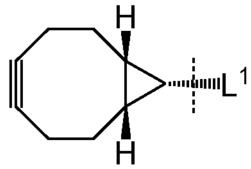 |

(B): an alginic acid derivative represented by formula (HB-II) below, comprising an azide group introduced via an amide bond and a divalent linker ($-L^2-$) at any one or more carboxyl groups of alginic acid:

[C9]

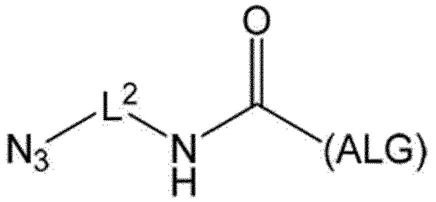

(II)

[in the formula (HB-II), (ALG) represents alginic acid; -NHCO- represents an amide bond via any carboxyl group of alginic acid; and $-L^2-$ represents a linker selected from the group consisting of the partial structural formulae shown in the following tables (excluding the parts outside the dashed lines at both ends of each formula)]:

[Table 3-1]

| No. | $-L^2-$ | |
|---|---|---|
| (L2-1) | 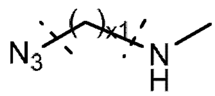 | x1=2-6 |
| (L2-2a) | 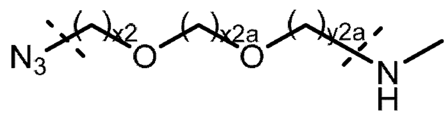 | x2=2-6<br>x2a=2-6<br>y2a=2-6 |
| (L2-2b) | 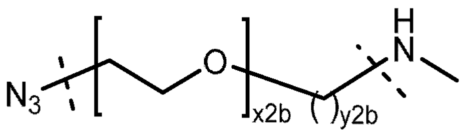 | x2b=1-6<br>y2b=2-6 |
| (L2-3) | 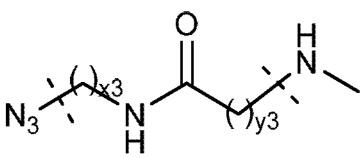 | x3=2-6<br>y3=1-6 |
| (L2-4) | 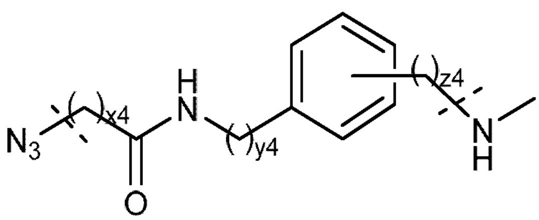 | x 4= 1-6<br>y4=1-6<br>z4=1-6 |

(continued)

| No. | -$L^2$- | |
|---|---|---|
| (L2-5a) | 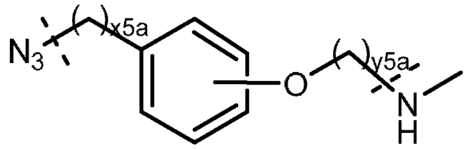 | x5a=0-4<br>y5a=2-6 |
| (L2-5b) | 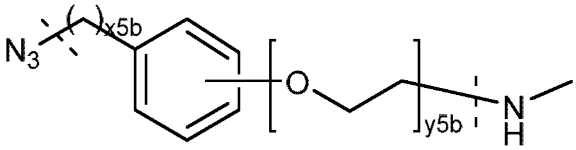 | x5b=0-4<br>y5b=1-6 |
| (L2-6a) | 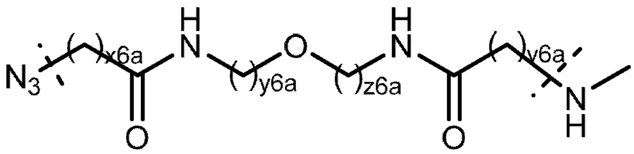 | x6a=1-6<br>y6a=2-6<br>z6a=2-6<br>v6a=1-6 |
| (L2-6b) | 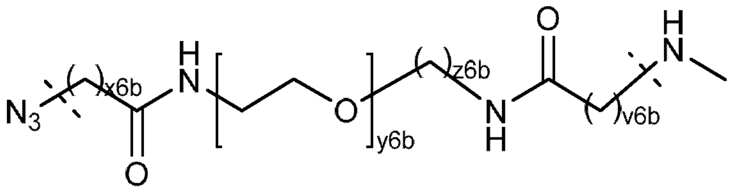 | x6b=1-6<br>y6b=1-6<br>z6b=2-6<br>v6b=1-6 |

[Table 3-2]

| No. | -$L^2$- | |
|---|---|---|
| (L2-7a) | 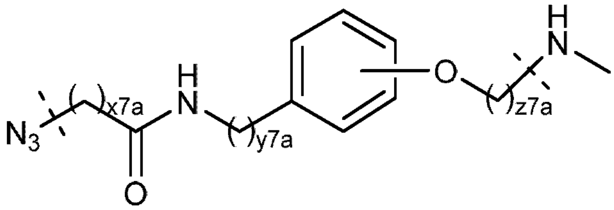 | x7a=1-6<br>y7a=1-6<br>z7a=2-6 |
| (L2-7b) | 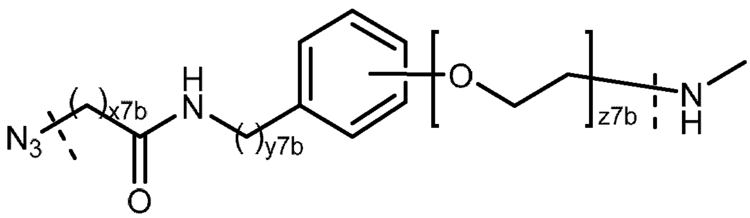 | x7b=1-6<br>y7b=1-6<br>z7b=1-6 |
| (L2-8a) | 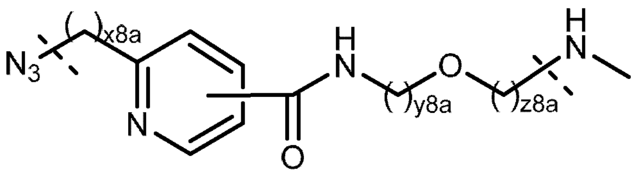 | x8a=0-4<br>y8a=2-6<br>z8a=3-6 |

(continued)

| No. | -$L^2$- | |
|---|---|---|
| (L2-8b) | 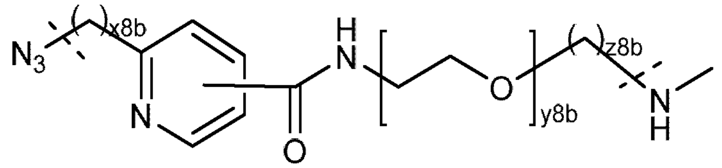 | x8b=0-4<br>y8b=1-6<br>z8b=3-6 |
| (L2-9a) | 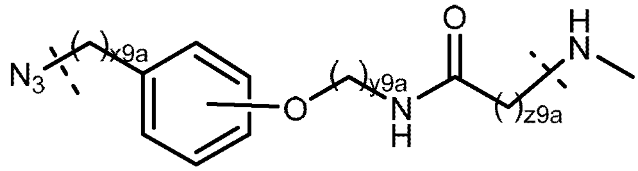 | x9a=0-4<br>y9a=2-6<br>z9a=1-6 |
| (L2-9b) | 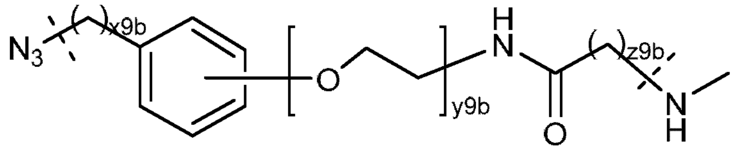 | x9b=0-4<br>y9b=1-6<br>z9b=1-6 |
| (L2-10) | 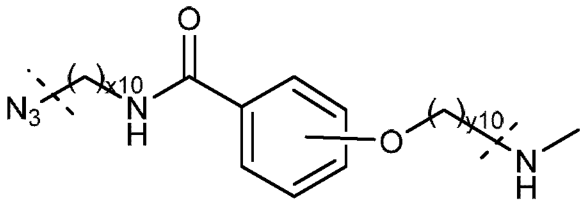 | x10=2-6<br>y10=2-6 |

(with the proviso that excluding chemical crosslinking by a combination of a derivative in which the -$L^1$- in the alginic acid derivative represented by the formula (HB-I) is any one linker selected from the group consisting of (L1-1), (L1-2a), (L1-2b), (L1-11) and (L1-12) and a derivative in which -$L^2$- in the alginic acid derivative represented by formula (HB-II) is the linker of (L2-10)).

[1-9] The transplantation device according to [1-8] above, wherein the chemically crosslinked alginic acid derivative is a crosslinked alginic acid in which any carboxyl group of a first alginic acid and any carboxyl group of a second alginic acid are linked via the following formula (HB-III-L):

[C10]

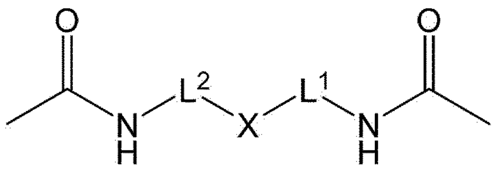

(III-L)

[in the formula (HB-III-L), the -CONH- and -NHCO- at either end represent amide bonds via any carboxyl groups of alginic acid;

-$L^1$- is defined as in [1-8] above;

-$L^2$- is defined as in [1-8] above; and X is a cyclic group selected from the group consisting of the partial structural formulae shown in the following tables (excluding the parts outside the dashed lines at both ends of each formula)]:

[Table 4-1]

| No. | X | No. | X |
|---|---|---|---|
| (TZ-1) | 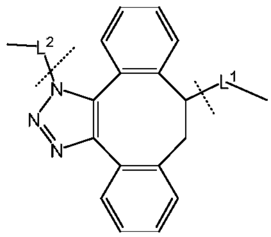 | (TZ-1-r) | 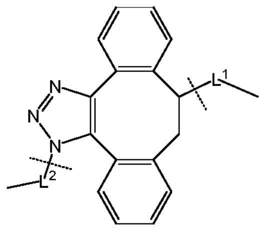 |
| (TZ-2) | 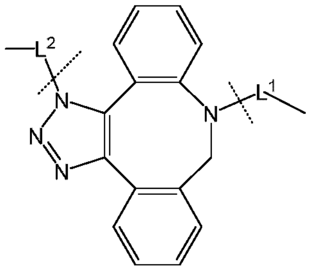 | (TZ-2-r) | 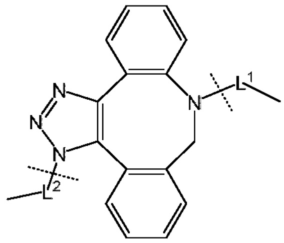 |
| (TZ-3) | 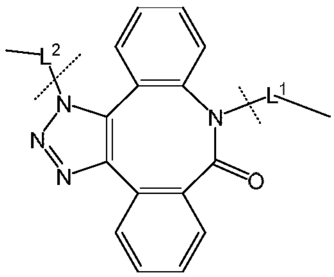 | (TZ-3-r) | 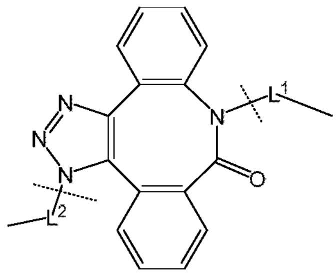 |
| (TZ-4) | 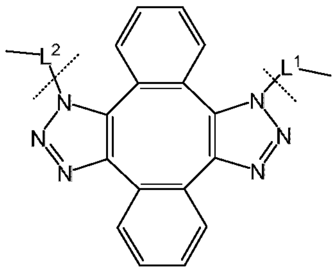 | (TZ-4-r) | 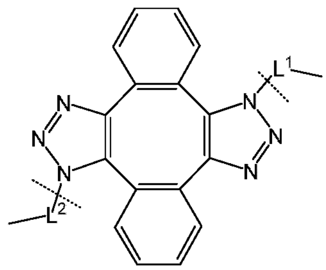 |
| (TZ-5) | 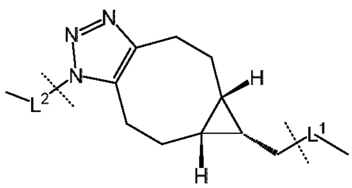 | (TZ-5-r) | 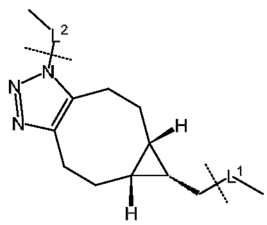 |
| (TZ-6) | 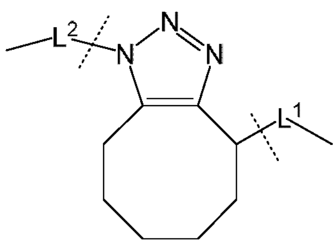 | (TZ-6-r) | 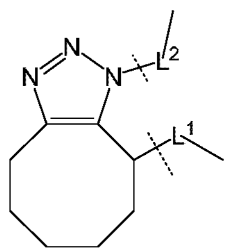 |

[Table 4-2]

| No. | X | No. | X |
|---|---|---|---|
| (TZ-7) | 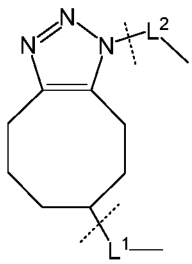 | (TZ-7-r) | 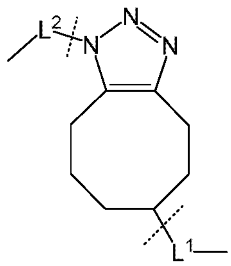 |
| (TZ-8) | 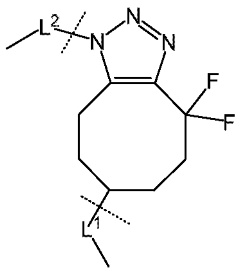 | (TZ-8-r) | 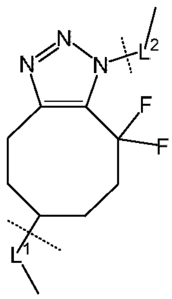 |
| (TZ-9) | 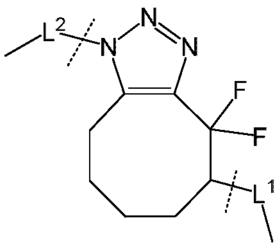 | (TZ-9-r) | 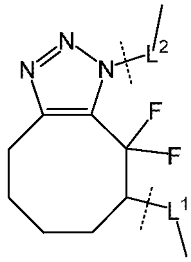 |
| (TZ-10) | 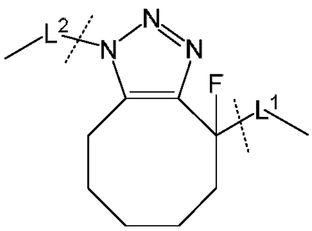 | (TZ-10-r) | 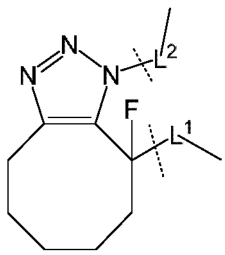 |
| (TZ-11) | 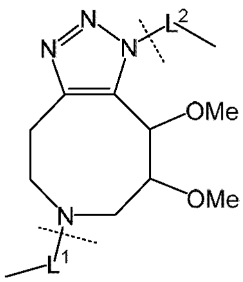 | (TZ-11-r) | 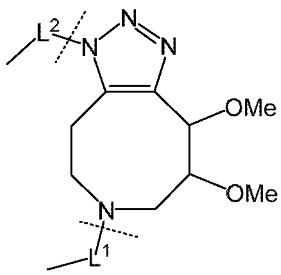 |

(continued)

| No. | X | No. | X |
|---|---|---|---|
| (TZ-12) | 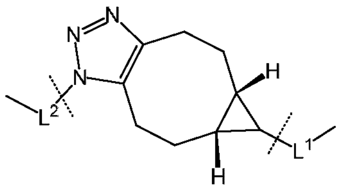 | (TZ-12-r) | 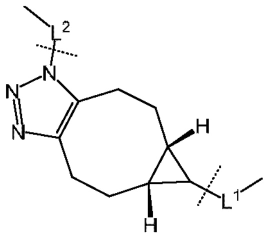 |

(with the proviso that excluding chemically crosslinked alginic acids formed from a combination of a derivative in which the -$L^1$- in the alginic acid derivative represented by formula (HB-I) is any one linker selected from the group consisting of (L1-1), (L1-2a), (L1-2b), (L1-11) and (L1-12) and a derivative in which -$L^2$- in the alginic acid derivative represented by formula (HB-II) is the linker of (L2-10)).

[1-10] The transplantation device according to [1-6] or [1-7] above, wherein the alginic acid derivative of formula (HA-I) is represented by following formula (EX-1-(I)-A-2):

[C11]

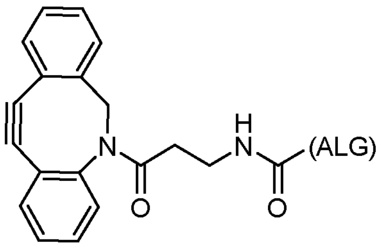

EX1-(I)-A-2

and the alginic acid derivative of formula (HA-II) is represented by following formula (EX-2-(II)-A-2):

[C12]

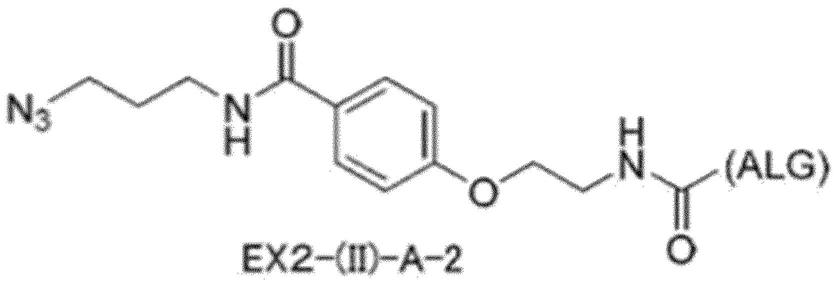

EX2-(II)-A-2

[1-11] The transplantation device according to [1-6] or [1-7] above, wherein the alginic acid derivative of formula (HA-I) is represented by following formula (EX-3-(I)-A-2):

[C13]

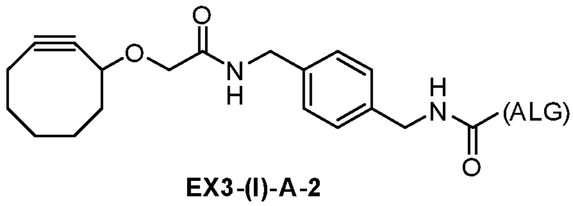

EX3-(I)-A-2

and the alginic acid derivative of formula (HA-II) is represented by following formula (EX-4-(II)-A-2)

[C14]

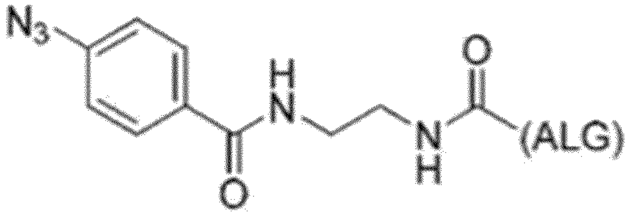

EX4-(II)-A-2

[1-12] The transplantation device according to [1-6] or [1-7] above, wherein the alginic acid derivative of the formula (HA-I) is the following formula (EX-1-(I)-A-2),

[C15]

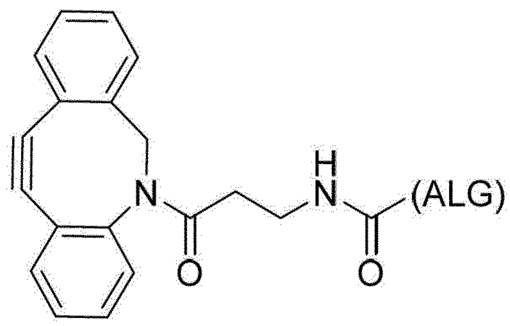

EX1-(I)-A-2

and the alginic acid of the formula (HA-II) is the following formula (EX-4-2-(II)-A-2),

[C16]

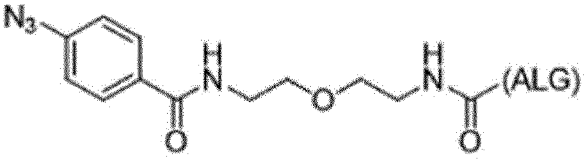

EX4-2-(II)-A-2

.

[1-13] The transplantation device according to [1-6] or [1-7] above, wherein the alginic acid derivative of the formula

(HA-I) is the following formula (EX-3-(I)-A-2),

[C17]

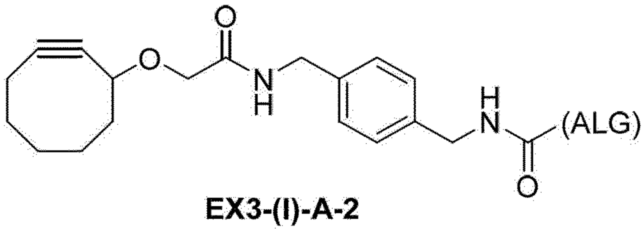

EX3-(I)-A-2

and the alginic acid of the formula (HA-II) is the following formula (EX-2-(II)-A-2),

[C18]

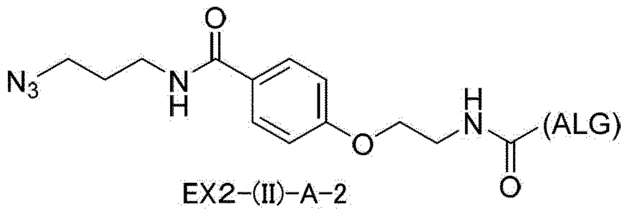

EX2-(II)-A-2

[1-14] The transplantation device according to [1-6] or [1-7] above, wherein the alginic acid derivative of the formula (HA-I) is the following formula (EX-3-(I)-A-2),

[C19]

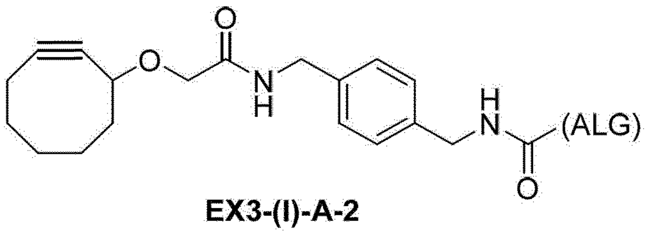

EX3-(I)-A-2

and the alginic acid of the formula (HA-II) is the following formula (EX-4-2-(II)-A-2),

[C20]

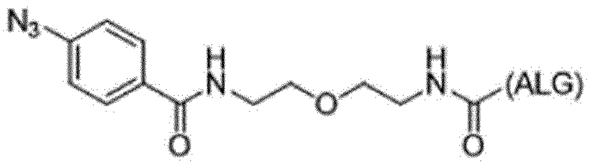

EX4-2-(II)-A-2

.

[1-15] The transplantation device according to any one of [1-1] to [1-14] above, wherein the pancreatic islets are human pancreatic islets or pig pancreatic islets.
[1-16] The transplantation device according to [1-15] above, wherein the pancreatic islets are pancreatic islets of an

adult pig.

[1-17] The transplantation device according to [1-15] above, wherein the pancreatic islets are fetal, neonatal or perinatal pig pancreatic islets.

[1-18] The transplantation device according to any one of [1-1] to [1-14] above, wherein the insulin-secreting cells or pancreatic islets are pancreatic islets, islet cells or beta cells obtained from a human donor, or pancreatic islets, islet cells or beta cells obtained from a pig donor, or pancreatic islets, islet cells or beta cells differentiated from human-derived stem cells.

[1-19] The transplantation device according to [1-18] above, wherein the insulin-secreting cells or pancreatic islets are pancreatic islets, islet cells or beta cells obtained from a human donor.

[1-20] The transplantation device according to any one of [1-1] to [1-19] above, wherein the hydrogel is further encapsulated in a semipermeable membrane.

[1-21] The transplantation device according to [1-20] above, wherein the semipermeable membrane is a dialysis membrane formed from a cellulose derivative.

[1-22] The transplantation device according to [1-21] above, wherein the cellulose derivative is cellulose acetate.

[1-23] The transplantation device according to any one of [1-1] to [1-22] above, wherein a transplantation site of the transplantation device is subcutaneous or intraperitoneal.

[1-24] The transplantation device according to any one of [1-1] to [1-23] above, wherein the transplantation device is from 0.1 to 5 mm thick.

[1-25] The transplantation device according to any one of [1-1] to [1-23] above, wherein the transplantation device is from 100 μm to less than 1000 μm thick.

[1-26] The transplantation device according to any one of [1-1] to [1-23] above, wherein the hydrogel is from 0.1 to 5 mm thick.

[1-27] The transplantation device according to any one of [1-1] to [1-23]above, wherein the hydrogen is from 100 μm to less than 500 μm thick.

[1-28] The transplantation device according to any one of [1-1] to [1-27] above, wherein the hydrogel comprising the insulin-secreting cells or pancreatic islets has been prepared and then encapsulated in a semipermeable membrane.

[1-29] The transplantation device according to any one of [1-1] to [1-28] above, obtained by suspending insulin-secreting cells or pancreatic islets in a solution of an alginic acid derivative that is hydrogelled by chemical crosslinking, enclosing the solution comprising the suspended insulin-secreting cells or pancreatic islets in a semipermeable membrane, and bringing the semipermeable membrane into contact with a solution comprising a divalent metal ion to thereby gel the alginic acid derivative inside the semipermeable membrane.

[1-30] The transplantation device according to [1-29] above, wherein the solution comprising a divalent metal ion is a solution comprising a calcium ion.

[1-31] The transplantation device according to any one of [1-1] to [1-30], wherein the hydrogel does not collapse when three hydrogels 10 mm in length and 20 mm in width have been shaken for 96 hours under conditions of reciprocating shaking at an amplitude of 25 mm and a shaking speed of 180 rpm with the temperature maintained at 37°C in 12 ml of 37°C physiological saline solution.

[1-32] The transplantation device according to any one of [1-1] to [1-31], wherein the collapse rate of the hydrogel is not more than 30% when three hydrogels 10 mm in length and 20 mm in width have been shaken for 96 hours under conditions of reciprocating shaking at an amplitude of 25 mm and a shaking speed of 180 rpm with the temperature maintained at 37°C in 12 ml of 37°C physiological saline solution.

[1-33] The transplantation device according to any one of [1-1] to [1-32], wherein almost no cells are shed from the hydrogel when three hydrogels 10 mm in length and 20 mm in width have been shaken for 24 hours under conditions of reciprocating shaking at an amplitude of 25 mm and a shaking speed of 180 rpm with the temperature maintained at 37°C in 12 ml of 37°C physiological saline solution.

[1-34] A transplantation device according to any one of [1-1] to [1-33], wherein when the number of cells encapsulated in the hydrogel is defined as 100%, the shedding rate of cells from the hydrogel after shaking agitation is not more than 30% when three hydrogels 10 mm in length and 20 mm in width have been shaken for 24 hours under conditions of reciprocating shaking at an amplitude of 25 mm and a shaking speed of 180 rpm with the temperature maintained at 37°C in 12 ml of 37°C physiological saline solution.

The second embodiment of the present invention is as shown in [2-1] to [2-34] below.

[2-1] A transplantation device comprising insulin-secreting cells or pancreatic islets encapsulated in a hydrogel, wherein the hydrogel has been gelled by chemical crosslinking of an alginic acid derivative, and wherein the thickness of the hydrogel is from 100 μm to less than 500 μm.

[2-2] The transplantation device according to [2-1] above, wherein the thickness of the hydrogel is from 150 μm to less than 500 μm.

[2-3] The transplantation device according to [2-1] or [2-2] above, wherein the thickness of the hydrogel is from 200 μm to less than 300 μm.

[2-4] The transplantation device according to any one of [2-1] to [2-3] above, wherein the hydrogel comprises, as crosslinking, chemical crosslinking formed between alginic acid derivatives.

[2-5] The transplantation device according to any one of [2-1] to [2-4] above, wherein the hydrogel comprises, as crosslinking, chemical crosslinking by triazole rings formed by a Huisgen reaction.

[2-6] The transplantation device according to any one of [2-1] to [2-5] above, wherein the chemical crosslinking is chemical crosslinking by a cyclic alkyne group introduced at any one or more carboxyl groups of alginic acid and an azide group introduced at any one or more carboxyl groups of alginic acid.

[2-7] The transplantation device according to [2-6] above, wherein the chemical crosslinking is chemical crosslinking by a cyclic alkyne group introduced at any one or more carboxyl groups of a first alginic acid and an azide group introduced at any one or more carboxyl groups of a second alginic acid.

[2-8] The transplantation device according to any one of [2-1] to [2-7] above, wherein the chemical crosslinking is chemical crosslinking by a combination of the alginic acid derivatives according to (A) and (B) below:

(A): an alginic acid derivative represented by formula (HA-I) below, comprising a cyclic alkyne group (Akn) introduced via an amide bond and a divalent linker ($-L^1-$) at any one or more carboxyl group groups of alginic acid:

[C21]

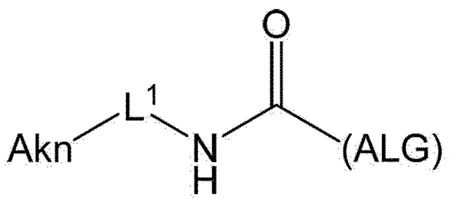

(I)

[in the formula (HA-I), (ALG) represents alginic acid; -NHCO- represents an amide bond via any carboxyl group of alginic acid; $-L^1-$ represents a divalent linker selected from the group consisting of the following partial structural formulae [excluding the parts outside the dashed lines at both ends of each formula]:

[C22]

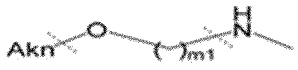

(LN-1) m1=2-6

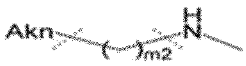

(LN-2) m2=1-6

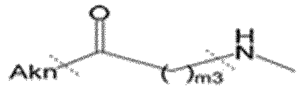

(LN-3) m3=1-6

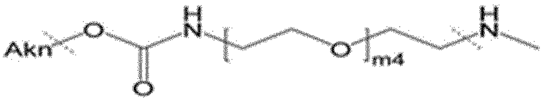

(LN-4) m4=1-6

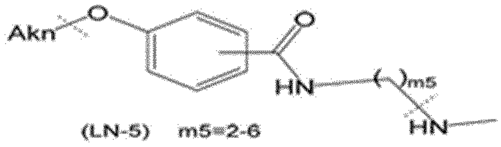

(LN-5) m5=2-6

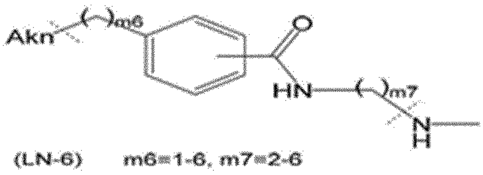

(LN-6) m6=1-6, m7=2-6

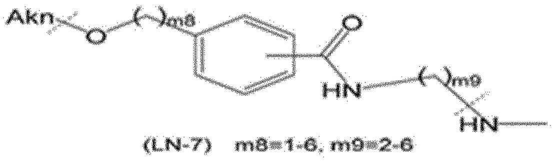

(LN-7) m8=1-6, m9=2-6

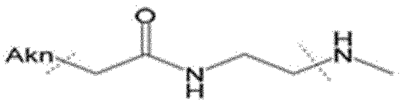

(LN-8)

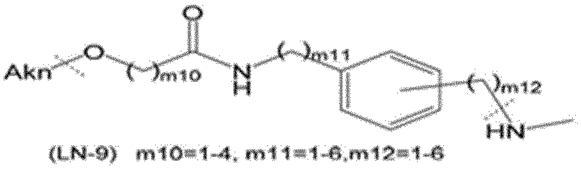

(LN-9) m10=1-4, m11=1-6,m12=1-6

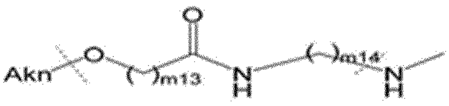

m13=1-4, m14=2~6

(LN-10)

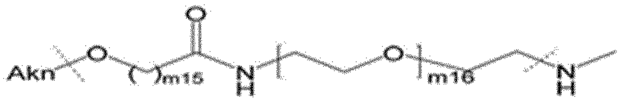

m15=1-4, m16=1-6

(LN-11)

and Akn is cyclic alkyne group selected from the following partial structural formulae (excluding the part to the right of the dashed line in each formula), in which the asterisks represent chiral centers]:

[C23]

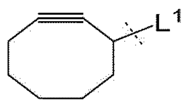

(AK-1)

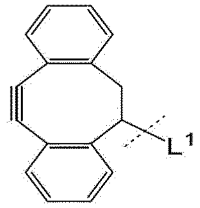

(AK-2)

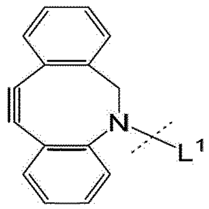

(AK-3)

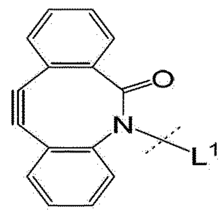

(AK-4)

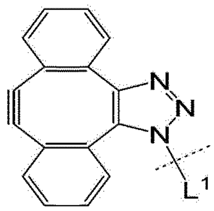

(AK-5)

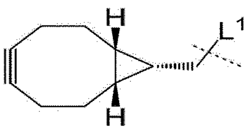

(AK-6)

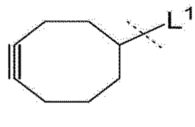

(AK-7)

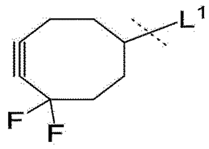

(AK-8)

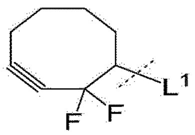

(AK-9)

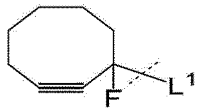

(AK-10)

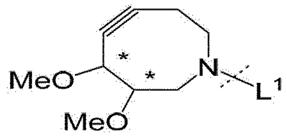

(AK-11)

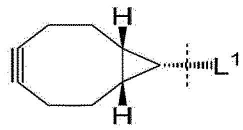

(AK-12)

(B): an alginic acid derivative represented by formula (HA-II) below, comprising an azide group introduced via an amide bond and a divalent linker (-$L^2$-) at any one or more carboxyl groups of alginic acid:

[C24]

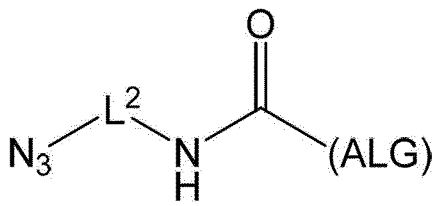

(II)

[in the formula (HA-II), (ALG) represents alginic acid; -NHCO- represents an amide bond via any carboxyl group of alginic acid; and -$L^2$- represents a divalent linker selected from the group consisting of the following partial structural formulae (excluding the parts outside the dashed lines at both ends of each formula)]:

[C25]

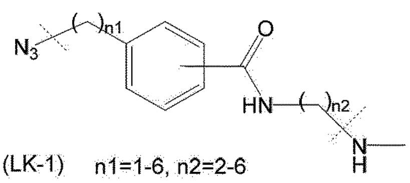

(LK-1) n1=1-6, n2=2-6

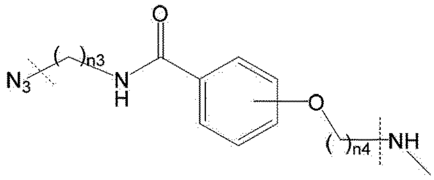

(LK-2) n3=2-6, n4=2-6

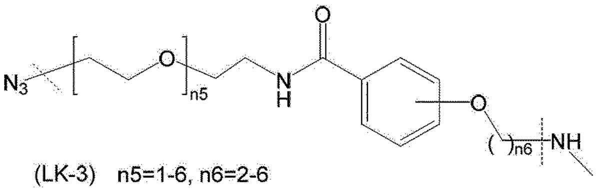

(LK-3) n5=1-6, n6=2-6

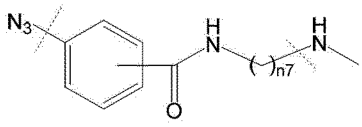

(LK-4) n7=2-6

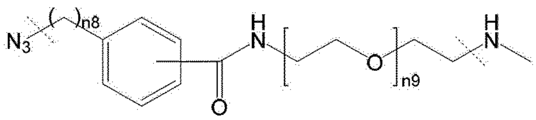

(LK-5) n8=1-4, n9=1-6

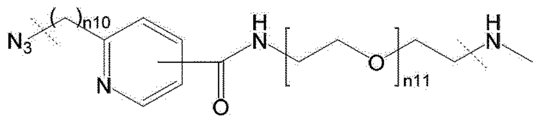

(LK-6) n10=1-4, n11=1-6

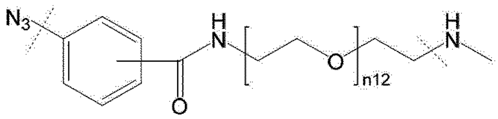

(LK-7) n12=1-6

[2-9] The transplantation device according to [2-8] above, wherein the chemically crosslinked alginic acid derivative is a crosslinked alginic acid in which any carboxyl group of a first alginic acid and any carboxyl group of a second alginic acid are linked via the following formula (HA-III-L):

[C26]

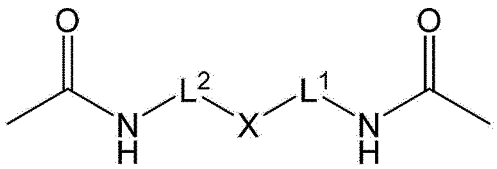

(III-L)

[in the formula (HA-III-L), the -CONH- and -NHCO- at either end represent amide bonds via any carboxyl group of alginic acid;

-$L^1$- is defined as in [2-8] above;

-$L^2$- is defined as in [2-8] above; and X is a cyclic group selected from the group consisting of the following partial structural formulae (excluding the parts outside the dashed lines at both ends of each formula), in which the asterisks represent chiral centers]:

[C27]

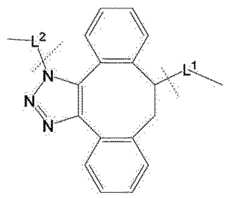
(TZ-1)

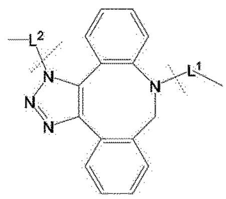
(TZ-2)

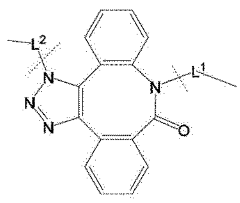
(TZ-3)

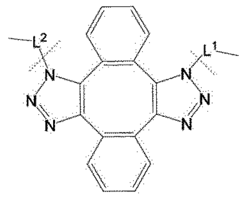
(TZ-4)

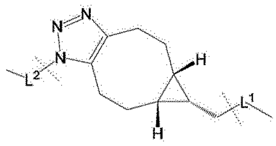
(TZ-5)

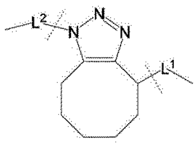
(TZ-6)

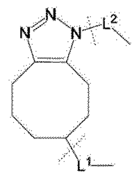
(TZ-7)

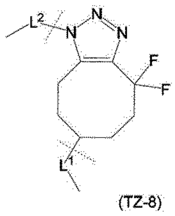
(TZ-8)

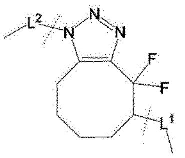
(TZ-9)

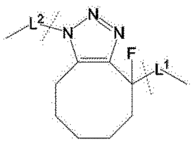
(TZ-10)

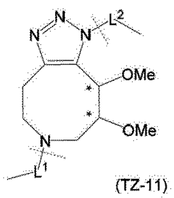
(TZ-11)

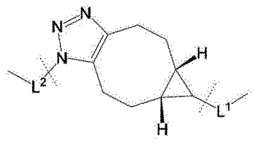
(TZ-12)

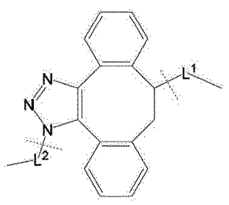
(TZ-1-r)

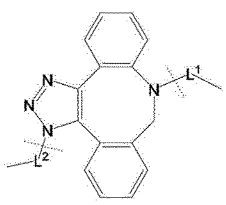
(TZ-2-r)

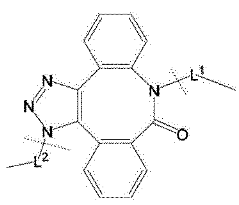
(TZ-3-r)

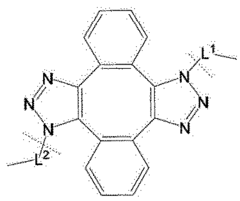
(TZ-4-r)

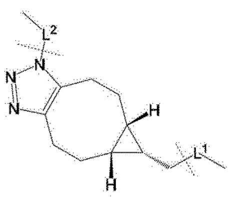
(TZ-5-r)

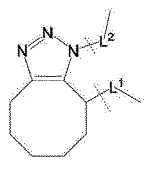
(TZ-6-r)

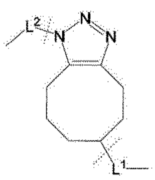
(TZ-7-r)

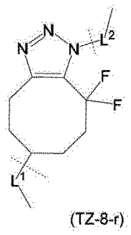
(TZ-8-r)

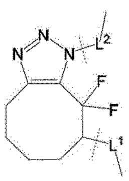
(TZ-9-r)

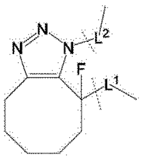
(TZ-10-r)

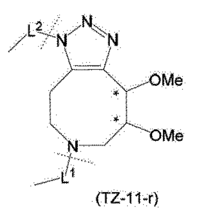
(TZ-11-r)

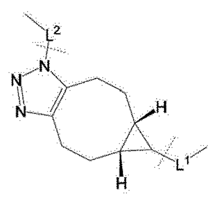
(TZ-12-r)

[2-10] The transplantation device according to any one of [2-1] to [2-7] above, wherein the chemical crosslinking is chemical crosslinking by a combination of the alginic acid derivatives according to (A) and (B) below:
(A): an alginic acid derivative represented by formula (HB-I) below, comprising a cyclic alkyne (Akn) introduced via an amide bond and a divalent linker (-$L^1$-) at any one or more carboxyl group groups of alginic acid:

[C28]

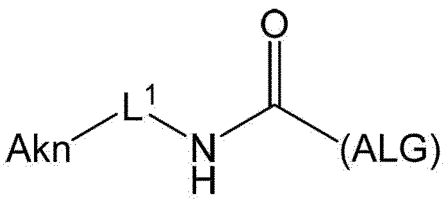

(I)

[in the formula (HB-I), (ALG) represents alginic acid; -NHCO- represents an amide bond via any carboxyl group of alginic acid; $-L^1-$ represents a linker selected from the group consisting of the partial structural formulae shown in the following tables (excluding the parts outside the dashed lines at both ends of each formula);

[Table 5-1]

| No. | $-L^1-$ | |
|---|---|---|
| (L1-1) | 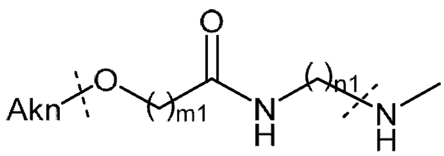 | m1=1-6<br>n1=2-6 |
| (L1-2a) | 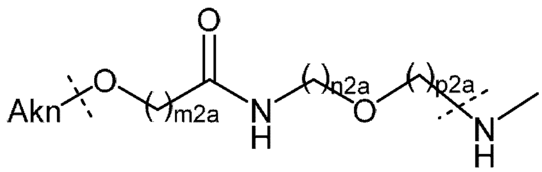 | m2a=1-6<br>n2a=2-6<br>p2a=2-6 |
| (L1-2b) | 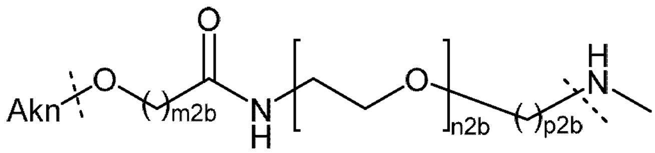 | m2b=1-6<br>n2b=1-6<br>p2b=2-6 |
| (L1-3) | 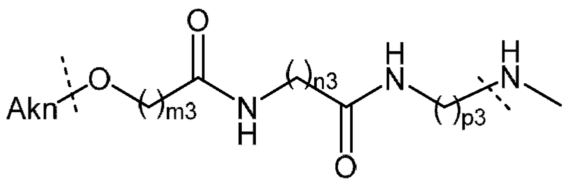 | m3=1-6<br>n3=1-6<br>p3=2-6 |
| (L1-4a) | 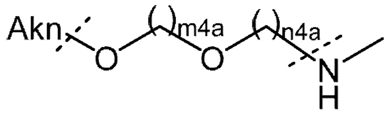 | m4a=2-6<br>n4a=2-6 |
| (L1-4b) | 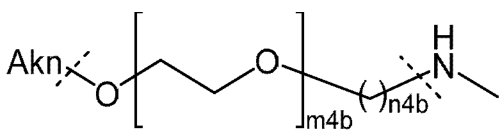 | m4b=0-6<br>n4b=2-6 |
| (L1-5a) | 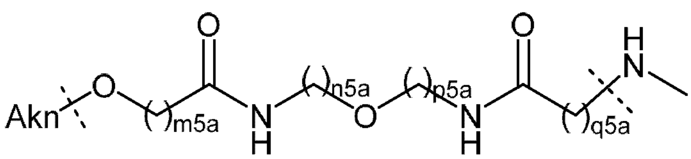 | m5a=1-6<br>n5a=2-6<br>p5a=2-6<br>q5a=1-6 |

(continued)

| No. | -L[1]- | |
|---|---|---|
| (L1-5b) | 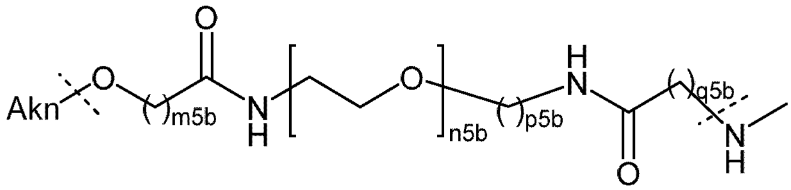 | m5b=1-6<br>n5b=1-6<br>p5b=2-6<br>q5b=1-6 |
| (L1-6a) | 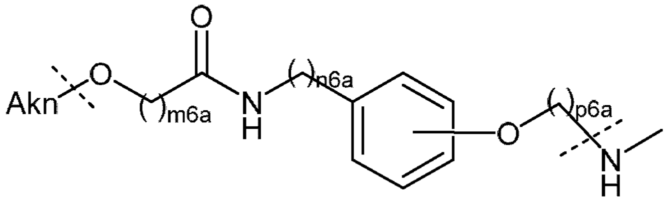 | m6a=1-6<br>n6a=0-6<br>p6a=2-6 |
| (L1-6b) | 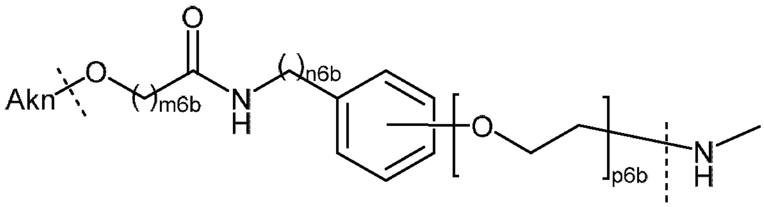 | m6b=1-6<br>n6b=1-6<br>p6b=2-6 |

[Table 5-2]

| No. | -L[1]- | |
|---|---|---|
| (L1-7) | 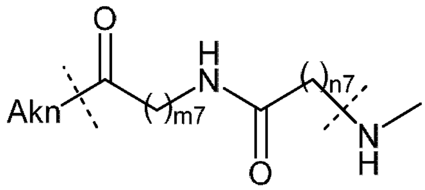 | m7=1-6<br>n7=1-6 |
| (L1-8a) | 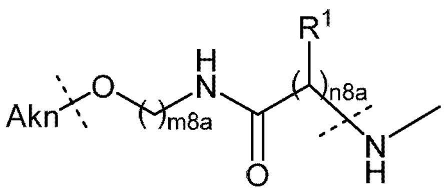 | m8a=2-6<br>n8a=1-6<br>$R^1$=H, Me, Et, Bn |
| (L1-8b) | 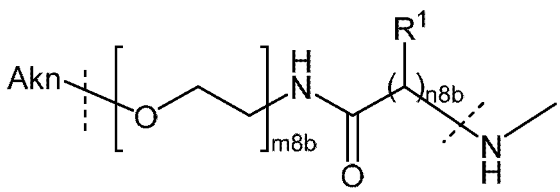 | m8b=1-6<br>n8b=1-6<br>$R^1$=H, Me, Et, Bn |
| (L1-9a) | 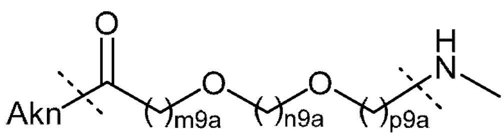 | m9a=1-6<br>n9a=2-6<br>p9a=2-6 |

(continued)

| No. | $-L^1-$ | |
|---|---|---|
| (L1-9b) | 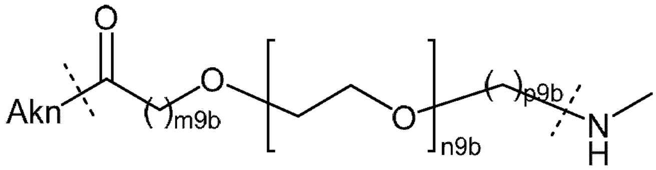 | m9b=1-6<br>n9b=1-6<br>p9b=2-6 |
| (L1-10) | 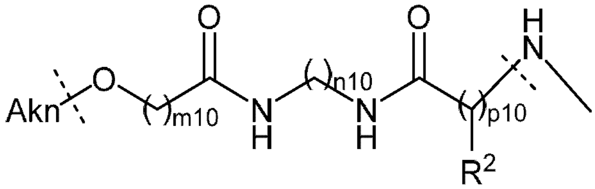 | m10=1-6<br>n10=2-6<br>p10=1-6<br>$R^2$=H, Me, Et, Bn |
| (L1-11) | 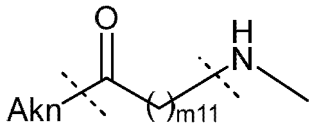 | m11=2-6 |
| (L1-12) | 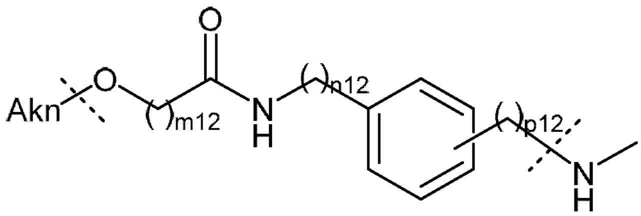 | m12=1-6<br>n12=0-6<br>p12=1-6 |

and Akn is a cyclic alkyne selected from the group consisting of the partial structural formula shown in the following table (excluding the part to the right of the dashed line in each formula)]:

[Table 6]

| No. | Akn | No. | Akn | No. | Akn |
|---|---|---|---|---|---|
| (AK-1) | 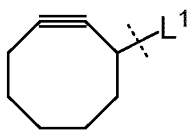 | (AK-5) | 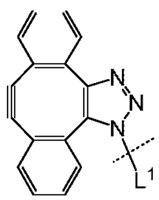 | (AK-9) | 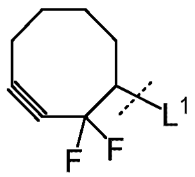 |
| (AK-2) | 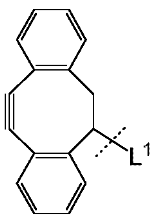 | (AK-6) | 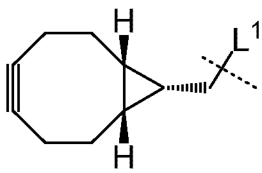 | (AK-10) | 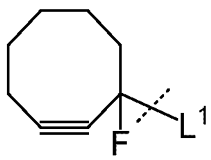 |
| (AK-3) | 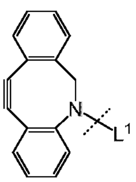 | (AK-7) | 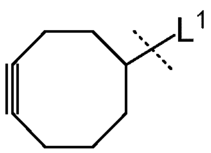 | (AK-11) | 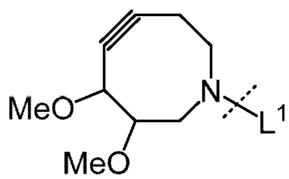 |

(continued)

| No. | Akn | No. | Akn | No. | Akn |
|---|---|---|---|---|---|
| (AK-4) | 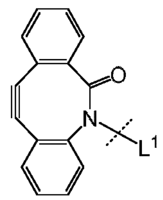 | (AK-8) | 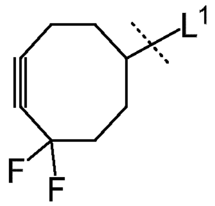 | (AK-12) | 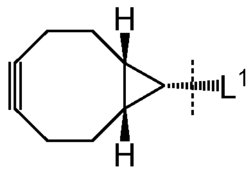 |

(B): an alginic acid derivative represented by formula (HB-II) below, comprising an azide group introduced via an amide bond and a divalent linker (-$L^2$-) at any one or more carboxyl groups of alginic acid:

[C29]

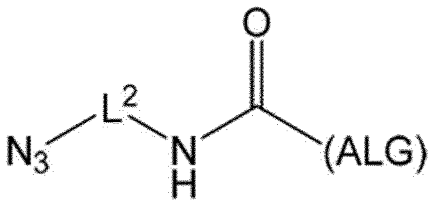

(II)

[in the formula (HB-II), (ALG) represents alginic acid; -NHCO- represents an amide bond via any carboxyl group of alginic acid; and -$L^2$- represents a linker selected from the group consisting of the partial structural formulae shown in the following tables (excluding the parts outside the dashed lines at both ends of each formula)]:

[Table 7-1]

| No. | -$L^2$- | |
|---|---|---|
| (L2-1) | 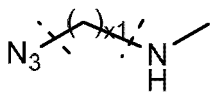 | x1=2-6 |
| (L2-2a) | 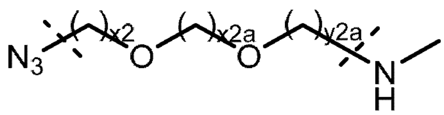 | x2=2-6<br>x2a=2-6<br>y2a=2-6 |
| (L2-2b) | 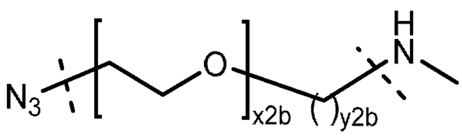 | x2b=1-6<br>y2b=2-6 |
| (L2-3) | 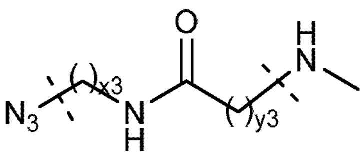 | x3=2-6<br>y3=1-6 |
| (L2-4) | 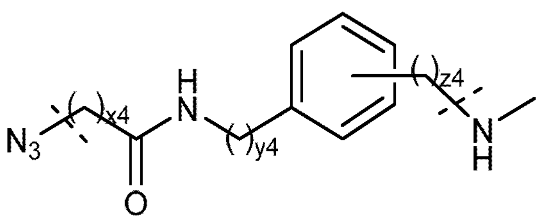 | x 4= 1-6<br>y4=1-6<br>z4=1-6 |

(continued)

| No. | -L²- | |
|---|---|---|
| (L2-5a) | 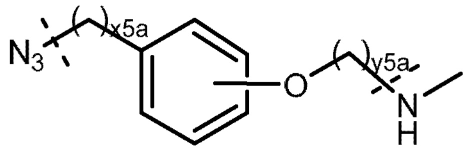 | x5a=0-4<br>y5a=2-6 |
| (L2-5b) | 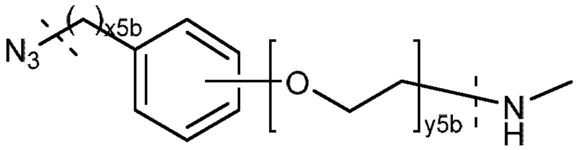 | x5b=0-4<br>y5b=1-6 |
| (L2-6a) | 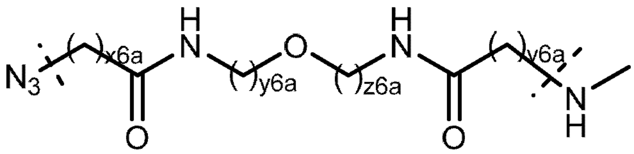 | x6a=1-6<br>y6a=2-6<br>z6a=2-6<br>v6a=1-6 |
| (L2-6b) | 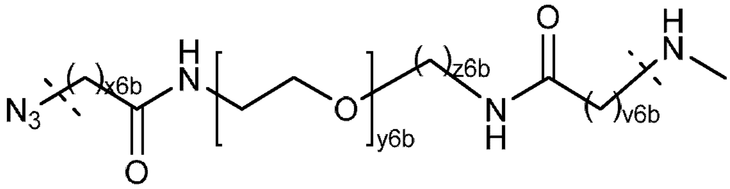 | x6b=1-6<br>y6b=1-6<br>z6b=2-6<br>v6b=1-6 |

[Table 7-2]

| No. | -L²- | |
|---|---|---|
| (L2-7a) | 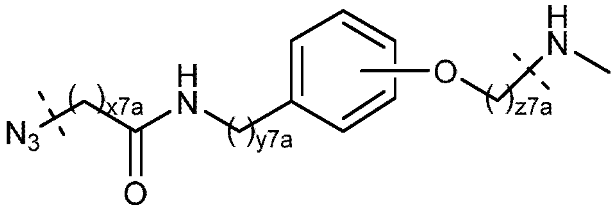 | x7a=1-6<br>y7a=1-6<br>z7a=2-6 |
| (L2-7b) | 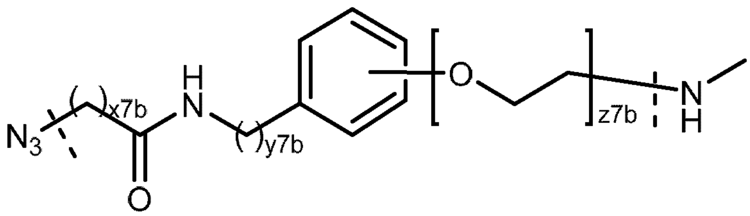 | x7b=1-6<br>y7b=1-6<br>z7b=1-6 |
| (L2-8a) | 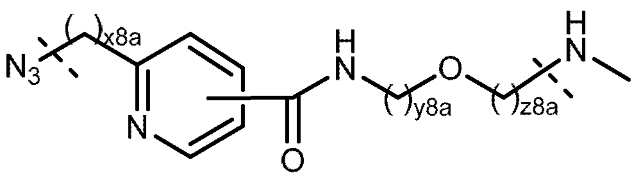 | x8a=0-4<br>y8a=2-6<br>z8a=3-6 |

(continued)

| No. | -$L^2$- | |
|---|---|---|
| (L2-8b) | 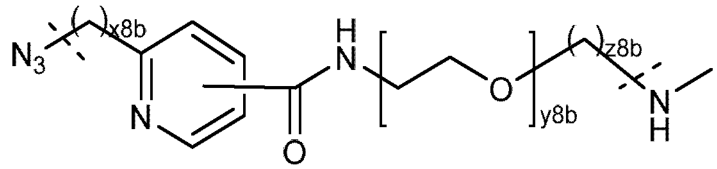 | x8b=0-4<br>y8b=1-6<br>z8b=3-6 |
| (L2-9a) | 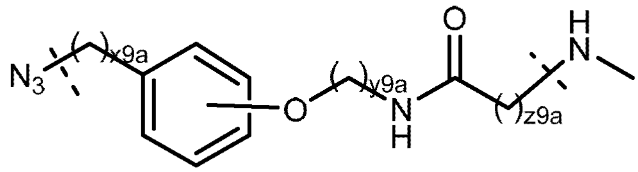 | x9a=0-4<br>y9a=2-6<br>z9a=1-6 |
| (L2-9b) | 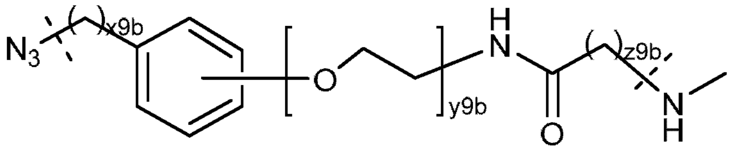 | x9b=0-4<br>y9b=1-6<br>z9b=1-6 |
| (L2-10) | 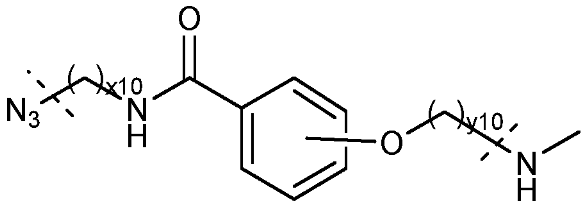 | x10=2-6<br>y10=2-6 |

(with the proviso that excluding chemical crosslinking by a combination of a derivative in which the -$L^1$- in the alginic acid derivative represented by formula (HB-I) is any one linker selected from the group consisting of (L1-1), (L1-2a), (L1-2b), (L1-11) and (L1-12) and a derivative in which -$L^2$- in the alginic acid derivative represented by formula (HB-II) is the linker of (L2-10).

[2-11] The transplantation device according to [2-10] above, wherein the chemically crosslinked alginic acid derivative is a crosslinked alginic acid in which any carboxyl group of a first alginic acid and any carboxyl group of a second alginic acid are linked via the following formula (HB-III-L):

[C30]

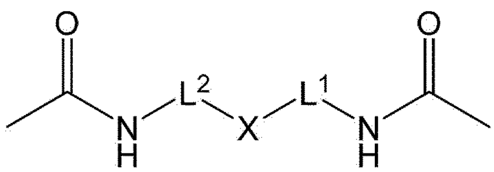

(III-L)

[in the formula (HB-III-L), the -CONH- and -NHCO- at either end represent amide bonds via any carboxyl group of alginic acid;

-$L^1$- is defined as in [2-10] above;

-$L^2$- is defined as in [2-10] above; and X is a cyclic group selected from the group consisting of the partial structural formulae shown in the following tables (excluding the parts outside the dashed lines at both ends of each formula)]:

[Table 8-1]

| No. | X | No. | X |
|---|---|---|---|
| (TZ-1) | 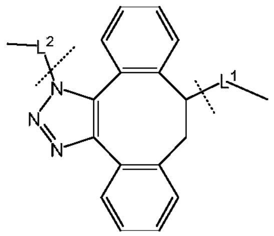 | (TZ-1-r) | 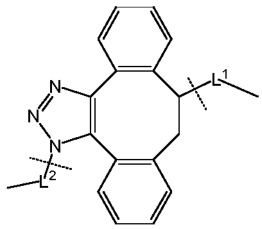 |
| (TZ-2) | 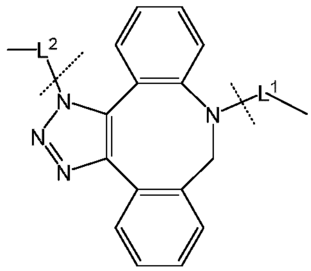 | (TZ-2-r) | 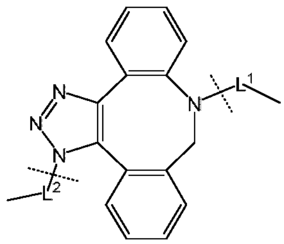 |
| (TZ-3) | 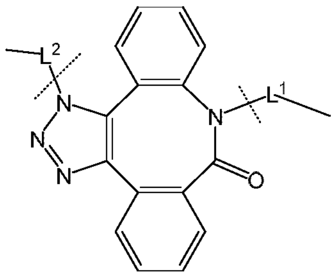 | (TZ-3-r) | 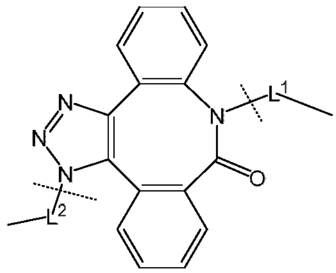 |
| (TZ-4) | 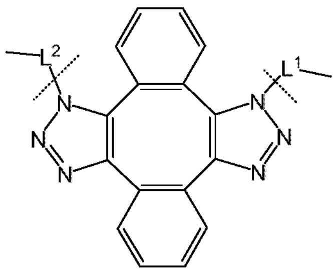 | (TZ-4-r) | 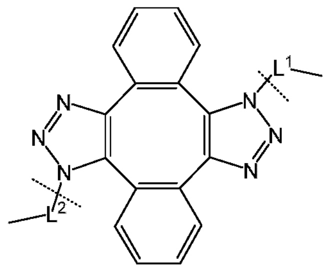 |
| (TZ-5) | 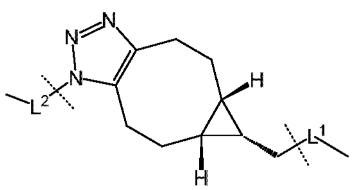 | (TZ-5-r) | 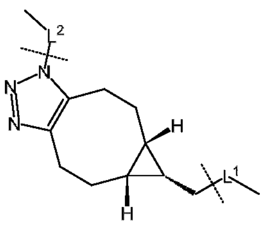 |
| (TZ-6) | 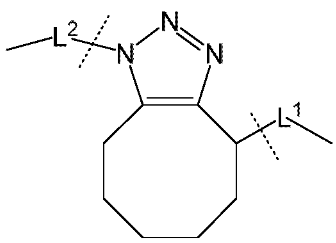 | (TZ-6-r) | 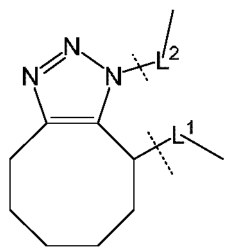 |

[Table 8-2]

| No. | X | No. | X |
|---|---|---|---|
| (TZ-7) | 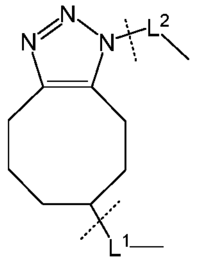 | (TZ-7-r) | 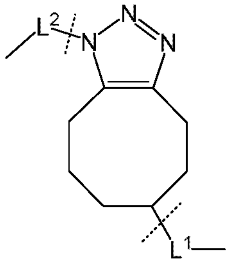 |
| (TZ-8) | 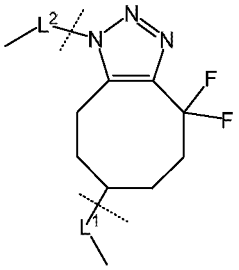 | (TZ-8-r) | 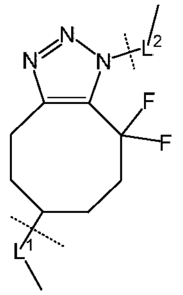 |
| (TZ-9) | 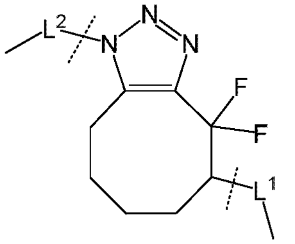 | (TZ-9-r) | 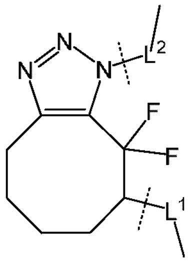 |
| (TZ-10) | 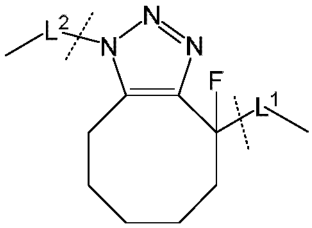 | (TZ-10-r) | 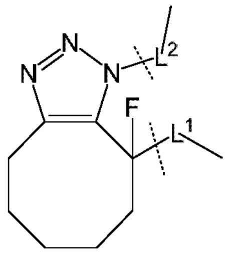 |
| (TZ-11) | 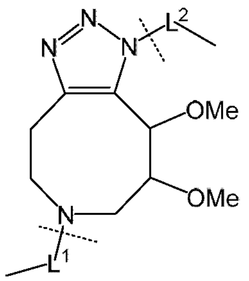 | (TZ-11-r) | 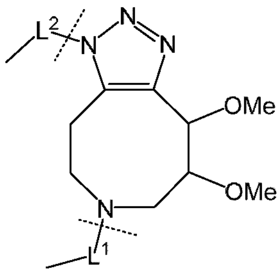 |

(continued)

| No. | X | No. | X |
|---|---|---|---|
| (TZ-12) | 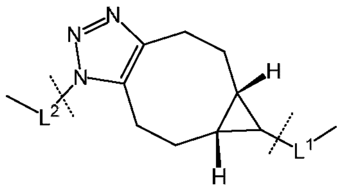 | (TZ-12-r) | 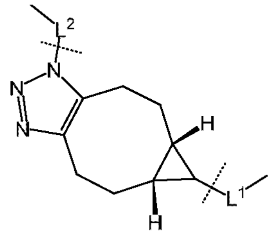 |

(with the proviso that excluding chemically crosslinked alginic acids formed from a combination of a derivative in which the -$L^1$- in the alginic acid derivative represented by formula (HB-I) is any one linker selected from the group consisting of (L1-1), (L1-2a), (L1-2b), (L1-11) and (L1-12) and a derivative in which -$L^2$- in the alginic acid derivative represented by formula (HB-II) is the linker of (L2-10)).

2-12] The transplantation device according to [2-8] or [2-9] above, wherein the alginic acid derivative of the formula (HA-I) is the following formula (EX-1-(I)-A-2),

[C31]

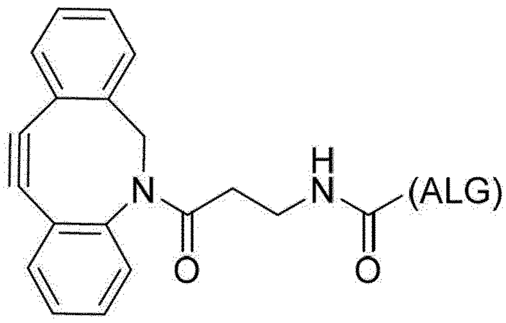

EX1-(I)-A-2

and the alginic acid of the formula (HA-II) is the following formula (EX-2-(II)-A-2),

[C32]

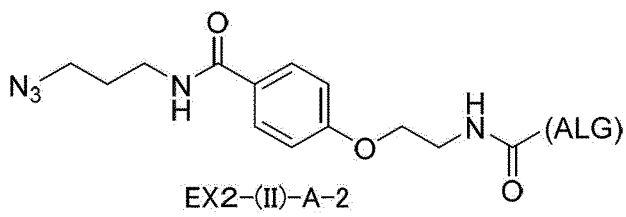

EX2-(II)-A-2

[2-13] The transplantation device according to [2-8] or [2-9] above, wherein the alginic acid derivative of the formula (HA-I) is the following formula (EX-3-(I)-A-2),

[C33]

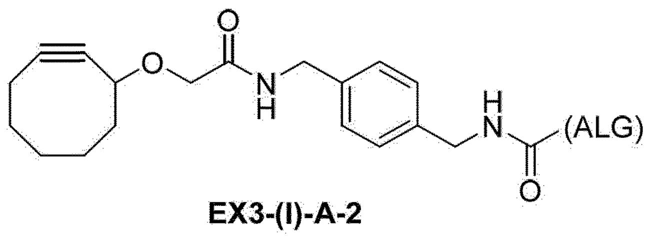

EX3-(I)-A-2

and the alginic acid of the formula (HA-II) is the following formula (EX-4-(II)-A-2),

[C34]

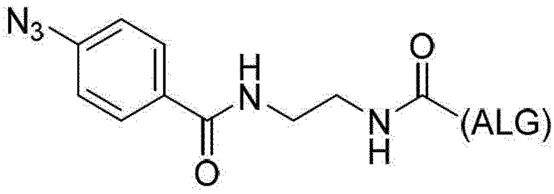

EX4-(II)-A-2

.

[2-14] The transplantation device according to [2-8] or [2-9] above, wherein the alginic acid derivative of the formula (HA-I) is the following formula (EX-1-(I)-A-2),

[C35]

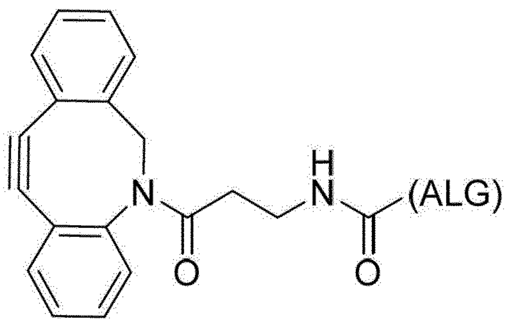

EX1-(I)-A-2

and the alginic acid of the formula (HA-II) is the following formula (EX-4-2-(II)-A-2):

[C36]

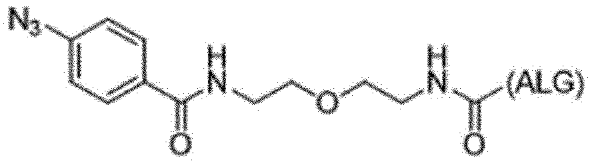

EX4-2-(II)-A-2

.

[2-15] The transplantation device according to [2-8] or [2-9] above, wherein the alginic acid derivative of the formula

(HA-I) is the following formula (EX-3-(I)-A-2),

[C37]

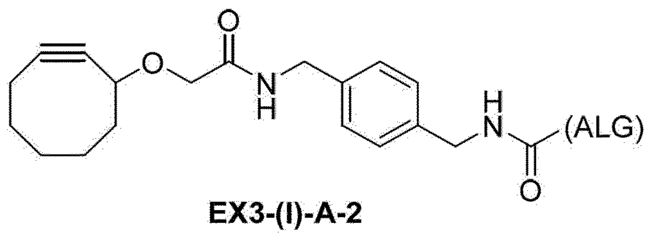

EX3-(I)-A-2

and the alginic acid of the formula (HA-II) is the following formula (EX-2-(II)-A-2),

[C38]

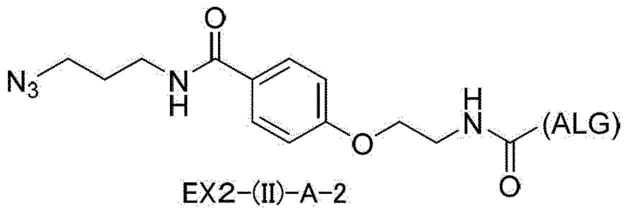

EX2-(II)-A-2

[2-16] The transplantation device according to [2-8] or [2-9] above, wherein the alginic acid derivative of the formula (HA-I) is the following formula (EX-3-(I)-A-2),

[C39]

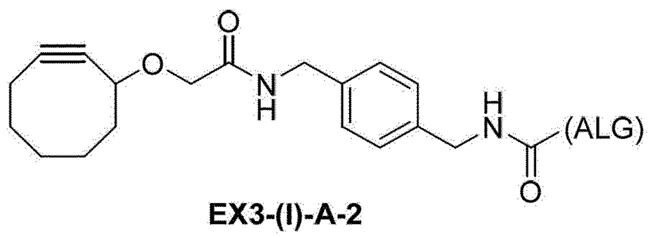

EX3-(I)-A-2

and the alginic acid of the formula (HA-II) is the following formula (EX-4-2-(II)-A-2):

[C40]

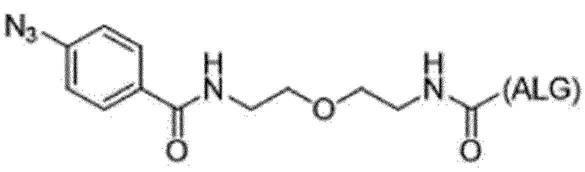

EX4-2-(II)-A-2

[2-17] The transplantation device according to any one of [2-1] to [2-16] above, wherein the pancreatic islets are human pancreatic islets or pig pancreatic islets.
[2-18] The transplantation device according to [2-17] above, wherein the pancreatic islets are pancreatic islets of an adult pig.

[2-19] The transplantation device according to [2-17] above, wherein the pancreatic islets are fetal, neonatal or perinatal pig islets.

[2-20] The transplantation device according to any one of [2-1] to [2-19] above, wherein the insulin-secreting cells or pancreatic islets are pancreatic islets, islet cells or beta cells obtained from a human donor, or pancreatic islets, islet cells or beta cells obtained from a pig donor, or pancreatic islets, islet cells or beta cells differentiated from human-derived stem cells.

[2-21] The transplantation device according to [2-20] above, wherein the insulin-secreting cells or pancreatic islets are pancreatic islets, islet cells or beta cells obtained from a human donor.

[2-22] The transplantation device according to any one of [2-1] to [2-21] above, wherein the hydrogel is further covered with a semipermeable membrane.

[2-23] The transplantation device according to [2-22] above, wherein the semipermeable membrane is a dialysis membrane formed from a cellulose derivative.

[2-24] The transplantation device according to [2-23] above, wherein the cellulose derivative is cellulose acetate.

[2-25] The transplantation device according to any one of [2-1] to [2-24] above, wherein the transplantation site of the transplantation device is subcutaneous or intraperitoneal.

[2-26] The transplantation device according to any one of [2-1] to [2-25] above, wherein the thickness of the transplantation device is from 100 μm to less than 1,000 μm.

[2-27] The transplantation device according to [2-26] above, wherein the thickness of the transplantation device is from 150 μm to less than 500 μm.

[2-28] The transplantation device according to any one of [2-1] to [2-27] above, wherein the hydrogel comprising insulin-secreting cells or pancreatic islets is first prepared and then covered with a semipermeable membrane.

[2-29] The transplantation device according to any one of [2-1] to [2-28] above, obtained by suspending insulin-secreting cells or pancreatic islets in a solution of an alginic acid derivative to be hydrogelled by chemical crosslinking, enclosing the solution of the suspended insulin-secreting cells or pancreatic islets in a semipermeable membrane, and then bringing the semipermeable membrane into contact with a solution comprising a divalent metal ion to thereby gel the alginic acid derivative in the semipermeable membrane.

[2-30] The transplantation device according to [2-29] above, wherein the solution comprising a divalent metal ion is a solution comprising a calcium ion.

[2-31] The transplantation device according to any one of [2-1] to [2-30] above, wherein the hydrogel does not collapse when three hydrogels 10 mm in length and 20 mm in width have been shaken for 96 hours under conditions of reciprocating shaking at an amplitude of 25 mm and a shaking speed of 180 rpm with the temperature maintained at 37°C in 12 ml of 37°C physiological saline solution.

[2-32] The transplantation device according to any one of [2-1] to [2-31] above, wherein the collapse rate of the hydrogel is not more than 30% when three hydrogels 10 mm in length and 20 mm in width have been shaken for 96 hours under conditions of reciprocating shaking at an amplitude of 25 mm and a shaking speed of 180 rpm with the temperature maintained at 37°C in 12 ml of 37°C physiological saline solution.

[2-33] The transplantation device according to any one of [2-1] to [2-32], wherein almost no cells are shed from the hydrogel when three hydrogels 10 mm in length and 20 mm in width have been shaken for 24 hours under conditions of reciprocating shaking at an amplitude of 25 mm and a shaking speed of 180 rpm with the temperature maintained at 37°C in 12 ml of 37°C physiological saline solution.

[2-34] The transplantation device according to any one of [2-1] to [2-33], wherein when the number of cells encapsulated in the hydrogel is defined as 100%, the shedding rate of cells from the hydrogel after shaking agitation is not more than 30% when three hydrogels 10 mm in length and 20 mm in width have been shaken for 24 hours under conditions of reciprocating shaking at an amplitude of 25 mm and a shaking speed of 180 rpm with the temperature maintained at 37°C in 12 ml of 37°C physiological saline solution.

The third embodiment of the present invention is as shown in [3-1] to [3-4] below.

[3-1] A method for manufacturing a transplantation device comprising insulin-secreting cells or pancreatic islets enclosed in a hydrogel, the method comprising following steps (a) to (d):

Step (a): an optional step in which a pancreas is extracted from a living body, and pancreatic islets are isolated;
Step (b): a step in which cells or a tissue selected from the group consisting of insulin-secreting cells, pancreatic islets, pancreatic islet cells obtained by culture, and pancreatic islet cells obtained by differentiation from stem cells are mixed with a solution of an alginic acid derivative that can be hydrogelled by chemical crosslinking;
Step (c): a step of bringing the alginic acid derivative solution obtained in the step (b) into contact with a solution comprising a divalent metal ion to thereby prepare a gel with a thickness of 0.1 to 5 mm (100 to 5000 μm); and
Step (d): an optional step of encapsulating the gel obtained in the step (c) in a semipermeable membrane.

[3-2] A method for manufacturing a transplantation device comprising insulin-secreting cells or pancreatic islets enclosed in a hydrogel, the method comprising following steps (a) to (d):

Step (a): an optional step in which a pancreas is extracted from a living body, and pancreatic islets are isolated;
Step (b): a step in which cells or tissue selected from the group consisting of insulin-secreting cells, pancreatic islets, pancreatic islet cells obtained by culture, and pancreatic islet cells obtained by differentiation from stem cells are mixed with a solution of an alginic acid derivative capable of being hydrogelled by chemical crosslinking;
Step (c): a step of bringing the alginic acid derivative solution obtained in the step (b) into contact with a solution comprising a divalent metal ion to thereby prepare a gel with a thickness of from 100 $\mu$m to less than 500 $\mu$m.
Step (d): an optional step in which the gel obtained in the step (c) is covered with a semipermeable membrane.

[3-3] A method for manufacturing a transplantation device comprising insulin-secreting cells or pancreatic islets encapsulated in a hydrogel, including the following steps (a) to (d).

Step (a): an optional step in which a pancreas is extracted from a living body, and in which pancreatic islets are isolated;
Step (b): a step in which a tissue or cells selected from the group consisting of insulin-secreting cells, pancreatic islets, pancreatic islet cells obtained by culture, and pancreatic islet cells obtained by differentiation from stem cells are mixed with a solution of an alginic acid derivative that can be hydrogelled by chemical crosslinking;
Step (c): a step of encapsulating the alginic acid derivative solution prepared in the step (b) in a semipermeable membrane; and
Step (d): a step of bringing the semipermeable membrane obtained in the step (c) into contact with a solution comprising a divalent metal ion to thereby gel the alginic acid derivative solution inside the semipermeable membrane.

[3-4] The method for manufacturing a transplantation device according to any one of [3-1] to [3-3] above, wherein the solution comprising a divalent metal ion is a solution comprising a calcium ion.
[3-5] A method for manufacturing a transplantation device comprising insulin-secreting cells or pancreatic islets encapsulated in a hydrogel, including the following steps (a), (b), (e) and (d).

Step (a): an optional step in which a pancreas is extracted from a living body, and in which pancreatic islets are isolated;
Step (b): a step in which a tissue or cells selected from the group consisting of insulin-secreting cells, pancreatic islets, pancreatic islet cells obtained by culture, and pancreatic islet cells obtained by differentiation from stem cells are mixed with a solution of an alginic acid derivative that can be hydrogelled by chemical crosslinking;
Step (e): a step of making the alginic acid derivative solution obtained in Step (b) into a gel with an arbitrary shape;
Step (d): an optional step in which the gel obtained in Step (e) is covered with a semipermeable membrane.

[3-6] A method for manufacturing a transplantation device comprising insulin-secreting cells or pancreatic islets encapsulated in a hydrogel, including the following steps (a), (b), (e) and (f)

Step (a): an optional step in which a pancreas is extracted from a living body, and in which pancreatic islets are isolated;
Step (b): a step in which a tissue or cells selected from the group consisting of insulin-secreting cells, pancreatic islets, pancreatic islet cells obtained by culture, and pancreatic islet cells obtained by differentiation from stem cells are mixed with a solution of an alginic acid derivative that can be hydrogelled by chemical crosslinking;
Step (e): a step of putting the alginic acid derivative solution obtained in Step (b) into any container or onto any surface;
Step (f): a step of gelling the alginic acid derivative solution in the container or on the surface obtained in Step (e).

[3-7] The method for manufacturing a transplantation device according to [3-6] above, wherein the any container is a semipermeable membrane, beaker or petri dish.
[3-8] The method for manufacturing a transplantation device according to [3-6] above, wherein the any surface is a semipermeable membrane surface, plastic surface or glass surface.
[3-9] The method for manufacturing a transplantation device according to any one of [3-5] to [3-8] above, wherein the hydrogel is from 0.1 to 5 mm (100 to 5,000 $\mu$m) thick.
[3-10] The method for manufacturing a transplantation device according to any of [3-5] to [3-9] above, wherein the gelling of the alginic acid derivative solution does not include a step of bringing into contact with a divalent metal

ion or a solution thereof.

[3-11] A method for manufacturing a transplantation device comprising insulin-secreting cells or pancreatic islets encapsulated in a hydrogel, including the following steps (a) to (c) and (f).

Step (a): an optional step in which a pancreas is extracted from a living body, and in which pancreatic islets are isolated;

Step (b): a step in which a tissue or cells selected from the group consisting of insulin-secreting cells, pancreatic islets, pancreatic islet cells obtained by culture, and pancreatic islet cells obtained by differentiation from stem cells are mixed with a solution of an alginic acid derivative that can be hydrogelled by chemical crosslinking;

Step (c): a step of enclosing the alginic acid derivative solution obtained in Step (b) in a semipermeable membrane;

(Step f): a step of gelling the alginic acid derivative solution in the semipermeable membrane obtained in Step (c) to thereby prepare a gel with a thickness of 0.1 to 5 mm (100 to 5,000 μm).

[Effect of the Invention]

The present invention provides a novel transplantation device. Preferably, the transplantation device exhibits at least one of the following effects.

(1) Excellent biocompatibility and stability, with little cytotoxicity and almost no adhesion or inflammation at the transplantation site.

(2) Shape maintained long-term, with little dissolution of the gel.

(3) Blood glucose lowering effects are sustained and blood glucose can be regulated long-term.

(4) Can be used over a long period of time because the alginic acid gel inside the semipermeable membrane can maintain its shape long-term without dissolving, and the pancreatic islets can survive and maintain their function.

(5) Can be a highly stable medical material that is replaceable and capable of immune isolation, with little adhesion, inflammation or the like.

(6) Substance permeability into and out of the device is excellent.

(7) The hydrogel does not collapse or hardly collapses when the hydrogel is shaken.

(8) There is little shedding of islet cells contained in the hydrogel when the hydrogel is shaken. Further, in a transplantation device having a thin hydrogel (for example, less than 500 μm), preferably at least one effect from among (1) to (8) above and/or at least one effect from among (9) to (10) below is elicited.

(9) The cure rate when the device is transplanted is higher than for thick hydrogels.

(10) The ratio of the oxygen concentration of the center part to the oxygen concentration of the device surface is high.

A transplantation device of a more preferred embodiment provides excellent transplantation results and functionality, is novel in terms of the material, and can have a sustained blood glucose lowering effect and regulate blood glucose long-term when transplanted into diabetes patients (especially type I diabetes patients and insulin-depleted type II diabetes patients). It can also be collected when the functions of the insulin-secreting cells or pancreatic islets in the hydrogel have declined. Periodic replacement or additional transplantation is also possible. Furthermore, insulin-secreting cells differentiated from stem cells (iPS cells or the like) or human pancreatic islets may be used as the insulin-secreting cells or pancreatic islets that are enclosed in the hydrogel of the transplantation device. The device of a more preferred embodiment is thus useful.

[Brief Description of Drawings]

[Fig. 1]
Fig. 1 shows photographs of a flat alginic acid gel, (a) before transplantation and (b) after transplantation.
[Fig. 2]
Fig. 2 shows photographs of a flat alginic acid gel, (a) before transplantation and (b) after transplantation.
[Fig. 3]
Fig. 3 shows photographs of a flat alginic acid gel, (a) before transplantation and (b) after transplantation.
[Fig. 4-1]
Fig. 4-1 is a photograph of a prepared transplantation device.
[Fig. 4-2]
Fig. 4-2 is a photograph of a prepared transplantation device.

[Fig. 5-1]
Fig. 5-1 shows blood glucose levels in mice into which a transplantation device was transplanted (up to 75 days after transplantation).
[Fig. 5-2]
Fig. 5-2 shows blood glucose levels in mice into which a transplantation device was transplanted (up to 305 days after transplantation).
[Fig. 5-3]
Fig. 5-3 shows blood glucose levels in a mouse into which a transplantation device was re-transplanted (up to 26 days after relay transplantation).
[Fig. 6-1]
Fig. 6-1 shows body weight in mice into which a transplantation device was transplanted (up to 75 days after transplantation).
[Fig. 6-2]
Fig. 6-2 shows body weight in mice into which a transplantation device was transplanted (up to 305 days after transplantation).
[Fig. 6-3]
Fig. 6-3 shows body weight in a mouse into which a transplantation device was re-transplanted (up to 26 days after relay transplantation).
[Fig. 7-1]
Fig. 7-1 shows blood glucose levels in mice into which a transplantation device was transplanted (up to 305 days after transplantation).
[Fig. 7-2]
Fig. 7-2 shows blood glucose levels in mice into which a transplantation device was re-transplanted (up to 26 days after relay transplantation).
[Fig. 8-1]
Fig. 8-1 shows body weight in mice into which a transplantation device was transplanted (up to 305 days after transplantation).
[Fig. 8-2]
Fig. 8-2 shows body weight in mice into which a transplantation device was re-transplanted (up to 26 days after relay transplantation).
[Fig. 9-1]
Fig. 9-1 shows blood glucose levels in mice into which a transplantation device was transplanted (up to 305 days after transplantation).
[Fig. 9-2]
Fig. 9-2 shows blood glucose levels in mice into which a transplantation device was re-transplanted (up to 26 days after relay transplantation).
[Fig. 10-1]
Fig. 10-1 shows body weight in mice into which a transplantation device was transplanted (up to 305 days after transplantation).
[Fig. 10-2]
Fig. 10-2 shows body weight in mice into which a transplantation device was re-transplanted (up to 26 days after relay transplantation).
[Fig. 11-1]
Fig. 11-1 shows blood glucose levels in mice into which a transplantation device was transplanted (up to 178 days after transplantation).
[Fig. 11-2]
Fig. 11-2 shows blood glucose levels in mice into which a transplantation device was transplanted (up to day 178 after transplantation).
[Fig. 11-3]
Fig. 11-3 shows blood glucose levels in mice into which a transplantation device was re-transplanted (up to day 40 after relay transplantation).
[Fig. 11-4]
Fig. 11-4 shows blood glucose levels in mice into which a transplantation device was transplanted (up to day 178 after transplantation).
[Fig. 11-5]
Fig. 11-5 shows blood glucose levels in mice into which a transplantation device was re-transplanted (up to day 40 after relay transplantation).
[Fig. 12-1]

Fig. 12-1 shows body weight in mice into which a transplantation device was transplanted (up to day 178 after transplantation).
[Fig. 12-2]
Fig. 12-2 shows body weight in mice into which a transplantation device was transplanted (up to day 178 after transplantation).
[Fig. 12-3]
Fig. 12-3 shows body weight in mice into which a transplantation device was re-transplanted (up to day 40 after relay transplantation).
[Fig. 12-4]
Fig. 12-4 shows body weight in mice into which a transplantation device was transplanted (up to day 178 after transplantation).
[Fig. 12-5]
Fig. 12-5 shows body weight in mice into which a transplantation device was re-transplanted (up to day 40 after relay transplantation).
[Fig. 13-1]
Fig. 13-1 shows blood glucose levels in mice into which a transplantation device was transplanted (up to day 17 after transplantation).
[Fig. 13-2]
Fig. 13-2 shows blood glucose levels in mice into which a transplantation device was transplanted (up to day 17 after transplantation).
[Fig. 13-3]
Fig. 13-3 shows blood glucose levels in a mouse into which a transplantation device was transplanted (up to day 17 after transplantation).
[Fig. 13-4]
Fig. 13-4 shows blood glucose levels in mice into which a transplantation device was transplanted (up to day 17 after transplantation).
[Fig. 13-5]
Fig. 13-5 shows blood glucose levels in mice into which a transplantation device was transplanted (up to day 17 after transplantation).
[Fig. 14-1]
Fig. 14-1 shows body weight in mice into which a transplantation device was transplanted (up to day 17 after transplantation).
[Fig. 14-2]
Fig. 14-2 shows body weight in mice into which a transplantation device was transplanted (up to day 17 after transplantation).
[Fig. 14-3]
Fig. 14-3 shows body weight in a mouse into which a transplantation device was transplanted (up to day 17 after transplantation).
[Fig. 14-4]
Fig. 14-4 shows body weight in mice into which a transplantation device was transplanted (up to day 17 after transplantation).
[Fig. 14-5]
Fig. 14-5 shows body weight in mice into which a transplantation device was transplanted (up to day 17 after transplantation).
[Fig. 15-1]
Fig. 15-1 shows a site for evaluating oxygen concentration in a hydrogel.
[Fig. 15-2]
Fig. 15-2 shows oxygen concentration in a cross-section of a hydrogel.
[Fig. 15-3]
Fig. 15-3 shows oxygen concentration in a cross-section of a hydrogel.
[Fig. 15-4]
Fig. 15-4 shows oxygen concentration in a cross-section of a hydrogel.
[Fig. 16-1]
Fig. 16-1 shows the permeation rate of human insulin from a transplantation device.
[Fig. 16-2]
Fig. 16-2 shows the permeation rate of glucose from a transplantation device.
[Fig. 17]
Fig. 17 shows the collapse rates of hydrogels when shaken.

[Fig. 18-1]
Fig. 18-1 is a microscopic photograph of an external solution 24 hours after the start of a test (bar indicates 1,000 μm).
[Fig. 18-2]
Fig. 18-2 is a microscopic photograph of an external solution 24 hours after the start of a test (bar indicates 1,000 μm).

[Description of Embodiments]

Provided here is a device for transplanting cells and the like into a living body, prepared using an alginic acid derivative that is gelled by chemical crosslinking, and more specifically a transplantation device comprising a chemically crosslinked alginic acid gel encapsulating insulin-secreting cells, pancreatic islets or the like, together with a semipermeable membrane covering the gel as necessary and a method for manufacturing the device. The alginic acid derivative that is gelled by chemical crosslinking is for example an alginic acid derivative of formula (HA-I) or formula (HA-II) having a cyclic alkyne or azide group introduced via an amide bond and a divalent linker at any one or more carboxyl groups of alginic acid, and a novel crosslinked alginic acid can be obtained by performing a Huisgen reaction (1,3-dipolar cycloaddition reaction) using the alginic acid derivatives of formula (HA-I) and formula (HA-II). Alternatively, the alginic acid derivative that is gelled by chemical crosslinking may be for example an alginic acid derivative of formula (HB-I) or formula (HB-II) having a cyclic alkyne or azide group introduced via an amide bond and a divalent linker at any one or more carboxyl groups of alginic acid, and a novel crosslinked alginic acid can be obtained by performing a Huisgen reaction (1,3-dipolar cycloaddition reaction) using the alginic acid derivatives of formula (HB-I) and formula (HB-II).

The "transplantation device" uses a hydrogel enclosing insulin-secreting cells or pancreatic islets. The hydrogel is produced by chemical crosslinking of alginic acid derivatives. Thus, alginic acid derivatives that can be gelled by chemical crosslinking are used. The shape of the hydrogel enclosing the insulin-secreting cells or pancreatic islets is, for example, a flat plate shape. In the transplantation device, the hydrogel may be further encapsulated in a semipermeable membrane, and in this case a hydrogel enclosing insulin-secreting cells or pancreatic islets has been inserted into the semipermeable membrane.

The "insulin-secreting cells" used in the transplantation device are cells having the function of secreting insulin, such as for example insulin-secreting beta cells among the cells constituting pancreatic islets. The "insulin-secreting cells" may also be cells that have acquired insulin-secreting ability through differentiation, maturation, modification or the like, including for example cells with insulin-secreting ability obtained by differentiation of stem cells such as iPS cells, ES cells, somatic stem cells (such as mesenchymal stem cells) or the like, cells with insulin-secreting ability obtained by maturation of juvenile cells or progenitor cells, or cells on which insulin-secreting ability has been conferred by genetic recombination. Differentiation or maturation of such cells may include culturing the cells, so in other words cells obtained by maturation or differentiation may include cells obtained by culture.

A "pancreatic islet," also called an islet of Langerhans, is a cell mass made up of an average of about 2,000 pancreatic islet cells. Pancreatic islets are constituted from five kinds of cells: glucagon-secreting alpha cells, insulin-secreting beta cells, somatostatin-secreting delta cells, ghrelin-secreting epsilon cells and pancreatic polypeptide-secreting PP (pancreatic polypeptide) cells.

"Insulin-secreting cells or pancreatic islets" are also referred to as cells or tissue having the function of secreting biologically active products.

In this Description, "pancreatic islet cells" may be any that include at least one kind of cell out of the 5 kinds of cells described above, but preferably they include at least beta cells. In certain embodiments, the pancreatic islet cells may be a mixture comprising all of alpha cells, beta cells, delta cells, epsilon cells and PP cells, and may also be cells contained in pancreatic islets.

The "pancreatic islet cells" may also be cells that have become pancreatic islet cells through differentiation, maturation, modification or the like. In this case, the "pancreatic islet cells" may include pancreatic islet cells obtained by differentiation of stem cells such as iPS cells, ES cells, somatic stem cells (such as mesenchymal stem cells) or the like, and pancreatic islet cells obtained by maturation of juvenile cells or progenitor cells.

"The insulin-secreting cells or pancreatic islets (including pancreatic islet cells)" preferably have sufficient survivability and function to allow them to improve a patient's disease state when transplanted into a patient. One function of insulin-secreting cells, pancreatic islets and pancreatic islet cells is insulin secretion for example, and preferably glucose responsiveness is retained even after transplantation.

The "transplantation device" of certain embodiments is called a bio-artificial islet device, which is one example of a bioartificial organ. The cells provided in the bio-artificial islet device include the aforementioned "insulin-secreting cells", "pancreatic islets" or "pancreatic islet cells", and include insulin-secreting cells for example. The insulin-secreting cells may be cells contained in pancreatic islets collected from a human or pig, or pancreatic islets differentiated from stem cells (such as ES cells, iPS cells or somatic stem cells (for example, mesenchymal stem cells)).

In some cases, the transplantation device of the present invention may use cells other than insulin secreting cells, pancreatic islets and pancreatic islet cells.

The cells other than insulin-secreting cells, pancreatic islets and pancreatic islet cells may be any cells that can be used in cell transplantation, with no particular limits on the type of cells. One kind of cell may be used, or multiple kinds may be combined. Examples of cells that can be used by preference include animal cells, preferably cells from vertebrates, and especially cells from humans. The cells from vertebrates (especially cells from humans) may be either stem cells (such as universal cells or somatic stem cells), precursor cells, or mature cells. Universal cells that may be used include for example embryonic stem (ES) cells, germline stem (GS) cells or induced pluripotent stem (iPS) cells. Somatic stem cells that may be used include for example mesenchymal stem cells (MSC), hematopoietic stem cells, amniotic epithelial cells, umbilical cord blood cells, bone marrow-derived cells, myocardial stem cells, adipose-derived stem cells and neural stem cells.

Precursor cells and mature cells that can be used include cells from skin, dermis, epidermis, muscle, myocardium, nerves, bone, cartilage, endothelium, brain, epithelium, heart, kidney, liver, pancreas, oral cavity, cornea, bone marrow, umbilical cord blood, amniotic membrane and hair for example. Cells from humans that can be used include ES cells, iPS cells, MSC cells, cartilage cells, osteoblasts, osteoblast progenitor cells, mesenchymal cells, myoblasts, myocardial cells, myocardial blast cells, nerve cells, liver cells, fibroblasts, corneal endothelial cells, vascular endothelial cells, corneal epithelial cells, amniotic membrane cells, umbilical cord blood cells, bone marrow-derived cells and hematopoietic stem cells. The cells may be either autologous or allogenic cells. Out of these cells, ES cells, iPS cells and mesenchymal stem cells (MSC) for example may be used in certain embodiments.

The donor of the "insulin-secreting cells or pancreatic islets (including pancreatic islet cells)" is an animal, or preferably a vertebrate, and specifically may be a human, pig, monkey, rat, mouse or the like, or preferably a human or pig. In certain embodiments, the donor of the "insulin-secreting cells or pancreatic islets (including pancreatic islet cells)" is a pig from the perspective of relieving the donor shortage. The "insulin-secreting cells or pancreatic islets (including pancreatic islet cells)" may be either pancreatic islets obtained from a donor animal or insulin-secreting cells or pancreatic islet cells obtained from cells from a donor, and for example may be insulin-secreting cells or pancreatic islet cells that have been differentiated from human-derived ES cells or iPS cells.

When the "insulin-secreting cells or pancreatic islets (including pancreatic islet cells)" are from a pig, examples include adult pig islets or fetal, neonatal or perinatal pig islets. Such pancreatic islets may be used after being cultured appropriately, and fetal, neonatal or perinatal pig islets may also be matured before being used.

A method of incision and implantation, an injection method, or an endoscopic or laparoscopic method may be used as the method for transplanting the transplantation device.

The transplantation site is not particularly limited, and may be subcutaneous, intraperitoneal, intrahepatic, intramuscular, omental, subcapsular or the like, but subcutaneous or intraperitoneal transplantation is preferred.

A "semipermeable membrane" here is a membrane that allows passage only of molecules or ions below a specific size. When solutions of two different concentrations are brought into contact on either side of a semipermeable membrane in a system of a solute that does not penetrate the semipermeable membrane and a solvent that exhibits permeability, osmotic pressure is generated at a distance and only the solvent passes through the membrane. Although the transplantation device described in this Description may include a semipermeable membrane, this is not essential, and the semipermeable membrane may also be omitted. A transplantation device of certain embodiments is a hydrogel by itself (enclosing insulin-secreting cells or pancreatic islets for example), or in other words a hydrogel that is not encapsulated in a semipermeable membrane. A transplantation device in which the hydrogel is not encapsulated in a semipermeable membrane preferably has excellent biocompatibility and stability with little cytotoxicity and almost no adhesion or inflammation at the transplantation site and can maintain its shape long-term with little dissolution of the gel, and more preferably it can provide continuous blood glucose lowering effects and regulate blood glucose over a long period of time. In a transplantation device of certain other embodiments, the hydrogel is encapsulated in a semipermeable membrane. The semipermeable membrane may be for example a tube or a membrane used in dialysis, such as a dialysis tube, a cotton cellulose dialysis membrane, a regenerated cellulose dialysis membrane or a cellulose ester dialysis membrane, and examples of commercial products include the Cellu-Sep T Tubular Membrane (Membrane Filtration Products, Inc.), Spectra Biotech Membrane (Spectrum Chemical Corp. (today's Repligen)), Spectra/Por CE dialysis tube (Spectrum Chemical Corp. (today's Repligen)) and the like.

A semipermeable membrane prepared from a cellulose ester is preferred as the "semipermeable membrane", and specific examples include dialysis membranes such as the Spectra/Por CE dialysis tube (Spectrum Chemical Corp. (today's Repligen)). The cellulose ester is more preferably a polymer of cellulose acetate.

The semipermeable membrane used here contains a resin. The semipermeable membrane may be prepared for example by dissolving at least one kind of resin in a solvent and coagulating the dissolved resin. The resin is not particularly limited. For example, an ethylene-vinyl alcohol copolymer, polysulfone polymer or polyacrylonitrile polymer, a cellulose polymer such as cellulose acetate, a polyamide polymer, a polycarbonate polymer or the like may be used as the resin. A cellulose polymer such as cellulose acetate is more preferred.

The semipermeable membrane used here has a "molecular weight cutoff". The "molecular weight cutoff" means the largest molecular weight that is not substantially blocked by the membrane. Molecules having molecular weights

that exceed the molecular weight cutoff are substantially prevented from passing in and out of the semipermeable membrane. The "molecular weight cutoff" of the semipermeable membrane used here is preferably 100 kDa (kilodaltons). For example, the Spectra/Por CE dialysis tube (Spectrum Chemical Corp. (today's Repligen)) is a cellulose ester dialysis membrane that is sold with cutoff values [MWCO] of 100 to 500 Da (daltons), 0.5 to 1kDa, 3.5 to 5 kDa, 8 to 10 kDa, 20 kDa, 50 kDa, 100 kDa, 300 kDa and 1,000 kDa and the like. When the molecular weight cutoff value is greater than about 500,000 Daltons for example, molecules such as IgG and complements can penetrate the semipermeable membrane, but host cells such as immune cells are prevented from penetrating the semipermeable membrane, and insulin and nutrients and oxygen for the cells can pass through the semipermeable membrane. The unit Dalton symbol is Da, and 1,000 Da means 1 kDa.

The thickness of the transplantation device here is defined as discussed below. In certain embodiments, the thickness of the transplantation device is from 0.1 to 5 mm (100 to 5,000 μm), and preferably from 0.1 to 3 mm (100 to 3,000 μm). Alternatively, it is preferably from 0.5 to 5 mm (500 to 5,000 μm), or more preferably from 1 to 3 mm (1,000 to 3,000 μm). When the hydrogel encapsulating the insulin-secreting cells or pancreatic islets is covered with a semipermeable membrane, the thickness of the semipermeable membrane in the transplantation device is from 0.15 to 5 mm (150 to 5,000 μm), or preferably from 0.2 to 3 mm (200 to 3,000 μm). Alternatively, it is preferably from 0.5 to 5 mm (500 to 5,000 μm), or more preferably from 1 to 3 mm (1,000 to 3,000 μm).

In the case of a thin transplantation device, the thickness of the transplantation device is from 100 μm to less than 1,000 μm, or preferably from 100 μm to less than 500 μm. Alternatively, it is preferably from 150 μm to less than 1,000 μm, or more preferably from 150 μm to 500 μm. When the hydrogel encapsulating the insulin-secreting cells or pancreatic islets is covered with a semipermeable membrane, the thickness of the semipermeable membrane in the transplantation device is preferably from 150 μm to less than 1000 μm, or more preferably from 150 μm to less than 500 μm. Alternatively, it is preferably from 200 μm to less than 1,000 μm, or more preferably from 200 μm to 500 μm.

The thickness of the hydrogel is also defined as discussed below. In certain embodiments, the thickness of the hydrogel is from 0.1 to 5 mm (100 to 5,000 μm), or preferably from 0.1 to 3 mm (100 to 3,000 μm), or more preferably from 0.1 to 1 mm (100 to 1,000 μm). It may also be from 0.15 to 5 mm (150 to 5,000 μm), or preferably from 0.15 to 3 mm (150 to 3,000 μm), or more preferably from 0.15 to 1 mm (150 to 1,000 μm). It may also be from 0.2 to 5 mm (200 to 5,000 μm), or preferably from 0.2 to 3 mm (200 to 3,000 μm), or more preferably from 0.2 to 1 mm (200 to 1,000 μm). It may also be from 0.5 to 5 mm (500 to 5,000 μm), or preferably from 0.5 to 3 mm (500 to 3,000 μm), or more preferably from 0.5 to 1 mm (500 to 1,000 μm).

When the transplantation device includes a semipermeable membrane, the thickness of the hydrogel in the semipermeable membrane is from 0.1 to 3 mm (100 to 3,000 μm), or preferably from 0.1 to 2 mm (100 to 2,000 μm), or more preferably from 0.1 to 1 mm (100 to 1,000 μm). It may also be from 0.15 to 3 mm (150 to 3,000 μm), or preferably from 0.15 to 2 mm (150 to 2,000 μm), or more preferably from 0.15 to 1 mm (150 to 1,000 μm). It may also be from 0.2 to 3 mm (200 to 3,000 μm), or preferably from 0.2 to 2 mm (200 to 2,000 μm), or more preferably from 0.2 to 1 mm (200 to 1,000 μm). Alternatively, it is preferably from 1 to 3 mm (1,000 to 3,000 μm), or more preferably from 1.5 to 2 mm (1,500 to 2,000 μm).

When the transplantation device does not include a semipermeable membrane, the thickness of the hydrogel is from 0.1 to 5 mm (100 to 5,000 μm), or preferably from 0.1 to 3 mm (100 to 3,000 μm), or more preferably from 0.1 to 1 mm (100 to 1,000 μm). It may also be from 0.15 to 5 mm (150 to 5,000 μm), or preferably from 0.15 to 3 mm (150 to 3,000 μm), or more preferably from 0.15 to 1 mm (150 to 1,000 μm). It may also be from 0.2 to 5 mm (200 to 5,000 μm), or preferably from 0.2 to 3 mm (200 to 3,000 μm), or more preferably from 0.2 to 1 mm (200 to 1,000 μm). It may also be from 0.5 to 5 mm (500 to 5,000 μm), or preferably from 0.5 to 3 mm (500 to 3,000 μm), or more preferably from 0.5 to 1 mm (500 to 1,000 μm).

In the case of a thin hydrogel, the thickness of the hydrogel is from 100 μm to less than 500 μm, or preferably from 100 μm to less than 400 μm, or more preferably from 100 μm to less than 300 μm. It may also be from 150 μm to less than 500 μm, or preferably from 150 μm to less than 400 μm, or more preferably from 150 μm to less than 300 μm, or still more preferably from 200 μm to less than 300 μm. This thickness is the same whether or not the transplantation device includes a semipermeable membrane.

The shape of the transplantation device is not particularly limited as long as it is flat. Flat means a flat plate, indicating a plate shape with a wide area and a roughly uniform thickness. Examples of such plate shapes include triangular, rectangular, pentagonal and other polygonal and round flat plate shapes and the like. Moreover, preferably the transplantation device has the above thickness and a roughly uniform thickness across the entire plate. The variation in thickness in a plate-shaped transplantation device is preferably within ±20%, or more preferably within ±10%. The thickness of the transplantation device is the thickness of the thickest part of the transplantation device. For example, when the hydrogel is encapsulated in a dialysis tube (which is a semipermeable membrane) and both ends of the dialysis tube are sealed, the shape of the transplantation device appears at first glance like a rugby ball, with both ends somewhat thin and the middle thicker than the two ends. With a shape like this, the thickness of the transplantation device is the thickness near the center at the thickest part of the rugby ball.

The shape of the hydrogel is also not particularly limited as long as it is flat. Flat means a flat plate, indicating a plate shape with a wide area and a roughly uniform thickness. Examples of such plate shapes include triangular, rectangular, pentagonal and other polygonal and round flat plate shapes and the like. Moreover, preferably the hydrogel has the above thickness and a roughly uniform thickness across the entire plate. The variation in thickness of the hydrogel is preferably within ±20%, or more preferably within ±10%. The thickness of the hydrogel is the thickness of the thickest part of the hydrogel. In certain embodiments, the flat plate shaped hydrogel is a crosslinked alginic acid gel with a short diameter of 12 to 15 mm, a long diameter of 12 to 18 mm and a thickness of about 0.1 to 5 mm, and may also assume a round, rectangular, hexagonal or octagonal shape or the like for example. Expressed in terms of area, the size of the flat plate shaped hydrogel may also be represented as 144 to 270 $mm^2$ for example.

In this Description, "IEQ" is an abbreviation for Islet Equivalents, which is an international unit representing the volume of pancreatic islets with 1 IEQ being defined as a pancreatic islet with a diameter of 150 μm assuming a spherical islet shape

According to the criteria for fresh islet transplantation of the Japan Pancreatic and Islet Transplantation Study Group (Islet Transplantation Implementation Manual), "an islet volume of at least 5,000 IEQ/kg (patient body weight)" is a condition for transplantation of fresh islets, and this standard is also referenced here. The transplantation device can be set appropriately to a number of islets calculated to produce the desired therapeutic effect, and the device can also be set appropriately according to the patient's body weight, degree of symptoms and the like.

The volume of insulin-secreting cells can also be set appropriately according to the pancreatic islets.

The method for manufacturing the transplantation device is explained in more detail.

In the method for manufacturing the transplantation device, the "Step (a): an optional step in which a pancreas is extracted from a living body, and pancreatic islets are isolated" means that the Step (a) may be selected as desired. The "living body" is a human or non-human mammal for example, and a pig is an example of a non-human mammal. When the Step (a) is performed to isolate pig islets for example, a sterile viable pancreas can be obtained from an adult pig under sterile conditions, and pancreatic islet cells can be isolated by procedures well known in the field, or by the methods described in Shimoda et al., Cell Transplantation, Vol. 21, pp. 501-508, 2012, or by standard Ricordi techniques established in the Edmonton Protocol or the like. Islets from other non-human mammals and human islets can also be isolated in the same way as pig islets. The isolated islets may then be used as is or cultured and then used. The islets may be cultured for example according to the methods of Noguchi et al (Transplantation Proceedings, 42, 2084-2086 (2010)) by culturing for 1 day at 37°C in a moist atmosphere of 5% $CO_2$/95% air in medium (Connaught Medical Research Laboratory (CMRL)-based Miami-defined media #1 (MM1; Mediatech-Cellgro, Herndon, VA)-supplemented with 0.5% human serum albumin).

Next in the "Step (b): a step in which cells or tissue selected from the group consisting of insulin-secreting cells, pancreatic islets, pancreatic islet cells obtained by culture, and pancreatic islet cells obtained by differentiation from stem cells are mixed with a solution of an alginic acid derivative that can be hydrogelled by chemical crosslinking", the alginic acid derivative that can be hydrogelled by chemical crosslinking may be represented for example by formula (I) and formula (II) above. In Step (b), for example an 0.1 to 5 wt% aqueous solution or physiological saline solution of the alginic acid derivative is prepared, and the necessary amount of cells or tissue selected from the group consisting of insulin-secreting cells, pancreatic islets, pancreatic islet cells obtained by culture, and pancreatic islet cells obtained by differentiation from stem cells (for example, the pancreatic islets obtained in Step (a), insulin-secreting cells isolated from these islets, or pancreatic islet cells obtained by culturing islet cells isolated from these islets) is suspended appropriately in this solution.

The "solution of an alginic acid derivative that can be hydrogelled by chemical crosslinking" here may be for example two kinds of solutions, a solution of the alginic acid derivative represented by formula (HA-I) above and a solution of the alginic acid derivative represented by formula (HA-II) above. Alternatively, the "solution of an alginic acid derivative that is hydrogelled by chemical crosslinking" is two solutions, a solution of the alginic acid derivative represented by formula (HB-I) above and a solution of the alginic acid derivative represented by formula (HB-II) above. In this case, these two solutions and the solutions of these mixed with cells or tissues are prepared separately rather than being mixed in the step (b). At this stage, the cells or tissue may be mixed with only one of the two solutions or may be mixed with both.

Next, in the "Step (c): a step of bringing the alginic acid derivative solution obtained in step (b) into contact with a solution containing a divalent metal ion to prepare a gel with a thickness of 0.1 to 5 mm (100 to 5000 μm) or a thickness of 100 μm or more and less than 500 μm", the alginic acid derivative solution prepared in Step (b) containing the suspended cells or tissue (such as pancreatic islets) is gelled. At this stage, the solution of the alginic acid derivative represented by formula (HA-I) or formula (HB-I) and the solution of the alginic acid derivative represented by formula (HA-II) or formula (HB-II) may be mixed in appropriate amounts according to the introduction rates of the chemical crosslinking groups in each. A solution containing a divalent metal ion can then be brought into contact with this mixed solution to simultaneously promote both ion crosslinking and chemical crosslinking and prepare a gel. More specifically, the gel may be prepared as described below in Example 5 under [Manufacturing flat alginic acid gel] <Common preparation

methods>.

Next, the "Step (d): an optional step of encapsulating the gel obtained in step (c) in a semipermeable membrane" means that the Step (d) may be selected as desired. When the Step (d) is performed, the gel obtained in Step (c) is encapsulated in a semipermeable membrane by methods known in the field or analogous methods. For example, the gel is inserted in the semipermeable membrane (such as a semipermeable membrane tube that has been sealed at one end), and the other end is sealed to encapsulate the gel.

Alternatively, in the "Step (c): a step of encapsulating the alginic acid derivative solution obtained in step (b) in a semipermeable membrane", the alginic acid derivative solution prepared in Step (b) containing the suspended cells or tissue (such as pancreatic islets) is encapsulated in the semipermeable membrane by methods known in the field or analogous methods. In this case, the solution of the alginic acid derivative of formula (HA-I) or formula (HB-I) and the solution of the alginic acid derivative of formula (HA-II) or formula (HB-II) may be mixed in appropriate amounts according to the introduction rates of the chemical crosslinking groups in each. The resulting mixed solution containing the suspended cells or tissue (such as pancreatic islets) is then inserted into the semipermeable membrane (such as a semipermeable membrane tube that has been sealed at one end), and the other end is sealed to encapsulate the solution.

Next, in the "Step (d): a step of bringing the semipermeable membrane obtained in step (c) into contact with a solution containing a divalent metal ion to thereby gel the alginic acid solution inside the semipermeable membrane", the semipermeable membrane containing the alginic acid solution obtained in step (c) is brought into contact with a solution containing a divalent metal ion to thereby gel the alginic acid solution inside the semipermeable membrane.

The device obtained from Step (d) may also be washed with a solvent such as physiological saline. It may also be cultured for a predetermined period of time in medium.

In the "Step (e): a step of making the alginic acid derivative solution obtained in Step (b) into a gel with an arbitrary shape" or the " Step (e): a step of putting the alginic acid derivative solution obtained in Step (b) into any container or onto any surface", the alginic acid derivative solution obtained in Step (b) with cells or tissue (such as pancreatic islets) suspended therein is made into a specific shape using any container, surface or the like according to known methods in the field or analogous methods, and gelled by promoting chemical crosslinking. At this time, appropriate amounts of the solution of the alginic acid derivative of formula (HA-I) or (HB-I) and the solution of the alginic acid derivative of formula (HA-II) or (HB-II) may be mixed in advance according to the introduction rates of the chemical crosslinking groups in each.

The any container is a container capable of holding the solution in a specific shape, and may be a semipermeable membrane, beaker or petri dish for example, or preferably a semipermeable membrane.

The any surface is a surface on which the solution can be laid in a specific shape, and may be a semipermeable membrane surface, plastic surface or glass surface, or preferably a semipermeable membrane surface.

In the " Step (f): a step of gelling the alginic acid derivative solution in the container or on the surface obtained in Step (e)" or the "(Step f): a step of gelling the alginic acid derivative solution in the semipermeable membrane obtained in Step (c) to thereby prepare a gel with a thickness of 0.1 to 5 mm (100 to 5,000 $\mu$m)", gelling is accomplished by chemically crosslinking a solution held in any container, surface or semipermeable membrane. At this time, a gel may be prepared by simultaneously performing chemical crosslinking and ion crosslinking through contact with a solution containing a divalent metal ion, or a gel may be prepared by chemical crosslinking without contacting a divalent metal ion or a solution thereof.

The solution containing the "divalent metal ion" used in the transplantation device may be a solution containing a calcium ion, barium ion, strontium ion or the like. Preferably it is a solution containing a calcium ion or barium ion, and more preferably a solution containing a calcium ion.

The solution containing the divalent metal ion may be obtained by dissolving a salt of a divalent metal ion in a solvent for example. Examples of salts of divalent metal ions include calcium chloride, barium chloride, strontium chloride and the like. The solvent may be water, physiological saline, or HEPES buffer solution, for example.

In certain embodiments, the solution containing the divalent metal ion is a solution containing a calcium ion and is preferably an aqueous solution containing calcium chloride.

It is desirable to appropriately adjust the amount of the solution containing the divalent metal ion according to the amount and molecular weight of the alginic acid derivative and the like.

When the transplantation device has a semipermeable membrane, the hydrogel in the device may be encapsulated by first encapsulating a solution of the alginic acid derivative in the semipermeable membrane, and then bringing it into contact with the divalent metal ion, or else the hydrogel may be gelled first and then encapsulated in the semipermeable membrane.

"Contact" here may mean immersing the semipermeable membrane containing the encapsulated alginic acid derivative solution in a divalent metal ion solution or applying the divalent metal ion solution to the semipermeable membrane containing the encapsulated alginic acid derivative solution or the like.

References to the hydrogel used in the transplantation device indicate a polymer having a three-dimensional mesh structure that is insoluble in water, and a swelled body of this polymer due to water. This hydrogel may simply be

called a gel here.

The molecular weights of the molecules that can pass through the mesh base structure of this gel can be varied with a great degree of freedom by changing the concentration of the polymer used in preparing the hydrogel. That is, it is thought that the mesh size of the gel mesh structure is smaller when the polymer concentration is high, and larger when the polymer concentration is low. If the mesh size of the mesh structure is too large, antibodies and the like will invade the mesh structure. In this case, rejection reactions to the insulin-secreting cells or pancreatic islets in the gel are more likely. Rejection reactions inhibit production of necessary substances such as insulin.

Common examples of hydrogel materials include polymers such as collagen, hyaluronan, gelatin, fibronectin, elastin, tenascin, laminin, vitronectin, polypeptides, heparan sulfate, chondroitin, chondroitin sulfate, keratan, keratan sulfate, dermatan sulfate, carrageenan, heparin, chitin, chitosan, alginic acid salts, alginic acid derivatives, agarose, agar, cellulose, methyl cellulose, carboxymethyl cellulose, glycogen and derivatives of these, as well as fibrin, fibrinogen, thrombin, polyglutamic acid, polylactic acid, polyglycolic acid, lactic acid-glycolic acid copolymer, vinyl alcohol polymers, gellan gum, xanthan gum, galactomannan, guar gum, locust bean gum, tara gum and the like.

An alginic acid derivative is preferred here as the hydrogel material from the standpoint of biocompatibility and long-term survival and functional maintenance of the pancreatic islets.

Each embodiment of the alginic acid derivative is explained in detail below.

1. Alginic acid

In the present Description, references to alginic acid pertain to at least one kind of alginic acid (also called "alginate") selected from the group consisting of alginic acid, the alginic acid esters and the salts of these (such as sodium alginate). The alginic acid used may be either naturally derived or synthetic, but a naturally derived alginic acid is preferred. A preferred alginic acid is a bioabsorbable polysaccharide that is extracted from natural brown algae such as Lessonia, Macrocystis, Laminaria, Ascophyllum, Durvillea, Ecklonia cava, Eisenia bicyclis and Saccharina japonica, and is a polymer obtained by linear polymerization of two kinds of uronic acid, D-mannuronic acid (M) and L-guluronic acid (G). More specifically, this is a block copolymer comprising a homopolymer fraction of D-mannuronic acid (MM fraction), a homopolymer fraction of L-guluronic acid (GG fraction), and a fraction of randomly arranged D-mannuronic acid and L-guluronic acid (M/G fraction) in arbitrary combination.

In this Description, alginic acid is sometimes expressed as (ALG)-COOH, where (ALG) is alginic acid and -COOH is any one carboxyl group of alginic acid.

In certain embodiments, the alginic acid is sodium alginate. Commercial sodium alginate may be used as the sodium alginate. In the examples described below, the sodium alginates A-1, A-2, A-3, B-1, B-2 and B-3 described in the tables below (sold by Mochida Pharmaceutical Co., Ltd.) are used as the sodium alginate. The following table shows the viscosity (1 w/w% aqueous solution), weight-average molecular weight and M/G ratio of each sodium alginate.

[Table 9]

| Sodium alginate | 1 w/w% viscosity (mPa s) | Weight-average molecular weight | | M/G ratio |
|---|---|---|---|---|
| | | GPC | GPC-MALS | |
| A-1 | 10 to 40 | 300,000 to 700,000 | 60,000 to 130,000 | 0.5 to 1.8 |
| A-2 | 50 to 150 | 700,000 to 1,400,000 | 130,000 to 200,000 | |
| A-3 | 300 to 600 | 1,400,000 to 2,000,000 | 200,000 to 400,000 | |
| B-1 | 10 to 40 | 150,000 to 800,000 | 60,000 to 130,000 | 0.1 to 0.5 |
| B-2 | 70 to 150 | 800,000 to 1,500,000 | 130,000 to 200,000 | |
| B-3 | 400 to 600 | 1,500,000 to 2,500,000 | 200,000 to 350,000 | |

The respective physical property values of the sodium alginates A-1, A-2, A-3, B-1, B-2 and B-3 were measured by the methods described below. The measurement methods are not limited to these, and the physical property values may differ from those given above depending on the measurement method.

[Measuring viscosity of sodium alginate]

This was measured by the rotational viscometer method (using a cone plate rotational viscometer) according to the viscosity measurement methods of the Japanese Pharmacopoeia (16th Edition). The specific measurement conditions are as follows. The sample solution was prepared using MilliQ water. A cone plate rotational viscometer

(RheoStress RS600 rheometer (Thermo Haake GmbH), sensor: 35/1) was used as the measurement equipment. The rotation was set at 1 rpm when measuring a 1 w/w% sodium alginate solution. For the read time, the solution was measured for 2 minutes and the average value from 1 minute to 2 minutes after starting was used. The average of three measured values was used as the measurement value. The measurement temperature was 20°C.

[Measuring weight-average molecular weight of sodium alginate]

This was measured by two measurement methods, (1) gel permeation chromatography (GPC) and (2) GPC-MALS. The measurement conditions are as follows.

[Pre-treatment methods]

An eluent was added to dissolve the sample, which was then filtered through an 0.45-micron membrane filter to obtain a measurement solution.

(1) Gel permeation chromatography (GPC) measurement

[Measurement conditions (relative molecular weight distribution measurement)]

Columns: TSKgel GMPW-XI, × 2 + G2500PW-XI, (7.8 mm I.D. × 300 mm × 3)
Eluent: 200 mM sodium nitrate aqueous solution
Flow rate: 1.0 ml/min
Concentration: 0.05%
Detector: RI detector
Column temperature: 40°C
Injection volume: 200 μl
Molecular weight standards: Standard pullulan, glucose

(2) GPC-MALS measurement

[Refractive index increment (dn/dc) measurement (measurement conditions)]

Differential refractometer: Optilab T-rEX
Measurement wavelength: 658 nm
Measurement temperature: 40°C
Solvent: 200 mM sodium nitrate aqueous solution
Sample concentration: 0.5 to 2.5 mg/ml (5 concentrations)

[Measurement conditions (absolute molecular weight distribution measurement)]

Columns: TSKgel GMPW-XI, × 2 + G2500PW-XI, (7.8 mm I.D. × 300 mm × 3)
Eluent: 200 mM sodium nitrate aqueous solution
Flow rate: 1.0 ml/min
Concentration: 0.05%
Detectors: RI detector, light scattering detector (MALS)
Column temperature: 40°C
Injection volume: 200 μl

In this Description, the molecular weights of alginic acid, alginic acid derivatives, crosslinked alginates and crosslinked alginic acids may be given in units of Da (Daltons).
The constituent ratio of D-mannuronic acid and L-guluronic acid (M/G ratio) in an alginate differs principally according to the type of seaweed or other organism from which it is derived and may also be affected by the organism's

habitat and season, with a wide range from high-G alginate (M/G ratio about 0.2) to high-M alginate (M/G ratio about 5). The gelling ability of the alginate and the properties of the resulting gel are affected by the M/G ratio, and in general, the gel strength is known to be greater the higher the proportion of G. The M/G ratio also affects the hardness, fragility, water absorption, flexibility and the like of the gel. The M/G ratio of the alginic acid and/or salt thereof used here is normally from 0.2 to 4.0, or preferably from 0.4 to 3.0, or still more preferably from 0.5 to 3.0.

When numerical ranges are indicated with "from" and "to" this Description, the numbers after "from" and "to" are the minimum and maximum values of the range, respectively.

The "alginic acid ester" and "alginic acid salt" used in this Description are not particularly limited, but because these will be reacted with a crosslinking agent, they must have no functional groups that would impede the crosslinking reaction. Desirable examples of alginic acid esters include propylene glycol alginate and the like.

In this Description, examples of alginic acid salts include monovalent salts of alginic acid and divalent salts of alginic acid. Preferred examples of monovalent alginic acid salts include sodium alginate, potassium alginate and ammonium alginate, of which sodium alginate and potassium alginate are more preferred, and sodium alginate is particularly preferred. Preferred examples of divalent alginic acid salts include calcium alginate, magnesium alginate, barium alginate and strontium alginate.

Alginic acid is a high molecular weight polysaccharide, and its molecular weight is difficult to determine accurately, but generally the weight-average molecular weight is in the range of 1,000 to 10,000,000, or preferably 10,000 to 8,000,000, or more preferably 20,000 to 3,000,000. It is known that in molecular weight measurement of naturally derived high molecular weight substances, values may differ depending on the measurement method.

For example, the weight-average molecular weight as measured by gel permeation chromatography (GPC) or gel filtration chromatography (which together are sometimes called size exclusion chromatography) is preferably at least 100,000, or more preferably at least 500,000, and is preferably not more than 5,000,000, or more preferably not more than 3,000,000. The preferred range is from 100,000 to 5,000,000, or more preferably from 150,000 to 3,000,000.

The absolute weight-average molecular weight can also be measured by the GPC-MALS method. The weight-average molecular weight (absolute molecular weight) as measured by GPC-MALS is preferably at least 10,000, or more preferably at least 50,000, or still more preferably at least 60,000, and is preferably not more than 1,000,000, or more preferably not more than 800,000, or still more preferably not more than 700,000, or particularly preferably not more than 500,000. The preferred range is from 10,000 to 1,000,000, or more preferably from 50,000 to 800,000, or still more preferably from 60,000 to 700,000, or particularly preferably from 60,000 to 500,000.

When the molecular weight of a high molecular weight polysaccharide is calculated by such methods, a measurement error of 10% to 20% is normal. Thus, a value of 400,000 may vary in the range of 320,000 to 480,000, a value of 500,000 may vary in the range of 400,000 to 600,000, and a value of 1,000,000 may vary in the range of 800,000 to 1,200,000 for example.

The molecular weight of an alginate can be measured by ordinary methods.

Typical conditions for molecular weight measurement using gel filtration chromatography are described in the examples of this Description below. For example, a Superose 6 Increase 10/300 GL column (GE Healthcare Science) may be used as the column, a 10 mmol/L phosphate buffer containing 0.15 mol/L NaCl (pH 7.4) may be used as the development solvent, and blue dextran, thyroglobulin, ferritin, aldolase, conalbumin, ovalbumin, ribonuclease A and aprotinin may be used as molecular weight standards.

The viscosity of the alginic acid used in this Description is not particularly limited, but when measured in a 1 w/w% aqueous alginate solution, the viscosity is preferably from 10 mPa·s to 1,000 mPa·s, or more preferably from 50 mPa·s to 800 mPa·s.

The viscosity of an aqueous solution of an alginic acid can be measured by ordinary methods. For example, it can be measured by rotational viscometry using a coaxial double cylindrical rotational viscometer, single cylindrical rotary viscometer (Brookfield viscometer), conical plate rotational viscometer (cone plate viscometer) or the like. Preferably it is measured according to the viscosity measurement methods of the Japanese Pharmacopoeia (16th Edition). More preferably, a cone plate viscometer is used.

When first extracted from brown algae, alginates have a high molecular weight and a high viscosity, but the molecular weight and viscosity are reduced by the processes of heat drying, purification and the like. Alginic acids with different molecular weights can be manufactured by methods such as controlling the temperature and other conditions during the manufacturing process, selecting the brown algae used as raw materials, and fractioning the molecular weights in the manufacturing process. An alginate having the desired molecular weight can also be obtained by mixing alginates from different lots having different molecular weights or viscosities.

In some embodiments of the alginic acid used in this Description, the alginic acid has not been subjected to low endotoxin treatment, while in other embodiments the alginic acid has been subjected to low endotoxin treatment. "Low endotoxin" means that the level of endotoxins is so low that there is substantially no risk of causing inflammation or fever. A low endotoxin treated alginate is more preferred.

Low endotoxin treatment may be performed by known methods or analogous methods. For example, it can be

performed by the methods of Kan et al for purifying sodium hyaluronate (see for example Japanese Patent Application Publication No. H09-324001, etc.), the methods of Yoshida et al for purifying beta 1,3-glucan (see for example Japanese Patent Application Publication No. H08-269102, etc.), the methods of William et al for purifying biopolymer salts such as alginates and gellan gum (see for example Japanese Translation of PCT Application No. 2002-530440, etc.), the methods of James et al for purifying polysaccharides (see for example WO 93/13136, etc.), the methods of Lewis et al (see for example U.S. Patent No. 5589591B, etc.), or the methods of Herman Frank for purifying alginates (see for example Appl. Microbiol. Biotechnol. (1994) 40:638-643, etc.) or the like or analogous methods. Low endotoxin treatment is not limited to these methods, and may also be performed by known methods such as washing, filtration with a filter (endotoxin removal filter, charged filter or the like), ultrafiltration, column purification (using an endotoxin adsorption affinity column, gel filtration column, ion-exchange resin column or the like), adsorption to a hydrophobic substance, resin, activated carbon or the like, organic solvent treatment (organic solvent extraction, deposition/sedimentation by addition of an organic solvent or the like), surfactant treatment (see for example Japanese Patent Application Publication No. 2005-036036) or the like, or by a suitable combination of these methods. Known methods such as centrifugation may also be combined with the steps of such treatment. The treatment is preferably selected appropriately according to the type of alginic acid.

The endotoxin level may be confirmed by known methods, such as limulus reagent (LAL) testing or a method using an Endospecy™ ES-24S set (Seikagaku Corp.).

There are no particular limitations on the endotoxin treatment method used, but the resulting endotoxin content of the treated alginate is preferably not more than 500 endotoxin units (EU)/g, or more preferably not more than 100 EU/g, or still more preferably not more than 50 EU/g, or particularly preferably not more than 30 EU/g when measured with a limulus reagent (LAL). Low endotoxin treated sodium alginate is available as a commercial product such as Sea Matrix™ (Mochida Pharmaceutical Co., Ltd.) or Pronova™ UP LVG (FMC BioPolymer).

2. Alginic acid derivatives

Novel alginic acid derivatives are provided in this Description. An alginic acid derivative in this Description has a reactive group or a reactive group complementary to that reactive group in a Huisgen reaction introduced at any one or more carboxyl groups of alginic acid via an amide bond and a divalent linker.

More specifically, these are an alginic acid derivative represented by formula (HA-I) or formula (HB-I) below [in which (ALG), $-L^1-$ and Akn are defined as in the first embodiment above]:

[C41]

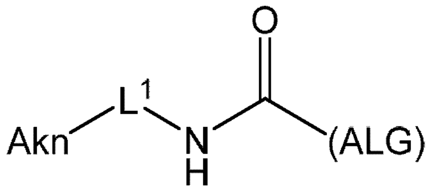

(I)

and an alginic acid derivative represented by formula (HA-II) or formula (HB-II) below [in which (ALG) and $-L^2-$ are defined as in the fourth embodiment above]:

[C42]

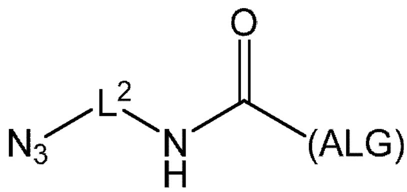

(II)

Any linear group may be used as the divalent linker ($-L^1-$ or $-L^2-$) as long as it does not impede the reaction between the reactive group and the reactive group complementary to that reactive group. Specifically, this may be a linear alkylene group ($-(CH_2)_n-$, n = 1 to 30) (in which $-CH_2-$ may be substituted with one or more (such as 1 to 10, or 1 to 5) groups such as -C(=O)-, -CONH-, -O-, -NH-, -S-, a benzene ring or a heterocyclic ring (a 5- to 6-membered aromatic

or non-aromatic heterocyclic ring such as a pyridine ring, piperidine ring or piperazine ring), and a hydrogen atom of the $-CH_2-$ may also be substituted with one or more (such as 1 to 10, or 1 to 5) groups selected from the oxo (=O), hydroxyl (-OH) and $C_{1-6}$ alkyl groups (such as methyl, ethyl, n-propyl and iso-propyl groups) and halogen atoms (such as a fluorine, chlorine, bromine and iodine atoms)).

The alginic acid derivatives represented by formula (HA-I) or formula (HB-I) and formula (HA-II) or formula (HB-II), which are novel alginic acid derivatives in the present Description, can be manufactured by the methods of the following formulae.

[C43]

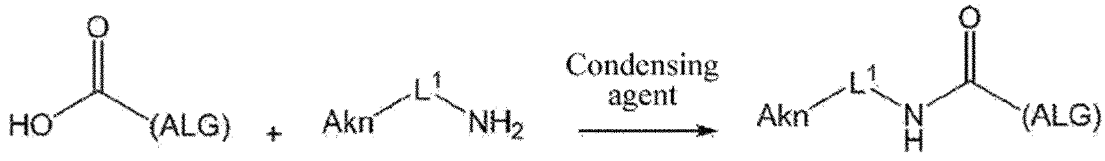


Alginic acid (AM-1) (I)

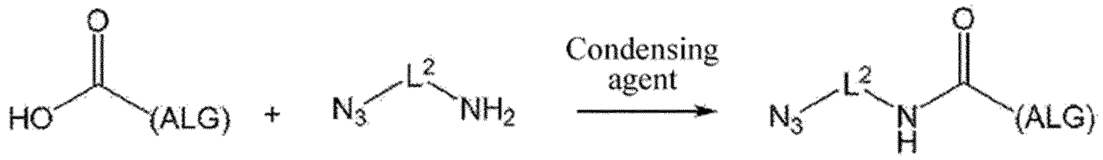


Alginic acid (AM-2) (II)

The weight-average molecular weight of an alginic acid derivative represented by formula (HA-I), formula (HA-II), formula (HB- I) or formula (HB- II) in this Description is from 100,000 Da to 3,000,000 Da, or preferably from 300,000 Da to 2,500,000 Da, or still more preferably from 500,000 Da to 2,000,000 Da. The molecular weights of both alginic acid derivatives can be determined by the methods described below.

In this Description, the Akn-$L^1$-NH- group of formula (HA-I) or formula (HB- I) need not be bound to all of the carboxyl groups of the constituent units of alginic acid, and the $N_3$-$L^2$-NH- group of formula (HA-II) or formula (HB-II) need not be bound to all of the carboxyl groups of the constituent units of alginic acid.

In this Description, when the Akn-$L^1$-NH- group of formula (HA-I) or formula (HB-I) is called a reactive group, the $N_3$-$L^2$-NH- group of formula (HA-II) or formula (HB-II) is called a complementary reactive group. Conversely, when the $N_3$-$L^2$-NH- group of formula (HA-II) or formula (HB-II) is called a reactive group, the Akn-$L^1$-NH- group of formula (HA-I) or formula (HB-I) is called a complementary reactive group.

In this Description, the introduction rate of the reactive group or complementary reactive group is 0.1% to 30% or 1% to 30%, or preferably 2% to 20%, or more preferably 3% to 10% of each.

The introduction rate of the reactive group or complementary reactive group is a value representing the number of uronic acid monosaccharide units having introduced reactive groups or complementary reactive groups as a percentage of the uronic acid monosaccharide units that are repeating units of the alginate. Unless otherwise specified, the % value used as the introduction rate of the reactive group or complementary reactive group in the alginic acid derivative (formula (HA-I), formula (HA-II), formula (HB-I) or formula (HB-II)) in this Description is a mol% value. The introduction rate of the reactive group or complementary reactive group can be determined by the methods described in the examples below.

In this Description, the cyclic alkyne group (Akn) in formula (HA-I) or formula (HB-I) and the azide group in formula (HA-II) or formula (HB-II) form a triazole ring by a Huisgen reaction, thereby forming a crosslink.

3. Huisgen reaction

A Huisgen reaction (1,3-dipolar cycloaddition reaction) is a condensation reaction between compounds having a terminal azide group and a terminal alkyne group as shown in the formula below. The reaction efficiently yields a disubstituted 1,2,3-triazole ring, and has the feature of producing no excess by-products. Although it is believed that 1,4- or 1,5-disubstituted triazole rings may be produced in the reaction, a copper catalyst may be used to regioselectively obtain a triazole ring.

[C44]

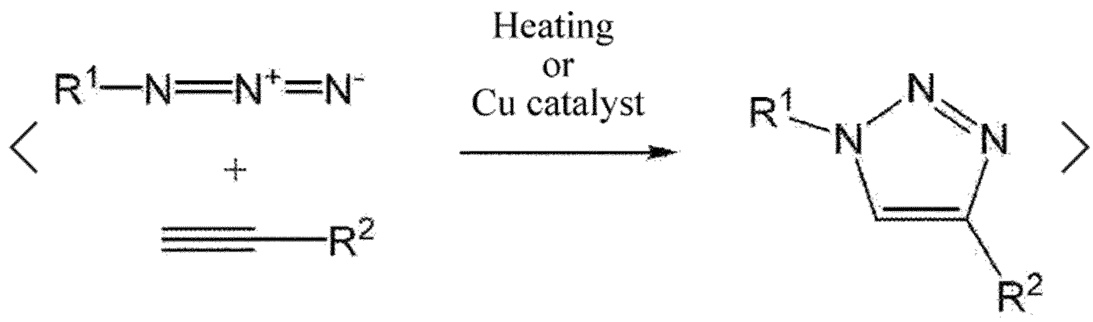


Wittig and Krebs have also reported on a Huisgen reaction that does not use a copper catalyst. That is, in this reaction ($R^3$ = phenyl in the formula below) a cycloadduct is obtained by simply mixing cyclooctyne and phenyl azide. Because the triple bond of cyclooctyne is greatly distorted in this reaction, elimination of the distortion caused by the reaction with the phenyl azide acts as a driving force, and the reaction progresses spontaneously without the need for a catalyst.

[C45]

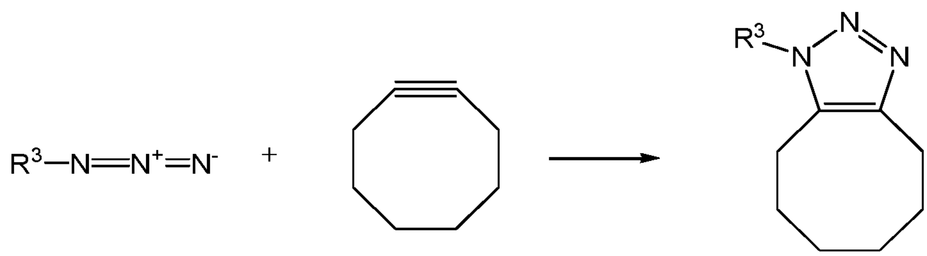

Thus, the Huisgen reaction may use an azide compound having a substituted primary azide, secondary azide, tertiary azide, aromatic azide or the like together with a compound having a terminal or cyclic alkyne group, which is a reactive group complementary to the azide group. Moreover, because it is mainly only the azide and alkyne groups that react in the Huisgen reaction, various functional groups (such as ester, carboxyl, alkenyl, hydroxyl and amino groups and the like) may also be substituted in the reaction substrate.

In certain embodiments, the cyclic alkyne group (cyclooctyl group) described in the first embodiment for example is used as the alkyne group in the Huisgen reaction so that crosslinking by 1,2,3-triazole rings is formed easily, efficiently and in a short amount of time between alginic acid molecules without producing undesirable by-products and without using a copper catalyst so as to avoid cytotoxicity from the copper catalyst.

In a preferred embodiment of the method of crosslinking the alginic acid derivatives, almost no undesirable by-products are formed by the reaction (Huisgen reaction). In this case, various bioactive molecules can be incorporated when alginic acid is used to prepare novel forms of biocompatible materials or to form alginic acid hydrogels, and cellular materials can also be incorporated into alginic acid hydrogels for use in reconstructive surgery or gene therapy.

4. Crosslinked alginic acid

Crosslinked alginic acids include (i) those crosslinked via divalent metal ion bonds, (ii) those crosslinked via chemical bonds, and (iii) those crosslinked via both divalent metal ion bonds and chemical bonds. All of these crosslinked alginic acids have the property of forming gels, semi-solids and in some cases sponge-like forms.

When a crosslinked alginic acid is crosslinked via divalent metal ion bonds, the reaction progresses ultra-rapidly and is reversible, while when a crosslinked alginic acid is crosslinked via chemical bonds, the reaction progresses slowly under relatively mild conditions, and is irreversible. The physical properties of a crosslinked alginic acid can be adjusted for example by such methods as changing the concentration of the aqueous solution (such as a calcium carbonate aqueous solution) containing the divalent metal ion or changing the introduction rate of the reactive group introduced into the alginic acid or the like.

A variety of alginic acid structures can be prepared using the above crosslinking reactions. For example, a specific structure can be prepared instantaneously from an alginic acid solution by an ion crosslinking reaction, and a crosslinking reaction via chemical bonds can then be used to structurally reinforce this structure (to give it long-term stability for example). Alternatively, for example in a crosslinked alginic acid structure that has been crosslinked via both divalent metal ion bonds and chemical bonds, the divalent metal ions incorporated by ion crosslinking can be reversibly released to create a structure having only crosslinking via chemical bonds.

A crosslinked alginic acid structure using an alginic acid derivative of a preferred embodiment is stable because it contains crosslinking by chemical bonds, and is also advantageous because it can maintain its shape long-term in comparison with a crosslinked alginic acid structure having only ion crosslinking using sodium alginate.

A crosslinked alginic acid of a certain embodiment can be obtained by mixing the alginic acid derivatives of formula (HA-I) above and formula (HA-II) above and performing a Huisgen reaction. A crosslinked alginic acid of a certain embodiment can be obtained by mixing the alginic acid derivatives of formula (HB-I) above and formula (HB-II) above and performing a Huisgen reaction.

The crosslinked alginic acid of a certain embodiment forms a three-dimensional mesh structure via chemical crosslinking (crosslinking by triazole rings formed from alkyne and azide groups). Preferred alginic acid derivatives are those having improved stability of the crosslinked alginic acid after crosslinking.

The crosslinked alginic acid of certain embodiments is a crosslinked alginic acid in which any carboxyl group of a first alginic acid and any carboxyl group of a second alginic acid are amide bonded via the following formula (HA-III-L) or formula (HB-III-L):

[C46]

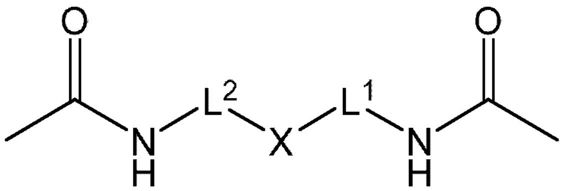

(III-L)

[in formula (HA-III-L) or formula (HB-III-L), the -CONH- and -NHCO- at either end represent amide bonds via any carboxyl group of alginic acid; and $-L^1$, $-L^2$- and X are defined as in the seventh embodiment above].

In certain embodiments, the mixing ratio of the alginic acid derivative of formula (HA-I) or formula (HB-I) and the alginic acid derivative of formula (HA-II) or formula (HB-II) (weight ratio of formula (HA-I) or formula (HB-I) derivative:formula (HA-II) or formula (HB-II) derivative) is 1:1 to 1.5:1 for example, or preferably 1.2:1 to 1.5:1, or 1:1 to 1.2:1, or more preferably 1:1 when preparing the crosslinked alginic acid.

In certain embodiments, the mixing ratio of the alginic acid derivative of formula (HA-II) or formula (HB-II) and the alginic acid derivative of formula (HA-I) or formula (HB-I) (weight ratio of formula (HA-II) or formula (HB-II) derivative:formula (HA-I) or formula (HB-I) derivative) is 1:1 to 4.0:1 for example, or preferably 1.5:1 to 4.0:1, or 1.2:1 to 1.5:1, or 1:1 to 1.2:1, or more preferably 1:1 when preparing the crosslinked alginic acid.

In certain embodiments, the mixing ratio of the alginic acid derivative of formula (HA-I) or formula (HB-I) and the alginic acid derivative of formula (HA-II) or formula (HB-II) when preparing the crosslinked alginic acid is more preferably such that the ratio of the introduction rates (mol%) of the reactive groups of the alginic acid derivative of formula (HA-I) or formula (HB-I) and the alginic acid derivative of formula (HA-II) or formula (HB-II) is 1:1 to 1.5:1 for example, or preferably 1.2:1 to 1.5:1, or 1:1 to 1.2:1, or more preferably 1:1.

In certain embodiments, the mixing ratio of the alginic acid derivative of formula (HA-II) or formula (HB-II) and the alginic acid derivative of formula (HA-I) or formula (HB-I)when preparing the crosslinked alginic acid is more preferably such that the ratio of the introduction rates (mol%) of the reactive groups of the alginic acid derivative of formula (HA-II) or formula (HB-II) and the alginic acid derivative of formula (HA-I) or formula (HB-I) is 1:1 to 4.0:1 for example, or preferably 1.5:1 to 4.0:1, or 1.2:1 to 1.5:1, or 1:1 to 1.2:1, or more preferably 1:1.

In the mixing ratios above, the alginic acid derivative of formula (HA-II) or formula (HB-II) may be substituted for the alginic acid derivative of formula (HA-I) or formula (HB-I), and the alginic acid derivative of formula (HA-I) or formula (HB-I) may be substituted for the alginic acid derivative of formula (HA-II) or formula (HB-II).

In the crosslinked alginic acid, it is not necessary that all of the carboxyl groups of the constituent units of the alginic acid have the crosslink of formula (HA-III-L) or formula (HB-III-L) above. The introduction rate of the crosslink represented by formula (HA-III-L) or formula (HB-III-L) above in the crosslinked alginic acid (also called the crosslinking rate) is in the range of 0.1% to 80%, or 0.3% to 60%, or 0.5% to 30%, or 1.0% to 10% for example.

The concentration of the alginic acid derivative of formula (HA-I), formula (HA-II), formula (HB-I) or formula (HB II) in the Huisgen reaction for obtaining the crosslinked alginic acid is normally in the range of 1 to 500 mg/ml, or preferably 5 to 100 mg/ml.

The reaction temperature in the Huisgen reaction is normally an external temperature in the range of 4°C to 60°C, or preferably an external temperature in the range of 15°C to 40°C.

The stirring time for forming the crosslinked alginic acid (hydrogel) is a few seconds to 24 hours, or a few seconds to 12 hours, or a few seconds to 30 minutes, or a few seconds to 10 minutes for example.

The reaction solvent or reaction solution used in the Huisgen reaction is not particularly limited, and examples include tap water, pure water (such as distilled water, deionized water, RO water, RO-EDI water or the like), ultrapure water, cell culture medium, phosphate-buffered saline (PBS), saline, and HEPES buffer solution, of which ultrapure water is preferred.

The crosslinked alginic acid of certain embodiments is a crosslinked alginic acid comprising as crosslinking both chemical crosslinking by triazole rings formed by a Huisgen reaction and ion crosslinking partially formed by calcium ions.

5. Crosslinked alginic acid structure

The crosslinked alginic acid structure can be obtained by a method that includes subjecting the above alginic acid derivatives to a crosslinking reaction. It can be prepared by the following methods for example, but the methods are not limited to these.

[Mixing method]

A mixed solution of alginic acid derivatives obtained by mixing the alginic acid derivative of formula (HA-I) or formula (HB-I) with the alginic acid derivative of formula (HA-II) or formula (HB- II) is dripped into a solution containing a divalent metal ion to obtain a crosslinked alginic acid structure, which is a specific structure formed by chemical crosslinking (crosslinking by triazole rings formed from alkyne groups and azide groups in a Huisgen reaction) and ion crosslinking (crosslinking partially formed by divalent metal ions).

[Coating method]

A solution comprising the alginic acid derivative of formula (HA-I) ) or formula (HB-I) is dripped or the like into a solution containing a divalent metal ion to obtain a specific partially crosslinked structure. The resulting gel or other structure for example can then be added to a solution comprising the alginic acid structure of formula (HA-II) or formula (HB-II) above to perform a further crosslinking reaction (Huisgen reaction) on the surface of the like of the previous structure and thereby obtain a crosslinked alginic acid. This method can also be implemented with the alginic acid derivative of formula (HA-II) or formula (HB-II) substituted for the alginic acid derivative of formula (HA-I) or formula (HB-I) and with the alginic acid derivative of formula (HA-I) or formula (HB-I) substituted for the alginic acid derivative of formula (HA-II) or formula (HB-II).

The divalent metal ion used in this method is not particularly limited, but examples include calcium ions, magnesium ions, barium ions, strontium ions, zinc ions and the like, and a calcium ion is preferred.

The solution containing the calcium ion used in this method is not particularly limited but may be an aqueous solution such as a calcium chloride aqueous solution, calcium carbonate aqueous solution, calcium gluconate aqueous solution or the like for example, and a calcium chloride aqueous solution is preferred.

The calcium ion concentration of the calcium ion-containing solution used in this method is not particularly limited but may be from 1 mM to 1 M for example, or preferably from 5 mM to 500 mM, or more preferably from 10 mM to 300 mM.

The solvent or solution used in this method is not particularly limited, but examples include tap water, pure water (such as distilled water, deionized water, RO water or RO-EDI water), ultrapure water, cell culture medium, phosphate-buffered saline (PBS), physiological saline, and HEPES buffer solution, and ultrapure water is preferred.

Examples of specific crosslinked alginic acid structures include fibrous structures, fibers, beads, gels and nearly spherical gels. A preferred crosslinked alginic acid structure has improved stability. The crosslinked alginic acid structure may also have the ability to retain contents within the structure (content retention property).

The physical properties of the alginic acid gel can be adjusted by adjusting the physical property values such as hardness, elasticity, repulsive force, rupture force, stress at break and the like.

6. Biocompatibility of alginic acid derivative and crosslinked alginic acid structure

In this Description, the alginic acid derivative or crosslinked alginic acid structure has biocompatibility. In this Description, biocompatibility means the property of not causing reactions such as interactions between a biomaterial (in this case, an alginic acid derivative represented by formula (HA-I), formula (HA- II) or a crosslinked alginic acid structure manufactured using such alginic acid derivatives) and a living body, or local reactions in tissue adjacent to the biomaterial, or systemic reactions and the like.

In this Description, the biocompatibility of the alginic acid derivative or crosslinked alginic acid structure is confirmed in the examples relating to biocompatibility below.

7. Stability of crosslinked alginic acid structure

The stability of the crosslinked alginic acid structure can be confirmed for example by measuring gel stability, and its permeability can be confirmed by measuring gel permeability.

[Method for measuring gel stability]

Phosphate buffered saline (PBS) is added to a crosslinked alginic acid structure gel in a container, and the concentration (μg/ml) of alginic acid leaking into the PBS is measured. The measured alginic acid concentration is divided by the total alginic acid concentration obtained by decomposing the crosslinked alginic acid structure gel, and the resulting value expressed as a percentage is used as the gel collapse rate. Specifically, gel stability can be determined by the methods described in the examples below.

In this Description, the gel collapse rate of the crosslinked alginic acid structure is preferably from 0% to 90%, or more preferably from 0% to 70%, or still more preferably from 0% to 50%. The stability of the crosslinked alginic acid structure is greater the lower the concentration of the alginic acid leaking into an aqueous solution, or in other words the lower the gel collapse rate.

[Method for measuring gel permeation rate]

A crosslinked alginic acid structure gel containing fluorescein isothiocyanate-dextran is prepared, physiological saline is added to the gel in a container, and the concentration of dextran leaking into the physiological saline is measured. The measured dextran concentration is divided by the total dextran concentration obtained by decomposing the crosslinked alginic acid structure gel containing the fluorescein isothiocyanate-dextran, and the resulting value expressed as a percentage is used as the gel permeation rate. Specifically, the gel permeation rate can be determined by the methods described in the examples below.

The gel permeation rate of the crosslinked alginic acid 24 hours after addition of the physiological saline is preferably from 0% to 90%, or more preferably from 0% to 70%, or still more preferably from 0% to 50% when the gel contains dextran with a molecular weight of 2,000,000. When it contains dextran with a molecular weight of 150,000, assuming that the intended use of the crosslinked alginic acid structure gel is releasing and producing proteins and antibodies, the gel permeation rate is preferably from 1% to 100%, or more preferably from 10% to 100%, or still more preferably from 30% to 100%, while if the intended use is as an immune barrier, the gel permeation rate is preferably from 0% to 90%, or more preferably from 0% to 70%, or still more preferably from 0% to 50%.

The lower the permeation rate of the crosslinked alginic acid structure, the lower the permeability of the contents or non-gel substances, while the higher the permeation rate, the higher the permeability of the contents or non-gel substances.

The gel permeation rate can be adjusted by adjusting the molecular weight and concentration of the alginic acid used, the type and introduction rate of the crosslinking group introduced into the alginic acid, the type and concentration of the divalent metal ion used for gelling, or a combination of these.

[Method for preparing crosslinked alginic acid structure gel containing contents]

For example, a crosslinked alginic acid structure gel containing fluorescein isothiocyanate-dextran contents can be prepared by the following methods.

(1) A solution of the alginic acid derivative represented by formula (HA-I) or formula (HB-I) is mixed with a fluorescein isothiocyanate-dextran solution.

(2) The mixed solution obtained in (1) is mixed with a solution of the alginic acid derivative represented by formula (HA-II) or formula (HB-II).

(If formula (HA-I) or formula (HB-I) is substituted for formula (HA-II) or formula (HB-II) in step (1), then formula (HA-II) or formula (HB-II) is substituted for formula (HA-I) or formula (HB-I) in step (2)).

(3) The mixed solution obtained in (2) is dripped into a solution containing a calcium ion to obtain a gel that forms chemical crosslinks and ion crosslinks in solution, thereby yielding a crosslinked alginic acid structure gel containing fluorescein isothiocyanate-dextran.

8. Method for synthesizing alginic acid derivative

The alginic acid derivative represented by formula (HA-I) or formula (HA-II) can be manufactured with reference to PCT/JP2019/023478 (submitted June 13, 2019).

9. Use of alginic acid derivatives and crosslinked alginic acid structure

As discussed above, the alginic acid derivatives may be used to prepare a transplantation device. Apart from the transplantation device, the alginic acid derivatives may also be used in place of conventional alginic acid in a wide range of fields including foodstuffs, medicine, cosmetics, fibers, paper and the like. Specifically, preferred applications of the alginic acid derivatives and ocrosslinked alginic acid structure include medical materials such as wound dressings, postoperative adhesion prevention materials, sustained drug release materials, cell culture substrates and cell transplant substrates.

When used as a medical material, the crosslinked alginic acid structure may be in the form of a tube, fiber, bead, gel, nearly spherical gel or the like; a bead, gel or nearly spherical gel is preferred, and a nearly spherical gel is more preferred.

A particularly preferred embodiment of the transplantation device using the alginic acid derivatives has excellent biocompatibility and stability, with little cytotoxicity and almost no adhesion or inflammation at the transplantation site, and can maintain its shape long-term with little dissolution of the gel (whether or not a semipermeable membrane is included), and can maintain blood glucose lowering effects and regulate blood glucose for a long period of time.

The cure rate in the present invention is represented as the ratio of the number of cured cases to the number of transplanted cases a predetermined time after transplantation when a hydrogel encapsulating insulin-secreting cells or pancreatic islets, or a transplantation device containing this hydrogel, is transplanted into a diabetic patient or a diabetic model animal.

For example, when a hydrogel encapsulating insulin-secreting cells or pancreatic islets, or a transplantation device containing this hydrogel, is transplanted into multiple diabetic model mice obtained by the methods of Example 7, a mouse is considered cured if its blood glucose level is not more than 300 mg/dl a predetermined time after transplantation (however, the value is allowed to exceed 300 mg/dl up to 3 times during that period), and the cure rate is the ratio of the number of cured mice to the number of diabetic model mice tested.

The cure rate is for example at least 20%, or preferably at least 35%, or more preferably at least 50% 2 weeks after transplantation. It is also at least 20%, or preferably at least 35%, or more preferably at least 50% one month after transplantation.

The oxygen permeation rate in the present invention is represented as the ratio of the center part oxygen concentration given 100% as the surface oxygen concentration of a hydrogel encapsulating insulin-secreting cells or pancreatic islets, or a transplantation device containing this hydrogel. The center part is the part roughly in the middle in the vertical direction, the horizontal direction and the thickness direction of a flat gel.

The oxygen permeation rate may be determined by measuring the hydrogel or device, or may be determined by calculation. When it is determined by measurement, for example it may be calculated from the measured values of the surface oxygen concentration and center part oxygen concentration as measured using an OXY-1 ST trace needle-type oxygen sensor manufactured by PreSens for example.

When the oxygen permeation rate is determined by calculation, the reaction-diffusion equation for the oxygen concentration when the hydrogel is first arranged in a cartesian coordinate system in air is as follows.

[Math. 1]

$$\frac{\partial C_{(x,y,z,t)}}{\partial t} = D_{Ae}\left(\frac{\partial^2 C_{(x,y,z,t)}}{\partial x^2} + \frac{\partial^2 C_{(x,y,z,t)}}{\partial y^2} + \frac{\partial^2 C_{(x,y,z,t)}}{\partial z^2}\right) - Q_{O_2}X$$

Given $Q_{02}X = r_{02}$ (0th order reaction), the oxygen concentration in a steady state ($(\partial C/\partial t) = 0$) is represented by the following formula.

[Math. 2]

$$0 = D_{Ae}\left(\frac{\partial^2 C_{(x,y,z)}}{\partial x^2} + \frac{\partial^2 C_{(x,y,z)}}{\partial y^2} + \frac{\partial^2 C_{(x,y,z)}}{\partial z^2}\right) - r_{O_2}$$

Divided equally with the x direction as x = iΔx, the y direction as y = jΔy and the z direction as z = kΔz, the concentration is differentialized by the nodal values of C(x,y,z) = Ci,j,k to obtain the following formula, which is used for calculation. With the proviso that, Δx = Δy = Δz = Δ.

[Math. 3]

$$C_{i,j,k} = \frac{1}{6}\left[C_{i-1,j,k} + C_{i+1,j,k} + C_{i,j+1,k} + C_{i,j-1,k} + C_{i,j,k+1} + C_{i,j,k-1} - \frac{\Delta^2 \cdot r_{O_2}}{D_{Ae}}\right]$$

The oxygen permeation rate may be any other than 0%, such as 1 to 100%, or preferably 10 to 100%, or more preferably 25 to 100%, or still more preferably 50 to 100%, or especially 75 to 100%. Also, preferably it is 25 to 50%, or more preferably 50 to 75%, or especially 75 to 100%.

The substance permeation rate in the present invention means the substance permeation rate from the hydrogel or transplantation device after a specific substance is enclosed in a hydrogel encapsulating insulin-secreting cells or pancreatic islets, or in a transplantation device containing such a hydrogel, and this is set for a specific time in a shaken solution. The substance is exemplified by, for instance, glucose and insulin.

For example, this may be the insulin permeation rate or glucose permeation rate when 500 mg of human insulin or 250 mg of glucose is enclosed in a hydrogel or transplantation device, and this is shaken for 24 hours at room temperature in 40 ml of physiological saline containing 0.01 Tween 20.

The insulin permeation rate is at least 50%, or preferably at least 70%, or more preferably at least 80%. The glucose permeation rate is at least 50%, or preferably at least 70%, or more preferably at least 80%.

When the hydrogel of the present invention is shaken under predetermined conditions, there is no or very little breakage, decomposition or dissolution. For example, when three hydrogels 10 mm long, 20 mm wide and 0.1 to 5 mm thick or about 20 $mm^3$ to 1,000 $mm^3$ square, or three hydrogels 10 mm long, 20 mm wide and 100 μm to less than 500 μm thick or 20 $mm^3$ to less than 100 $mm^3$ square, or three hydrogels 10 mm long, 20 mm wide and 250 μm thick or roughly 50 $mm^3$ square are prepared, added to 12 ml of 37°C physiological saline solution in a 15 ml conical tube, and shaken back and forth with a shaking incubator (for example, a medium-sized constant-temperature shaker (Taitec Corp. BR-43FL MR Bioshaker®)) at an amplitude of 25 mm and a shaking speed of 180 rpm with the temperature maintained at 37°C, there is no or very little breakage, decomposition or dissolution.

For there to be little decomposition or dissolution of the hydrogel means that for example given 100% as the alginic acid in the hydrogel, and using the ratio of alginic acid eluted into the solution under the above shaking conditions as the collapse rate, the collapse rate is not more than 40%, or preferably not more than 20%, or more preferably not more than 10%. In particular, the collapse rate is not more than 30% or preferably not more than 20% or more preferably not more than 10% if the collapse rate is the ratio of alginic acid eluted into the solution after being shaken for 24 hours under the above conditions. Furthermore, the collapse rate is not more than 30%, or preferably not more than 20%, or more preferably not more than 10% if the collapse rate is the ratio of alginic acid eluted into the solution after being shaken for 96 hours under the above conditions.

No cells or few cells are shed from the hydrogel of the present invention when the hydrogel is shaken under specific conditions. For example, when three hydrogels 10 mm long, 20 mm wide and 0.1 to 5 mm thick or about 20 $mm^3$ to 1,000 $mm^3$ square, or three hydrogels 10 mm long, 20 mm wide and 100 μm to less than 500 μm thick or about 20 $mm^3$ to less than 100 $mm^3$ square, or three hydrogels 10 mm long, 20 mm wide and 250 μm thick or roughly 50 $mm^3$ square (each encapsulating cells) are prepared, added to 12 ml of 37°C physiological saline solution in a 15 ml conical tube, and shaken back and forth with a shaking incubator (for example, a medium-sized constant-temperature shaker (Taitec Corp. BR-43FL MR Bioshaker®)) at an amplitude of 25 mm and a shaking speed of 180 rpm with the temperature maintained at 37°C, no cells or few cells are shed from the hydrogel.

For there to be little shedding of cells from the hydrogel means that for example given 100% as the number of cells in the hydrogel at the start of testing, the shedding rate is not more than 30%, or preferably not more than 20%, or more preferably not more than 10% if the shedding rate is the ratio of cells shed from the hydrogel when shaken under the above conditions. In particular, the shedding rate is not more than 30%, or preferably not more than 20%, or more

preferably not more than 10% after 24 hours of shaking under the above conditions.

The entire contents of all literature and publications cited in this Description are incorporated by reference in this Description regardless of their purpose. Moreover, the contents disclosed in the Claims, Description and drawings of Japanese Patent Application No. 2019-122063 (submitted June 28, 2019) and International Patent Application PCT/JP 2020/25324 (submitted June 26, 2020) are incorporated by reference in this Description.

Moreover, the objectives, features, advantages and ideas of the present invention are clear to a person skilled in the art from the descriptions of this Description, and the present invention can be easily implemented by a person skilled in the art based on the descriptions of this Description. The best mode and specific examples for implementing the invention are used to illustrate preferred embodiments of the present invention, and the present invention is not limited to these because they are given for purposes of example or explanation. Based on the descriptions of this Description, a person skilled in the art can understand that various modifications are possible within the intent and scope of the present invention as disclosed in this Description.

## Examples

Examples and test examples are given next in order to explain the present invention in detail, but these are only examples and test examples that do not limit the present invention and may be altered without departing from the scope of the present invention.

A JEOL JNM-ECX400 FT-NMR (JEOL) was used for nuclear magnetic resonance (NMR) spectrum measurement. Liquid chromatography-mass spectrometry (LC-Mass) was performed by the following methods. A [UPLC] Waters Aquity UPLC system and a BEH C18 column (2.1 mm × 50 mm, 1.7 μm) (Waters) were used under gradient conditions with a mobile phase of acetonitrile:0.05% trifluoroacetic acid aqueous solution = 5:95 (0 minutes) to 95:5 (1.0 minute) to 95:5 (1.6 minutes) to 5:95 (2.0 minutes).

In the NMR signal patterns of the $^1$H-NMR data, s means a singlet, d a doublet, t a triplet, q a quartet and m a multiplet, br means broad, J is the coupling constant, Hz means hertz, $CDCl_3$ is deuterated chloroform, DMSO-$D_6$ is deuterated dimethylsulfoxide, and $D_2O$ is deuterium. The $^1$H-NMR data does not include signals that cannot be confirmed because they are broadband, such as hydroxyl (OH), amino ($NH_2$) and carboxyl (COOH) group proton signals.

In the LC-Mass data, M means molecular weight, RT means retention time, and $[M + H]^+$ and $[M + Na]^+$ indicate molecular ion peaks.

"Room temperature" in the examples normally indicates a temperature from 0°C to about 35°C.

In the examples, the introduction rate (mol%) of a reactive substituent is the molar number of introduced reactive substituents as a percentage of the molar number of monosaccharide (guluronic acid and mannuronic acid) units constituting the alginic acid as calculated by $^1$H-NMR ($D_2O$).

In the examples, sodium alginate having the physical properties shown in Table 10 above was used as the sodium alginate before introduction of the reactive group or complementary reactive group.

Table 12 shows the physical property values (specifically, reactive group introduction rates (mol%), molecular weights and weight-average molecular weights (× 10,000 Da)) of the alginic acid derivatives with introduced reactive groups (Example 1a, Example 2a) obtained in (Example 1) to (Example 4-2).

(Example 1) Synthesis of alginic acids having introduced dibenzocyclooctyne-amine groups (Examples 1a, 1b, 1c, 1d and 1e):

[C47]

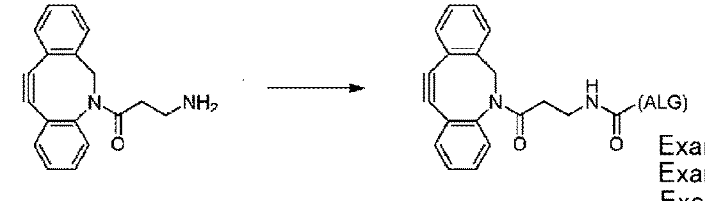

EX1-SM

Example 1a:EX1-(I)-A-2a
Example 1b:EX1-(I)-A-2b
Example 1c:EX1-(I)-A-2c
Example 1d:EX1-(I)-A-2d
Example 1e:EX1-(I)-A-2e

(Example 1a) Synthesis of alginic acid (EX1-(I)-A-2a) having introduced dibenzocyclooctyne-amine group: 4-(4,6-dimethoxy-1,3,5-triazin-2-yl)-4-methylmorpholinium chloride (DMT-MM) (111.65 mg) and 1-molar sodium bicarbonate water (403.5 μl) were added to 43.6 ml of an aqueous solution of sodium alginate (Mochida Pharmaceutical: A-2) adjusted to 1 wt%. An ethanol solution (2 ml) of commercial dibenzocyclooctyne-amine [CAS: 1255942-06-3] (EX1-SM, 83.62 mg) was dripped into this solution, and stirred for 18 hours at room temperature. Sodium chloride (400 mg) was added, ethanol (87.2 ml) was added, and the mixture was stirred for 30 minutes at room temperature. The resulting precipitate was collected by filtration, washed with ethanol, and dried under reduced pressure to obtain the title compound EX1-(I)-A-2a (376 mg) as a light-yellow solid.

The introduction rate of the reactive substituent (dibenzocyclooctyne-amino group) was 6.9 mol% (NMR integration ratio).

(Example 1b) Synthesis of alginic acid (EX1-(I)-A-2b) having introduced dibenzocyclooctyne-amine group:

4-(4,6-dimethoxy-1,3,5-triazin-2-yl)-4-methylmorpholinium chloride (DMT-MM) (330 mg) and 1-molar sodium bicarbonate water (300 μl) were added to 120 ml of an aqueous solution of sodium alginate (Mochida Pharmaceutical: A-2) adjusted to 1 wt%. An ethanol solution (12 ml) of commercial dibenzocyclooctyne-amine [CAS: 1255942-06-3] (EX1-SM, 80 mg) was dripped into this solution, and the mixture was stirred for 4 hours at 30°C. Sodium chloride (1.2 g) was added, ethanol (240 ml) was added, and the mixture was stirred for 30 minutes at room temperature. The resulting precipitate was collected by filtration, washed with ethanol, and dried under reduced pressure to obtain the title compound EX1-(I)-A-2b (1.19 g) as a white solid.

This white solid was dissolved in 80 ml of water, freeze dried, and then dried for 23 hours at 40°C to obtain the title compound EX1-(I)-A-2b (1.2 g) as a white amorphous substance.

The introduction rate of the reactive substituent (dibenzocyclooctyne-amine group) was 5.0 mol% (NMR integration ratio).

(Example 1c) Synthesis of alginic acid (EX1-(I)-A-2c) having introduced dibenzocyclooctyne-amine group:

4-(4,6-dimethoxy-1,3,5-triazin-2-yl)-4-methylmorpholinium chloride (DMT-MM) (167 mg) and 1-molar sodium bicarbonate water (151 μl) were added to 120 ml of an aqueous solution of sodium alginate (Mochida Pharmaceutical: A-2) adjusted to 1 wt%. An ethanol solution (12 ml) of commercial dibenzocyclooctyne-amine [CAS: 1255942-06-3] (EX1-SM, 42 mg) was dripped into this solution, and the mixture was stirred for 3.5 hours at 30°C. Sodium chloride (1.2 g) was added, ethanol (240 ml) was added, and the mixture was stirred for 30 minutes at room temperature. The resulting precipitate was collected by filtration, washed with ethanol, and dried under reduced pressure to obtain the title compound EX1-(I)-A-2c (1.2 g) as a white solid.

This white solid was dissolved in 80 ml of water, freeze dried, and then dried for 23 hours at 40°C to obtain the title compound EX1-(I)-A-2c (1.15 g) as a white amorphous substance.

The introduction rate of the reactive substituent (dibenzocyclooctyne-amine group) was 2.3 mol% (NMR integration ratio).

(Example 1d) Synthesis of alginic acid (EX1-(I)-A-2d) having introduced dibenzocyclooctyne-amine group:

4-(4,6-dimethoxy-1,3,5-triazin-2-yl)-4-methylmorpholinium chloride (DMT-MM) (281 mg) and 1-molar sodium bicarbonate water (253 μl) were added to 201 ml of an aqueous solution of sodium alginate (Mochida Pharmaceutical: A-2) adjusted to 1 wt%. An ethanol solution (20 ml) of commercial dibenzocyclooctyne-amine [CAS: 1255942-06-3] (EX1-SM, 70 mg) was dripped into this solution, and the mixture was stirred for 3 hours at 32°C. Sodium chloride (2.01 g) was added, ethanol (402 ml) was added, and the mixture was stirred for 30 minutes at room temperature. The resulting precipitate was collected by filtration, washed with ethanol, and dried under reduced pressure to obtain the title compound EX1-(I)-A-2d (1.94 g) as a white solid.

This white solid was dissolved in 130 ml of water, freeze dried, and then dried for 2 hours at room temperature to obtain the title compound EX1-(I)-A-2d (1.84 g) as a white amorphous substance.

The introduction rate of the reactive substituent (dibenzocyclooctyne-amine group) was 2.4 mol% (NMR integration ratio).

(Example 1e) Synthesis of alginic acid (EX1-(I)-A-2e) having introduced dibenzocyclooctyne-amine group

4-(4,6-dimethoxy-1,3,5-triazin-2-yl)-4-methylmorpholinium chloride (DMT-MM) (307 mg) and 1-molar sodium bicarbonate water (277 μl) were added to 250 ml of an aqueous solution of sodium alginate (Mochida Pharmaceutical: A-2) adjusted to 1 wt%. An ethanol solution (25 ml) of commercial dibenzocyclooctyne-amine [CAS: 1255942-06-3]

(EX1-SM, 77 mg) was added to this solution, which was then stirred for 3 hours at 32°C. Sodium chloride (2.5 g) was added to the reaction solution, followed by ethanol (500 ml), and the mixture was stirred for 30 minutes at room temperature. The resulting precipitate was collected by filtration, washed with ethanol, and dried under reduced pressure to obtain the title compound EX1-(I)-A-2e (2.42 g) as a white solid.

This white solid was dissolved in 160 ml of water, freeze dried, and then dried for 2 hours at room temperature to obtain the title compound EX1-(I)-A-2e (2.34 g) as a white amorphous substance.

The introduction rate of the reactive substituent (dibenzocyclooctyne-amine group) was 2.2 mol% (NMR integration ratio).

(Example 2) Synthesis of alginic acids having introduced 4-(2-aminoethoxy)-N-(3-azidopropyl) benzamide groups (Examples 2a, 2b, 2c and 2d and 2e):

[C48]

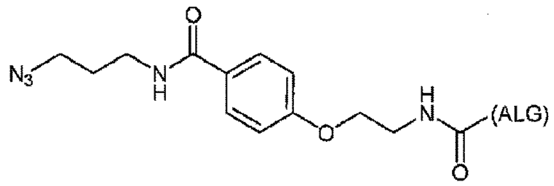

Example 2a:EX2-(II)-A-2a
Example 2b:EX2-(II)-A-2b
Example 2c:EX2-(II)-A-2c
Example 2d:EX2-(II)-A-2d
Example 2e:EX2-(II)-A-2e

<Step 1> Synthesis of methyl 4-(2-((tert-butoxycarbonyl)amino)ethoxy) benzoate (Compound EX2-IM-1):

[C49]

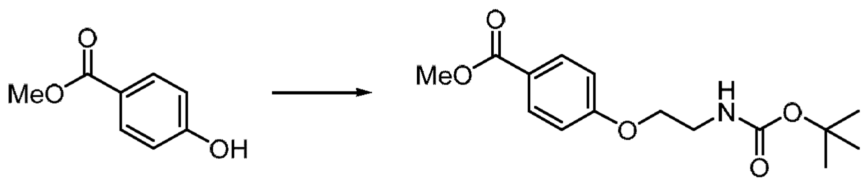

EX2-SM EX2-IM-1

A diethyl azodicarboxylate solution (40% toluene solution, 1.92 ml) was added under ice cooling and stirring to a tetrahydrofuran (2.59 ml) solution of triphenylphosphine (0.96 g), and the mixture was stirred for 20 minutes at room temperature. A tetrahydrofuran (1.1 ml) solution of commercial methyl 4-hydroxybenzoate [CAS: 99-76-3] (Compound EX2-SM, 0.37 g) and 2-(tert-butoxycarbonyl) ethanolamine [CAS: 26690-80-2] (0.39 g) was added to this solution under ice cooling and stirring, and the mixture was stirred for 17 hours at room temperature. The reaction solution was concentrated under reduced pressure, and the residue was purified by silica gel column chromatography (5% ethyl acetate/n-heptane to 40% ethyl acetate/n-heptane) to obtain a mixture of a Compound 1 and Compound 2. This mixture was dissolved in methyl tert-butyl ether (20 ml) and washed twice with 1N-sodium hydroxide aqueous solution (5 ml) and then once with brine (5 ml). The organic layer was dried with anhydrous sodium sulfate, and the solvent was distilled off under reduced pressure to obtain the compound EX2-IM-1 (0.45 g) as a pink oily substance.

NMR data ($CDCl_3$) (δ: ppm): 7.98 (2H, d, J = 8.8 Hz), 6.90 (2H, d, J = 8.8 Hz), 4.97 (1H, br s), 4.07 (2H, t, J = 5.2 Hz), 3.88 (3H, s), 3.56 (2H, q, J = 5.2 Hz), 1.45 (9H, s)

<Step 2> Synthesis of 4-(2-aminoethoxy)-N-(3-azidopropyl) benzamide hydrochloride (Compound EX2-IM-3):

[C50]

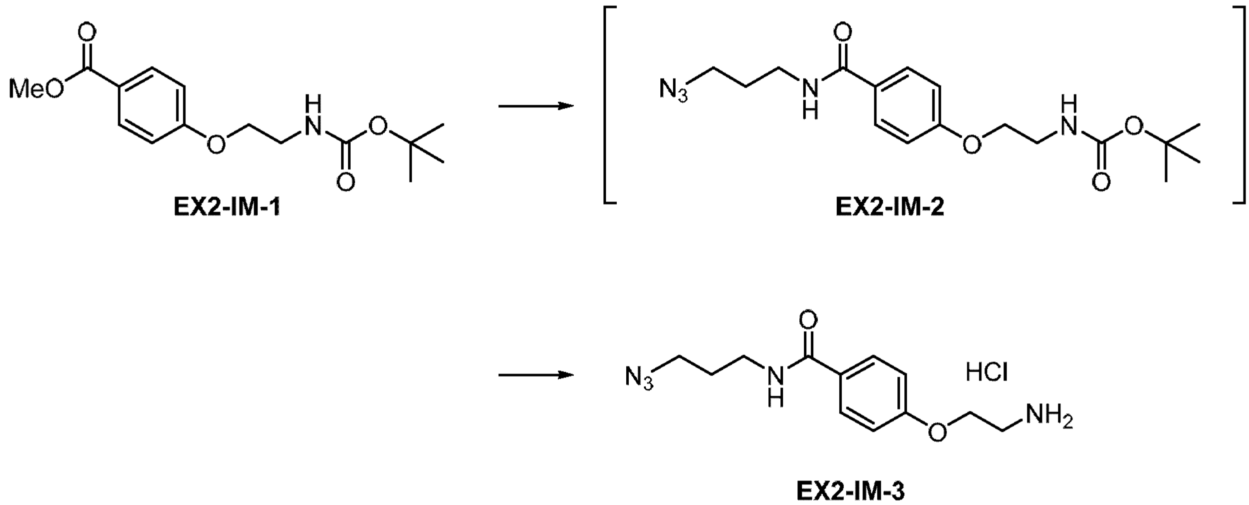

EX2-IM-1

EX2-IM-2

EX2-IM-3

Lithium hydroxide monohydrate (0.25 g) was added to a methanol (4.4 ml) solution of the compound EX2-IM-1 (0.44 g) obtained in <Step 1> of the (Example 2), and the mixture was stirred for 3 hours and 30 minutes at 60°C. 1N-hydrochloric acid (5 ml) was added to the reaction solution, which was then extracted three times with ethyl acetate (10 ml). The organic layer was washed successively with water (5 ml) and brine (5 ml) and dried with anhydrous sodium sulfate, and the solvent was distilled off under reduced pressure. The residue was dissolved in acetonitrile (4.4 ml), and 3-azidopropane-1-amine [CAS: 88192-19-2] (0.15 g) and O-(7-azabenzotriazole-1-yl)-N,N,N',N'-tetramethyluronium hexafluorophosphate salt (0.57 g) were added. N,N-diisopropylethylamine (0.52 ml) was then added under ice cooling and stirring, and the mixture was stirred for 5 hours at room temperature. Water (10 ml) was added to the reaction solution, which was then extracted 3 times with ethyl acetate (15 ml), the organic layer was dried with anhydrous sodium sulfate, and the solvent was distilled off under reduced pressure. The residue was purified by silica gel column chromatography (16% ethyl acetate/n-heptane to 100% ethyl acetate) to obtain a fraction containing the compound EX2-IM-2 (0.71 g).

4N-hydrogen chloride/1,4-dioxane (4.9 ml) was added to the fraction (0.71 g) containing the compound EX2-IM-2, and the mixture was stirred for 20 minutes at room temperature. Diisopropyl ether was added to the reaction solution, and the precipitate was filtered out to obtain the title compound EX2-IM-3 (0.49 g) as a white solid.

NMR data ($CDCl_3$) (δ: ppm): 7.60 (2H, d, J = 8.8 Hz), 6.93 (2H, d, J = 8.8 Hz), 4.19 (2H, t, J = 4.8 Hz), 3.31 to 3.29 (6H, m), 1.77 to 1.71 (2H, m), LC-MS: M (free amine) = 263, RT = 0.54 (minutes), $[M + H]^+$ = 264

(Example 2a) Synthesis of alginic acid (compound EX2-(II)-A-2a) having introduced 4-(2-aminoethoxy)-N-(3-azidopropyl)benzamide group:

[C51]

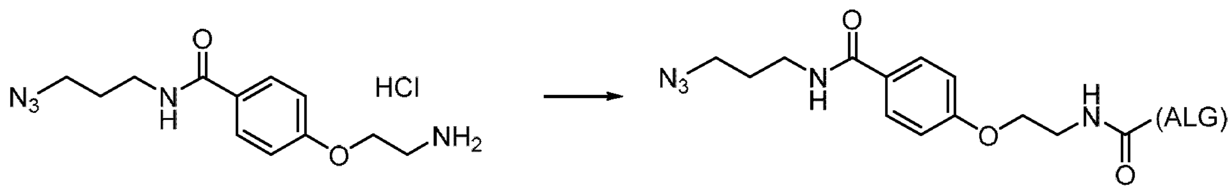

EX2-IM-3

EX2-(II)-A-2a

4-(4,6-dimethoxy-1,3,5-triazin-2-yl)-4-methylmorpholinium chloride (DMT-MM) (50.19 mg), the compound EX2-IM-3 (54.37 mg) obtained in <Step 2> of the (Example 2), and 1-molar sodium bicarbonate water (181.4 μl) were added under ice cooling and stirring to 19.6 ml of an aqueous solution of sodium alginate (Mochida Pharmaceutical: A-2)

adjusted to 1 wt%, and the mixture was stirred for 5 hours at room temperature. Sodium chloride (200 mg) was added, ethanol (39.2 ml) was added, and the mixture was stirred for 30 minutes at room temperature. The resulting precipitate was collected by filtration, washed with ethanol, and dried under reduced pressure to obtain the title compound EX2-(II)-A-2a (198 mg) as a white solid.

The introduction rate of the reactive substituent (4-(2-aminoethoxy)-N-(3-azidopropyl)benzamide group) was 6.1 mol% (NMR integration ratio).

(Example 2b) Synthesis of alginic acid (compound EX2-(II)-A-2b) having introduced 4-(2-aminoethoxy)-N-(3-azidopropyl)benzamide group:

[C52]

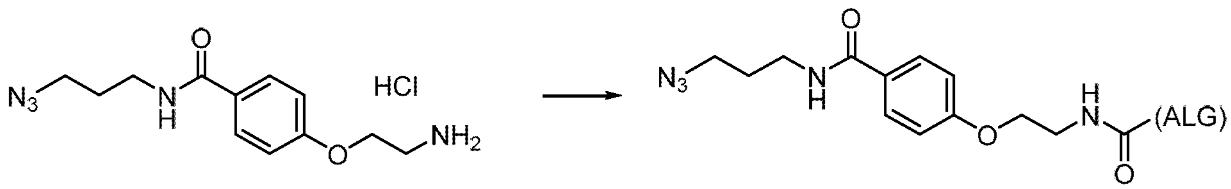

**EX2-IM-3** **EX2-(II)-A-2b**

4-(4,6-dimethoxy-1,3,5-triazin-2-yl)-4-methylmorpholinium chloride (DMT-MM) (330 mg), the compound EX2-IM-3 (90 mg) obtained in <Step 2> of the (Example 2), and 1-molar sodium bicarbonate water (450 μl) were added under ice cooling and stirring to 120 ml of an aqueous solution of sodium alginate (Mochida Pharmaceutical: A-2) adjusted to 1 wt%, and the mixture was stirred for 4 hours at 30°C. Sodium chloride (1.2 g) was added, ethanol (240 ml) was added, and the mixture was stirred for 30 minutes at room temperature. The resulting precipitate was collected by filtration, washed with ethanol, and dried under reduced pressure to obtain the target compound EX2-(II)-A-2b (1.22 g) as a white solid.

This white solid was dissolved in 80 ml of water, freeze dried, and dried for 23 hours at 40°C to obtain the title compound EX3-(II)-A-2b (1.19 g) as a white amorphous substance.

The introduction rate of the reactive substituent (4-(2-aminoethoxy)-N-(3-azidopropyl)benzamide group) was 4.9 mol% (NMR integration ratio).

(Example 2c) Synthesis of alginic acid (compound EX2-(II)-A-2c) having introduced 4-(2-aminoethoxy)-N-(3-azidopropyl) benzamide group:

[C53]

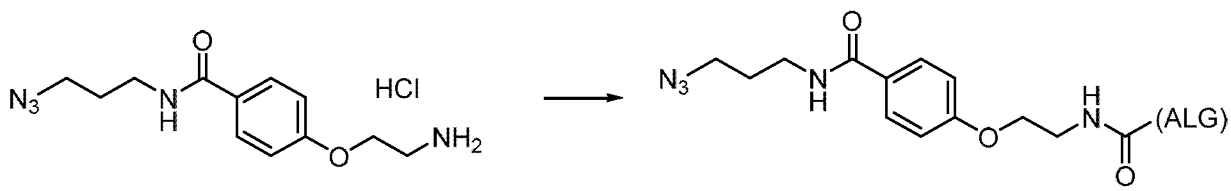

**EX2-IM-3** **EX2-(II)-A-2c**

4-(4,6-dimethoxy-1,3,5-triazin-2-yl)-4-methylmorpholinium chloride (DMT-MM) (167 mg), the compound EX2-IM-3 (45 mg) obtained in <Step 2> of the (Example 2), and 1-molar sodium bicarbonate water (227 μl) were added under ice cooling and stirring to 120 ml of an aqueous solution of sodium alginate (Mochida Pharmaceutical: A-2) adjusted to 1 wt%, and the mixture was stirred for 3.5 hours at 30°C. Sodium chloride (1.2 g) was added, ethanol (240 ml) was added, and the mixture was stirred for 30 minutes at room temperature. The resulting precipitate was collected by filtration, washed with ethanol, and dried under reduced pressure to obtain the title compound EX2-(II)-A-2c (1.22 g) as

a white solid.

This white solid was dissolved in 80 ml of water, freeze dried, and dried for 22 hours at 40°C to obtain the title compound EX2-(II)-A-2c (1.15 g) as a white amorphous substance.

The introduction rate of the reactive substituent (4-(2-aminoethoxy)-N-(3-azidopropyl)benzamide group) was 2.3 mol% (NMR integration ratio).

(Example 2d) Synthesis of alginic acid (compound EX2-(II)-A-2d) having introduced 4-(2-aminoethoxy)-N-(3-azidopropyl) benzamide group:

[C54]

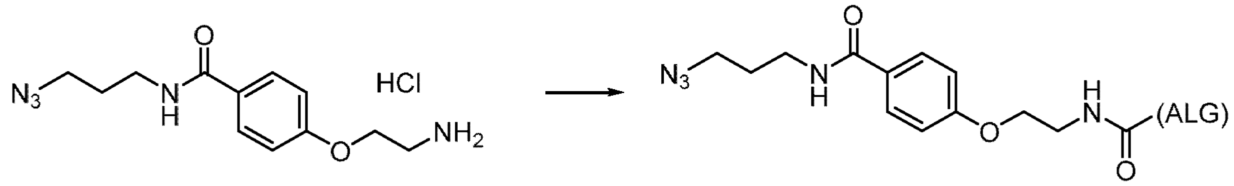

**EX2-IM-3** **EX2-(II)-A-2d**

4-(4,6-dimethoxy-1,3,5-triazin-2-yl)-4-methylmorpholinium chloride (DMT-MM) (307 mg), the compound EX2-IM-3 obtained in <Step 2> of the (Example 2) (83 mg), and 1-molar sodium bicarbonate water (416 μl) were added to 220 ml of an aqueous solution of sodium alginate (Mochida Pharmaceutical: A-2) adjusted to 1 wt%, and the mixture was stirred for 3 hours at 32°C. Sodium chloride (2.2 g) was added, ethanol (440 ml) was added, and the mixture was stirred for 30 minutes at room temperature. The resulting precipitate was collected by filtration, washed with ethanol, and dried under reduced pressure to obtain the title compound EX2-(II)- A-2d (2.13 g) as a white solid.

This white solid was dissolved in 130 ml of water, freeze dried, and dried for 2 hours at room temperature to obtain the title compound EX2-(II)-A-2d (1.99 g) as a white amorphous substance.

The introduction rate of the reactive substituent (4-(2-aminoethoxy)-N-(3-azidopropyl)benzamide group) was 2.3 mol% (NMR integration ratio).

(Example 2e) Synthesis of alginic acid (compound EX2-(II)-A-2e) having introduced 4-(2-aminoethoxy)-N-(3-azidopropyl) benzamide group:

[C55]

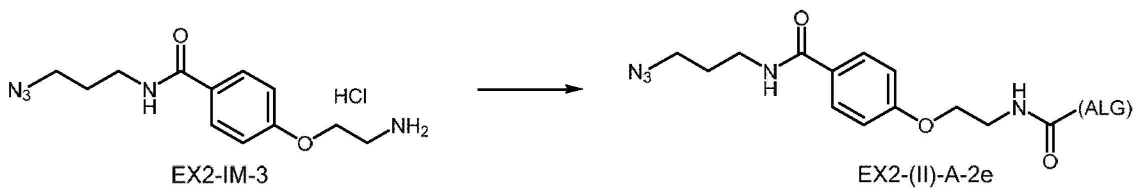

EX2-IM-3 EX2-(II)-A-2e

4-(4,6-dimethoxy-1,3,5-triazin-2-yl)-4-methylmorpholinium chloride (DMT-MM) (377 mg), the compound EX2-IM-3 obtained in <Step 2> of the (Example 2) (102 mg), and 1-molar sodium bicarbonate water (511 μl) were added to 270 ml of an aqueous solution of sodium alginate (Mochida Pharmaceutical: A-2) adjusted to 1 wt%, and the mixture was stirred for 3 hours at 32°C. Sodium chloride (2.7 g) was added to the reaction solution, followed by ethanol (540 ml), and the mixture was stirred for 30 minutes at room temperature. The resulting precipitate was collected by filtration, washed with ethanol, and dried under reduced pressure to obtain the title compound EX2-(II)- A-2e (2.60 g) as a white solid.

This white solid was dissolved in 160 ml of water, freeze dried, and dried for 2 hours at room temperature to obtain the title compound EX2-(II)-A-2e (2.56 g) as a white amorphous substance.

The introduction rate of the reactive substituent (4-(2-aminoethoxy)-N-(3-azidopropyl)benzamide group) was 2.4 mol% (NMR integration ratio).

(Example 3) Synthesis of alginic acids having introduced N-(4-(aminoethyl)benzyl)-2-(cyclooct-2-yn-1-yloxy) acetamide groups (Examples 3a, 3b and 3c):

[C56]

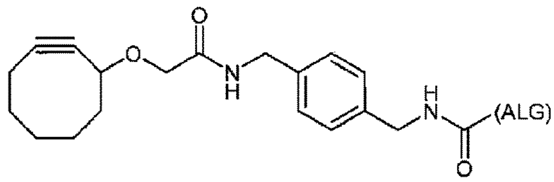

Example 3a:EX3-(I)-A-2a
Example 3b:EX3-(I)-A-2b
Example 3c:EX3-(I)-A-2c

<Step 1> Synthesis of tert-butyl (4-(4((2,2,2-trifluoroacetamido)methyl)benzyl)carbamate (Compound EX3-IM-1):

[C57]

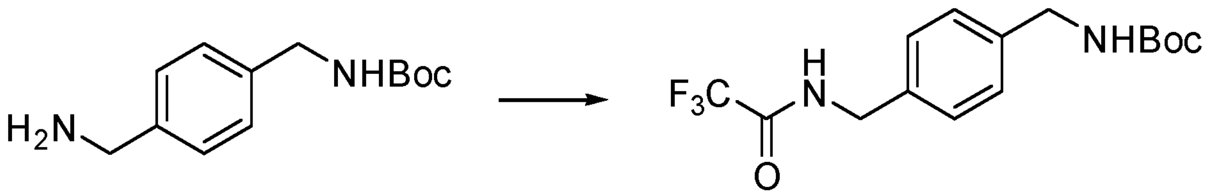

**EX3-SM1** **EX3-IM-1**

With reference to methods known in the literature (Bioorganic & Medicinal Chemistry (2003) 11: 4189-4206), ethyl trifluoroacetate (0.44 ml) was dripped under ice cooling and stirring into a mixture of triethylamine (0.39 ml), methanol (6.67 ml) and tert-butyl (4-(aminoethyl)benzyl)carbamate [CAS: 108468-80-4] (EX3-SM1, 0.67 g) synthesized from 1,4-bis(aminomethyl) benzene [CAS: 539-48-0]. The reaction mixture was warmed to room temperature and stirred for 5 hours at that temperature. The reaction was stopped with water (10 ml), and the mixture was extracted 3 times with ethyl acetate (10 ml). The collected organic layer was washed with brine (5 ml) and dried with anhydrous sodium sulfate, and the dried organic layer was filtered and then concentrated to obtain the title compound EX3-IM-1 (0.671 g) as a light-yellow amorphous substance.

NMR data ($CDCl_3$) (δ: ppm): 7.29 (2H, d, J = 8.4 Hz), 7.25 (2H, d, J = 7.6 Hz), 6.51 (1H, br s), 4.86 (1H, br s), 4.51 (2H, d, J = 5.2 Hz), 4.31 (2H, d, J = 6.0 Hz), 1.46 (9H, s), LC-MS: M = 332, RT = 0.97 (minutes), $[M + Na]^+ = 355$

<Step 2> Synthesis of N-(4-(aminoethyl)benzyl)-2,2,2-trifluoroacetamide hydrochloride (compound EX3-IM-2):

[C58]

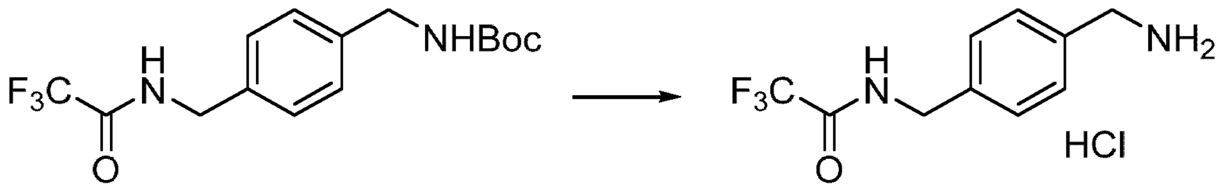

**EX3-IM-1** **EX3-IM-2**

4N-hydrogen chloride/1,4-dioxane (3.5 ml) was added under water cooling and stirring to a 1,4-dioxane solution (3.5 ml) of the compound EX3-IM-1 (0.5 g) obtained in <Step 1> of the (Example 3), and the mixture was stirred for 3 hours at room temperature. Diisopropyl ether (40 ml) was added to the reaction solution, and the precipitate was filtered out to obtain the title compound EX3-IM-2 (0.36 g) as a white solid.

NMR data ($D_2O$) (δ: ppm): δ: 7.29 (2H, d, J = 8.0 Hz), 7.25 (2H, d, J = 8.4 Hz), 4.38 (2H, s), 4.02 (2H, s), LC-MS: M (free amine) = 232, RT = 0.53 (minutes), [M + H]+ = 233

<Step 3> Synthesis of N-(4-((2-(cyclooct-2-yn-1-yloxy)acetamido)methyl)benzyl)-2,2,2-trifluoroacetamide (compound EX3-IM-3):

[C59]

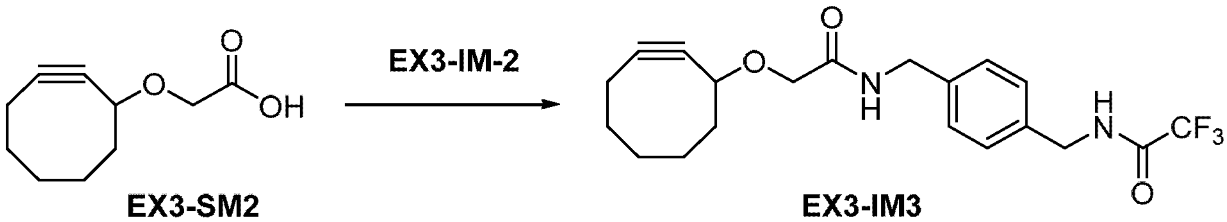


EX3-SM2 EX3-IM3

Following methods known in the literature (Org. Process Res. Dev. (2018) 22: 108-110), the compound EX3-IM-2 (0.26 g) obtained in <Step 2> of the (Example 3) and N,N-diisopropylethylamine (0.51 ml) were dripped under ice-cooling and stirring into an acetonitrile (1.7 ml) solution of O-(7-azabenzotriazol-1-yl)-N,N,N',N'-tetramethyluronium hexafluorophosphate salt (0.26 g) and a carboxylic acid [CAS: 917756-42-4] (EX3-SM2, 0.17 g) synthesized from cycloheptene [CAS: 628-92-2], and the mixture was stirred for 1 hour and 30 minutes at room temperature. Water (5 ml) was added to stop the reaction, and the mixture was extracted 3 times with ethyl acetate (5 ml). The organic layer was washed with brine (3 ml) and dried with anhydrous sodium sulfate. The dried organic layer was filtered, and the solvent was distilled off under reduced pressure. The residue was purified by silica gel column chromatography (12% ethyl acetate/n-heptane to 100% ethyl acetate) to obtain the title compound EX3-IM-3 (0.189 g) as a white amorphous substance.

NMR data ($CDCl_3$) (δ: ppm): δ: 7.31 (2H, d, J = 8.4 Hz), 7.26 (2H, d, J = 8.0 Hz, overlapped with solvent peak), 6.84 (1H, br s), 6.52 (1H, br s), 4.52 (2H, d, J = 6.0 Hz), 4.49 (2H, d, J = 6.4 Hz), 4.26 to 4.23 (1H, m), 4.11 (1H, d, J = 15.2 Hz), 3.94 (1H, d, J = 15.2 Hz), 2.26 to 2.09 (3H, m), 2.00 to 1.58 (6H, m), 1.48 to 1.44 (1H, m), LC-MS: M = 396, RT = 0.99 (minutes), [M + H]+ = 397

<Step 4> Synthesis of N-(4-(aminomethyl)benzyl)-2-(cyclooct-2-yn-1-yloxy)acetoamide (compound EX3-IM-4):

[C60]

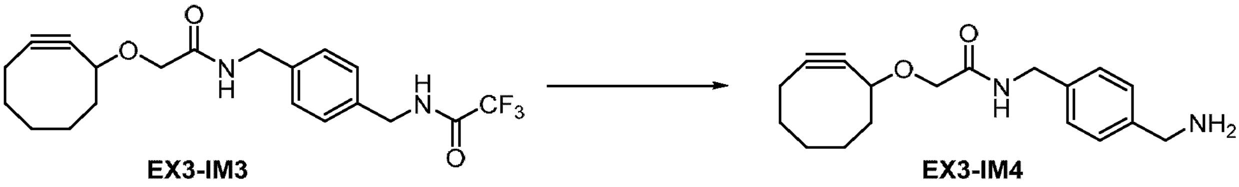

EX3-IM3 EX3-IM4

A potassium carbonate (0.126 g) aqueous solution (0.9 ml) was dripped under ice cooling and stirring into a mixture of the compound EX3-IM-3 (0.18 g) obtained in <Step 3> of the (Example 3) and methanol (1.8 ml), and the mixture was stirred for 17 hours and 30 minutes at room temperature. The methanol was distilled off under reduced pressure, and the mixture was extracted 3 times with ethyl acetate (5 ml). The organic layer was washed with brine (5 ml), and dried with anhydrous sodium sulfate. The organic layer was filtered and the solvent was distilled off under reduced pressure to obtain the title crude compound EX3-IM-4 (0.13 g) as a light yellow oily substance.

NMR data ($CDCl_3$) (δ: ppm): 7.28 to 7.28 (4H, m), 6.80 (1H, br s), 4.48 (2H, d, J = 6.0 Hz), 4.26 to 4.21 (1H,

m), 4.11 (1H, d, J = 15.2 Hz), 3.93 (1H, d, J = 15.2 Hz), 3.86 (2H, s), 2.28 to 2.07 (3H, m), 1.99 to 1.40 (7H, m, overlapped with solvent peak), LC-MS: M = 300, RT = 0.68 (minutes), [M + H]+ = 301

(Example 3a) Synthesis of alginic acid having introduced N-(4-aminomethyl)benzyl)-2-(cyclooct-2-yn-1-yloxy)acetoamide group (EX3-(I)-A-2a)

[C61]

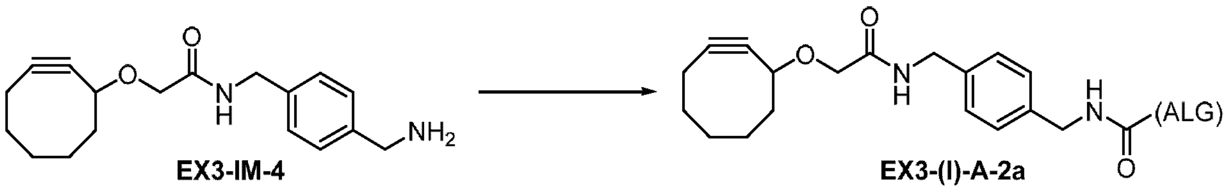

4-(4,6-dimethoxy-1,3,5-triazin-2-yl)-4-methylmorpholinium chloride (DMT-MM) (183 mg) was added under stirring at room temperature to 60 ml of an aqueous solution of sodium alginate (Mochida Pharmaceutical: A-2) adjusted to 1 wt%. An ethanol (5 ml) solution of the compound EX3-IM-4 (41.2 mg) obtained in <Step 4> of the (Example 3) was then dripped in at the same temperature, and the mixture was stirred for 4 hours at 40°C. This was cooled to room temperature and sodium chloride 600 mg) was added, followed by ethanol (119 ml), and the mixture was stirred for 30 minutes. The resulting precipitate was collected by filtration, washed 3 times with ethanol (5 ml), and dried under reduced pressure to obtain the title compound EX3-(I)-A-2a as a white solid. This white solid was dissolved in 40 ml of water, freeze dried, and then dried for 22 hours at 40°C to obtain the title compound EX3-(I)-A-2a (597 mg) as a white cottony substance.

The introduction rate of the reactive substituent (N-(4-(aminomethyl)benzyl)-2-(cyclooct-2-yn-1-yloxy)acetamide group) was 3.7 mol% (NMR integration ratio).

(Example 3b) Synthesis of alginic acid having introduced N-(4-(aminomethyl)benzyl)-2-(cyclooct-2-yn-1-yloxy) acetamide group (EX3-(I)-A-2b)

[C62]

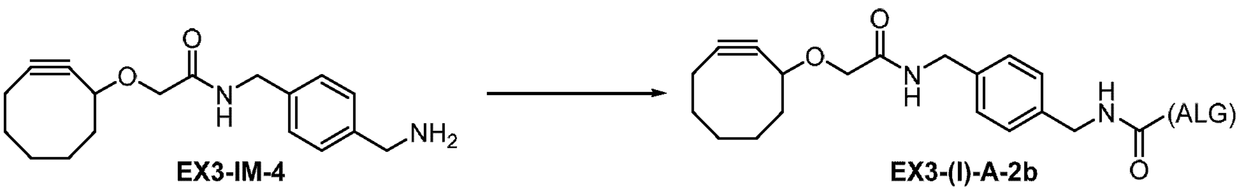

4-(4,6-dimethoxy-1,3,5-triazin-2-yl)-4-methylmorpholinium chloride (DMT-MM) (405 mg), an ethanol (29 ml) solution of the compound EX3-IM-4 (121 mg) obtained in <Step 4> of the (Example 3), and 1-molar sodium bicarbonate water (366 μl) were added to 290 ml of an aqueous solution of sodium alginate (Mochida Pharmaceutical: A-2) adjusted to 1 wt%, and the mixture was stirred for 3 hours and 20 minutes at 32°C. Sodium chloride (2.9 g) was added, ethanol (580 ml) was added, and the mixture was stirred for 30 minutes at room temperature. The resulting precipitate was collected by filtration, washed with ethanol, and dried under reduced pressure to obtain the title compound EX3-(I)-A-2b (2.67 g) as a white solid

This white solid was dissolved in 180 ml of water, freeze dried, dried for 6 hours at 40°C, and then stored under refrigeration. This was then dried again for 5 hours at 40°C to obtain the title compound EX3-(I)-A-2b (2.77 g) as a white amorphous substance.

The introduction rate of the reactive substituent (N-(4-(aminomethyl)benzyl)-2-(cyclooct-2-yn-1-yloxy)acetamide group) was 2.1 mol% (NMR integration ratio).

(Example 3c) Synthesis of alginic acid having introduced N-(4-(aminomethyl)benzyl)-2-(cyclooct-2-yn-1-yloxy) acetamide group (EX3-(I)-A-2c):

[C63]

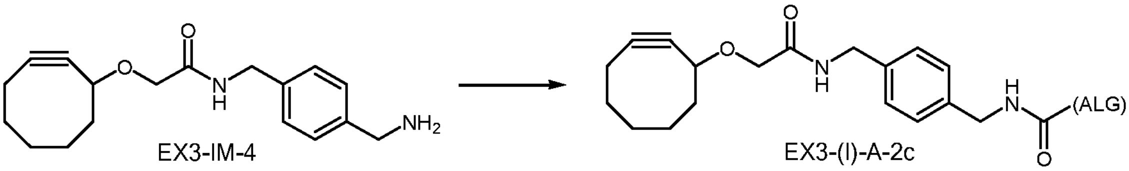

EX3-IM-4 EX3-(I)-A-2c

4-(4,6-dimethoxy-1,3,5-triazin-2-yl)-4-methylmorpholinium chloride (DMT-MM) (363 mg), an ethanol (26 ml) solution of the compound EX3-IM-4 (109 mg) obtained in <Step 4> of the (Example 3), and 1-molar sodium bicarbonate water (328 μl) were added to 260 ml of an aqueous solution of sodium alginate (Mochida Pharmaceutical: A-2) adjusted to 1 wt%, and the mixture was stirred for 3 hours at 32°C. Sodium chloride (2.6 g) was added to the reaction solution, followed by ethanol (520 ml), and the mixture was stirred for 30 minutes at room temperature. The resulting precipitate was collected by filtration, washed with ethanol, and dried under reduced pressure to obtain the title compound EX3-(I)-A-2c (2.69 g) as a white solid

This white solid was dissolved in 180 ml of water, freeze dried, and dried for 2.5 hours at 40°C to obtain the title compound EX3-(I)-A-2c (2.42 g) as a white amorphous substance.

The introduction rate of the reactive substituent (N-(4-(aminomethyl)benzyl)-2-(cyclooct-2-yn-1-yloxy)acetamide group) was 2.0 mol% (NMR integration ratio).

(Example 4)

Synthesis of alginic acid (Example 4) having introduced N-(2-aminoethyl)-4-azidobenzamide group:

[C64]

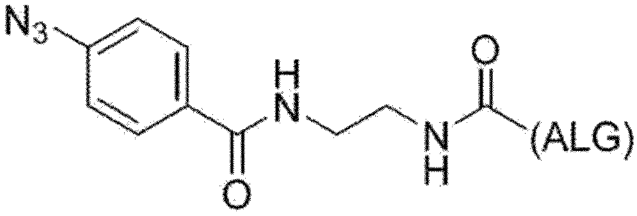

Example 4:EX4-(II)-A-2

<Step 1> Synthesis of tert-butyl(2-(4-azidobenzamido)ethyl)carbamate (Compound EX4-IM-1):

[C65]

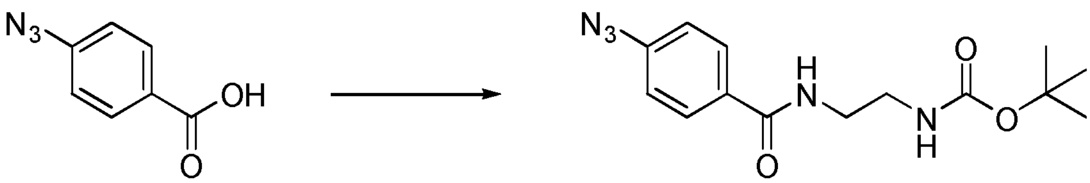

**EX4-SM** **EX4-IM-1**

Thionyl chloride (783 μl) and N,N-dimethylformamide (3 μl) were added to 700 mg of 4-azidobenzoic acid (EX4-SM) [CAS: 6427-66-3], and the mixture was stirred for 1 hour at 70°C. The reaction solution was concentrated under reduced pressure, and azeotropically distilled by adding 1 ml of methylene chloride to the residue, and the same operations were repeated. A methylene chloride (7.0 ml) solution of tert-butyl(2-aminoethyl)carbamate [CAS: 57260-73-8] (825 mg) and pyridine (1.04 ml) was added under ice-water cooling to a methylene chloride (3 ml) solution of the resulting light-yellow solid, and the mixture was stirred for 1 hour at room temperature. The reaction solution was diluted with tert-butyl methyl ether (30 ml), and washed successively with water (10 ml), saturated sodium bicarbonate water (5 ml), 0.5 N citric acid (5 ml, twice), water (5 ml) and brine (5 ml). The organic layer was dried with anhydrous sodium sulfate and concentrated under reduced pressure. The residue was triturated with tert-butyl methyl ether/heptane, and the solids were filtered and washed with tert-butyl methyl ether/heptane to obtain the title compound EX4-IM-1 (1.1 g) as a white solid.

NMR data ($CDCl_3$) (δ: ppm): 7.83 (2H, d, J=8 Hz), 7.26 (1H, br s), 7.05 (2H, d, J=8 Hz), 4.97 (1H, br s), 3.55 (2H, q, J=5 Hz), 3.45-3.37 (2H, m), 1.43 (9H, s), LC-MS: M=305, RT=0.90 (minutes), $[M+H]^+$ = 306, $[M+Na]^+$ = 328

<Step 2> Synthesis of N-(2-aminoethyl)-4-azidobenzamide hydrochloride (Compound EX4-IM-2):

[C66]

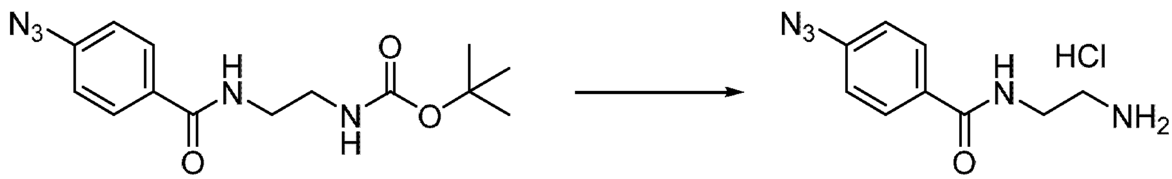

EX4-IM-1 EX4-IM-2

The compound (EX4-IM-1, 500 mg) obtained in <Step 1> of the (Example 4) was suspended in 1,4-dioxane (1.5 ml). 4N-hydrogen chloride/dioxane solution (3.5 ml) was added under ice water cooling, and the mixture was stirred for 2.5 hours at room temperature. Diisopropyl ether (10.5 ml) was added to the reaction solution, which was then stirred for 50 minutes at room temperature. The solids were filtered, washed with diisopropyl ether, and dried under reduced pressure to obtain the title compound EX4-IM-2 (365 mg) as a light beige solid.

NMR data (DMSO-$d_6$) (δ: ppm): 8.68 (1H, t, J=6 Hz), 7.93 (2H, d, J=9 Hz), 7.82 (1H, br s), 7.22 (2H, d, J=9 Hz), 3.49 (2H, q, J=6 Hz), 2.97 (2H, t, J=6 Hz), LC-MS: M (free amine) = 205, RT = 0.56 (minutes), $[M+H]^+$ = 206

<Step 3-1> Synthesis of alginic acid (EX4-(II)-A2) having introduced N-(2-aminoethyl)-4-azidobenzamide group

[C67]

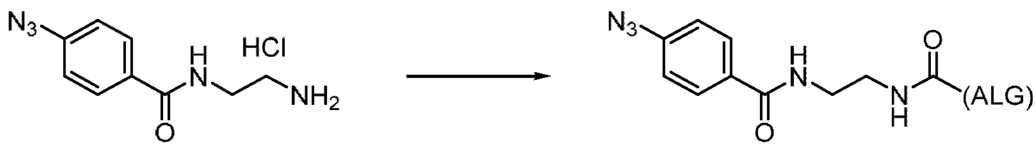

EX4-IM-2 EX4-(II)-A-2

4-(4,6-dimethoxy-1,3,5-triazin-2-yl)-4-methylmorpholinium chloride (DMT-MM) (167 mg), the compound EX4-IM-2 (37 mg) obtained in <Step 2> of the (Example 4), and 1-molar sodium bicarbonate water (227 μl) were added to 60 ml of an aqueous solution of sodium alginate (Mochida Pharmaceutical: A-2) adjusted to 1 wt%, and the mixture was stirred for 3.5 hours at 30°C. Sodium chloride (600 mg) was added, ethanol (120 ml) was added, and the mixture was stirred for 30 minutes at room temperature. The resulting precipitate was collected by filtration, washed with ethanol, and dried under reduced pressure to obtain the title compound EX4-(II)-A-2 (615 mg) as a white powder.

This white solid was dissolved in 40 ml of water, freeze dried, and dried for 22 hours at 40°C to obtain the title compound EX4-(II)-A-2 (583 mg) as a white amorphous substance.

The introduction rate of the reactive substituent (N-(2-aminoethyl)-4-azidobenzamide group) was 5.3 mol% (NMR integration ratio).

(Example 4-2) Synthesis N-(2-(2-aminoethoxy)ethyl)-4-azidobenzamide groups (Examples 4-2a and 4-2b)

[C68]

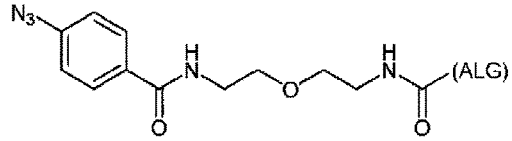

Example 4a:EX4-2-(II)-A-2a
Example 4b:EX4-2-(II)-A-2b

<Step 1> Synthesis of tert-butyl(2-(2-(4-azidobenzamido)ethoxy)ethyl)carbamate (Compound EX4-2-IM-1)

[C69]

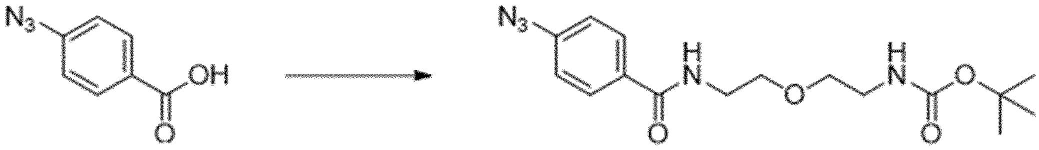

EX4-SM EX4-2-1M-1

4-azidobenzoic acid [CAS: 6427-66-3] (EX4-SM, 300 mg) and tert-butyl(2-(2-aminoethoxy)ethyl)carbamate [CAS: 127828-22-2] (376 mg) were dissolved in acetonitrile (6.0 ml). O-(7-azabenzotriazole-1-yl)-N,N,N',N'-tetramethyluronium hexafluorophosphate salt (0.77 g) and diisopropyl ethylamine (707 μl) were added, and the mixture was stirred for 16 hours at room temperature. Ethyl acetate (20 ml) and water (10 ml) were added to separate the reaction solution. The organic layer was washed successively with water (10 ml) and brine (5 ml), dried with anhydrous sodium sulfate, and concentrated under reduced pressure. The residue was purified by silica gel column chromatography (20% ethyl acetate/n-heptane to 70% ethyl acetate/n-heptane) to obtain the title compound EX4-2-IM-1 (673 mg) as a light-yellow gum-like substance.

NMR data ($CDCl_3$) (δ: ppm): 7.83 (2H, d, J=9 Hz), 7.08 (2H, d, J=9 Hz), 6.61 (1H, br s), 4.84 (1H, br s), 3.68-3.64 (4H, m), 3.56 (2H, t, J=5 Hz), 3.34 (2H, q, J=5 Hz), 1.44 (9H, s)

<Step 2> Synthesis of N-(2-(2-aminoethoxy)ethyl)-4-azidobenzamide hydrochloride (Compound EX4-2-IM-2):

[C70]

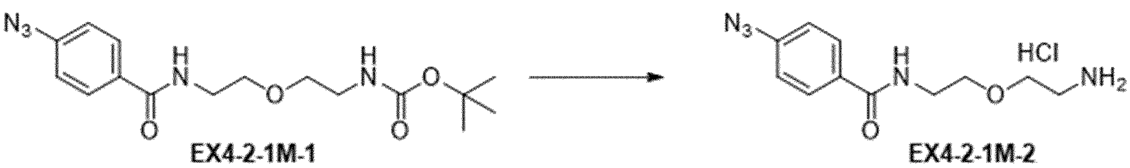

EX4-2-1M-1 EX4-2-1M-2

4N-hydrogen chloride/1,4-dioxane (4.7 ml) was added under ice water cooling to the compound (EX4-2-IM-1, 670 mg) obtained in <Step 1> of the (Example 4-2), and the mixture was stirred for 2 hours at room temperature. Diisopropyl ether (14 ml) was added to the reaction solution, which was then stirred for 30 minutes. The resulting solid was collected by filtration, washed with diisopropyl ether, and dried under reduced pressure to obtain the title compound

EX4-2-IM-2 (604 mg) as a light beige solid.

NMR data (DMSO-$d_6$) (δ: ppm): 8.61 (1H, t, J=6 Hz), 7.95 (3H, br s), 7.93 (2H, d, J=9 Hz), 7.20 (2H, d, J=9 Hz), 3.62 (2H, t, J=5 Hz), 3.57 (2H, t, J=6 Hz), 3.46 (2H, q, J=6 Hz), 3.02-2.93 (2H, m), LC-MS (free amine): RT = 0.57 (minutes), $[M+H]^+$ = 250

(Example 4-2a) Synthesis of alginic acid (EX4-2-(II)-A-2a) having introduced N-(2-(2-aminoethoxy)ethyl)-4-azidobenzamide group:

[C71]

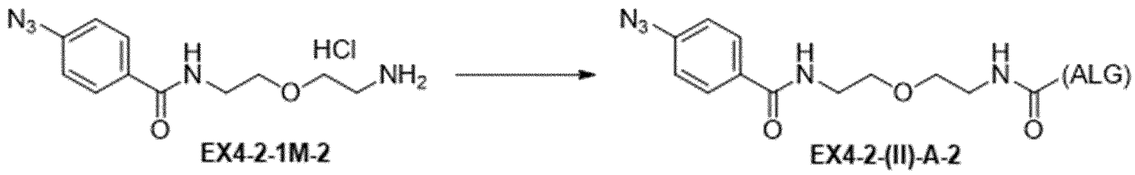

EX4-2-1M-2 EX4-2-(II)-A-2

4-(4,6-dimethoxy-1,3,5-triazin-2-yl)-4-methylmorpholinium chloride (DMT-MM) (366.09 mg) and 1-molar sodium bicarbonate water (274.51 μl) were added to 118 ml of an aqueous solution of sodium alginate (Mochida Pharmaceutical: A-2) adjusted to 1 wt%. A water (1 ml) and ethanol (1 ml) solution of the compound EX4-2-IM-2 (78.44 mg) obtained in <Step 2> of the (Example 4-2) was then added at room temperature, and the mixture was stirred for 4 hours at 40°C. Sodium chloride (1.2 g) was added at room temperature, ethanol (237 ml) was added, and the mixture was stirred for 30 minutes at room temperature. The resulting precipitate was collected by filtration, washed with ethanol, and dried under reduced pressure to obtain the title compound EX4-2-(II)-A-2a (1.17 g) as a white solid.

This white solid was dissolved in 80 ml of water and freeze dried to obtain the title compound EX4-2-(II)-A-2a (1.14 g) as a white cottony substance.

The introduction rate of the reactive substituent (N-(2-(2-aminoethoxy)ethyl)-4-azidobenzamide group) was 2.72 mol% (NMR integration ratio).

(Example 4-2b) Synthesis of alginic acid (EX4-2-(II)-A-2b) having introduced N-(2-(2-aminoethoxy)ethyl)-4-azidobenzamide group:

[C72]

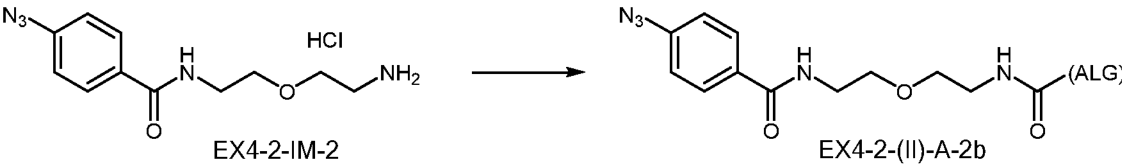

EX4-2-IM-2 EX4-2-(II)-A-2b

4-(4,6-dimethoxy-1,3,5-triazin-2-yl)-4-methylmorpholinium chloride (DMT-MM) (279 mg) and 1-molar sodium bicarbonate water (378 μl) were added to 200 ml of an aqueous solution of sodium alginate (Mochida Pharmaceutical: A-2) adjusted to 1 wt%. The compound EX4-2-IM-2 (77 mg) obtained in <Step 2> of the (Example 4-2) was then added at room temperature, and the mixture was stirred for 3.25 hours at 32°C. Sodium chloride (2.0 g) was added to the reaction solution, followed by ethanol (400 ml), and the mixture was stirred for 30 minutes at room temperature. The resulting precipitate was collected by filtration, washed with ethanol, and dried under reduced pressure to obtain the title compound EX4-2-(II)-A-2b (1.93 g) as a white solid.

This white solid was dissolved in 130 ml of water and freeze dried to obtain the title compound EX4-2-(II)-A-2b (1.85 g) as a white amorphous substance.

The introduction rate of the reactive group (N-(2-(2-aminoethoxy)ethyl)-4-azidobenzamide group) was 2.3 mol% (NMR integration ratio).

[Table 10]

| Example | Measurement wavelength (nm) | Molecular weight (Da) | Weight-average molecular weight (Da) | Introduction rate (mol%) |
|---|---|---|---|---|
| 1a | 280 | 12,000 to 2,650,000 | 1,530,000 | 6. 9(∗) |
| 2a | 255 | 15.000 to 2.530.000 | 1.510.000 | 6. 1(∗) |
| (∗)NMR integration ratio | | | | |

[Measuring introduction rate of reactive group or complementary reactive group]

The introduction rate of the reactive group or complementary reactive group is a percentage value representing the number of introduced reactive groups or complementary reactive groups relative to the total uronic acid monosaccharide units that are repeating units of the alginic acid.

In these examples, the introduction rate of the reactive group or complementary reactive group (mol%) is calculated based on the $^1$H-NMR integration ratio. An amount of alginic acid necessary for calculating the introduction rate is measured by the carbazole-sulfuric acid method using a calibration curve, and the amount of the reactive group or complementary reactive group is measured by the absorbance measurement method using a calibration curve.

[Molecular weight measurement]

The alginic acid solids having introduced reactive groups or complementary reactive groups obtained in the examples were each dissolved in 10 mmol/L phosphate buffer, (pH 7.4) containing 0.15 mol/L NaCl to prepare 0.1% solutions, which were then passed through a polyether sulfone filter (Minisart High Flow Filter, Sartorius) with a pore size of 0.22 microns to remove insoluble matter, after which samples for gel filtration were prepared. The spectrum of each sample was measured with a DU-800 spectrophotometer (Beckman-Coulter), and the measurement wavelength for each compound in gel filtration was determined. A differential refractometer was used for compounds lacking characteristic absorption wavelengths.

200 μl of each sample for gel filtration was supplied to a Superose 6 Increase 10/300 GL column (GE Healthcare Science). Gel filtration was performed at room temperature at a flow rate of 0.8 ml/min using an AKTA Explorer 10S as the chromatograph unit and 10 mmol/L phosphate buffer, (pH 7.4) containing 0.15 mol/L NaCl as the developing solvent. An elution profile was prepared for each sample by monitoring absorbance at the wavelength determined for that compound. The resulting chromatogram was analyzed with Unicorn 5.31 software (GE Healthcare Science) to determine the peak range.

To determine the molecular weights of the alginic acids having introduced reactive groups or complementary reactive groups, gel filtration was performed using blue dextran (molecular weight 2,000,000 Da, SIGMA), thyroglobulin (molecular weight 669,000 Da, GE Healthcare Science), ferritin (molecular weight 440,000 Da, GE Healthcare Science), aldolase (molecular weight 158,000 Da, GE Healthcare Science), conalbumin (molecular weight 75,000 Da, GE Healthcare Science), ovalbumin (molecular weight 44,000 Da, GE Healthcare Science), ribonuclease A (molecular weight 13,700 Da, GE Healthcare Science and aprotinin (molecular weight 6,500 Da, GE Healthcare Science) as standard substances under the same conditions used for the alginic acids having introduced reactive groups or complementary reactive groups, and the elution volume of each component was determined with Unicorn software. The elution volume of each component was plotted on the horizontal axis and the logarithm of the molecular weight on the vertical axis, and a calibration curve was prepared by linear regression. Two curves were prepared, one for blue dextran to ferritin and one for ferritin to aprotinin.

The calibration curves were used to calculate the molecular weight (Mi) at elution time i in the chromatogram obtained above. Absorbance at elution time i was then read and given as Hi. The weight-average molecular weight (Mw) was determined by the following formula from these data.

[Math. 4]

$$Mw=\frac{\sum_{i=1}^{\infty}(Hi\times Mi)}{\sum_{i=1}^{\infty}Hi}$$

(Example 5)

Manufacturing flat alginic acid gel

[Preparing alginic acid solution]

1.6 wt% or 3.3 wt% aqueous solutions were prepared using the alginic acid derivatives prepared in each example, and filter sterilized with a Minisart High Flow (Sartorius, 0.2 μm, Cat. 16532GUK). The salt concentration of each filter sterilized aqueous solution was adjusted to obtain a 1.5 wt% or 3.0 wt% physiological saline solution.

Alginic acids of Example 1 (a, b, c): 1.5 wt% physiological saline solutions (solutions of Examples 5-1a, 5-1b and 5-1c)
Alginic acids of Example 2 (a, b, c): 3.0 wt% physiological saline solutions (solutions of Examples 5-2a, 5-2b and 5-2c)
Alginic acid of Example 3(a): 1.5 wt% physiological saline solution (solution of Example 5-3a)
Alginic acid of Example 4: 3.0 wt% physiological saline solution (solution of Example 5-4).

Alternatively, 1.0 wt% aqueous physiological saline solutions are prepared using the alginic acid derivatives obtained in the examples, and filter sterilized with a Millex GV 0.22 μm (Millipore, 0.22 μm, Cat. SLGV033RS).

Alginic acid of Example 1(d): 1.0 wt% physiological saline solution (solution of Example 5-1d)
Alginic acid of Example 2(d): 1.0 wt% physiological saline solution (solution of Example 5-2d)
Alginic acid of Example 3(b): 1.0 wt% physiological saline solution (solution of Example 5-3b)
Alginic acid of Example 4-2(a): 1.0 wt% physiological saline solution (solution of Example 5-5a).
Alginic acid of Example 1(e): 0.5 wt% saline solution (solution of Example 5-1e)
Alginic acid of Example 2(e): 0.5 wt% saline solution (solution of Example 5-2e)
Alginic acid of Example 3(c): 0.5 wt% saline solution (solution of Example 5-3b)
Alginic acid of Example 4-2(b): 0.5 wt% saline solution (solution of Example 5-5b)

In the examples below, each of these alginic acid saline solutions was adjusted to a suitable concentration and used in testing.

Chemically crosslinked alginic acid gels are prepared by combining the alginic acids prepared in Example 1 (a, b, c) or Example 3(a) with the alginic acids prepared in Example 2 (a, b, c) or Example 4.

More specifically, the solutions of Example 5-1 (a, b, c) are combined with the solutions of Example 5-2 (a, b, c), and the solution of Example 5-3a was combined with the solution of Example 5-4.

Chemically crosslinked alginic acid gels are also prepared by combining the alginic acid prepared in Example 1(d) or Example 3(b) with the alginic acid prepared in Example 2(d) or Example 4-2(a).

More specifically, the solution of Example 5-1d is combined with the solution of Example 5-2d, the solution of Example 5-1d is combined with the solution of Example 5-5a, the solution of Example 5-3b is combined with the solution of Example 5-2d, and the solution of Example 5-3b is combined with the solution of Example 5-5a.

Chemically crosslinked alginic acid gels are also prepared by combining the alginic acid prepared in Example 1(e) or Example 3(c) with the alginic acid prepared in Example 2(e) or Example 4-2(b).

More specifically, the solution of Example 5-1e is combined with the solution of Example 5-2e, the solution of Example 5-1e is combined with the solution of Example 5-5b, the solution of Example 5-3c is combined with the solution of Example 5-2e, and the solution of Example 5-3c is combined with the solution of Example 5-5b.

[Manufacturing flat alginic acid gels]

<Common preparation methods>

2 ml of a 55 mmol/L calcium chloride (CaCh) aqueous solution is added to a 3.5 cm culture dish, and 400 μl of the alginic acid solution is dripped in with a P1000 pipette and left standing for 10 minutes. If the gel started to solidify after 5 minutes or more during this process, the culture dish is shaken by hand to solidify the rim of the alginic acid. After 10 minutes, an additional 4 ml of calcium chloride solution is added and left standing for 5 minutes. This is washed 3 times with brine to obtain a flat alginic acid gel.

The flat gel here is an alginic acid gel with a short diameter of 12 to 15 mm, a long diameter of 12 to 18 mm and a thickness of about 0.5 to 5 mm for example, and may be circular, rectangular, hexangular, octagonal or the like in shape, with no particular limitations.

(Example 6)

Biocompatibility test of alginic acid gel

[Example 6-1: Manufacturing flat alginic acid gels for transplantation]

Flat alginic acid gels were manufactured by the methods for [Manufacturing flat alginic acid gel] described in Example 5 using a 55 mmol/L calcium chloride aqueous solution. The following flat alginic acid gels were prepared using an aqueous solution of sodium alginate (Mochida Pharmaceutical: B-2) adjusted to 1 wt%, an aqueous solution of sodium alginate (Mochida Pharmaceutical: A-2) adjusted to 1 wt%, a solution obtained by mixing the solution of Example 5-1b and the solution of Example 5-2b at a volume ratio of 2:1, a solution obtained by mixing the solution of Example 5-1d and the solution of Example 5-2d at a volume ratio of 1: 1, a solution obtained by mixing the solution of Example 5-1d and the solution of Example 5-5 at a volume ratio of 1: 1, a solution obtained by mixing the solution of Example 5-3b and the solution of Example 5-2d at a volume ratio of 1:1, and a solution obtained by mixing the solution of Example 5-3b and the solution of Example 5-5a at a volume ratio of 1:1 as the alginic acid solutions.

The prepared flat alginic acid gels were cultured overnight in D-MEM medium. The next day they were transferred to serum-free D-MEM medium, then to physiological saline, and left for at least 1 hour to obtain alginic acid gels for transplantation into animals.

Example 6-1a:

A flat alginic acid gel with a short diameter of 12mm, a long diameter of 15 mm and a thickness of 5 mm was obtained using an aqueous solution of sodium alginate (Mochida Pharmaceutical: B-2) adjusted to 1 wt%. Fig. 1(a) shows a photograph of this flat alginic acid gel.

Example 6-1b:

A flat alginic acid gel with a short diameter of 12 mm, a long diameter of 12 mm and a thickness of 4 mm was obtained using a mixture of the solution of the Example 5-1b and the solution of the Example 5-2b mixed at a volume ratio of 2:1. This was prepared to a concentration of 1% of the chemical crosslinking groups. Fig. 2(a) shows a photograph of this flat alginic acid gel.

Example 6-1c:

A flat alginic acid gel with a short diameter of 12 mm, a long diameter of 12 mm and a thickness of 4 mm was obtained using a mixture of the solution of the Example 5-1b and the solution of the Example 5-2b mixed at a volume ratio of 2:1. This was prepared to a concentration of 2% of the chemical crosslinking groups. Fig. 3(a) shows a photograph of this flat alginic acid gel.

Example 6-1d:

A flat alginic acid gel with a short diameter of about 12 mm, a long diameter of about 12 mm and a thickness of about 4 mm was obtained using an aqueous solution of sodium alginate (Mochida Pharmaceutical: A-2) adjusted to 1 wt%.

Example 6-1e:

A flat alginic acid gel with a short diameter of about 12 mm, a long diameter of about 12 mm and a thickness of about 4 mm was obtained using a mixture of the solution of the Example 5-1d and the solution of the Example 5-2d mixed at a volume ratio of 1:1. This was prepared to a concentration of 1% of the chemical crosslinking groups.

Example 6-1f:

A flat alginic acid gel with a short diameter of about 12 mm, a long diameter of about 12 mm and a thickness of about 4 mm was obtained using a mixture of the solution of the Example 5-1d and the solution of the Example 5-5a mixed at a volume ratio of 1:1. This was prepared to a concentration of 1% of the chemical crosslinking groups.

Example 6-1g:

A flat alginic acid gel with a short diameter of about 12 mm, a long diameter of about 12 mm and a thickness of about 4 mm was obtained using a mixture of the solution of the Example 5-3b and the solution of the Example 5-2d mixed at a volume ratio of 1:1. This was prepared to a concentration of 1% of the chemical crosslinking groups.

Example 6-1h:

A flat alginic acid gel with a short diameter of about 12 mm, a long diameter of about 12 mm and a thickness of about 4 mm was obtained using a mixture of the solution of the Example 5-3b and the solution of the Example 5-5a mixed at a volume ratio of 1:1. This was prepared to a concentration of 1% of the chemical crosslinking groups.

[Example 6-2: Test of transplantation of flat alginic acid gels into animals]

The alginic acid gels prepared in Examples 6-1a to 6-1c were transplanted intraperitoneally into healthy C57BL/6NCr mice. After 5 weeks the abdomens were opened and the gels extracted, and the gel condition was confirmed. Intraperitoneal adhesion and inflammation were also confirmed.

The alginic acid gels prepared in Examples 6-1d to 6-1h were also transplanted intraperitoneally into healthy C57BL/6NCr mice. After 1, 2 and 4 weeks the abdomens were opened and the gels extracted, and the gel condition was confirmed. Intraperitoneal adhesion and inflammation were also confirmed.

Alginic acid gel of Example 6-1a: The extracted alginic acid gel had fallen apart and had not maintained its shape, and only a small amount of remaining gel could be confirmed and recovered. A photograph of the gel condition is shown in Fig. 1(b).

Alginic acid gel of Example 6-1b: There was no change in the size of the extracted alginic acid gel. A photograph of the gel condition is shown in Fig. 2(b).

Alginic acid gel of Example 6-1c: The extracted alginic acid gel was cracked, but generally maintained its original shape, and there was no change in the gel size. A photograph of the gel condition is shown in Fig. 3(b).

Alginic acid gel of Example 6-1d: The alginic acid gel extracted after 1 week had fallen apart and had not maintained its shape, and only a small amount of remaining gel could be confirmed and recovered.

Alginic acid gel of Example 6-1f: The alginic acid gels extracted after 1 week, 2 weeks and 4 weeks showed no change in the size of the gel.

Alginic acid gel of Example 6-1g: There was no change in the gel size of the alginic acid gels extracted after 1 week and 2 weeks. The alginic acid gel extracted after 4 weeks was cracked, but generally maintained its original shape, and there was no change in the size of the gel.

Alginic acid gel of Example 6-1h: The alginic acid gels extracted after 1 week and 2 weeks showed no change in the size of the gel. The alginic acid gel extracted after 4 weeks was cracked, but generally maintained its original shape, and there was no change in the size of the gel.

When the alginic acid gels of Example 6-1a, Example 6-1b and Example 6-1c were transplanted and the abdomens were opened and checked after 5 weeks, there was no inflammation or adhesion between intraperitoneal organs. The greater omentum and intestinal membrane where the gels were embedded also exhibited no adhesion or inflammation. There was no adhesion or inflammation of the liver to which the broken gel had adhered.

When the alginic acid gels of Example 6-1d, Example 6-1f, Example 6-1g and Example 6-1h were transplanted and the abdomens were opened and checked after 1 week, 2 weeks and 4 weeks, there was no inflammation or adhesion between intraperitoneal organs. The greater omentum and intestinal membrane where the gels were embedded also exhibited no adhesion or inflammation. There was no adhesion or inflammation of the liver to which the broken gel had adhered.

[Example 6-3: Test to confirm cell survival in flat alginic acid gels]

MIN6 cells, an established strain of pancreatic Langerhans islet beta cells, ($5 \times 10^6$ cells) were added to the alginic acid solutions used to prepare the alginic acid gels of Example 6-1a, Example 6-1b, Example 6-1c, Example 6-1d, Example 6-1e, Example 6-1f, Example 6-1g and Example 6-1h, alginic acid gels were prepared and cultured for 3 to 4 weeks in D-MEM medium, and the survival of the MIN6 cells was confirmed under a microscope.

Cell proliferation was good in the alginic acid gels of the Example 6-1a, Example 6-1b, Example 6-1c, Example 6-1d, Example 6-1e, Example 6-1f, Example 6-1g and Example 6-1h, and adequate cell survival was confirmed under a microscope.

(Example 7)

Evaluating transplantation device by intraperitoneal transplantation into diabetic model mice

[Isolation of pig pancreatic islets]

Sterile viable pancreases were obtained from adult pigs under sterile conditions, and pancreatic islet cells were isolated by procedures well known in the field or by the methods described in Shimoda: Cell Transplantation, Vol. 21, pp. 501-508, 2012, or by standard Ricordi techniques established in the Edmonton Protocol.

[Methods for culturing isolated pancreatic islets]

The isolated pancreatic islets were then cultured for 1 day at 37°C in a moist atmosphere of 5% $CO_2$/95% air in medium (Connaught Medical Research Laboratory (CMRL)-based Miami-defined media #1 (MM1; Mediatech-Cellgro, Herndon, VA) supplemented with 0.5% human serum albumin) according to the methods of Noguchi et al (Transplantation Proceedings, 42, 2084-2086 (2010)). The cultured islets were used for producing a transplantation device.

[Preparation of transplantation device]

Using the Example 1b with an introduction rate of 5.0 mol% of the crosslinking group and the Example 2b with an introduction rate of 4.9 mol% of the crosslinking group, a 1.5 wt% physiological saline solution of Example 5-1b and a 3.0 wt% physiological saline solution of Example 5-2b are prepared from each.

A 2 wt% alginic acid solution corresponding to an introduction rate of 5.0 mol% of the crosslinking group can be prepared by mixing the solution of the Example 5-1b and the solution of the Example 5-2b at a volume ratio of 2:1.

The solutions of Example 5-1b and Example 5-2b are each diluted by 2x and 4x to prepare solutions that are then mixed at a volume ratio of 2:1 to prepare solutions.

The mixed solution of the 2x diluted solutions is taken as the solution of Example 7-1, and the mixed solution of the 4x diluted solutions is taken as the solution of the Example 7-2.

The solution of the Example 5-1c and the solution of the Example 5-2c are mixed by the same methods at a volume ratio of 2:1 to obtain a mixed solution of 4× diluted solutions, which is taken as the solution of Example 7-3.

The transplantation devices prepared using the solution of Example 7-1 and the solution of Example 7-2 (100 μl, 200 μl) as well as the solution of Example 7-3 (100 μl, 200 μl) are as follows.

<Transplantation devices>

Example 7-1a: Transplantation device prepared using 100 μl of the solution of Example 7-1
Example 7-1b: Transplantation device prepared using 200 μl of the solution of Example 7-1
Example 7-2a: Transplantation device prepared using 100 μl of the solution of Example 7-2
Example 7-2b: Transplantation device prepared using 200 μl of the solution of Example 7-2
Example 7-3a: Transplantation device prepared using 100 μl of the solution of Example 7-3
Example 7-3b: Transplantation device prepared using 200 μl of the solution of Example 7-3

To prepare 100 μl or 200 μl of 0.5 wt% to 1.0 wt% alginic acid solutions containing pig pancreatic islets, pancreatic islet pellets dispensed in the amount needed for one device were suspended in the alginic acid solution (B1) of Example 5-1b, and this was mixed with the alginic acid solution (C1) of Example 5-2b to obtain solutions of Example 7-1a, Example 7-1b, Example 7-2a and Example 7-2b containing suspended pig pancreatic islets. The alginic acid

solution (B1) of Example 5-1b and the alginic acid solution (C1) of Example 5-2b were adjusted with physiological saline to concentrations depending on the amount of pellets (10 to 30 μl) corresponding to a pig islet volume of 10,000 IEQ per device.

To prepare 100 μl or 200 μl of 0.5 wt% to 1.0 wt% alginic acid solutions containing pig pancreatic islets, moreover, pancreatic islet pellets dispensed in the amount needed for one device were suspended in the alginic acid solution (B1) of Example 5-1c, and this was mixed with the alginic acid solution (C-1) of Example 5-2c to obtain solutions of Example 7-3a and Example 7-3b containing suspended pig pancreatic islets. The alginic acid solution (B1) of Example 5-1c and the alginic acid solution (C1) of Example 5-2c were adjusted with physiological saline to concentrations depending on the amount of pellets (10 to 30 μl) corresponding a pig islet volume of 10,000 IEQ per device.

The pig islet-containing alginic acid solutions prepared in Example 7-1a, Example 7-2a, Example 7-2b and Example 7-3b were immediately encapsulated in semipermeable membranes (Spectrum Chemical Corp. Spectra/Por CE dialysis tube (fractional molecular weight 100,000)) by heal sealing one end of each semipermeable membrane, inserting the alginic acid solution, and sealing with a titanium clip, and these were then immersed for 10 to 15 minutes in a 55 mmol/L CaCh solution to gel the alginic acid gel inside the device.

After gelling, each transplantation device was washed for 3 minutes with physiological saline and cultured overnight in transplantation medium (M199-nicotinamide-FBS + P/S). This was then immersed for 30 minutes in serum-free medium for transplantation (M199 + P/S) and immersed and washed for 30 minutes in physiological saline + P/S for washing before transplantation to obtain a device for mouse transplantation. Fig. 4-1 shows a photograph of a prepared transplantation device.

- Size of transplantation device 10 mm high × 26 mm wide × about 2 mm thick
- Culture conditions: M199 + nicotinamide + fetal bovine serum + penicillin/streptomycin (P/S), O/N
- Washing conditions:

    1) Serum-free medium for transplantation (M199+P/S), 30 min., r.t.
    2) Physiological saline (saline+P/S), 30 min, r.t.

[Table 11]

| Transplantation device | Alginic acid solution | Alginic acid |
|---|---|---|
| Example 7-1 (a, b) | Corresponding to 2x dilution of solutions of Example 5-1b and Example 5-2b | Alginic acids of Example 1b and Example 2b |
| Example 7-2 (a, b) | Corresponding to 4x dilution of solutions of Example 5-1b and Example 5-2b | Alginic acids of Example 1b and Example 2b |
| Example 7-3 (a, b) | Corresponding to 4x dilution of solutions of Example 5-1c and Example 5-2c | Alginic acids of Example 1c and Example 2c |

[Evaluation of transplantation device (transplantation test)]

Animals:

Diabetes model mice from wild-type immune normal mice (C57BL/6NCr). 13 to 17 weeks old, male, 25 to 35 g. Diabetes model was established after 1 week by single intravenous tail injections of 110 mg/kg of Streptozocin solution. Individuals with a blood glucose level of not less than 300 mg/dl and not more than 600 mg/dl were used as needed as diabetes model mice.

Administration methods, transplantation methods

Each mouse was anesthetized by intraperitoneal administration of 0.25 to 0.3 ml of a mixture of 3 kinds of anesthetic (Domitor/Midazolam/Butorphanol) and shaved abdominally and disinfected under anesthesia, after which a roughly 2 cm midline incision was made in the abdomen, and the washed transplantation device was set simply in the abdominal cavity to perform transplantation without fixing. After transplantation the abdomen was closed, and the animal was awakened by subcutaneous injection of 0.25 to 0.3 of medetomidine antagonist (Antisedan). The mouse was kept warm on a heat pad during surgery. No immunosuppressant was administered. No fluid replacement, antibiotic or antiinflammatory was administered.

Methods for measuring blood glucose and body weight:

Casual blood glucose levels were measured before transplantation and at a regular time during the day from day 1 after transplantation to a few days after. Using one drop of blood from a knife wound to the tail, blood glucose levels were measured using a Glutest Neo Alpha and Glutest Neo Sensor. Body weight was measured with an electronic scale immediately before casual blood glucose measurement. If the casual blood glucose level of a mouse with a transplanted device was less than 300 mg/dl, the diabetes was judged to be cured.

Fig. 5-1 shows blood glucose levels and Fig. 6-1 shows body weight up to day 75 after transplantation using the transplantation device of Example 7-2a. Fig. 5-2 shows blood glucose levels and Fig. 6-2 shows body weight up to day 305 after transplantation. Fig. 5-3 shows blood glucose levels and Fig. 6-3 shows body weight up to day 26 after relay transplantation, in which the transplantation device was removed on day 305 after transplantation and re-transplanted into another diabetes model mouse (transplanting a device into a diabetes model mouse, removing the transplanted device after a predetermined amount of time and re-transplanting the removed device into another diabetes model mouse in this way is called "relay transplantation"). In Figs. 5-1 to 5-3 and Figs. 6-1 to 6-3, #1 and #2 are the numbers identifying the individual mice receiving transplantation.

There were no abnormal changes in body weight, and blood glucose levels remained normal for 75 days. There were also no abnormal changes in body weight and blood glucose levels remained normal for 305 days after transplantation and 26 days after subsequent relay transplantation.

As with the Example 7-2a, there were no abnormal changes in body weight and blood glucose levels remained normal for 75 days using the transplantation device of Example 7-2b.

Fig. 7-1 shows blood glucose levels and Fig. 8-1 shows body weight up to day 305 after transplantation using the transplantation device of Example 7-2b. Furthermore, Fig. 7-2 shows blood glucose levels and Fig. 8-2 shows body weight up to day 26 after relay transplantation, in which the transplantation device was removed on day 305 after transplantation and re-transplanted into a different diabetes model mouse.

There were no abnormal changes in body weight, and blood glucose levels remained normal for 305 days after transplantation and for 26 days after subsequent relay transplantation.

As with the Example 7-2a, there were no abnormal changes in body weight and blood glucose levels remained normal for 75 days using the transplantation device of Example 7-3b.

Fig. 9-1 shows blood glucose levels and Fig. 10-1 shows body weight up to day 305 after transplantation using the transplantation device of Example 7-3b. Furthermore, Fig. 9-2 shows blood glucose levels and Fig. 10-2 shows body weight up to day 26 after relay transplantation, in which the transplantation device was removed on day 305 after transplantation and re-transplanted into a different diabetes model mouse. In cases #2 and #3 the device was removed part way through, and the test was terminated.

There were no abnormal changes in body weight, and blood glucose levels remained normal for 305 days after transplantation and for 26 days after subsequent relay transplantation.

A transplantation device was also prepared in the same way as the above transplantation device by enclosing pancreatic islets in a semipermeable membrane without using any alginic acid derivative. When this was transplanted into a mouse by the methods described above under 'Evaluation of transplantation device (transplantation test)" above, no blood glucose lowering effect was observed in the diabetic mouse.

Evaluation of tissue reactivity:

Tissue reactivity was evaluated as follows.

Several weeks after transplantation or after casual blood glucose elevation, the mice with the transplanted devices were anesthetized with a mixture of 3 kinds of anesthetic and disinfected abdominally under anesthesia, a roughly 4 cm midline incision was made in the abdomen, and the transplantation device was searched out among the intra-abdominal organs. If part of the device was seen among the organs it was slowly extracted with tweezers to determine whether the device could be extracted by itself. The condition of the surface of the extracted device was then observed.

<Observation items>

1. Investigate whether there is angiogenesis on the device surface. If angiogenesis exists, observe whether it has progressed to the level of capillaries or thick blood vessels.
2. Next, observe whether there is adhesion or attachment to the organs, peritoneum, omentum or the like. Investigate whether organs can be easily detached, or if sharp detachment is necessary.

3. When there is direct adhesion to the organs, confirm which part of the device (the entire surface, or a part, or a side, or a device fold or sealing part or the like) adheres to which organ.
4. Confirm whether there is inflammation or the like on the organ side.

*Abdomen is closed after device extraction, and the mouse is awakened by subcutaneous injection of an antagonist. The mouse is kept warm on a heat pad during surgery.

Using the transplantation device of Example 7-1a, when the device was extracted 10 weeks after transplantation and tissue reactivity was observed, (1) there was no angiogenesis on the device surface, (2) the device had not adhered to the organs, peritoneum, omentum or the like, (3) the organs could be easily detached, and there was no direct adhesion to the organs, and (4) there was no inflammation or the like on the organ side.

The condition of the surviving pancreatic islet cells in the extracted device was investigated by observing the pancreatic islet cells dispersed in the alginic acid gel under a microscope, either without staining or after staining by (a) Dithizone pancreatic islet cell staining, (b) Dithizone pancreatic islet cell staining, (c) fluorescent staining of living cells with FDA, and (d) fluorescent staining of dead cells with PI. As a result, adequate survival of the pancreatic islet cells in the device was observed.

The device was extracted 10 weeks after transplantation and opened, and the shape of the alginic acid gel in the device was confirmed. As a result, it was shown that an alginic acid gel in a transplantation device that is left for a long period of time in vivo still maintains its shape.

(Example 8) Evaluation 2 of transplantation device by intraperitoneal transplantation into diabetes model mice

[Methods for isolating pig pancreatic islets and culturing isolated islets]

The pancreatic islets used in preparing the transplantation device were obtained by the isolation and culturing methods described in Example 7.

[Preparing transplantation device]

Following methods similar to Example 7, the solution of Example 5-1c and the solution of Example 5-2c were mixed at a volume ratio of 2:1, and diluted 4 times to obtain a mixed solution for Example 8.

The transplantation devices prepared from the solution of Example 8 (100 μl, 75 μl, 50 μl) were as follows.

<Transplantation devices>

Example 8-1 Transplantation device prepared using 100 μl of solution of Example 8
Example 8-2 Transplantation device prepared using 75 μl of solution of Example 8
Example 8-3 Transplantation device prepared using 50 μl of solution of Example 8

To prepare 100 μl, 75 μl and 50 μl of 0.5 wt% alginic acid solution containing pig pancreatic islets, pancreatic islet pellets dispensed for one device were suspended in the alginic acid solution (B1) of Example 5-1c, and this was mixed with the alginic acid solution (C1) of Example 5-2c to obtain a solution of Example 8 containing suspended pig pancreatic islets. The concentrations of the alginic acid solution (B1) of Example 5-1b and the alginic acid solution (C1) of Example 5-2b were adjusted with saline according to the amount of pellets (10 to 30 μl) for 10,000 IEQ of pig pancreatic islets per device.

The pig pancreatic islet-containing alginic acid solutions prepared for Example 8 were rapidly enclosed in a semipermeable membrane (SpectraPor CE dialysis tube (molecular weight cutoff 100,000), manufactured by Spectrum) which had been heat sealed at one end in advance and was then heat sealed at the other end after the alginic acid solution was added, and this was immersed for 10 to 15 minutes in a 55 mmol L$CaCl_2$ solution to gel the alginic acid solution in the device.

After gelling, the transplantation device was washed for three minutes with saline, and cultured overnight in transplantation medium (M199-nicotinamide-FBS+P/S). This was then immersed for 30 minutes in serum-free medium for transplantation (M199+P/S), and then immersed and washed for 30 minutes in saline + P/S for washing before transplantation, to obtain a device for mouse transplantation. Fig 4-2 shows a photograph of the prepared transplantation device.

- Size of transplantation device: L 10 mm × W 26 mm × Thickness about 0.25 to 0.8 mm
- Size of hydrogels in transplantation devices:

  Example 8-1: L 10 mm × W 20 mm × T about 0.5 mm (500 μm)
  Example 8-2: L 10 mm × W 20 mm× T about 0.375 mm (375 μm)
  Example 8-3: L 10 mm × W 20 mm × T about 0.25 mm (250 μm)
  •Culture conditions: M199 + Nicotinamide + Fetal bovine serum + Penicillin/Streptomycin (P/S), O/N
  •Washing conditions:

    1) Serum-free medium for transplantation (M199+P/S), 30 min, r.t.
    2) Physiological saline (saline+P/S), 30 min, r.t.

[Evaluation of transplantation device (transplantation test)]

This was performed according to Example 7.
Fig. 11-1 shows blood glucose levels and Fig. 12-1 shows changes in body weight up to day 178 after transplantation using the transplantation device of Example 8-1.
Fig. 11-2 shows blood glucose levels and Fig. 12-2 shows changes in body weight up to day 178 after transplantation using the transplantation device of Example 8-2. Furthermore, Fig. 11-3 shows blood glucose levels and Fig. 12-3 shows changes in body weight up to day 40 after the transplantation device was removed on day 178 after transplantation and relay transplanted.
Fig. 11-4 shows blood glucose levels and Fig. 12-4 shows changes in body weight up to day 178 after transplantation using the transplantation device of Example 8-3. Furthermore, Fig. 11-5 shows blood glucose levels and Fig. 12-5 shows changes in body weight up to day 40 after the transplantation device was removed on day 178 after transplantation and relay transplanted.
In Figs. 11-1 to 11-5 and 12-1 to 12-5, a # followed by a number represents the number of an individual mouse receiving transplantation.
There were no abnormal changes in body weight, and blood glucose levels remained normal for 178 days. There were also no abnormal changes in body weight and blood glucose levels remained stable for 40 days after further relay transplantation.
The transplanted devices were subjected to GsIs to confirm the insulin-secreting ability of the device.

(Example 9) Evaluation 3 of transplantation device by intraperitoneal transplantation into diabetes model mice

The following devices were prepared by the methods of Example 7.

<Transplantation devices>

Example 9-1: Transplantation device prepared using 200 μl of aqueous solution of sodium alginate (Mochida Pharmaceutical: B-2) adjusted to 1 wt%
Example 9-2: Transplantation device prepared using 200 μl of solution manufactured by the same methods as in Example 7-1
Example 9-3: Transplantation device prepared using 200 μl of solution manufactured by the same methods as in Example 7-2
Example 9-4: Transplantation device prepared using 100 μl of solution manufactured by the same methods as in Example 7-2
Example 9-5: Transplantation device prepared using 200 μl of solution manufactured by the same methods as in Example 7-3
Example 9-6: Transplantation device prepared using 100 μl of solution manufactured by the same methods as in Example 7-3
Example 9-7: Transplantation device prepared using 50 μl of solution manufactured by the same methods as in Example 7-3

- Size of transplantation device: L 10 mm × W 26 mm × Thickness about 0.25 to 1.3 mm
- Size of hydrogels in transplantation devices:

Example 9-1, 9-2, 9-3 and 9-5: L about 10 mm × W about 20 mm × T about 1 mm (1,000 μm)

Examples 9-4 and 9-6: L about 10 mm × W about 20 mm × T about 0.5 mm (500 μm)

Example 9-7: L about 10 mm × W about 20 mm × T about 0.25 mm (250 μm)

A transplantation test was performed according to Example 7. The devices of Examples 9-1 to 9-7 were each transplanted into multiple diabetes model mice. Those mice whose blood glucose levels were not more than 300 mg/dl during the 178 days after transplantation (however, the value was allowed to exceed 300 mg/dl up to 3 times during that period) were considered cured mice, and the ratio of the number of cured mice to the number of diabetes model mice receiving transplantation was calculated as the cure rate, with the results shown below.

[Table 12]

| | Alginic acid solution | Alginic acid | Volume of solution | Number of diabetes model mice | Number of cured mice | Cure rate |
|---|---|---|---|---|---|---|
| Example 9-1 | 1% B-2 alginic acid solution | B-2 alginic acid | 200μl | 30 | 5 | 16.7% |
| Example 9-2 | Corresponding to 2x dilution of solutions of Examples 5-1b and 5-2b | Alginic acids of Examples 1b and 2b | 200μl | 7 | 0 | 0.0% |
| Example 9-3 | Corresponding to 4x dilution of solutions of Examples 5-1b and 5-2b | | 200μl | 11 | 3 | 27.3% |
| Example 9-4 | | | 100μl | 7 | 3 | 42.9% |
| Example 9-5 | Corresponding to 4x dilution of solutions of Examples 5-1b and 5-2b | Alginic acids of Examples 1c and 2c | 200μl | 10 | 4 | 40.0% |
| Example 9-6 | | | 100μl | 13 | 5 | 38.5% |
| Example 9-7 | | | 50μl | 21 | 13 | 61.9% |

It was confirmed that the cure rate of the diabetes model mice was greater using the transplantation devices containing hydrogels manufactured with the alginic acid derivatives of the invention of the present application than using the transplantation devices containing hydrogels manufactured with natural alginic acid. It was also confirmed that the cure rate was higher the thinner the hydrogel, with a cure rate of about 40% at a thickness of 500 μm or more and a higher cure rate of about 62% at a thickness of 250 μm. The cure rate of 0.0% seen with Example 9-2 is due to the small N number, and it is expected that the cure rate would be equivalent to that of Example 9-3 if the N number were larger.

(Example 10) Evaluation 4 of transplantation device by intraperitoneal transplantation into diabetes model mice

[Preparing transplantation devices]

Devices were prepared according to Example 7 using MIN6 cells ($2.5 \times 10^6$ cells), which are stem cells of pancreatic islet of Langerhans beta cells, for the pancreatic islets.
A 2% solution of A-2 alginic acid was diluted 4 times to obtain the solution of Example 10-1.
The solution of Example 5-1e and the solution of Example 5-2e were mixed at a volume ratio of 1:1 to obtain the solution of Example 10-2.
The solution of Example 5-1e and the solution of Example 5-5b were mixed at a volume ratio of 1:1 to obtain the solution of Example 10-3.
The solution of Example 5-3c and the solution of Example 5-2e were mixed at a volume ratio of 1:1 to obtain the solution of Example 10-4.
The solution of Example 5-3c and the solution of Example 5-5b were mixed at a volume ratio of 1:1 to obtain the solution of Example 10-5.

Examples 10-2 to 10-5 each had a 0.5% concentration of the chemical crosslinking group.
The transplantation devices prepared with the solutions (50 μl each) of Examples 10-1 to 10-5 were as follows.

<Transplantation devices>

Example 10-1: Transplantation device prepared using 50 μl of solution of Example 10-1
Example 10-2: Transplantation device prepared using 50 μl of solution of Example 10-2
Example 10-3: Transplantation device prepared using 50 μl of solution of Example 10-3
Example 10-4: Transplantation device prepared using 50 μl of solution of Example 10-4
Example 10-5: Transplantation device prepared using 50 μl of solution of Example 10-5

- Size of transplantation device: L 10 mm × W 26 mm × Thickness about 0.4 mm
- Size of hydrogels in transplantation devices: L about 10 mm × W about 20 mm × T about 0.25 mm (250 μm)

[Evaluation of transplantation device (transplantation test)]

This was performed according to Example 7.
Fig. 13-1 shows blood glucose levels and Fig. 14-1 shows changes in body weight up to day 17 after transplantation using the transplantation device of Example 10-1.
Fig. 13-2 shows blood glucose levels and Fig. 14-2 shows changes in body weight up to day 17 after transplantation using the transplantation device of Example 10-2.
Fig. 13-3 shows blood glucose levels and Fig. 14-3 shows changes in body weight up to day 17 after transplantation using the transplantation device of Example 10-3.
Fig. 13-4 shows blood glucose levels and Fig. 14-4 shows changes in body weight up to day 17 after transplantation using the transplantation device of Example 10-4.
Fig. 13-5 shows blood glucose levels and Fig. 14-5 shows changes in body weight up to day 17 after transplantation using the transplantation device of Example 10-5.
In Figs. 13-1 to 13-5 and 14-1 to 14-5, a # followed by a number represents the number of an individual mouse receiving transplantation.
There were no abnormal weight changes, and blood glucose levels remained normal for 12 to 17 days.

Evaluation of tissue reactivity

Tissue reactivity was evaluated according to Example 7.
Using the transplantation devices of Examples 10-1 to 10-5, the devices were removed 2 weeks after transplantation and observed for tissue reactivity, with the result that (1) there was no angiogenesis on the device surfaces, (2) there was no adhesion of the devices to the organs, peritoneum, omentum or the like, (3) the organs could be easily detached, and there was no direct adhesion to the organs, and (4) no inflammation or the like was observed on the organ side.
The devices were opened 2 weeks after transplantation, and the shape of the alginic acid gel in the device was confirmed. As a result, it was shown that an alginic acid gel in a transplantation device that is left for a long period of time in vivo still maintains its shape.

(Example 11) Evaluating oxygen permeability of transplantation device

[Oxygen permeability simulation]

Hydrogels 10 mm long by 20 mm wide and 1 mm thick in Example 11-1, 0.5 mm thick in Example 11-2 and 0.25 mm thick in Example 11-3 were subjected to an oxygen permeability simulation under the following conditions.
In the simulation, it was presumed that the oxygen concentration on the hydrogel surface was uniform and constant, that the specific cell respiration rate was constant, that oxygen consumption in the hydrogel was uniform irrespective of cell volume, and that oxygen diffusion occurred evenly from the entire surface of the gel to the center.
First, the reaction-diffusion equation for the oxygen concentration when the hydrogel is arranged in a cartesian coordinate system in air is as follows.

[Math. 5]

$$\frac{\partial C_{(x,y,z,t)}}{\partial t} = D_{Ae}(\frac{\partial^2 C_{(x,y,z,t)}}{\partial x^2} + \frac{\partial^2 C_{(x,y,z,t)}}{\partial y^2} + \frac{\partial^2 C_{(x,y,z,t)}}{\partial z^2}) - Q_{O_2}X$$

Given $Q_{02}X = r_{02}$ (0th order reaction), the oxygen concentration in a steady state (($\partial C/\partial t$) = 0) is calculated and represented by the following formula. $r_{02}$ was calculated using the specific respiratory rate of the pancreatic islets as described in Table 13, the total number of pancreatic islets contained in the hydrogel and the shape of the hydrogel.

[Math. 6]

$$\frac{\partial C_{(x,y,z,t)}}{\partial t} = D_{Ae}(\frac{\partial^2 C_{(x,y,z,t)}}{\partial x^2} + \frac{\partial^2 C_{(x,y,z,t)}}{\partial y^2} + \frac{\partial^2 C_{(x,y,z,t)}}{\partial z^2}) - Q_{O_2}X$$

Divided equally with the x direction as x = i$\Delta$x, the y direction as y = j$\Delta$y and the z direction as z = k$\Delta$z, the concentration is differentialized by the nodal values of C(x,y,z) = Ci,j,k to obtain the following formula, which is used for calculation. With the proviso that, $\Delta x = \Delta y = \Delta z = \Delta$.

[Math. 7]

$$C_{i,j,k} = \frac{1}{6}\left[C_{i-1,j,k} + C_{i+1,j,k} + C_{i,j+1,k} + C_{i,j-1,k} + C_{i,j,k+1} + C_{i,j,k-1} - \frac{\Delta^2 \cdot r_{O_2}}{D_{Ae}}\right]$$

The ratio of the oxygen concentrations on the hydrogel surface and in the center part was simulated by the above formula. The coefficients used in the simulation were as follows, with reference to Biotechnology and Bioengineering Vol. 96, Issue 5, pp 990-998 (2007) and Theoretical biology and Medical Modelling 6:5 (2009).

[Table 13]

| Coefficient | Value (unit) |
|---|---|
| Effective dispersion coefficient $D_{Ae}$ of oxygen in hydrogel | 2.7*10^-9 ($m^2/s$) |
| Dissolved oxygen concentration on gel surface | 3430 ($mg/m^3$) |
| Specific respiratory rate of pancreatic islets | 0.06*10^(-12) (mol/sec/IEQ) |
| Total number of pancreatic islets in hydrogel | 10000 (IEQ) |
| Hydrogel shape (L × W × T) | 10 mm × 20 mm × 1 mm<br>10 mm × 20 mm × 0.5 mm<br>10 mm × 20 mm × 0.25 mm |
| Δ | 0.2 mm<br>0.1 mm<br>0.05 mm |

Fig. 15-1 shows a hydrogel, while Figs. 15-2 to 15-4 show oxygen permeability in cross-section at the location indicated by the black arrow. Fig. 15-2 has a legend which also applies to Figs. 15-3 and 15-4. Fig. 15-2 shows the hydrogel of Example 11-1 with a thickness of 1 mm, in which oxygen permeability was 100% to 75% on the surface but the oxygen permeation rate in the center part was not more than 25%. In Fig. 15-3 showing the hydrogel of Example 11-2, the oxygen permeation rate in the center has been raised to 50% to 25% by changing the thickness to 0.5 mm, while in Fig. 15-4 showing the hydrogel of Example 11-3 with a thickness of 0.25 mm, the oxygen permeation rate in the center is 75% to 50%, or at least half the oxygen concentration on the surface.

(Example 12) Evaluation of substance permeability from transplantation device [Preparation of transplantation device]

Using Example 1e having an introduction rate of 2.2 mol% and Example 2e having an introduction rate of 2.4 mol% of the crosslinking group, a 2 wt% aqueous solution of Example 12-1e and a 2 wt% aqueous solution of Example 12-2e were prepared. Physiological saline was used for preparing the aqueous solutions.

The solution of Example 12-1e and the solution of Example 12-2e can be mixed at a volume ratio of 1:1 to prepare a 2 wt% alginic acid solution. 25 $\mu$l of a solution obtained by mixing this solution with physiological saline at a volume ratio of 1:1 was then mixed with 25 $\mu$l of physiological saline solution containing 0.01% Tween 20 as well as 500 ng of human insulin or 250 $\mu$g of glucose, for a total of 50 $\mu$l. This solution was then rapidly enclosed in a semipermeable membrane (SpectraPor CE dialysis tube (molecular weight cutoff 100,000), manufactured by Spectrum) which had been heat sealed at one end in advance and was then heat sealed at the other end after the alginic acid solution was added, and this was immersed for 10 to 15 minutes in a 55 mmol L$CaCl_2$ solution to gel the alginic acid solution in the device. The final alginic acid concentration was 0.5%. Transplantation devices prepared using this solution were used for the hydrogels of Example 12-1 (containing insulin) and Example 12-2 (containing glucose).

- Hydrogel size: L 10 mm × W 20 mm × Thickness about 0.25 mm

[Test methods]

The hydrogel of Example 12-1 or Example 12-2 was shaken for 24 hours at room temperature in 40 ml of saline solution containing 0.01% Tween20, the insulin concentration or glucose concentration in the external solution was measured, and the human insulin permeation rate or glucose permeation rate was calculated relative to 100% when the human insulin or glucose encapsulated in the hydrogel has diffused into physiological saline solution.

Fig. 16-1 shows the insulin concentration and Fig. 16-2 shows the glucose concentration in the external solution up to 24 hours after the start of the test using the transplantation device of Example 12. Both the human insulin and glucose permeated rapidly, and 100% was confirmed to have permeated after 24 hours under stirring, confirming that the transplantation device of the present invention has excellent human insulin and glucose permeability.

(Example 13) Evaluating collapsibility of hydrogel

[Preparing transplantation device]

The following saline solutions of Examples 13-1 to 13-5 were prepared first.

[Table 14]

| Example | Alginic acid solution |
|---|---|
| Solution of Example 13-1 | 0.5% sodium alginate aqueous solution (Mochida Pharmaceutical: B-2) |
| Solution of Example 13-2 | 1% sodium alginate aqueous solution (Mochida Pharmaceutical: B-2) |
| Solution of Example 13-3 | 2x dilution of solution obtained by mixing 1.0 wt% saline solution of alginic acid of Example 1e with 1.0 wt% saline solution of alginic acid of Example 2e at a volume ratio of 1:1 (0.5 wt% concentration of chemical crosslinking group) |
| Solution of Example 13-4 | Solution obtained by mixing 1.0 wt% saline solution of alginic acid of Example 1e with 1.0 wt% saline solution of alginic acid of Example 2e at a volume ratio of 1:1 (1 wt% concentration of chemical crosslinking group) |
| Solution of Example 13-5 | 2x dilution of a solution obtained by mixing 1.0 wt% saline solution of alginic acid of Example 1b with 1.0 wt% saline solution of alginic acid of Example 2b at a volume ratio of 1:1 (0.5 wt% concentration of chemical crosslinking group) |

The transplantation devices prepared with the solutions of Examples 13-1 to 13-5 were as follows.

<Transplantation devices>

Example 13-1: Prepared using 50 μl of the solution of Example 13-1, which was then rapidly enclosed in a semipermeable membrane (SpectraPor CE dialysis tube (molecular weight cutoff 100,000), manufactured by Spectrum, heat sealed at one end in advance and then heat sealed at the other end after solution is added), and then immersed for 10 to 15 minutes in a 55 mmol $LCaCl_2$ solution to gel the alginic acid solution in the device. The alginic acid gel was then removed from the enclosing membrane as the transplantation device.

Example 13-2: Transplantation device prepared as in the Example 13-1 using 200 μl of the solution of Example 13-2.

Example 13-3a: Transplantation device prepared as in the Example 13-1 using 50 μl of the solution of Example 13-3.

Example 13-3b: Transplantation device prepared as in the Example 13-1 using 100 μl of the solution of Example 13-3.

Example 13-3c: Transplantation device prepared as in the Example 13-1 using 200 μl of the solution of Example 13-3.

Example 13-4: Transplantation device prepared as in the Example 13-1 using 200 μl of the solution of Example 13-4.

Example 13-5a: Transplantation device prepared as in the Example 13-1 using 50 μl of the solution of Example 13-5.

Example 13-5b: Transplantation device prepared as in the Example 13-1 using 100 μl of the solution of Example 13-5.

Example 13-5c: Transplantation device prepared as in the Example 13-1 using 200 μl of the solution of Example 13-5.

[Test Methods]

Three of the transplantation devices of any of Examples 13-1 to 13-5c were added to 12 ml of 37°C saline in a 15 ml conical tube, and shaken back and forth at an amplitude of 25 mm and a shaking speed of 180 rpm with the temperature maintained at 37°C using a constant-temperature shaker (Taitec Corp. BR-43FL MR Bioshaker®). External solution was collected 1.5 hours, 6 hours, 24 hours and 96 hours after the start of testing, and the alginic acid concentration was assayed by the carbazole sulfate method. Following external solution collection 96 hours after the start of testing, the transplantation devices were also collected and lyase treated to dissolve the alginic acid gel, and the alginic acid concentration of the residual gel was assayed.

The alginic acid content of the device was determined from the alginic acid concentration in the external solution after completion of testing and the alginic acid concentration in the residual gel, and given this as 100%, the alginic acid content leaking from the device was calculated from the alginic acid concentration confirmed in the external solution at each point after the start of testing, and used as the collapse rate.

Fig. 17 shows the collapse rate up to 96 hours after the start of testing using the transplantation devices of Example 13. Table 15 shows the collapse rates 24 hours and 96 hours after the start of testing.

[Table 15]

| Example | Collapse rate after 24 hours | Collapse rate after 96 hours |
|---|---|---|
| Example 13-1 | 125% | 112% |
| Example 13-2 | 40% | 44% |
| Example 13-3a | 5% | 4% |
| Example 13-3b | 7% | 3% |
| Example 13-3c | 8% | 8% |
| Example 13-4 | 7% | 6% |
| Example 13-5a | 0% | 0% |
| Example 13-5b | 0% | 0% |
| Example 13-5c | 1% | 2% |

With the transplantation devices of Examples 13-1 and 13-2 using natural alginic acids, the alginic acid in the

hydrogel rapidly dissolved and the hydrogel collapsed. With the transplantation devices of the present invention (Examples 13-3 to 13-5), however, excellent stability was confirmed since there was very little elution of alginic acid and the hydrogels did not collapse.

(Example 14) Model test of cell shedding from alginic acid hydrogel

[Preparing transplantation devices]

The prepared transplantation device were as follows.

<Transplantation devices>

Example 14-1: Prepared by adding 5 μl of a solution of polystyrene beads (106-125 μm, Polysciences Inc., Cat. No. 19824) in place of pancreatic islet cells to 45 μl of the solution of Example 13-1, and then rapidly enclosing this in a semipermeable membrane (SpectraPor CE dialysis tube (molecular weight cutoff 100,000), manufactured by Spectrum, heat sealed at one end in advance and then heat sealed at the other end after solution addition), which was then immersed for 10 to 15 minutes in a 55 mmol $LCaCl_2$ solution to gel the alginic acid solution in the device. The alginic acid gel was then removed from the enclosing membrane as the transplantation device.
Example 14-2: Transplantation device prepared by adding 5μl of polystyrene beads in place of islet cells to 45 μl of the solution of Example 13-3a, and then preparing the device as in Example 14-1.

[Test Methods]

Three of the transplantation devices of Example 14-1 or Example 14-2 were added to 12 ml of 37°C saline in a 15 ml conical tube, and shaken back and forth at an amplitude of 25 mm and a shaking speed of 180 rpm with the temperature maintained at 37°C using a constant-temperature shaker (Taitec Corp. BR-43FL MR Bioshaker®). External solution was collected 24 hours after the start of testing, and the polystyrene beads shed into the external solution were observed with a microscope.
Fig. 18-1 shows a photograph of the external solution 24 hours after the start of testing using the transplantation device of Example 14-1, and Fig. 18-2 shows a photograph of the external solution 24 hours after the start of testing using the transplantation device of Example 14-2.
With the transplantation device of Example 14-1 using natural alginic acid, many beads were observed in the external solution, and it was confirmed that most of the microbeads had been shed into the external solution by 24 hours after the start of testing. With the transplantation device of the present invention (Example 14-2), on the other hand, very little shedding of microbeads was observed (shedding rate estimated to be not more than 10%), so it appears that cells are not shed over a long period of time, confirming excellent stability.
It is clear from the above that a transplantation device of a preferred embodiment exhibits at least one of the following effects.

(1) Excellent biocompatibility and stability, with little cytotoxicity and almost no adhesion or inflammation at the transplantation site.
(2) Shape maintained long-term, with little dissolution of the gel.
(3) Blood glucose lowering effects can be continued and blood glucose can be regulated for a long period of time.
(4) Device can be used over a long period of time because the alginic acid gel inside the semipermeable membrane can maintain its shape long-term without dissolving, and the pancreatic islets can survive and maintain their function.
(5) Can be a highly stable medical material that is replaceable and capable of immune isolation, with little adhesion, inflammation or the like.
(6) Substance permeability into and out of the device is excellent.
(7) A thin device with a hydrogel thickness of less than 500 μm has a higher cure rate in animals implanted with the device than a device with a thickness of 500 μm or more.
(8) In the case of a thin device with a hydrogel thickness of less than 500 μm, the ratio of the oxygen concentration of the center part to the concentration of the device surface is higher than when the thickness is over 500 μm.
(9) The hydrogel does not collapse or hardly collapses when the hydrogel is shaken.
(10) There is little shedding of islet cells contained in the hydrogel when the hydrogel is shaken.

A transplantation device of a more preferred embodiment provides excellent transplantation results and functionality, is novel in terms of the material, and can have a sustained blood glucose lowering effect and regulate blood glucose long-term when transplanted into diabetes patients (especially type I diabetes patients and insulin-depleted type

II diabetes patients). It can also be collected when the functions of the insulin-secreting cells or pancreatic islets in the hydrogel have declined. Periodic replacement or additional transplantation is also possible. Furthermore, insulin-secreting cells differentiated from stem cells (iPS cells or the like), or human pancreatic islets may be used as the insulin-secreting cells or pancreatic islets that are enclosed in the hydrogel of the transplantation device. The transplantation device of a more preferred embodiment is thus useful.

## Claims

1. A transplantation device comprising insulin-secreting cells or pancreatic islets enclosed in a hydrogel,

   wherein the hydrogel is an alginic acid derivative that has been gelled by chemical crosslinking, and
   wherein the thickness of the hydrogel is from 100 μm to less than 500 μm.

2. The transplantation device according to Claim 1, wherein the hydrogel comprises, as crosslinking, chemical crosslinking formed between alginic acid derivatives.

3. The transplantation device according to Claim 1 or 2, wherein the hydrogel comprises, as crosslinking, chemical crosslinking by triazole rings formed by a Huisgen reaction.

4. The transplantation device according to any one of Claims 1 to 3, wherein the chemical crosslinking is chemical crosslinking by cyclic alkyne groups introduced at any one or more carboxyl groups of alginic acid and azide groups introduced at any one or more carboxyl groups of alginic acid.

5. The transplantation device according to any one of Claims 1 to 4,
   wherein the chemical crosslinking is chemical crosslinking by a combination of alginic acid derivatives according to (A) and (B) below:

   (A): an alginic acid derivative represented by formula (HA-1) below, comprising a cyclic alkyne group (Akn) introduced via an amide bond and a divalent linker ($-L^1-$) at any one or more carboxyl group groups of alginic acid:

[C73]

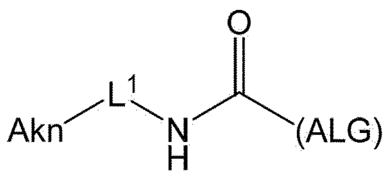

(I)

[in the formula (HA-1), (ALG) represents alginic acid; -NHCO- represents an amide bond via any carboxyl group of alginic acid; $-L^1-$ represents a divalent linker selected from the group consisting of following partial structural formulae (excluding parts outside dashed lines at both ends of each formula):

[C74]

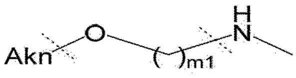

(LN-1) m1=2-6

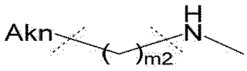

(LN-2) m2=1-6

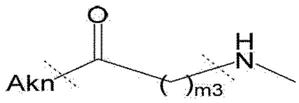

(LN-3) m3=1-6

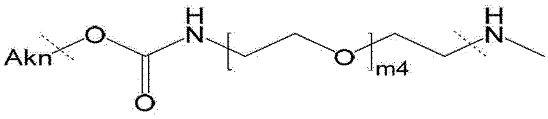

(LN-4) m4=1-6

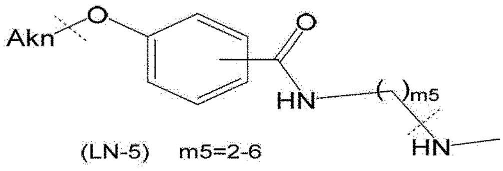

(LN-5) m5=2-6

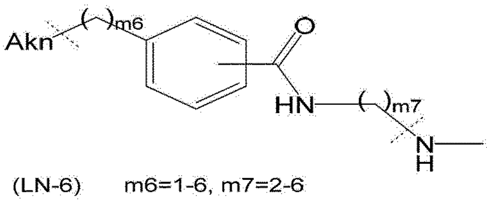

(LN-6) m6=1-6, m7=2-6

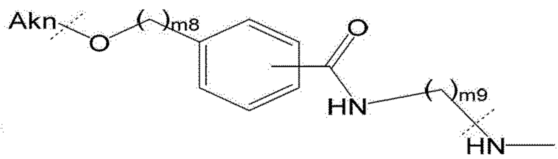

(LN-7) m8=1-6, m9=2-6

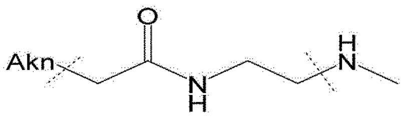

(LN-8)

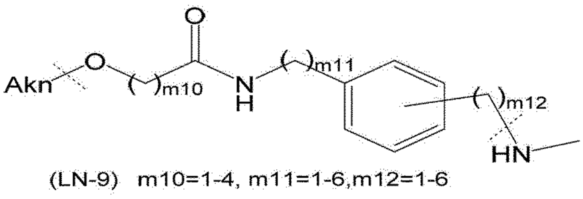

(LN-9) m10=1-4, m11=1-6,m12=1-6

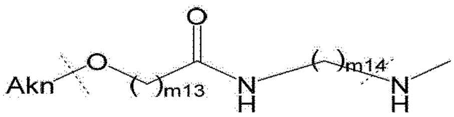

m13=1-4, m14=2~6

(LN-10)

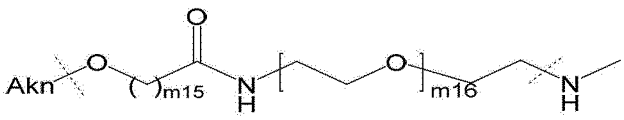

m15=1-4, m16=1-6

(LN-11)

and Akn is cyclic alkyne group selected from the following partial structural formulae (excluding part to the right of the dashed line in each formula), in which asterisks represent chiral centers]:

[C75]

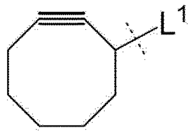
(AK-1)

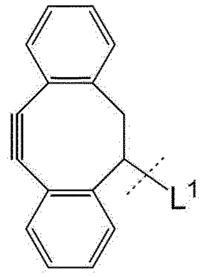
(AK-2)

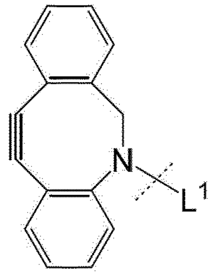
(AK-3)

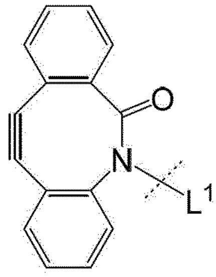
(AK-4)

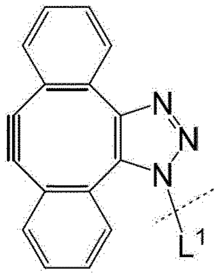
(AK-5)

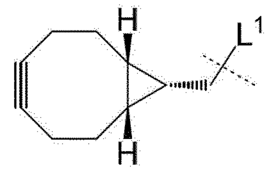
(AK-6)

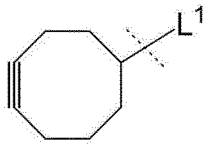
(AK-7)

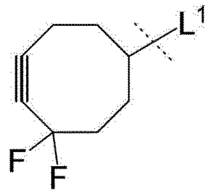
(AK-8)

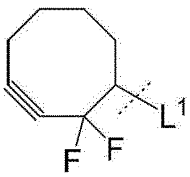
(AK-9)

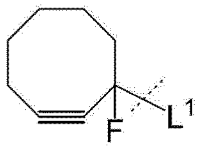
(AK-10)

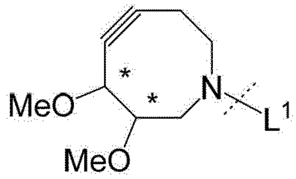
(AK-11)

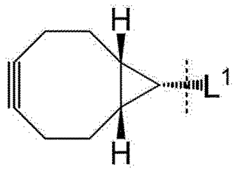
(AK-12)

(B): an alginic acid derivative represented by formula (HA-II) below, comprising an azide group introduced via an amide bond and a divalent linker ($-L^2-$) at any one or more carboxyl groups of alginic acid:

[C76]

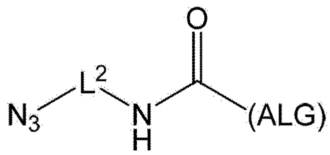
(II)

[in the formula (HA-II), (ALG) represents alginic acid; -NHCO- represents an amide bond via any carboxyl group of alginic acid; and $-L^2-$ represents a divalent linker selected from the group consisting of following partial structural formulae (excluding parts outside dashed lines at both ends of each formula)]:

[C77]

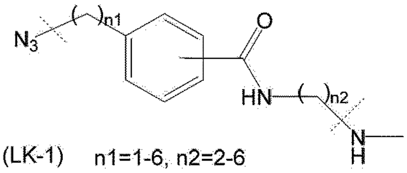

(LK-1) n1=1-6, n2=2-6

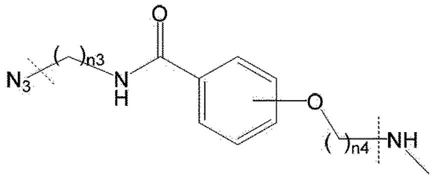

(LK-2) n3=2-6, n4=2-6

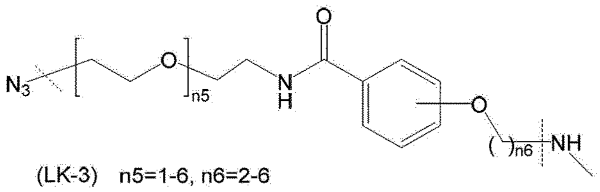

(LK-3) n5=1-6, n6=2-6

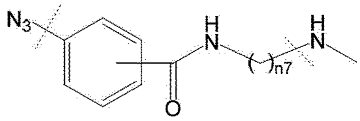

(LK-4) n7=2-6

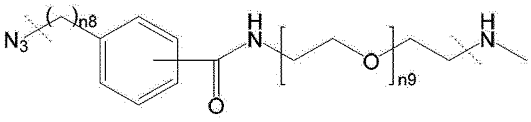

(LK-5) n8=1-4, n9=1-6

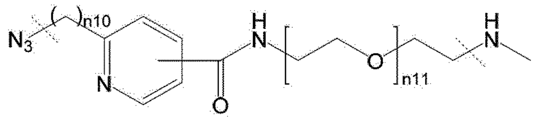

(LK-6) n10=1-4, n11=1-6

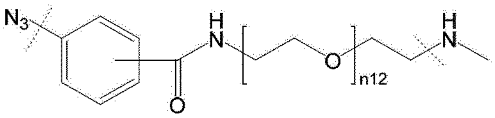

(LK-7) n12=1-6

.

**6.** The transplantation device according to Claim 5, wherein the chemically crosslinked alginic acid derivative is a crosslinked alginic acid in which any carboxyl group of a first alginic acid and any carboxyl group of a second alginic acid are linked via following formula (HA-III-L):

[C78]

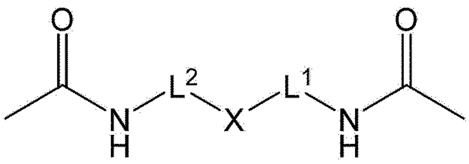

(III-L)

[in the formula (HA-III-L), -CONH- and -NHCO- at either end represent amide bonds via any carboxyl group of alginic acid;

-$L^1$- is defined as in Claim 5;

-$L^2$- is defined as in Claim 5; and X is a cyclic group selected from the group consisting of following partial structural formulae (excluding parts outside dashed lines at both ends of each formula), in which asterisks represent chiral centers]:

[C79]

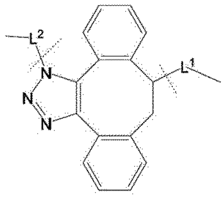
(TZ-1)

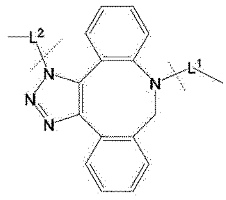
(TZ-2)

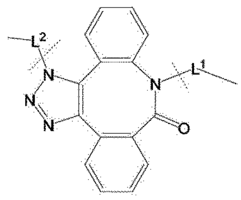
(TZ-3)

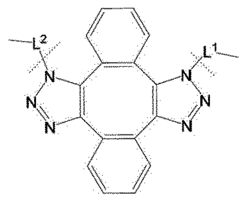
(TZ-4)

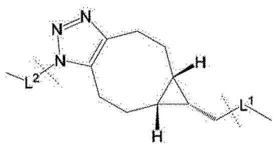
(TZ-5)

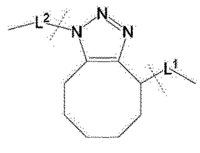
(TZ-6)

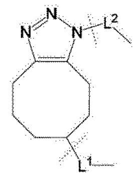
(TZ-7)

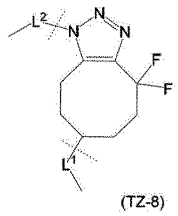
(TZ-8)

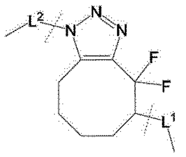
(TZ-9)

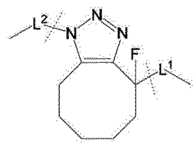
(TZ-10)

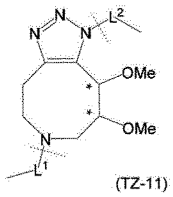
(TZ-11)

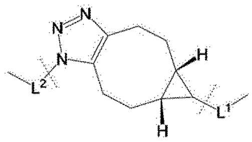
(TZ-12)

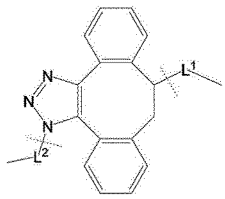
(TZ-1-r)

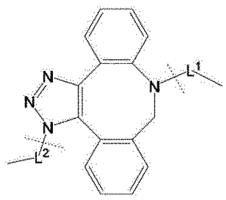
(TZ-2-r)

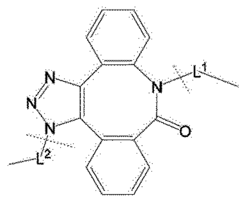
(TZ-3-r)

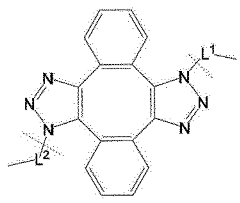
(TZ-4-r)

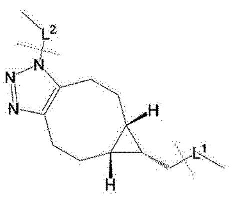
(TZ-5-r)

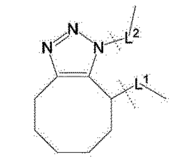
(TZ-6-r)

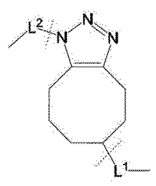
(TZ-7-r)

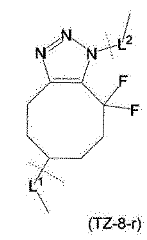
(TZ-8-r)

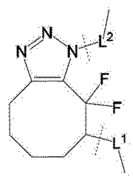
(TZ-9-r)

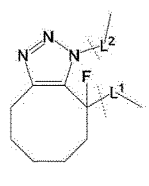
(TZ-10-r)

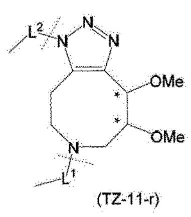
(TZ-11-r)

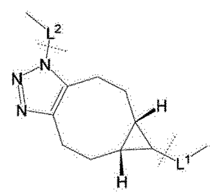
(TZ-12-r)

.

**7.** The transplantation device according to any one of Claims 1 to 4, wherein the chemical crosslinking is chemical crosslinking by a combination of alginic acid derivatives according to (A) and (B) below:

(A): an alginic acid derivative represented by formula (HB-I) below, comprising a cyclic alkyne (Akn) introduced via an amide bond and a divalent linker ($-L^1-$) at any one or more carboxyl group groups of alginic acid:

[C80]

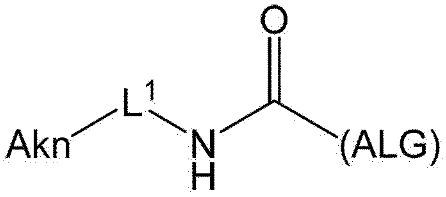

(I)

[in the formula (HB-1), (ALG) represents alginic acid; -NHCO- represents an amide bond via any carboxyl group of alginic acid; $-L^1-$ represents a linker selected from the group consisting of partial structural formulae shown in following tables [excluding parts outside the dashed lines at both ends of each formula];

[Table 16-1]

| No. | $-L^1-$ | |
|---|---|---|
| (L1-1) | 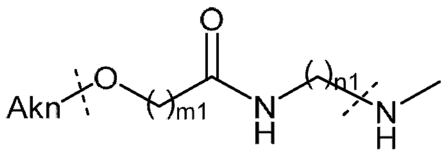 | m1=1-6<br>n1=2-6 |
| (L1-2a) | 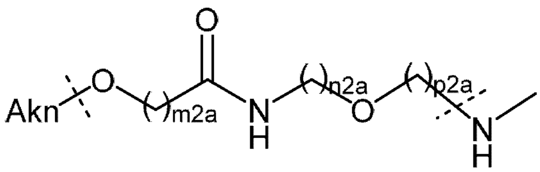 | m2a=1-6<br>n2a=2-6<br>p2a=2-6 |
| (L1-2b) | 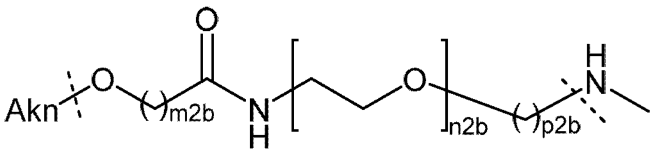 | m2b=1-6<br>n2b=1-6<br>p2b=2-6 |
| (L1-3) | 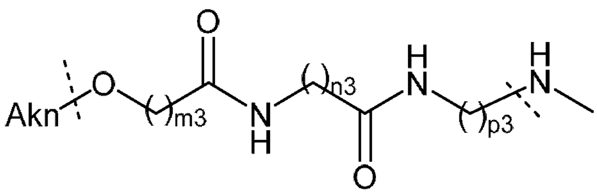 | m3=1-6<br>n3=1-6<br>p3=2-6 |
| (L1-4a) | 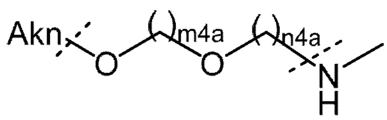 | m4a=2-6<br>n4a=2-6 |
| (L1-4b) | 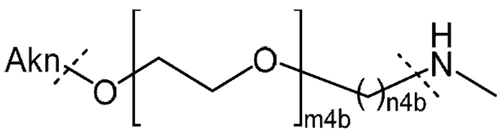 | m4b=0-6<br>n4b=2-6 |
| (L1-5a) | 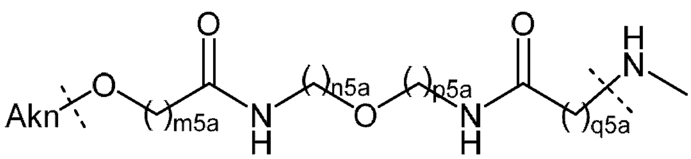 | m5a=1-6<br>n5a=2-6<br>p5a=2-6<br>q5a=1-6 |

(continued)

| No. | -$L^1$- | |
|---|---|---|
| (L1-5b) | 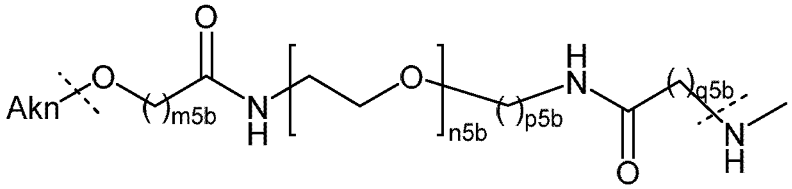 | m5b=1-6<br>n5b=1-6<br>p5b=2-6<br>q5b=1-6 |
| (L1-6a) | 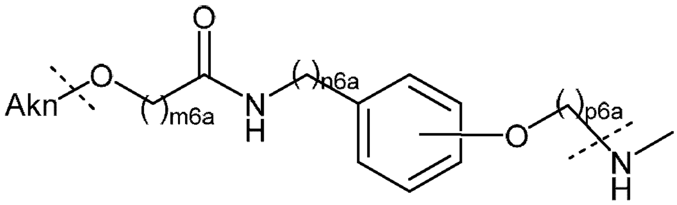 | m6a=1-6<br>n6a=0-6<br>p6a=2-6 |
| (L1-6b) | 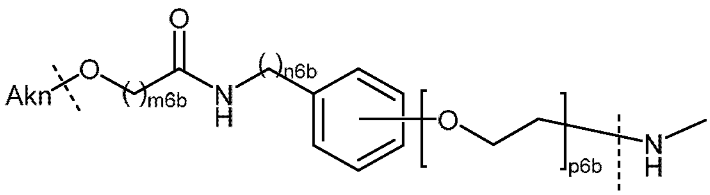 | m6b=1-6<br>n6b=1-6<br>p6b=2-6 |

[Table 16-2]

| No. | -$L^1$- | |
|---|---|---|
| (L1-7) | 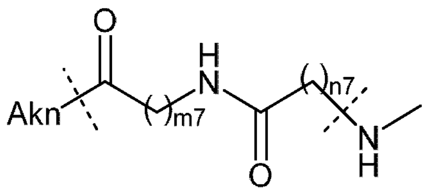 | m7=1-6<br>n7=1-6 |
| (L1-8a) | 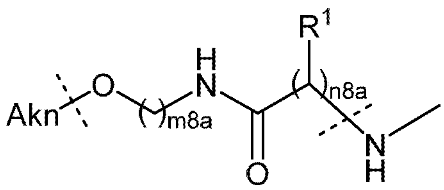 | m8a=2-6<br>n8a=1-6<br>$R^1$=H, Me, Et, Bn |
| (L1-8b) | 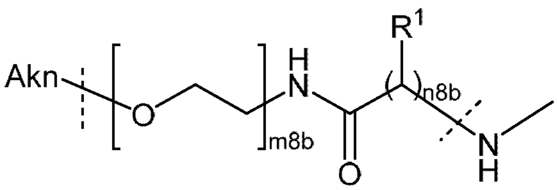 | m8b=1-6<br>n8b=1-6<br>$R^1$=H, Me, Et, Bn |
| (L1-9a) | 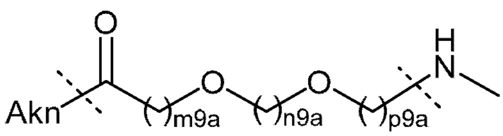 | m9a=1-6<br>n9a=2-6<br>p9a=2-6 |

(continued)

| No. | $-L^1-$ | |
|---|---|---|
| (L1-9b) | 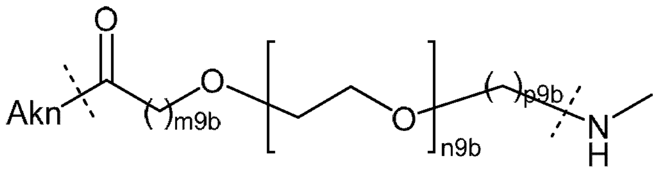 | m9b=1-6<br>n9b=1-6<br>p9b=2-6 |
| (L1-10) | 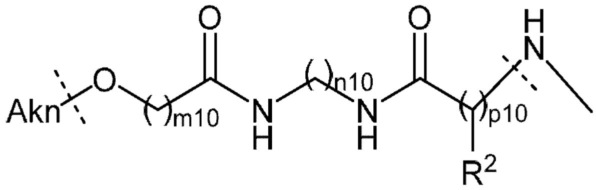 | m10=1-6<br>n10=2-6<br>p10=1-6<br>$R^2$=H, Me, Et, Bn |
| (L1-11) | 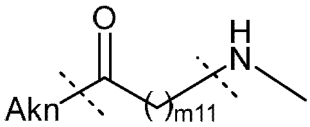 | m11 =2-6 |
| (L1-12) | 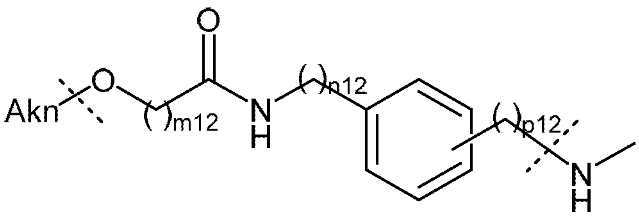 | m12=1-6<br>n 12=0-6<br>p12=1-6 |

and Akn is a cyclic alkyne selected from the group consisting of the partial structural formula described in a following table (excluding a part to the right of a dashed line in each formula)]:

[Table 17]

| No. | Akn | No. | Akn | No. | Akn |
|---|---|---|---|---|---|
| (AK-1) | 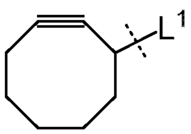 | (AK-5) | 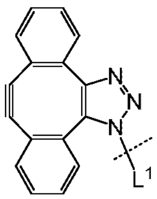 | (AK-9) | 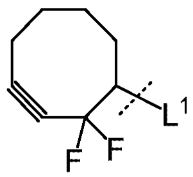 |
| (AK-2) | 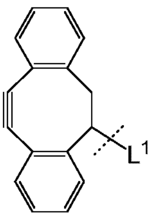 | (AK-6) | 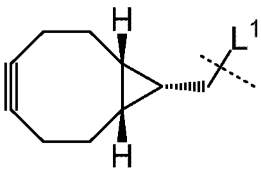 | (AK-10) | 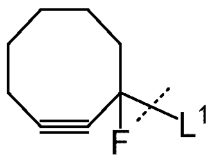 |
| (AK-3) | 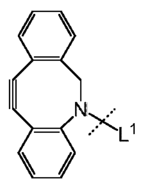 | (AK-7) | 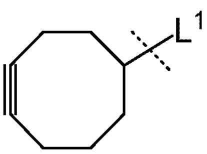 | (AK-11) | 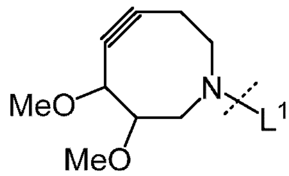 |

(continued)

| No. | Akn | No. | Akn | No. | Akn |
|---|---|---|---|---|---|
| (AK-4) | 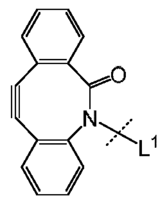 | (AK-8) | 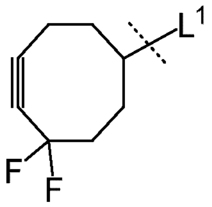 | (AK-12) | 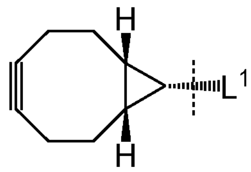 |

(B): an alginic acid derivative represented by formula (HB-II) below, comprising an azide group introduced via an amide bond and a divalent linker (-$L^2$-) at any one or more carboxyl groups of alginic acid:

[C81]

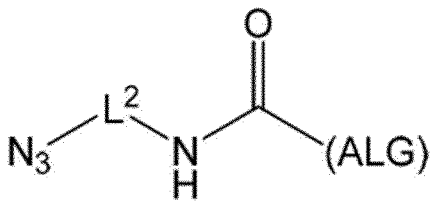

(II)

[in the formula (HB-II), (ALG) represents alginic acid; -NHCO- represents an amide bond via any carboxyl group of alginic acid; and -$L^2$- represents a linker selected from the group consisting of partial structural formulae shown in following tables (excluding parts outside dashed lines at both ends of each formula)]:

[Table 18-1]

| No. | -$L^2$- | |
|---|---|---|
| (L2-1) | 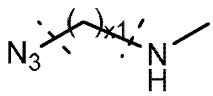 | x1=2-6 |
| (L2-2a) | 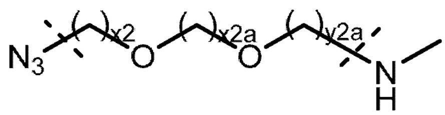 | x2=2-6<br>x2a=2-6<br>y2a=2-6 |
| (L2-2b) | 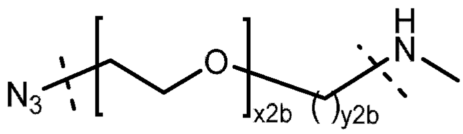 | x2b=1-6<br>y2b=2-6 |
| (L2-3) | 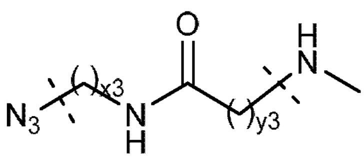 | x3=2-6<br>y3=1-6 |
| (L2-4) | 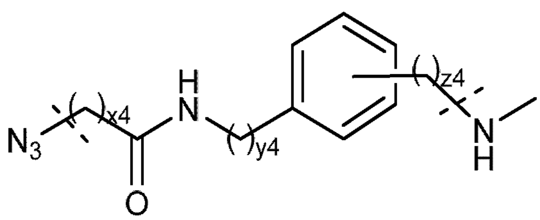 | x 4=1-6<br>y4=1-6<br>z4=1-6 |

(continued)

| No. | -L²- | |
|---|---|---|
| (L2-5a) | 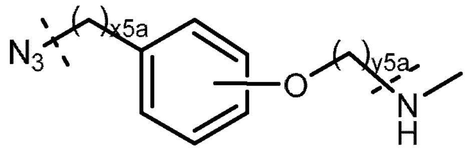 | x5a=0-4<br>y5a=2-6 |
| (L2-5b) | 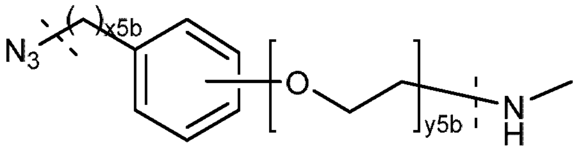 | x5b=0-4<br>y5b=1-6 |
| (L2-6a) | 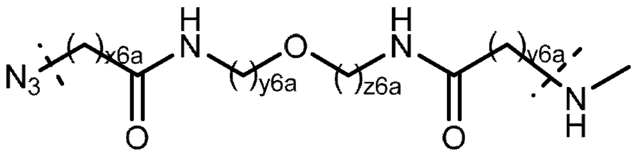 | x6a=1-6<br>y6a=2-6<br>z6a=2-6<br>v6a=1-6 |
| (L2-6b) | 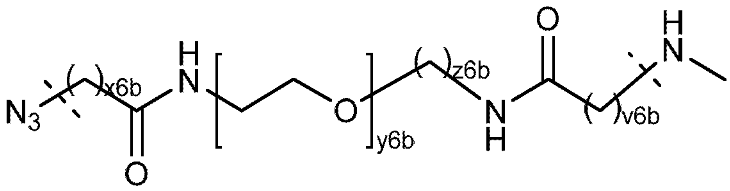 | x6b=1-6<br>y6b=1-6<br>z6b=2-6<br>v6b=1-6 |

[Table 18-2]

| No. | -L²- | |
|---|---|---|
| (L2-7a) | 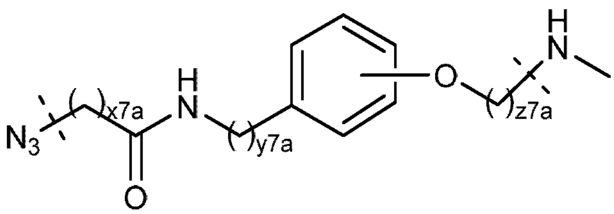 | x7a=1-6<br>y7a=1-6<br>z7a=2-6 |
| (L2-7b) | 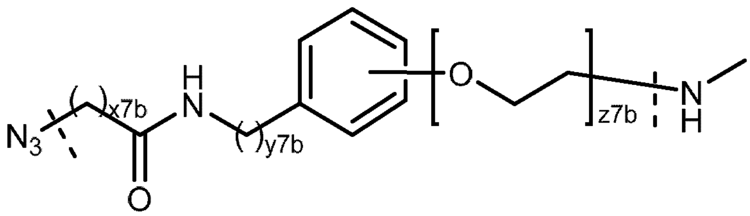 | x7b=1-6<br>y7b=1-6<br>z7b=1-6 |
| (L2-8a) | 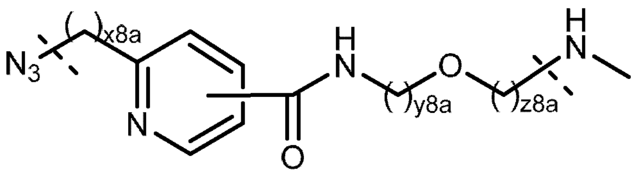 | x8a=0-4<br>y8a=2-6<br>z8a=3-6 |

(continued)

| No. | $-L^2-$ | |
|---|---|---|
| (L2-8b) | 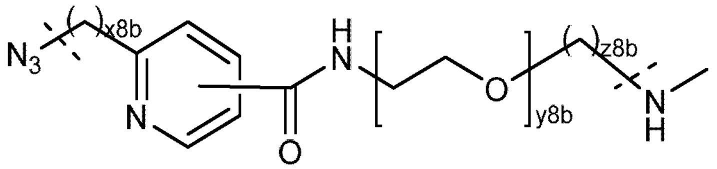 | x8b=0-4 y8b=1-6 z8b=3-6 |
| (L2-9a) | 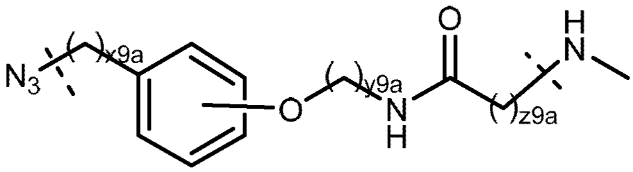 | x9a=0-4 y9a=2-6 z9a=1-6 |
| (L2-9b) | 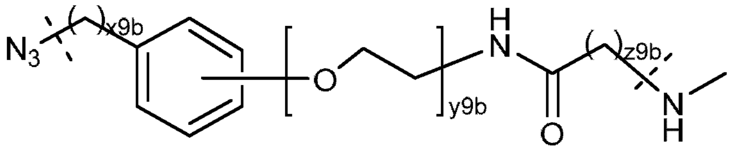 | x9b=0-4 y9b=1-6 z9b=1-6 |
| (L2-10) | 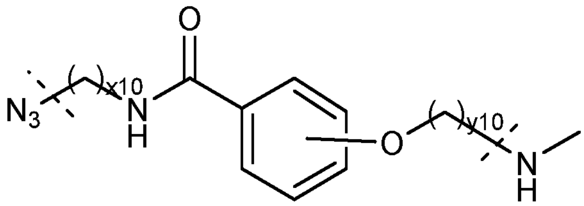 | x10=2-6 y10=2-6 |

(with the proviso that excluding chemical crosslinking by a combination of a derivative in which the $-L^1-$ in the alginic acid derivative represented by formula (HB-I) is any one linker selected from the group consisting of (L1-1), (L1-2a), (L1-2b), (L1-11) and (L1-12) and a derivative in which $-L^2-$ in the alginic acid derivative represented by formula (HB-II) is the linker of (L2-10)).

**8.** The transplantation device according to Claim 7, wherein the chemically crosslinked alginic acid derivative is a crosslinked alginic acid in which any carboxyl group of a first alginic acid and any carboxyl group of a second alginic acid are linked via following formula (HB-III-L):

[C82]

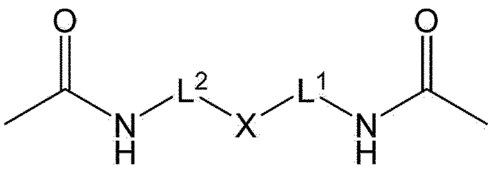

(III-L)

[in the formula (HB-III-L), the -CONH- and -NHCO- at either end represent amide bonds via any carboxyl group of alginic acid;

$-L^1-$ is defined as in Claim 7;

$-L^2-$ is defined as in Claim 7; and X is a cyclic group selected from the group consisting of partial structural formulae shown in following tables (excluding parts outside dashed lines at both ends of each formula)]:

[Table 19-1]

| No. | X | No. | X |
|---|---|---|---|
| (TZ-1) | 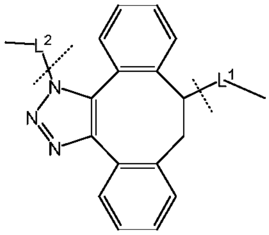 | (TZ-1-r) | 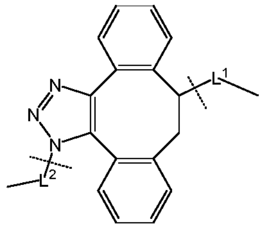 |
| (TZ-2) | 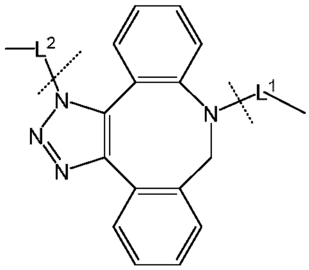 | (TZ-2-r) | 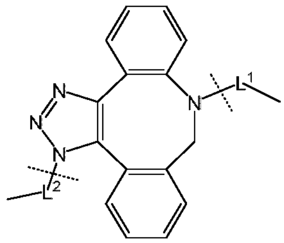 |
| (TZ-3) | 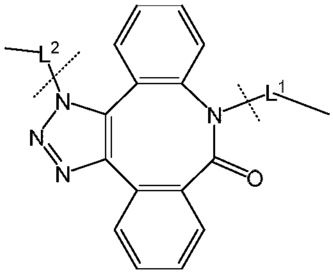 | (TZ-3-r) | 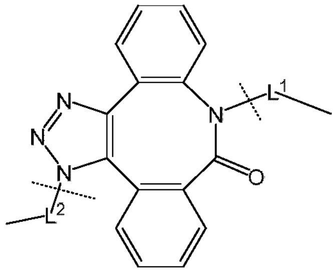 |
| (TZ-4) | 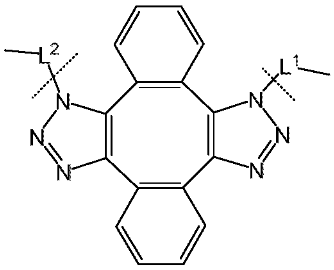 | (TZ-4-r) | 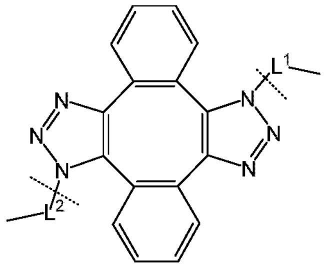 |
| (TZ-5) | 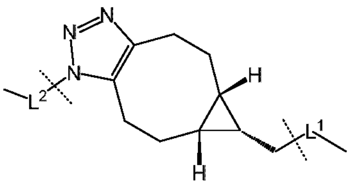 | (TZ-5-r) | 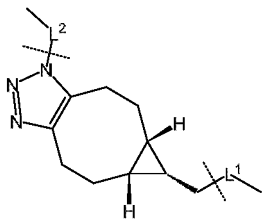 |
| (TZ-6) | 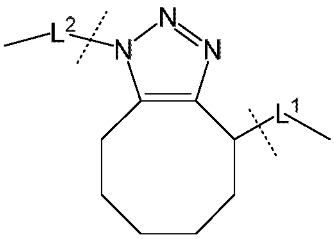 | (TZ-6-r) | 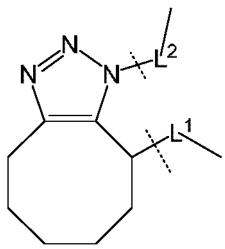 |

[Table 19-2]

| No. | X | No. | X |
|---|---|---|---|
| (TZ-7) | 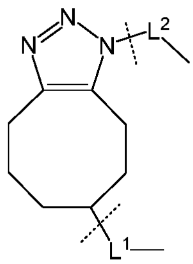 | (TZ-7-r) | 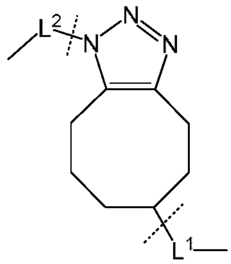 |
| (TZ-8) | 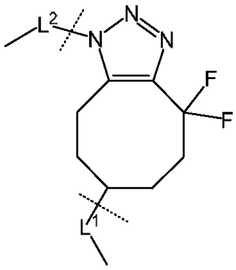 | (TZ-8-r) | 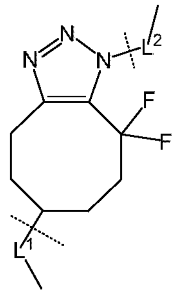 |
| (TZ-9) | 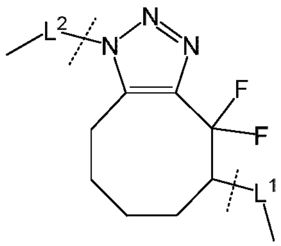 | (TZ-9-r) | 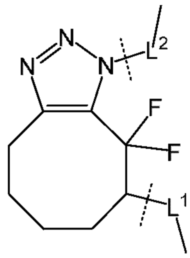 |
| (TZ-10) | 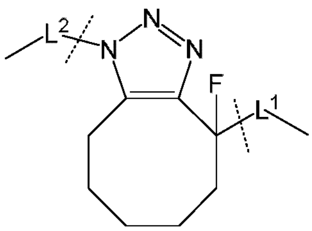 | (TZ-10-r) | 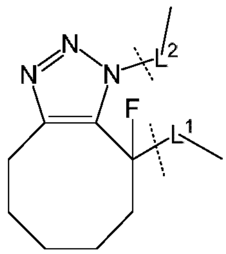 |
| (TZ-11) | 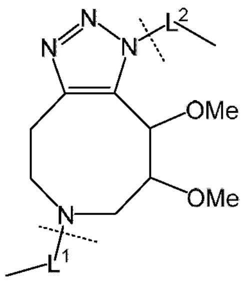 | (TZ-11-r) | 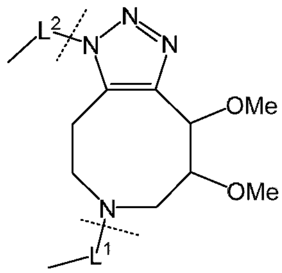 |

(continued)

| No. | X | No. | X |
|---|---|---|---|
| (TZ-12) | 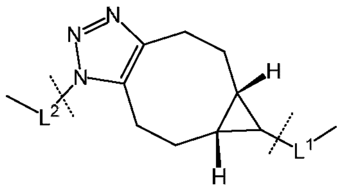 | (TZ-12-r) | 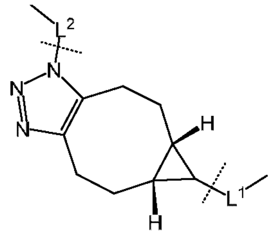 |

(with the proviso that excluding chemically crosslinked alginic acids formed from a combination of a derivative in which the -$L^1$- in the alginic acid derivative represented by formula (HB-1) is any one linker selected from the group consisting of (L1-1), (L1-2a), (L1-2b), (L1-11) and (L1-12) and a derivative in which -$L^2$- in the alginic acid derivative represented by formula (HB-II) is the linker of (L2-10)).

**9.** The transplantation device according to Claim 5 or 6,
wherein the alginic acid derivative of the formula (HA-1) is the following formula (EX-1-(I)-A-2),

[C83]

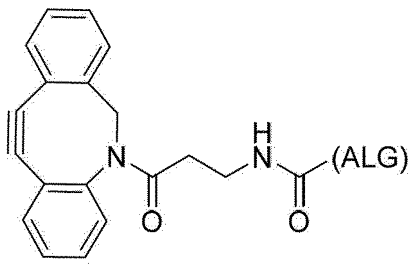

EX1-(I)-A-2

and the alginic acid of the formula (HA-II) is the following formula (EX-2-(II)-A-2),

[C84]

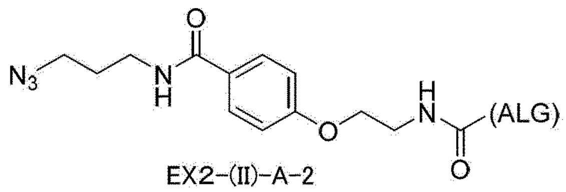

EX2-(II)-A-2

.

**10.** The transplantation device according to any one of Claims 1 to 9, wherein the pancreatic islets are human pancreatic islets or pig pancreatic islets.

**11.** The transplantation device according to Claim 10, wherein the pancreatic islets are pancreatic islets of an adult pig.

**12.** The transplantation device according to Claim 10, wherein the pancreatic islets are fetal, neonatal or perinatal pig pancreatic islets.

**13.** The transplantation device according to any one of Claims 1 to 12, wherein the hydrogel is further encapsulated in

a semipermeable membrane.

14. The transplantation device according to Claim 13, wherein the semipermeable membrane is a dialysis membrane formed from a cellulose derivative.

15. The transplantation device according to Claim 14, wherein the cellulose derivative is cellulose acetate.

Fig. 1

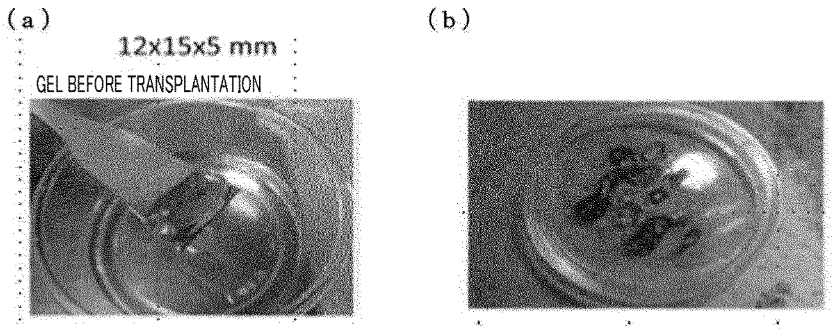

Fig. 2

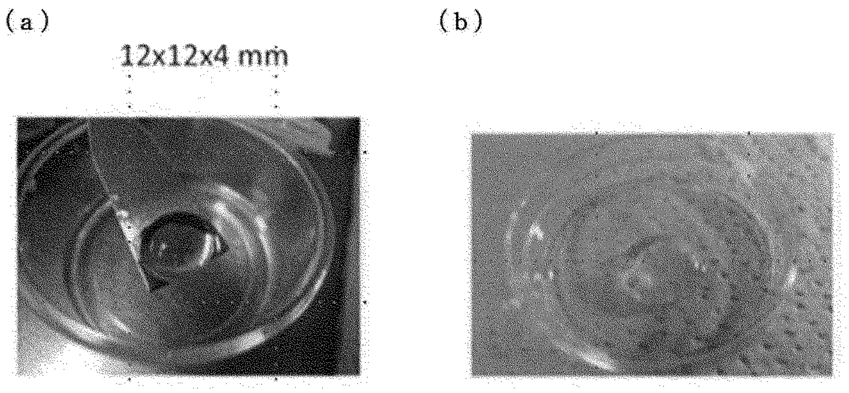

Fig. 3

(a)

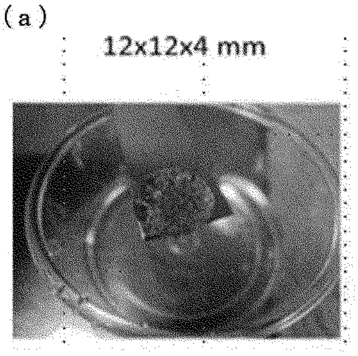


(b)

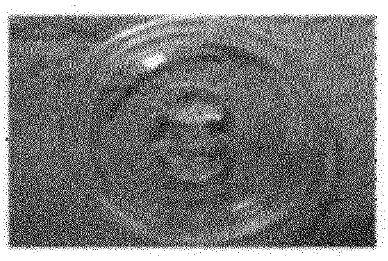

Fig. 4-1

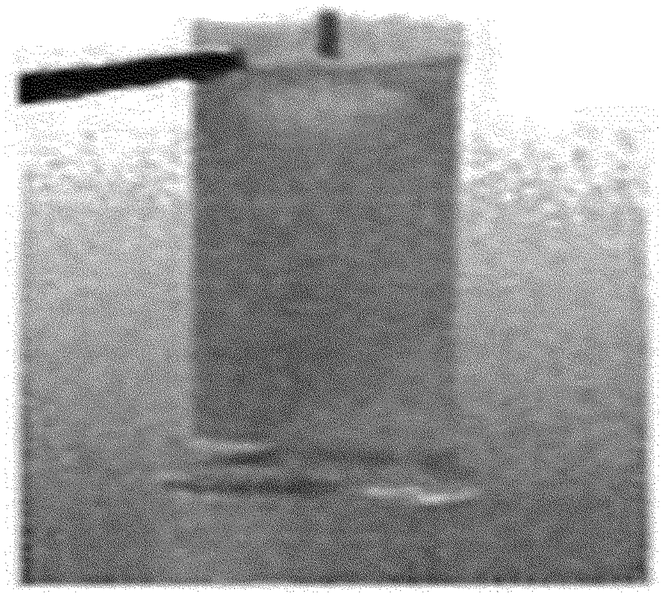

Fig. 4-2

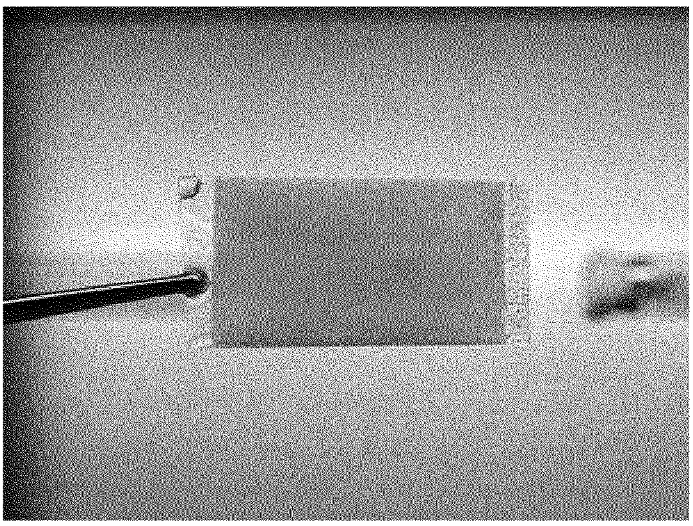

Fig. 5-1

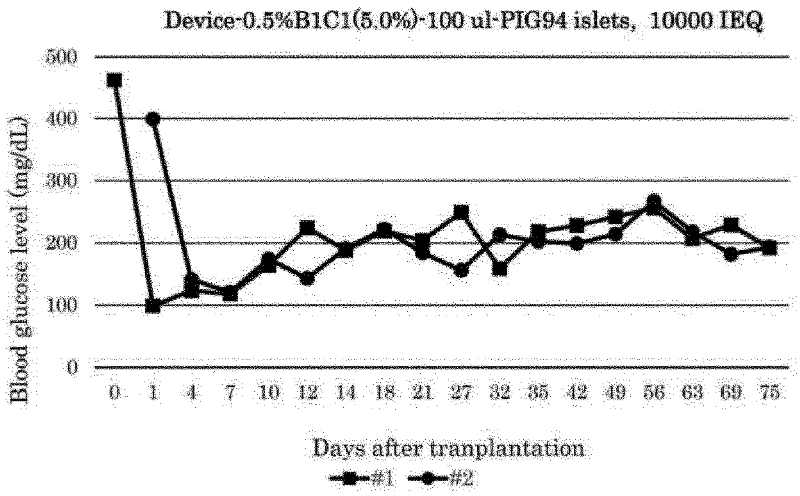
Device-0.5%B1C1(5.0%)-100 ul-PIG94 islets, 10000 IEQ
Blood glucose level (mg/dL)
500
400
300
200
100
0
0 1 4 7 10 12 14 18 21 27 32 35 42 49 56 63 69 75
Days after tranplantation
#1 #2

Fig. 5-2

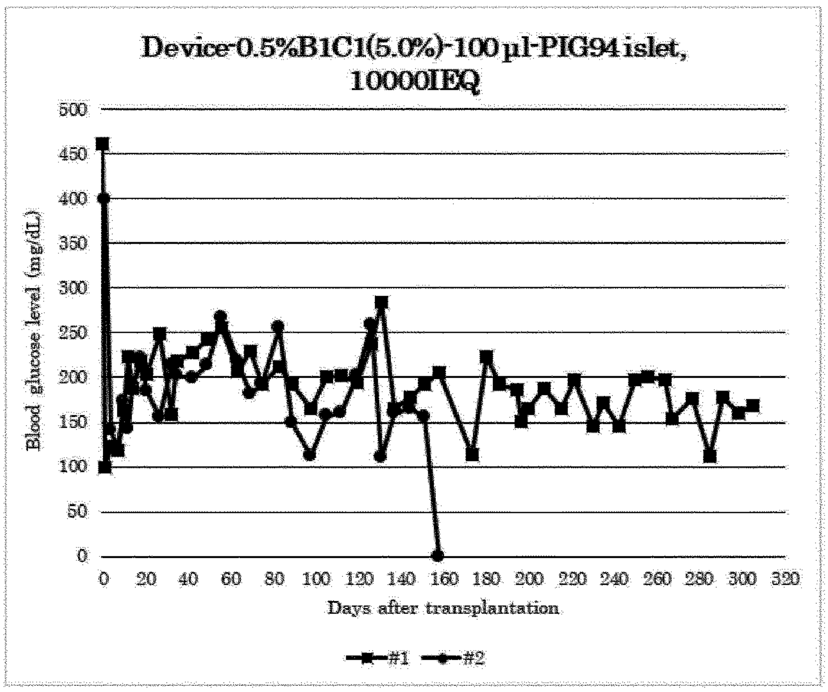
Device-0.5%B1C1(5.0%)-100µl-PIG94 islet, 10000IEQ
Blood glucose level (mg/dL)
500
450
400
350
300
250
200
150
100
50
0
0 20 40 60 80 100 120 140 160 180 200 220 240 260 280 300 320
Days after transplantation
#1 #2

Fig. 5-3

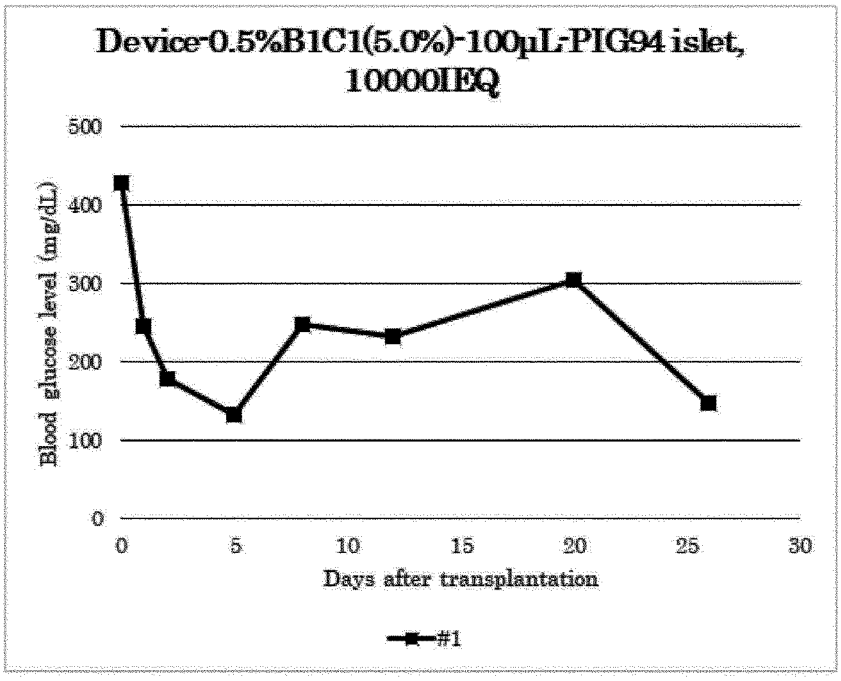
Device-0.5%B1C1(5.0%)-100μL-PIG94 islet, 10000IEQ
500
400
300
200
100
0
Blood glucose level (mg/dL)
0
5
10
15
20
25
30
Days after transplantation
#1

Fig. 6-1

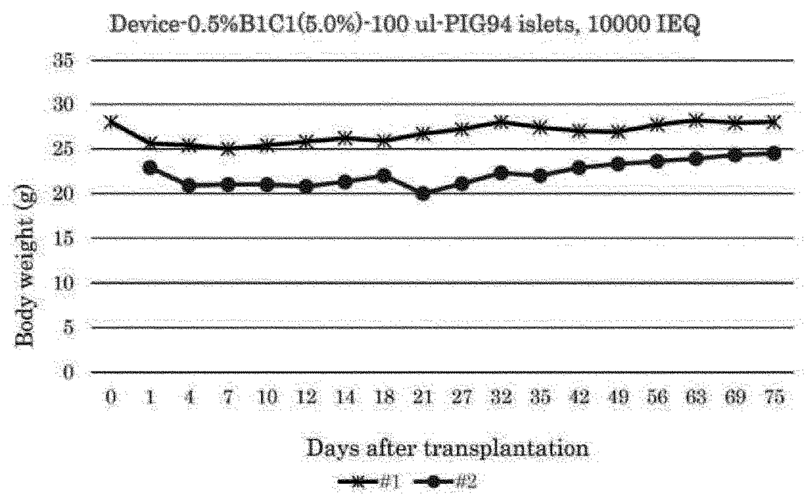
Device-0.5%B1C1(5.0%)-100 ul-PIG94 islets, 10000 IEQ
Body weight (g)
35
30
25
20
15
10
5
0
0 1 4 7 10 12 14 18 21 27 32 35 42 49 56 63 69 75
Days after transplantation
#1 #2

Fig. 6-2

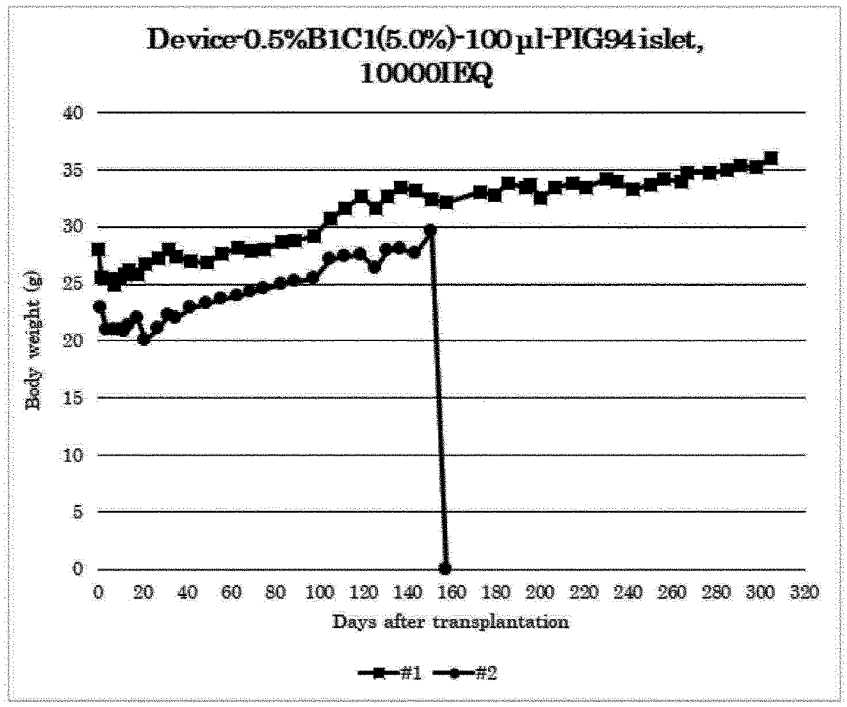
Device-0.5%B1C1(5.0%)-100 µl-PIG94 islet, 10000IEQ
Body weight (g)
40
35
30
25
20
15
10
5
0
0 20 40 60 80 100 120 140 160 180 200 220 240 260 280 300 320
Days after transplantation
#1 #2

Fig. 6-3

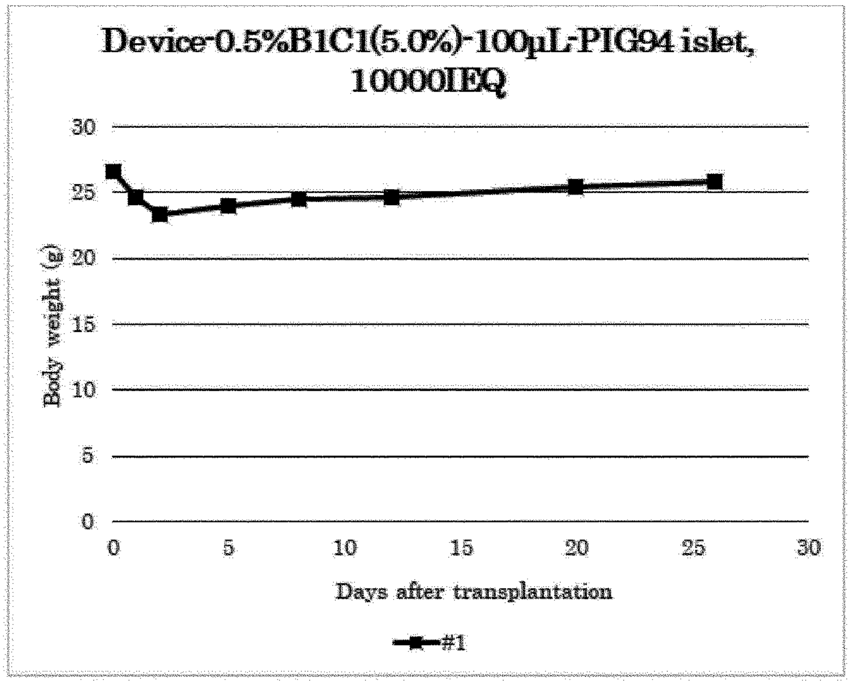
Device-0.5%B1C1(5.0%)-100μL-PIG94 islet, 10000IEQ
Body weight (g)
30
25
20
15
10
5
0
0
5
10
15
20
25
30
Days after transplantation
#1

Fig. 7-1

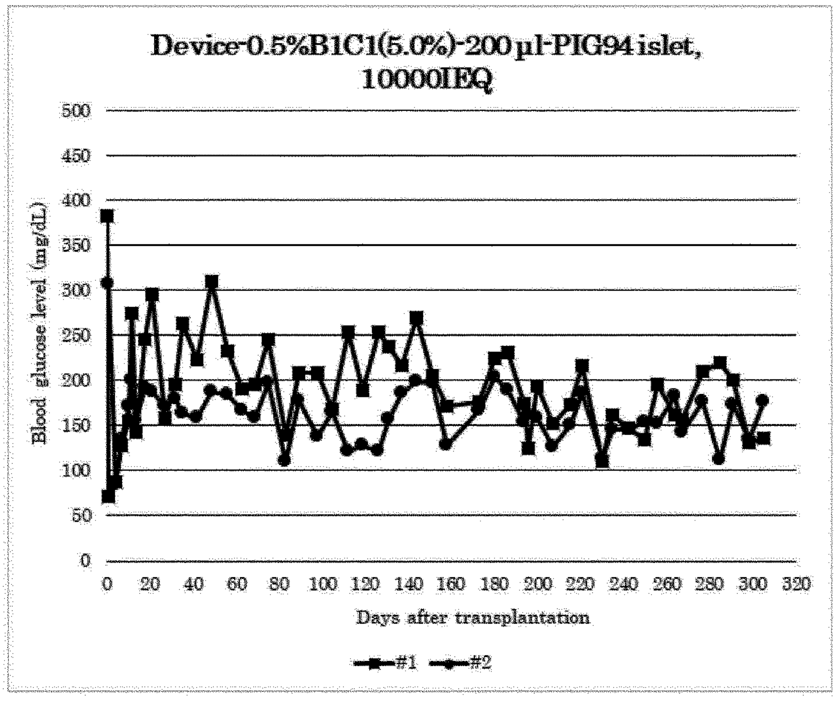
Device-0.5%B1C1(5.0%)-200 µl-PIG94 islet, 10000IEQ
Blood glucose level (mg/dL)
0
50
100
150
200
250
300
350
400
450
500
0 20 40 60 80 100 120 140 160 180 200 220 240 260 280 300 320
Days after transplantation
#1
#2

Fig. 7-2

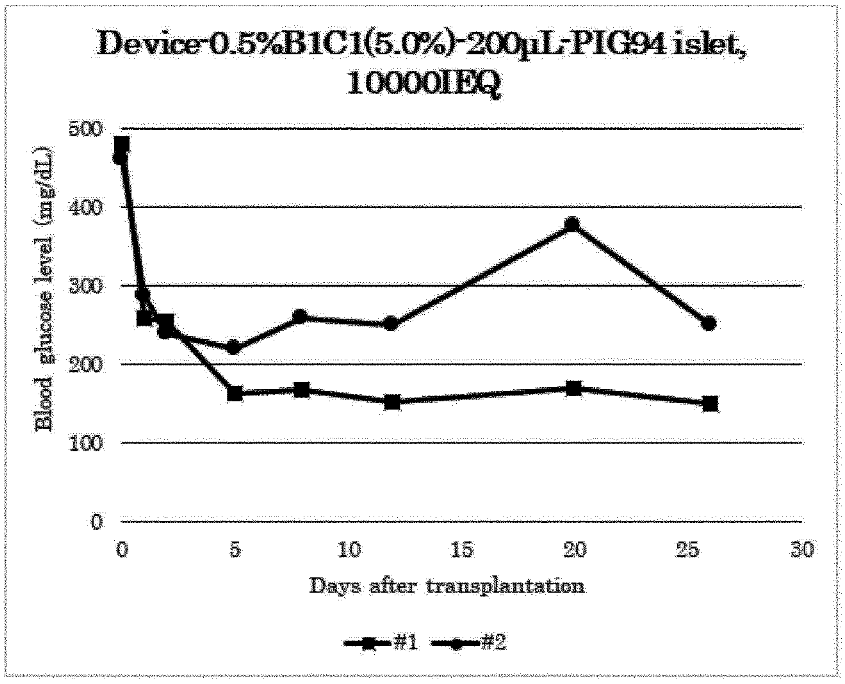
Device-0.5%B1C1(5.0%)-200µL-PIG94 islet, 10000IEQ
Blood glucose level (mg/dL)
0
100
200
300
400
500
0 5 10 15 20 25 30
Days after transplantation
#1
#2

Fig. 8-1

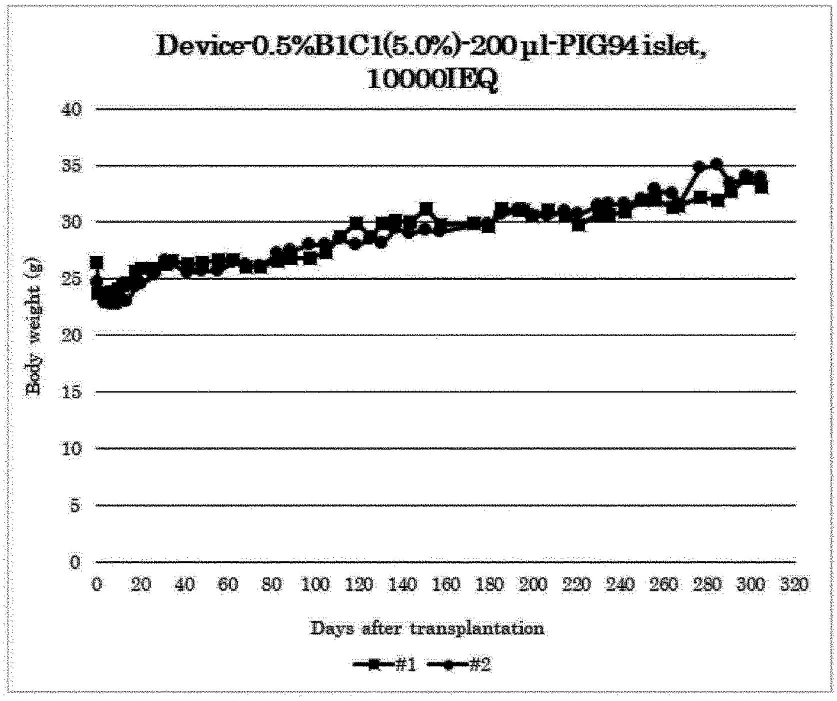
Device-0.5%B1C1(5.0%)-200 µl-PIG94 islet, 10000IEQ
40
35
30
25
20
15
10
5
0
Body weight (g)
0 20 40 60 80 100 120 140 160 180 200 220 240 260 280 300 320
Days after transplantation
#1
#2

Fig. 8-2

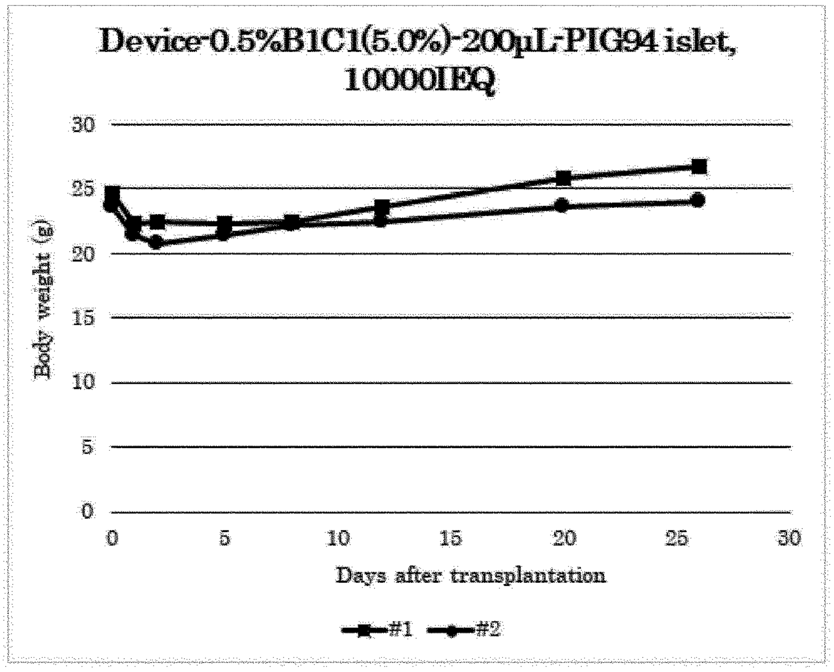
Device-0.5%B1C1(5.0%)-200µL-PIG94 islet, 10000IEQ
30
25
20
15
10
5
0
Body weight (g)
0 5 10 15 20 25 30
Days after transplantation
#1
#2

Fig. 9-1

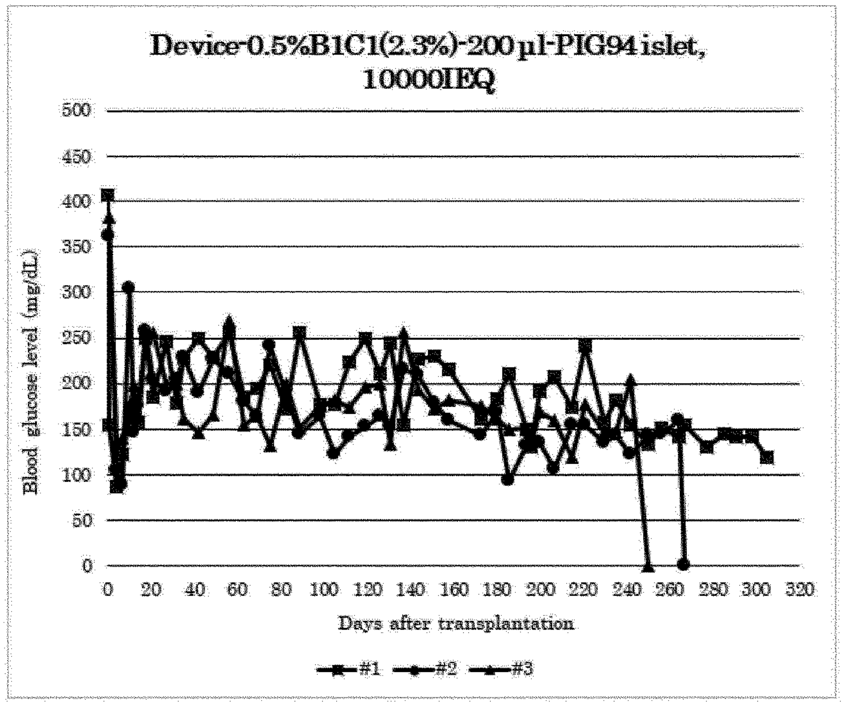
Device-0.5%B1C1(2.3%)-200 µl-PIG94 islet, 10000IEQ
Blood glucose level (mg/dL)
500
450
400
350
300
250
200
150
100
50
0
0 20 40 60 80 100 120 140 160 180 200 220 240 260 280 300 320
Days after transplantation
#1 #2 #3

Fig. 9-2

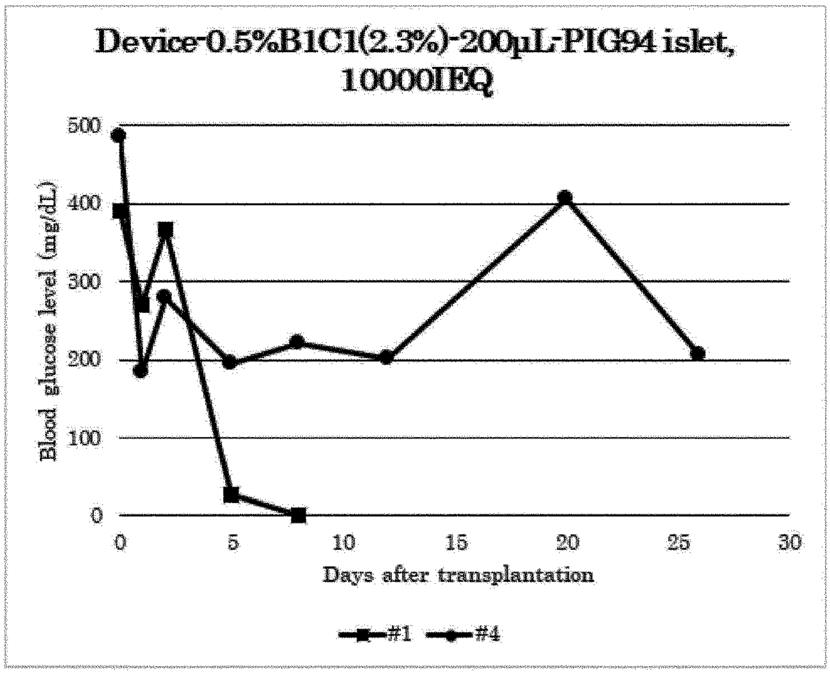
Device-0.5%B1C1(2.3%)-200µL-PIG94 islet, 10000IEQ
Blood glucose level (mg/dL)
500
400
300
200
100
0
0 5 10 15 20 25 30
Days after transplantation
#1 #4

Fig. 10-1

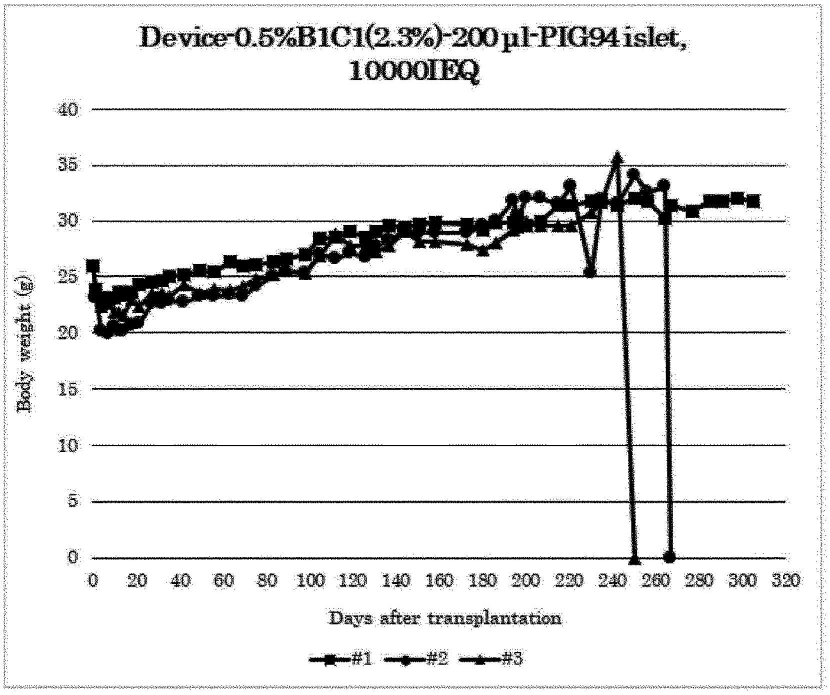


Fig. 10-2

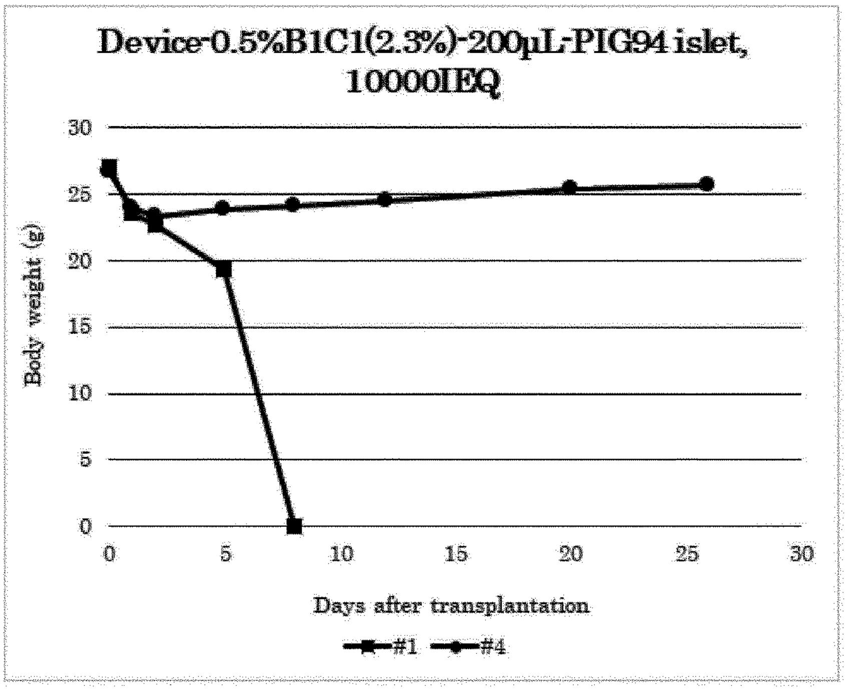

Fig. 11-1

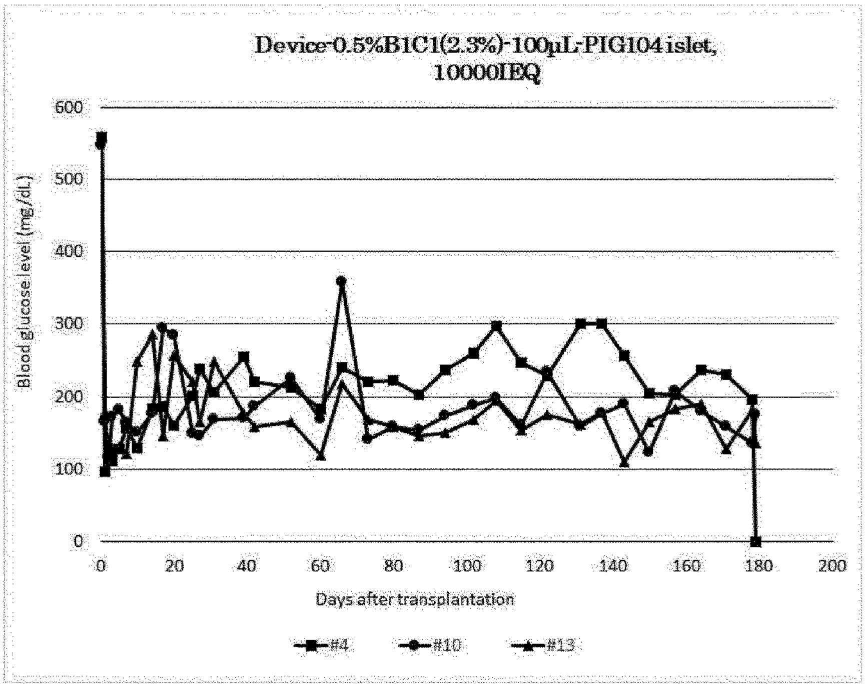


Fig. 11-2

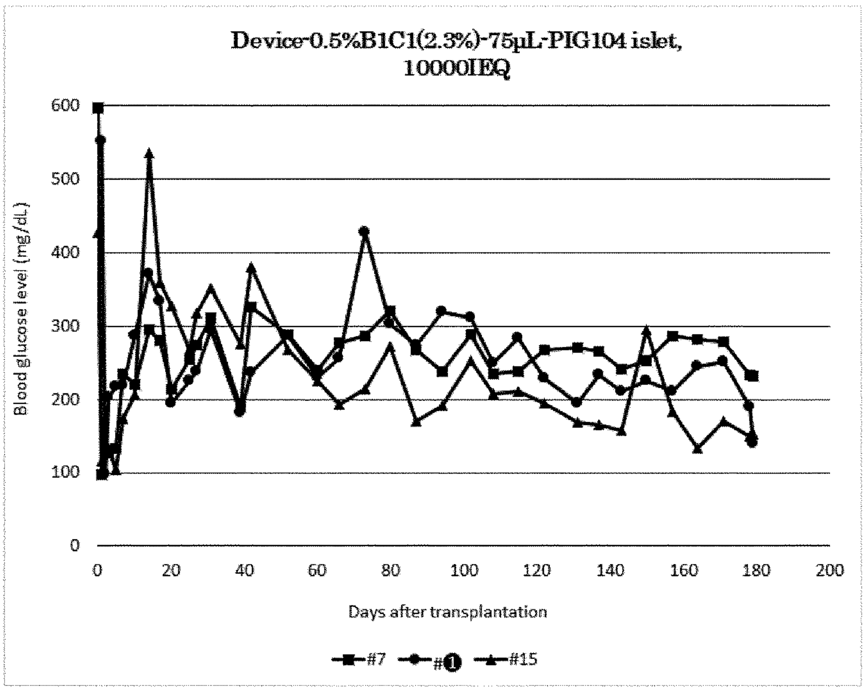

Fig. 11-3

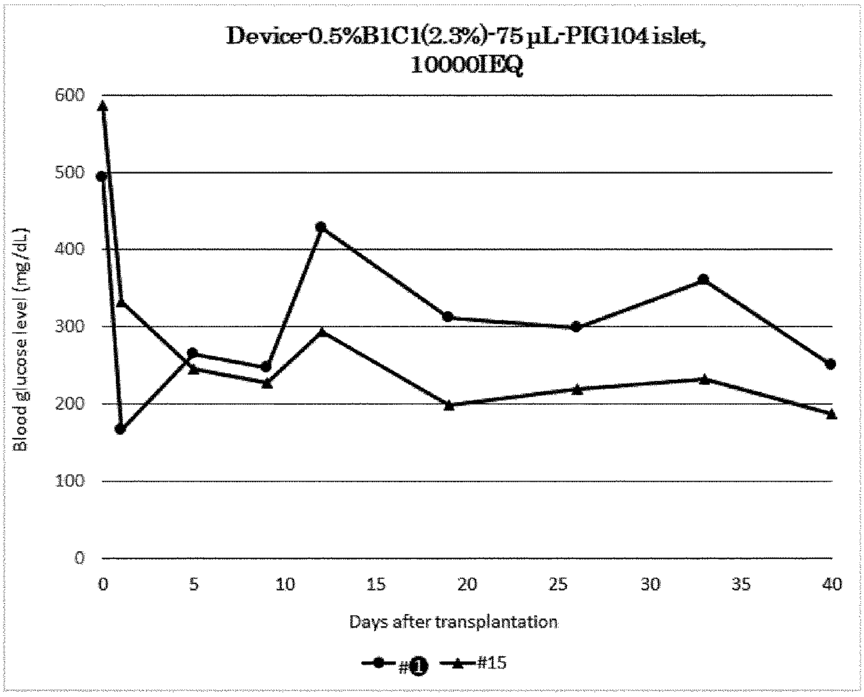


Fig. 11-4

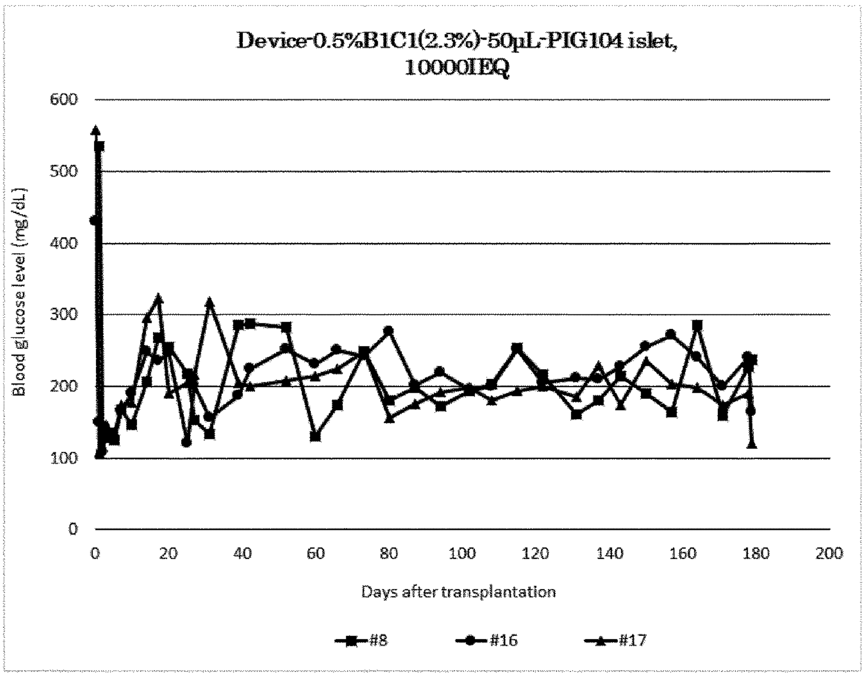

Fig. 11-5

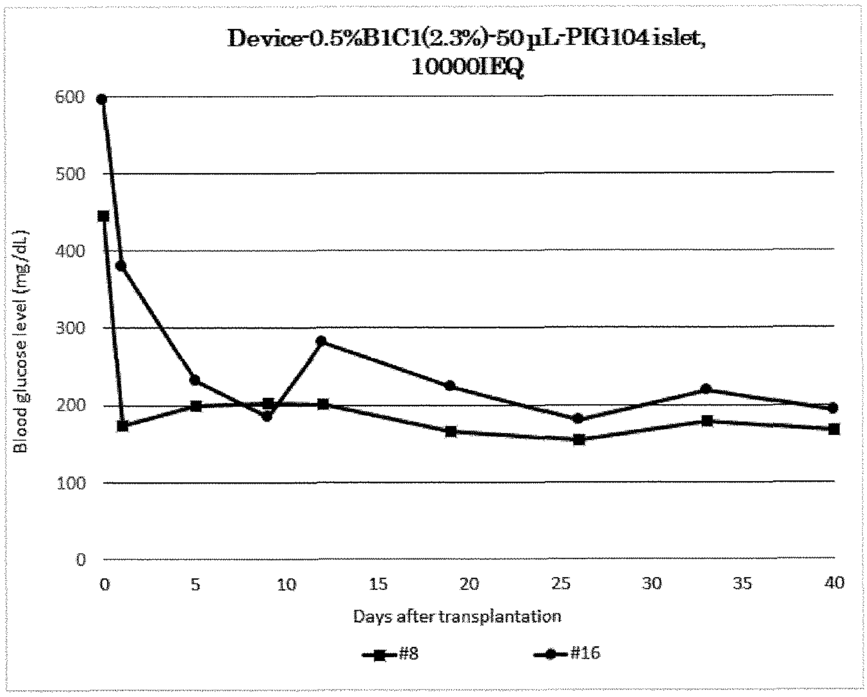
Device-0.5%B1C1(2.3%)-50μL-PIG104 islet,
10000IEQ
Blood glucose level (mg/dL)
600
500
400
300
200
100
0
0
5
10
15
20
25
30
35
40
Days after transplantation
#8
#16

Fig. 12-1

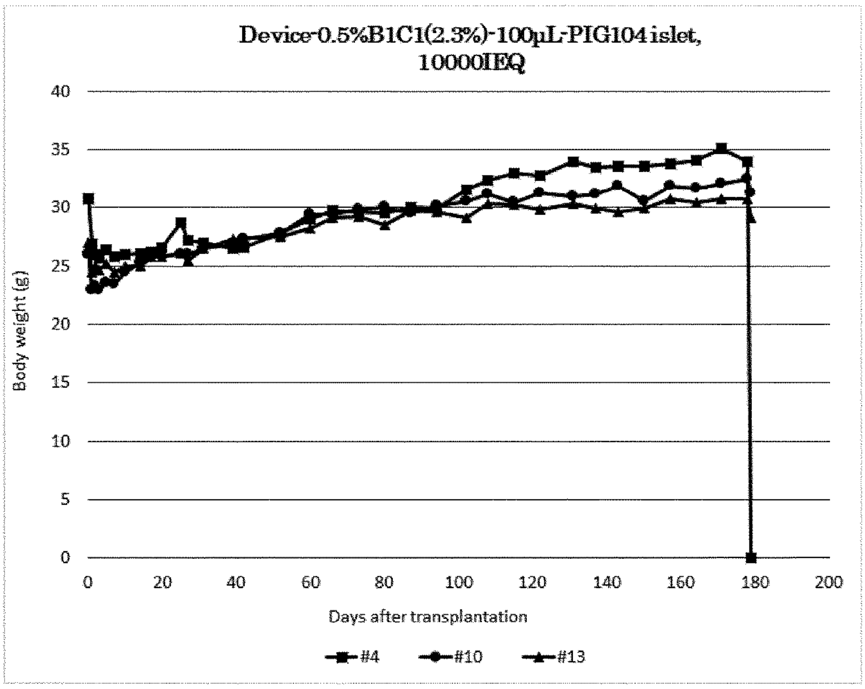


Fig. 12-2

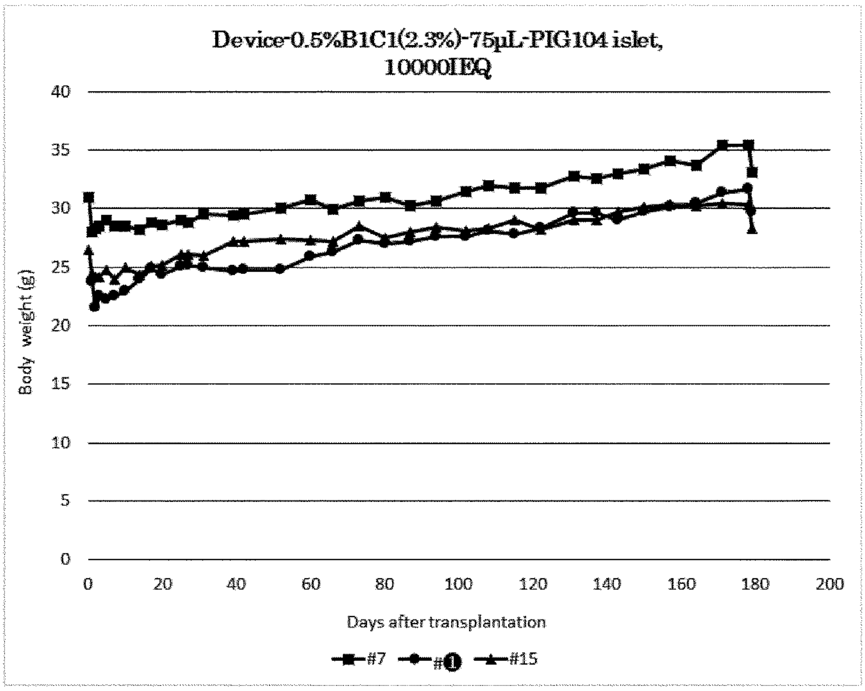

Fig. 12-3

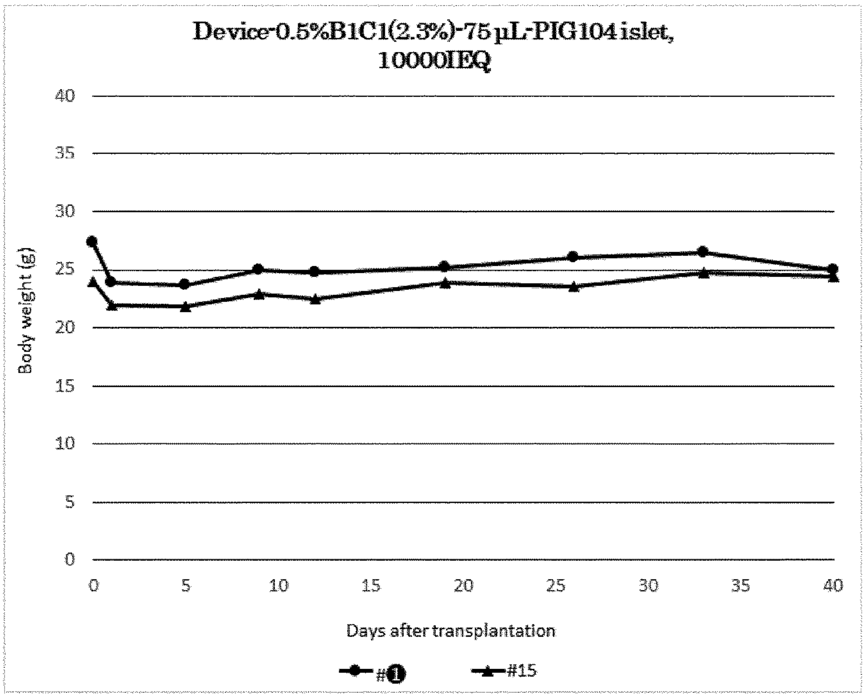


Fig. 12-4

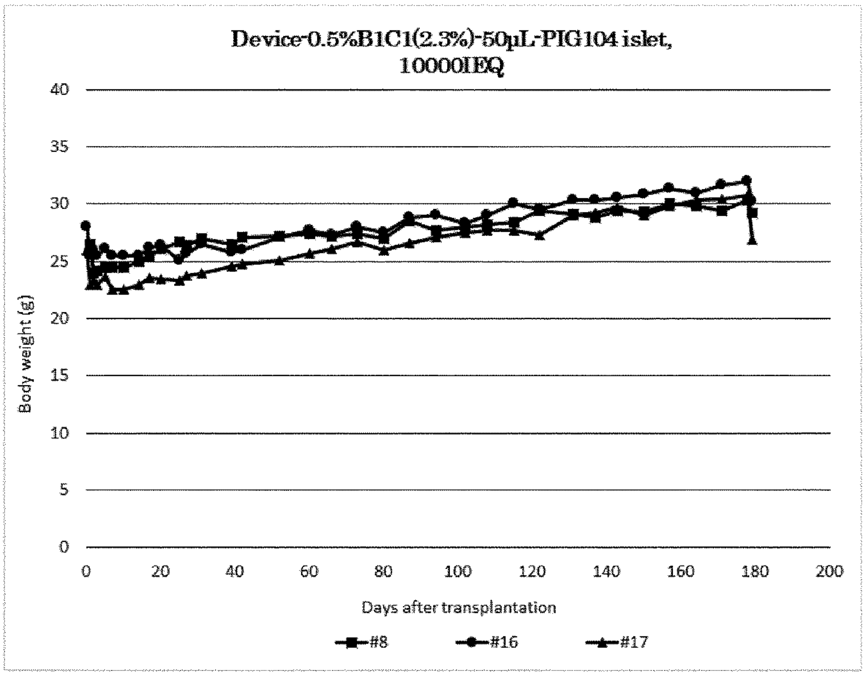

Fig. 12-5

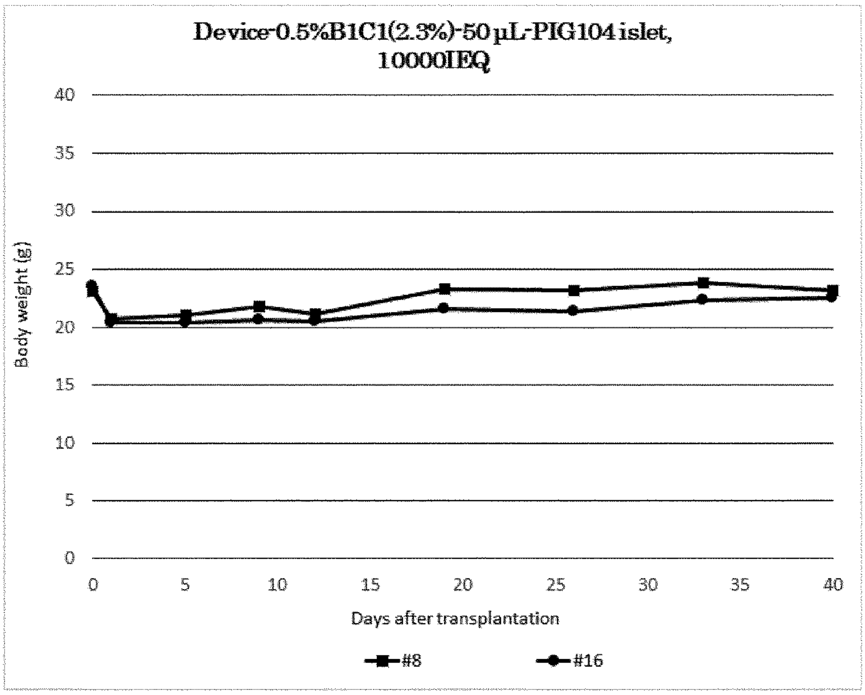
Device-0.5%B1C1(2.3%)-50 µL-PIG104 islet, 10000IEQ
Body weight (g)
40
35
30
25
20
15
10
5
0
0
5
10
15
20
25
30
35
40
Days after transplantation
#8
#16

Fig. 13-1

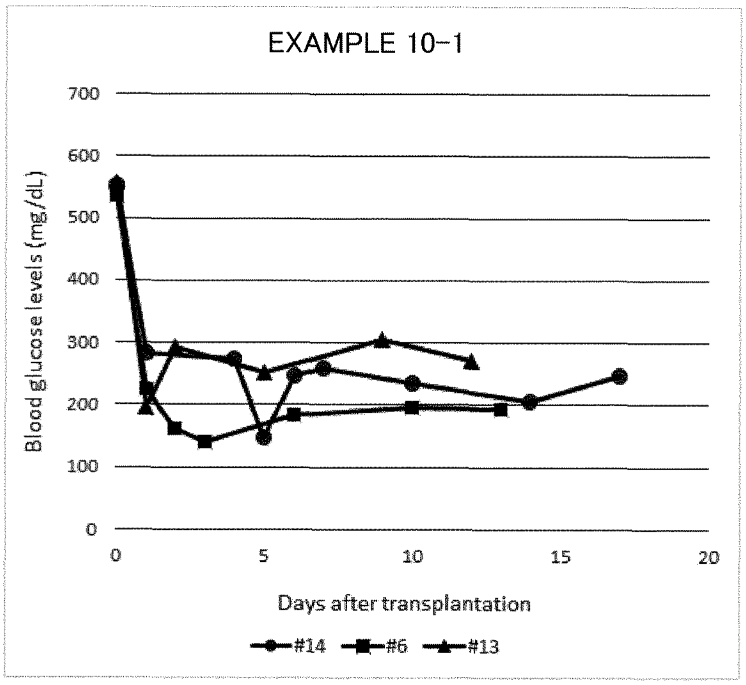


Fig. 13-2

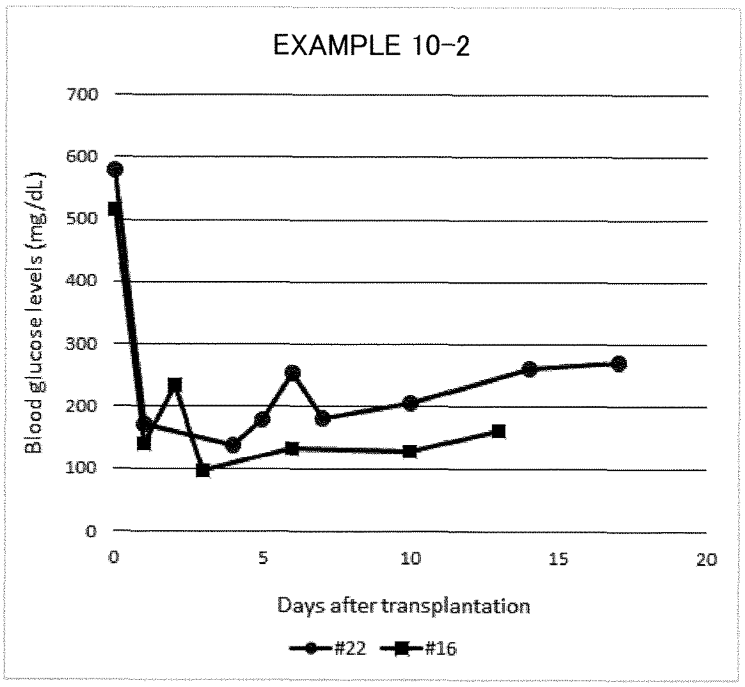

Fig. 13-3

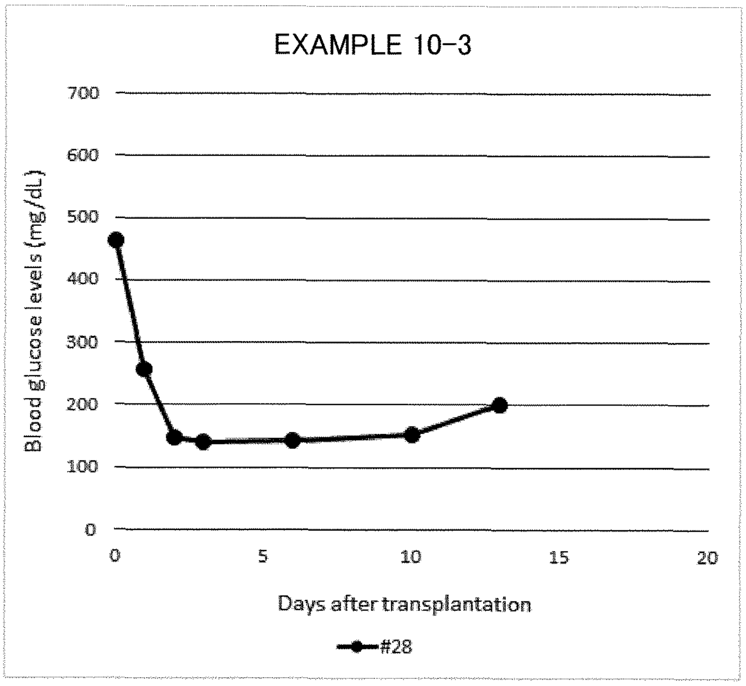


Fig. 13-4

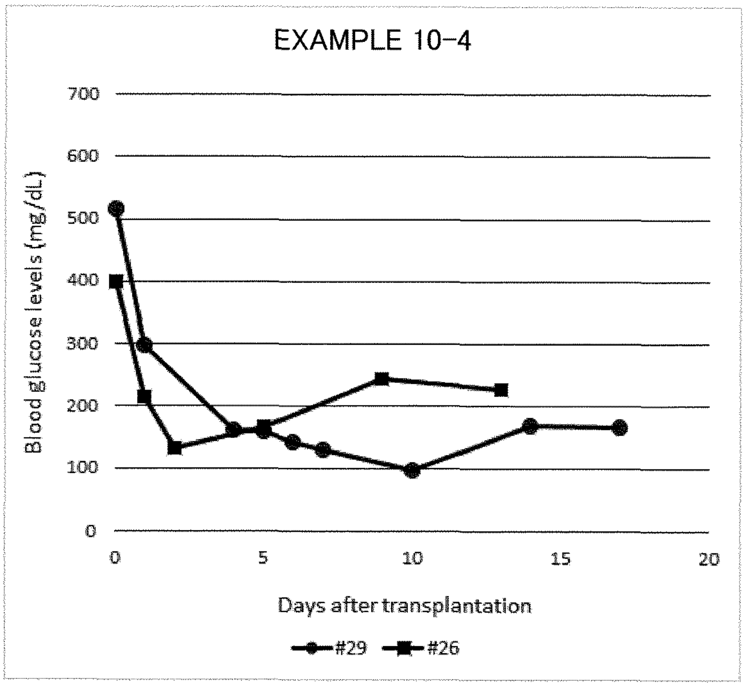

Fig. 13-5

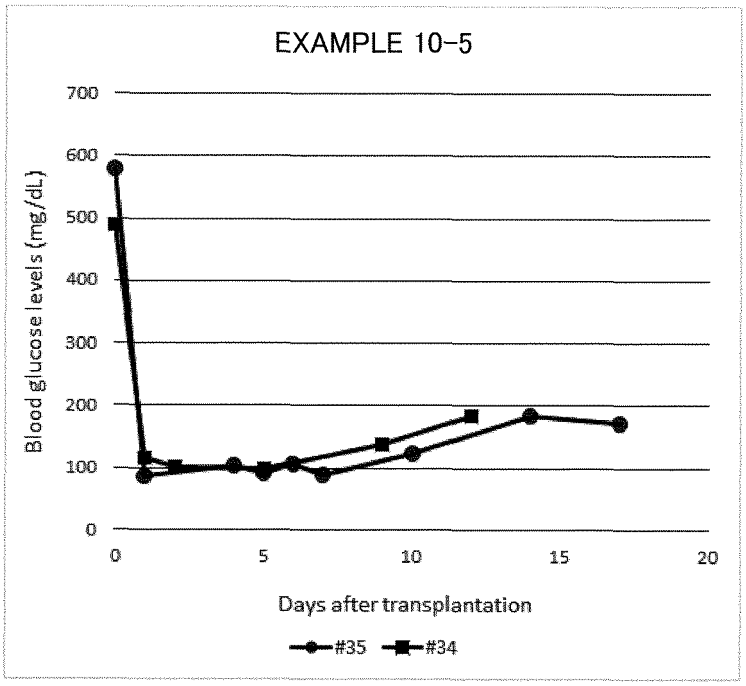
EXAMPLE 10-5
700
600
500
400
300
200
100
0
Blood glucose levels (mg/dL)
0
5
10
15
20
Days after transplantation
#35
#34

Fig. 14-1

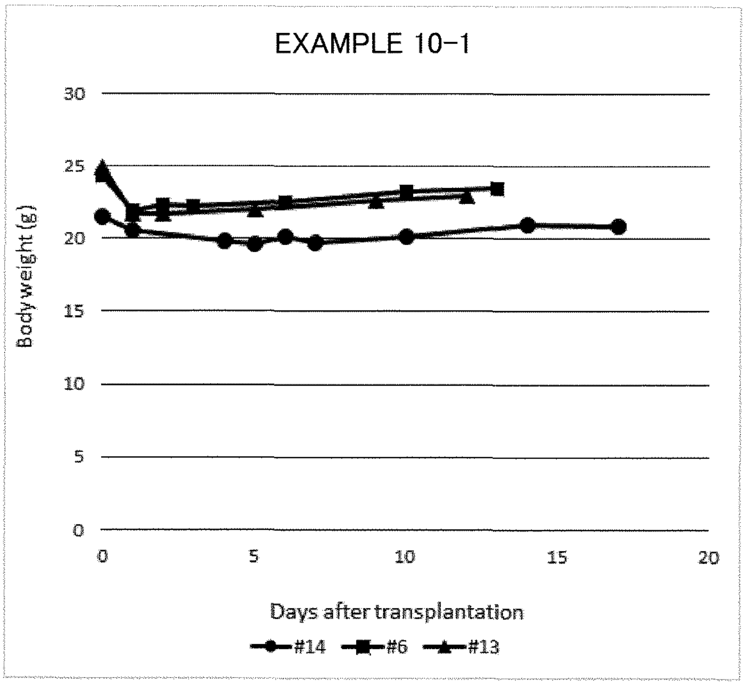


Fig. 14-2

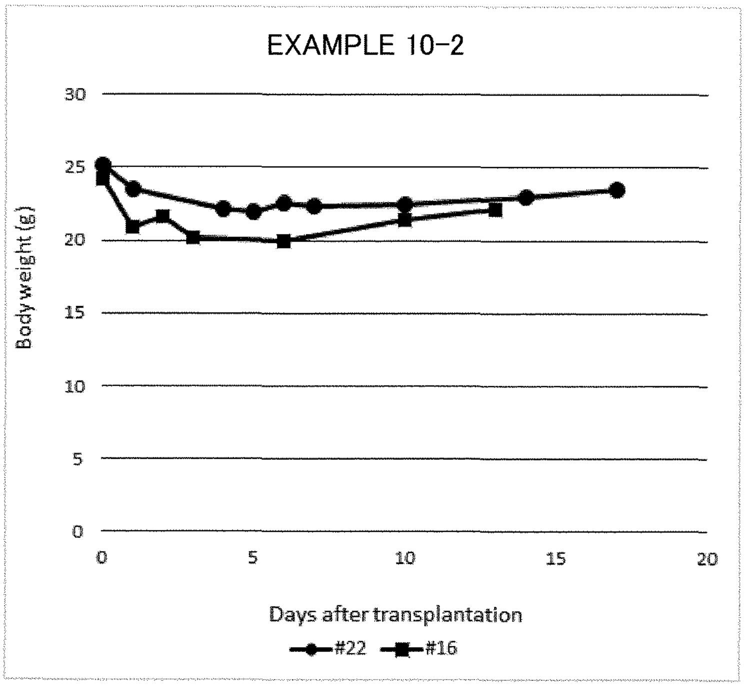

Fig. 14-3

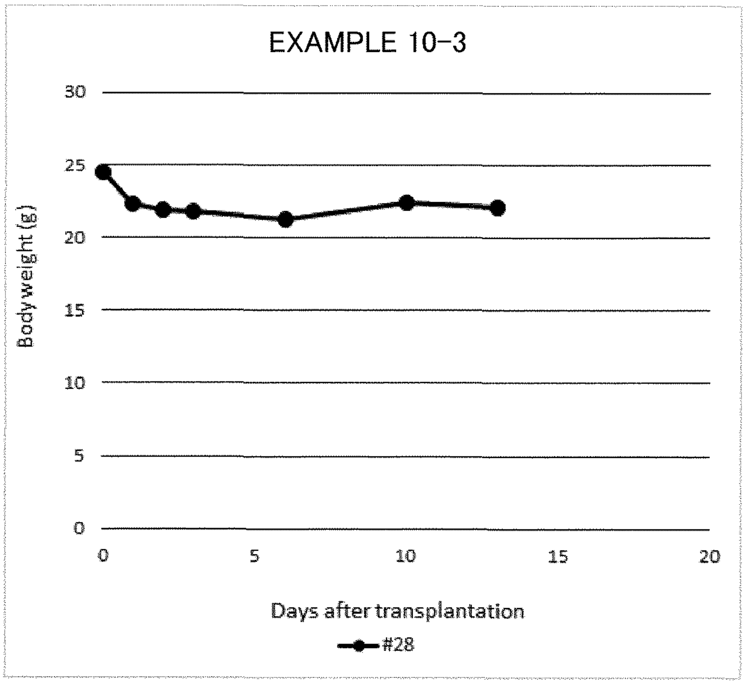


Fig. 14-4

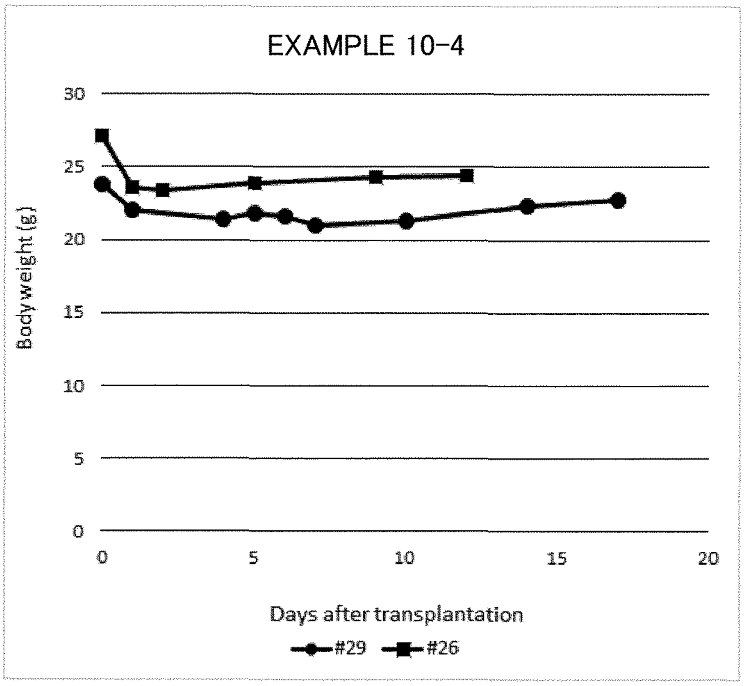

Fig. 14-5

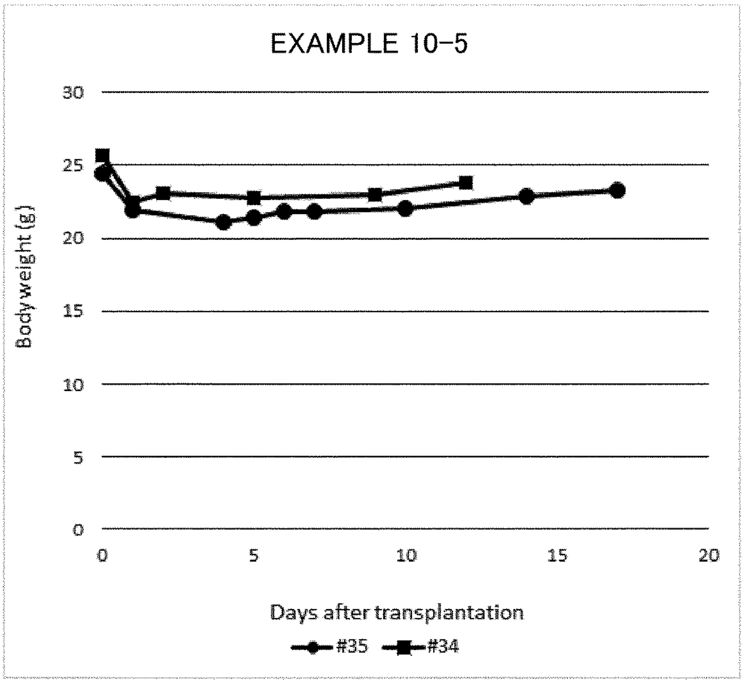
EXAMPLE 10-5
30
25
20
15
10
5
0
Bodyweight (g)
0
5
10
15
20
Days after transplantation
#35
#34

Fig. 15-1

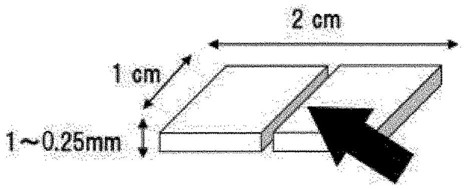


Fig. 15-2

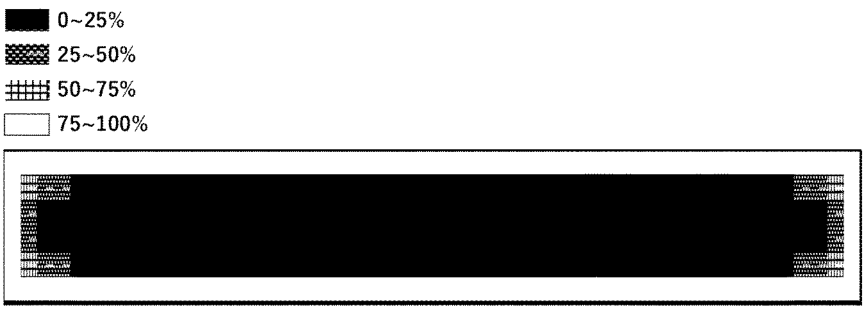

Fig. 15-3

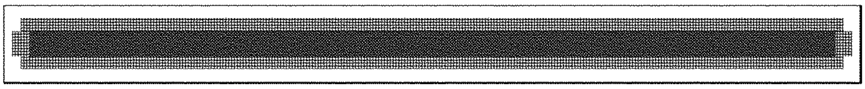

Fig. 15-4

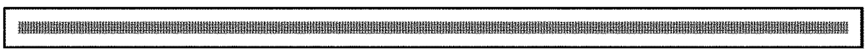

Fig. 16-1

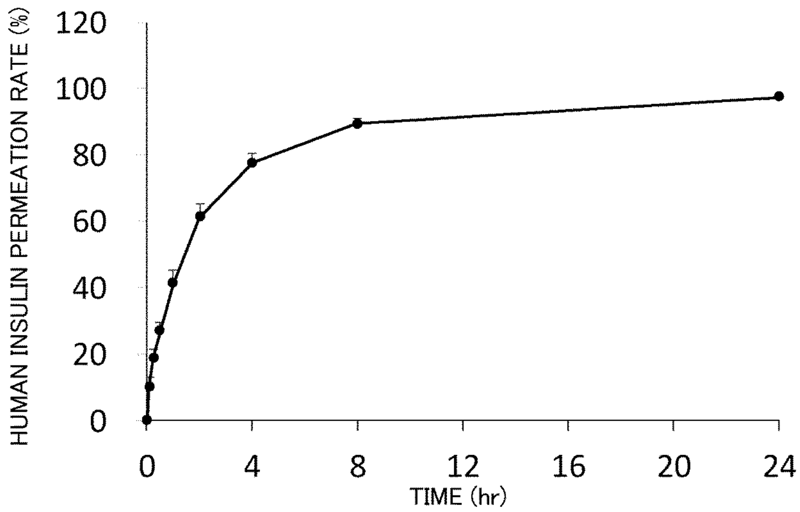


Fig. 16-2

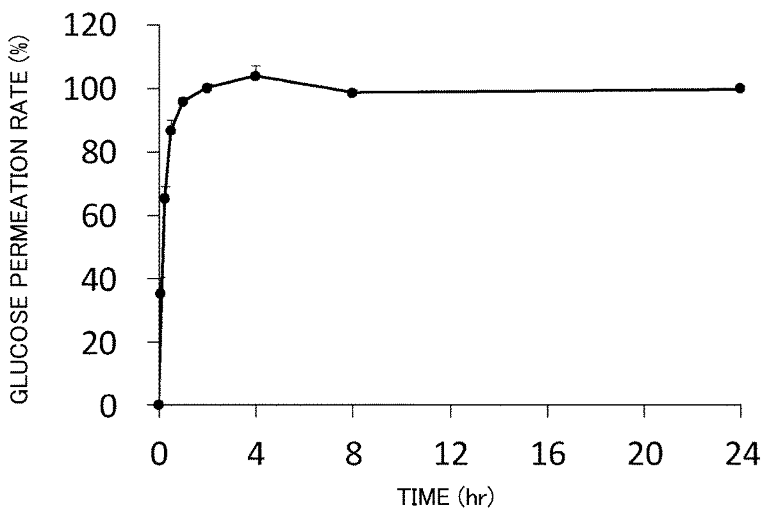

Fig. 17

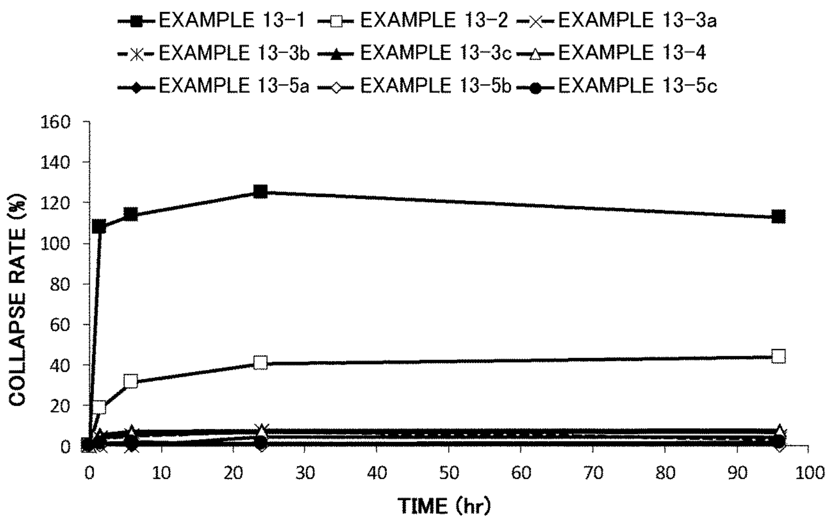
EXAMPLE 13-1
EXAMPLE 13-2
EXAMPLE 13-3a
EXAMPLE 13-3b
EXAMPLE 13-3c
EXAMPLE 13-4
EXAMPLE 13-5a
EXAMPLE 13-5b
EXAMPLE 13-5c
COLLAPSE RATE (%)
TIME (hr)
0
20
40
60
80
100
120
140
160
0
10
20
30
40
50
60
70
80
90
100

Fig. 18-1

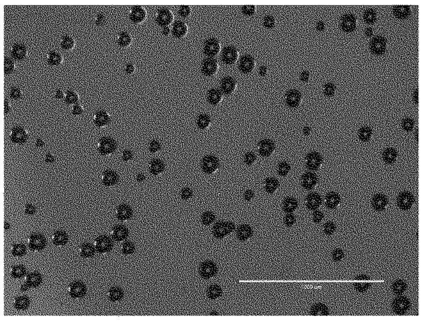

Fig. 18-2

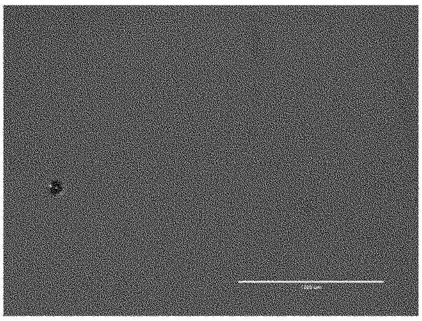

**INTERNATIONAL SEARCH REPORT**

International application No. PCT/JP2020/047944

A. CLASSIFICATION OF SUBJECT MATTER
Int. Cl. A61L27/38(2006.01)i, A61K35/39(2015.01)i, A61L27/20(2006.01)i, A61P3/10(2006.01)i
FI: A61L27/38, A61L27/20, A61K35/39, A61P3/10
According to International Patent Classification (IPC) or to both national classification and IPC

B. FIELDS SEARCHED
Minimum documentation searched (classification system followed by classification symbols)
Int. Cl. A61L27/38, A61K35/39, A61L27/20, A61P3/10

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | CN 105078923 A (DALIAN CHEMICAL PHYSICS INSTITUTE) 25 November 2015 (2015-11-25), paragraphs [0003], [0062]-[0068], claim 7 | 1-4, 10-12<br>10-15<br>5-9 |
| X<br>Y<br>A | JP 2019-512522 A (MILLENNIUM PHARMACEUTICALS, INC.) 16 May 2019 (2019-05-16), paragraphs [0006]-[0012], [0101], examples 3-4 | 1-4, 10<br>10-15<br>5-9 |
| Y | JP 2000-507202 A (ENCELLE INC.) 13 June 2000 (2000-06-13), p. 5, line 8 to p. 7, line 8, examples 2, 8 | 13-15 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09.02.2021 | 02.03.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | <br><br>Telephone No. |

Form PCT/ISA/210 (second sheet) (January 2015)

**INTERNATIONAL SEARCH REPORT**
Information on patent family members

International application No.
PCT/JP2020/047944

| Patent Documents referred to in the Report | Publication Date | Patent Family | Publication Date |
|---|---|---|---|
| CN 105078923 A | 25.11.2015 | (Family: none) | |
| JP 2019-512522 A | 16.05.2019 | US 2019/0100628 A1 paragraphs [0006]-[0012], [0107], examples 3, 4 | |
| JP 2000-507202 A | 13.06.2000 | US 5830492 A columns 1, 2, Background of the invention, examples 2, 8<br>EP 1321516 A2<br>CA 2332701 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 55157502 A
- JP 60258121 A
- WO 9528480 A
- WO 9219195 A
- JP 2017196150 A
- JP S55157502 B
- JP S60258121 A
- JP H09324001 B
- JP H08269102 B
- JP 2002530440 W
- WO 9313136 A
- US 5589591 B
- JP 2005036036 A
- JP 2019023478 W
- JP 2019122063 A
- JP 2020025324 W


### Non-patent literature cited in the description


- *Organ Biology,* 2017, vol. 24 (1), 7-12
- *Journal of Biomedical Materials Research,* 2015, vol. 103B (5), 1120-1132
- **SHIMODA et al.** *Cell Transplantation,* 2012, vol. 21, 501-508
- **NOGUCHI et al.** *Transplantation Proceedings,* 2010, vol. 42, 2084-2086
- *Appl. Microbiol. Biotechnol.,* 1994, vol. 40, 638-643
- *CHEMICAL ABSTRACTS,* 1255942-06-3
- *CHEMICAL ABSTRACTS,* 99-76-3
- *CHEMICAL ABSTRACTS,* 26690-80-2
- *CHEMICAL ABSTRACTS,* 88192-19-2
- *Bioorganic & Medicinal Chemistry,* 2003, vol. 11, 4189-4206
- *CHEMICAL ABSTRACTS,* 108468-80-4
- *CHEMICAL ABSTRACTS,* 539-48-0
- *Org. Process Res. Dev.,* 2018, vol. 22, 108-110
- *CHEMICAL ABSTRACTS,* 917756-42-4
- *CHEMICAL ABSTRACTS,* 628-92-2
- *CHEMICAL ABSTRACTS,* 6427-66-3
- *CHEMICAL ABSTRACTS,* 57260-73-8
- *CHEMICAL ABSTRACTS,* 127828-22-2
- **SHIMODA.** *Cell Transplantation,* 2012, vol. 21, 501-508
- *Biotechnology and Bioengineering,* 2007, vol. 96 (5), 990-998
- *Theoretical biology and Medical Modelling,* 2009, vol. 6, 5